# EUROPÄISCHE PATENTSCHRIFT

(11) **EP 2 994 467 B1**
(45) Veröffentlichungstag und Bekanntmachung des Hinweises auf die Patenterteilung: **01.08.2018**
(21) Anmeldenummer: 14726074.9
(22) Anmeldetag: 08.05.2014
(51) Int. Cl.: C07D 405/14, C07D 401/10, C07D 403/10, C07D 405/04, C07D 417/10, C07D 417/14, C07D 471/04, C07D 487/04, C07D 491/048, C07D 495/04, C07D 498/08, C07D 513/04, A61K 31/506, A61K 31/519, A61P 35/00

(54) **ARYLCHINAZOLINE**
ARYLQUINAZOLINES
ARYLCHINAZOLINE

(30) Priorität: 11.05.2013 DE 102013008118
(43) Veröffentlichungstag der Anmeldung: 16.03.2016
(73) Patentinhaber: Merck Patent GmbH, 64293 Darmstadt (DE)
(72) Erfinder: FUCHSS, Thomas, 64625 Bensheim-Auerbach (DE); EMDE, Ulrich, 64291 Darmstadt (DE); BUCHSTALLER, Hans-Peter, 64347 Griesheim (DE); MEDERSKI, Werner, 64673 Zwingenberg (DE)
(86) Internationale Anmeldenummer: PCT/EP2014/001236
(87) Internationale Veröffentlichungsnummer: WO 2014/183850

(56) Entgegenhaltungen:
- WO-A1-2011/113512

## Beschreibung

Die Erfindung betrifft Verbindungen der Formel (I) 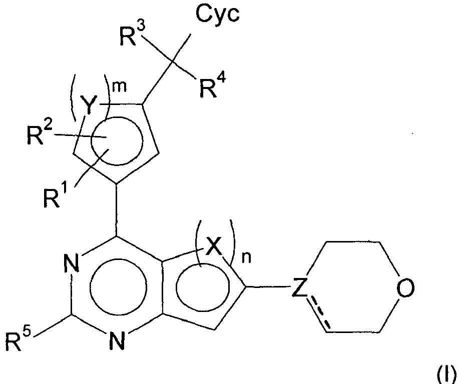 worin
- X: CH, CF, S oder N ist,
- Y: CH, S oder N ist,
- Z: C oder N ist,
- ----: bildet, wenn Z = C ist, zusammen mit der Einfachbindung eine Doppelbindung,
ist abwesend, wenn Z = N ist,
- n: 1 oder 2 ist, wobei
wenn n = 1 ist, ist X = S,
und wenn n = 2 ist, sind beide X = CH, oder das mit dem Pyrimidinring verknüpfte X ist CF und das nicht mit dem Pyrimidinring verknüpfte X ist CH, oder ein X ist CH und das andere X ist N;
- m: 1 oder 2 ist, wobei
wenn m = 1 ist, ist Y = S,
und wenn m = 2 ist, sind beide Y = CH, oder ein Y ist CH und das andere Y ist N;
- R¹, R², R³, R⁴: unabhängig voneinander H, Hal, CN, OH, CONH₂, CONH(LA) oder LA sind;
- R⁵: H, Hal, CN oder C=CH ist;
- Cyc: Phenyl ist, das unsubstituiert oder ein- oder zweifach unabhängig voneinander durch R⁶ substituiert sein kann, oder Het¹ ist;
- Het¹: ein ein- oder zweikerniger, 5-10 gliedriger Heterozyklus ist, mit 1-3 N-, O- und/oder S-Atomen, oder 1-4 N-Atomen, der unsubstituiert oder ein-, zwei oder dreifach unabhängig voneinander durch R⁶ substituiert sein kann, oder einfach durch Het² substituiert sein kann;
- R⁶: Hal, LA, Oxo, CN, oder NH₂ ist;
- LA: unverzweigtes oder verzweigtes Alkyl mit 1-5 C-Atomen ist, das gesättigt oder partiell ungesättigt sein kann, worin 1-3 H-Atome durch Hal, und/oder ein H-Atom durch CN oder Het², und/oder ein oder zwei CH₂-Gruppen durch O, NH, NH₂, N(CH₃) oder CO ersetzt sein können;
- Het²: ein 3-5 gliedriger aliphatischer Homo- oder Heterozyklus mit 0, 1, 2 oder 3 N-, O- und/oder S-Atomen ist, der unsubstituiert ist;
- Hal: F, Cl, Br oder I ist;
und/oder ihre physiologisch unbedenklichen Salze, Tautomere und/oder Stereoisomere, einschließlich deren Mischungen in allen Verhältnissen.

Die Verbindungen der Formel (I) können zur Hemmung von Serin-Threonin-Proteinkinasen sowie zur Sensibilisierung von Krebszellen gegenüber Antikrebsmitteln und/oder ionisierender Strahlung verwendet werden. Gegenstand der Erfindung ist auch die Verwendung der Verbindungen der Formel (I) in der Prophylaxe, Therapie oder Verlaufskontrolle von Krebs, Tumoren bzw. Metastasen, in Kombination mit Radiotherapie und/oder einem Antikrebsmittel. Die Erfindung betrifft ferner ein Verfahren zum Herstellen der Verbindungen der Formel (I) durch Umsetzen von Verbindungen der Formeln (IV) und (V) und gegebenenfalls Umwandeln einer Base oder Säure der Verbindungen der Formel (I) in eines ihrer Salze.

Die DNA-abhängige Proteinkinase (DNA-PK) ist eine Serin/Threonin-Proteinkinase, die in Verbindung mit DNA aktiviert wird. Biochemische und genetische Daten zeigen, dass DNA-PK (a) aus einer katalytischen Untereinheit, die man DNA-PKcs nennt, und (b) zwei regulatorischen Komponenten (Ku70 und Ku80) besteht. Funktional ist DNA-PK ein entscheidender Bestandteil einerseits der Reparatur von DNA-Doppelstrangbrüchen (DSBs) und anderseits der somatischen oder V(D)J Rekombination. Darüber hinaus werden DNA-PK und ihre Komponenten mit einer Vielzahl weiterer physiologischer Prozesse in Verbindung gebracht, darunter die Modulation der Chromatinstruktur sowie die telomere Wartung (Smith & Jackson (1999) Genes and Dev 13: 916; Goytisolo et al. (2001) Mol. Cell. Biol. 21: 3642; Williams et al. (2009) Cancer Res. 69: 2100).

Das menschliche Erbgut in Form der DNA ist fortwährend dem Angriff reaktiver Sauerstoff-Spezies (ROS) ausgesetzt, die vornehmlich als Nebenprodukte des oxidativen Metabolismus anfallen. ROS sind imstande, DNA-Schädigungen in Form von Einzelstrangbrüchen hervorzurufen. Doppelstrangbrüche können entstehen, wenn vorangegangene Einzelstrangbrüche in enger Nachbarschaft erfolgen. Darüber hinaus können Einzel- und Doppelstrangbrüche verursacht werden, wenn die DNA-Replikationsgabel auf beschädigte Basenmuster trifft. Des Weiteren sind körperfremde Einflüsse, wie ionisierende Strahlung (z.B. gamma- oder Partikel-Strahlung), und bestimmte Anti-Krebsmedikamente (z.B. Bleomycin) in der Lage, DNA-Doppelstrangbrüche hervorzurufen. DSB können ferner als Zwischenprodukte der somatischen Rekombination auftreten, einem Prozess, der bedeutend ist für die Ausbildung eines funktionalen Immunsystems aller Wirbeltiere. Werden DNA-Doppelstrangbrüche nicht oder fehlerhaft behoben, können Mutationen und/oder Chromosomenaberrationen auftreten, die in der Folge zum Zelltod führen können. Um den schwerwiegenden Gefährdungen, die von DNA-Doppelstrangbrüchen ausgehen, entgegenzuwirken, haben eukaryotische Zellen mehrere Mechanismen entwickelt, diese zu beheben. Höhere Eukaryoten bedienen sich dabei überwiegend der sogenannten nichthomologen Endverknüpfung (*non-homologous end-joining*), bei der die DNA-abhängige Proteinkinase die Schlüsselrolle einnimmt. Biochemische Untersuchungen haben gezeigt, dass DNA-PK am wirksamsten durch das Auftreten von DNA-DSBs aktiviert wird. Zelllinien, deren DNA-PK Komponenten mutiert und nicht funktional sind, erweisen sich als strahlungsempfindlich (Smith und Jackson, 1999).

DNA-PK gehört wegen Ihrer katalytischen Domäne, die in der etwa 500 Aminosäuren zählenden C-terminalen katalytischen Untereinheit (DNA-PKcs) liegt, zur Familie der *Phosphatidylinositol-3-kinase-related kinases (PIKK),* wobei DNA-PK keine Lipid-Kinase darstellt (Hartley et al. (1995) Cell 82: 849; Smith & Jackson (1999) Genes and Dev 13: 916; Lempiäinen & Halazonetis (2009) EMBO J. 28: 3067).

Es ist durch Izzard et al. (1999) Cancer Res. 59: 2581 beschrieben, dass der PI3-Kinase Inhibitor LY294002 in in-vitro Versuchen die Funktion von DNA-PK inhibiert. Der IC₅₀-Wert (Konzentration bei der 50 % der Enzymaktivität gehemmt ist) liegt bei wenig wirksamen 1,25 µM (5,0 mM ATP). Obwohl der Nachweis, dass der Inhibitor LY294002 Säugerzellen strahlungsempfindlicher werden lässt, d.h. die Zytotoxizität ionisierender Strahlung verstärkt wird, prinzipiell eine Anwendung bei der Bestrahlungstherapie von z.B. soliden Krebstumoren impliziert, konnte für LY294002 zellulär lediglich eine schwache Empfindlichkeitssteigerung gegenüber ionisierender Bestrahlung nachgewiesen werden (Rosenzweig et al. (1999) Clin. Cancer Res. 3: 1149). KuDOS Pharmaceuticals Ltd. haben die Leitstruktur LY294002 optimiert und verschiedene DNA-PK-Inhibitoren vorgestellt. Die Einführung einer Dibenzothiophenylgruppe führte zum Inhibitor NU-7441, einer ATPkompetitiven Verbindung mit einem IC₅₀-Wert von 20.0 nM (Hardcastle et al. (2005) J. Med. Chem. 48: 7829). KU-0060648 vereint inhibitorische Eigenschaften gegenüber DNA-PK mit einem verbesserten Löslichkeitsprofil im wässrigen Medium, jedoch werden die Kinasen der PI3K-lsoenzym-Familie durch KU-0060648 ebenfalls potent gehemmt. Das seit Langem bestehende Bedürfnis nach einem potenten und selektiven DNA-PK-Inhibitor ist folglich bisher nicht befriedigt worden.

Der Erfindung liegt die Aufgabe zugrunde, die im Stand der Technik aufgezeigten Nachteile zu überwinden und wirksame Inhibitoren der DNA-PK zu entwickeln, die selektiv gegenüber den verwandten Kinasen der PIKK Familie sowie von niedermolekularer Größe sind und insbesondere eine effektive Anwendung in der Krebstherapie als Radio- und Chemosensibilisierer ermöglichen - mit dem Ziel, die therapeutische Wirksamkeit bei gleichzeitiger Verringerung der Nebenwirkungen zu verbessern.

Die Aufgabe der Erfindung wird gemäß den unabhängigen Ansprüchen gelöst. Die Unteransprüche beinhalten bevorzugte Ausführungsformen. Erfindungsgemäß werden Verbindungen der Formel (I) bereitgestellt.

Überraschenderweise hat sich gezeigt, dass die erfindungsgemäßen Verbindungen mit inhibierenden Eigenschaften auf Serin-Threonin-Proteinkinasen versehen sind. Die Verbindungen der Formel (I) sind derart ausgestaltet, dass eine potente und selektive Hemmung von DNA-PK erfolgt. Die erfindungsgemäßen Verbindungen eröffnen damit völlig neue Möglichkeiten hinsichtlich der anti-cancerogenen Wirkung von Antikrebsmitteln. Dabei spielen die Verbindungen der Formel (I) eine therapeutische Rolle als Radio- und Chemosensibilisierer durch gezielte Hemmung der Reparatur von DNA-Doppelstrangbrüchen (*non-homologous end-joining*) bei der Behandlung von Krebs.

Es ist bisher aus WO 1992/07844 bekannt, dass 2,4-Diaminochinazolin-Derivate Verstärker von chemotherapeutischen Mitteln in der Krebsbehandlung sind. Die Derivate adressieren die Mehrfachresistenz von Tumorzellen infolge von Überexpression des mdr1-Gens, dessen Genprodukt einer Efflux-P-Glycoproteinpumpe die intrazelluläre Wirkstoffkonzentration gering hält. Weder sind physiko-chemische und pharmakologische Daten offenbart, noch ist ein vermarktetes Medikament bekannt. Andere Chinazolin-Derivate als DNA-PK-Inhibitoren sind in der WO 2011/113512 offenbart.

Mit der vorliegenden Erfindung wird eine neue Generation von DNA-PK-Inhibitoren bereitgestellt, die nicht nur zur spezifischen Hemmung befähigt sind, was insbesondere bei zellulären Assays zu Tage tritt. Darüber hinaus zeichnen sie sich auch durch das Fehlen der häufig beobachteten, unerwünschten Hemmung kardialer lonenkanäle, insbesondere des Kv1.11 hERG aus, dessen Blockade zu lebensbedrohlichen Arrhythmien führen kann.

Die erfindungsgemäßen Verbindungen und ihre Salze besitzen folglich wertvolle pharmakologische Eigenschaften bei gleichzeitig guter Verträglichkeit.

Im Rahmen der Erfindung sind die Verbindungen der Formel (I) so definiert, dass man darunter auch pharmazeutisch verwendbare Derivate, Salze, Solvate, Solvate von Salzen, Vorstufen der Verbindungen, Tautomere und optisch aktive Formen (wie z.B. Stereoisomere, Diastereomere, Enantiomere, Racemate) versteht. Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate. Unter pharmazeutisch verwendbaren Derivaten versteht man z.B. die Salze der erfindungsgemäßen Verbindungen als auch sogenannte Vorstufen der Verbindungen. Unter Vorstufen versteht man z.B. mit Alkyl- oder Acylgruppen, Zuckern oder Oligopeptiden abgewandelte Verbindungen der Formel (I), die im Organismus rasch zu den wirksamen erfindungsgemäßen Verbindungen gespalten werden. Hierzu gehören auch bioabbaubare Polymerderivate der erfindungsgemäßen Verbindungen, wie dies z.B. in Int. J. Pharm. 115, 61-67 (1995) beschrieben ist. Jegliche Verbindung, die *in vivo* in ein bioaktives Mittel, d.h. Verbindungen der Formel (I) umgewandelt werden kann, ist eine Vorstufe im Sinne dieser Erfindung. Jegliche biologisch aktive Verbindung, die aus der in-vivo Verstoffwechslung einer erfindungsgemäßen Verbindung resultiert, ist ein Metabolit im Sinne der vorliegenden Erfindung. Die Verbindungen der Formel (I) können ein oder mehrere chirale Zentren besitzen und daher in verschiedenen stereoisomeren Formen vorkommen. Die Formel (I) schließt alle diese Formen ein.

Gegenstand der Erfindung ist auch die Verwendung von Mischungen der Verbindungen der Formel (I), z.B. Gemische zweier Diastereomerer, z.B. im Verhältnis 1:1, 1:2, 1:3, 1:4, 1:5, 1:10, 1:100 oder 1:1000. Besonders bevorzugt handelt es sich dabei um Mischungen stereoisomerer Verbindungen.

Vor- und nachstehend haben die Reste X, Y, R¹, R², R³, R⁴, R⁵, R⁶, LA, Cyc, Het¹, Het² und Hal sowie m und n die bei der Formel (I) angegebenen Bedeutungen, sofern nicht ausdrücklich etwas anderes angegeben ist. Bei mehrfachem Auftreten einzelner Reste innerhalb einer Verbindung oder eines Radikals nehmen die Reste unabhängig voneinander die angegebenen Bedeutungen an, sofern nicht ausdrücklich etwas anderes angegeben ist. Den vorliegend verwendeten Bezeichnungen zur Definition der Verbindungen liegen im Allgemeinen die Regeln der IUPAC-Organisation für chemische Verbindungen und insbesondere organische Verbindungen zugrunde. Die Bezeichnungen zur Erläuterung der o.g. Verbindungen der Erfindung haben immer die nachstehenden Bedeutungen, sofern die Beschreibung oder die Ansprüche nicht etwas anderes anzeigen.

"LA" bezeichnet im Sinne der Erfindung einen gesättigten oder partiell ungesättigten Kohlenwasserstoffrest, der unverzweigt (linear) oder verzweigt ist und 1, 2, 3, 4 oder 5 C-Atome besitzt. Beispiele für LA sind Methyl, Ethyl, Propyl, Isopropyl, 1,1-, 1,2- oder 2,2-Dimethylpropyl, 1-Ethylpropyl, Butyl, Isobutyl, sek.-Butyl, tert.-Butyl. Der Kohlenwasserstoffrest kann aber auch substituiert sein dergestalt, dass 1-3 H-Atome durch Hal, und/oder ein H-Atom durch CN oder Het², und/oder ein oder zwei CH₂-Gruppen durch O, NH, N(CH₃) oder CO ersetzt sein können. Beispiele hierfür sind Methoxy, Methylsulfanyl, Ethoxy, Cyanomethoxy, 2-Propionitriloxy, Oxetan-3-yloxy, N-Methylaminocarbonyl, Carboxamido, 2-Methoxy-Ethoxy, 2,2,2-Trifluoro-Ethoxy, oder 2-Hydroxy-Ethoxy.

"Het¹" bezeichnet im Sinne der Erfindung einen ein- oder zweikernigen aliphatischen oder aromatischen Kohlenwasserstoff- Heterozyklus mit 3, 4, 5, 6, 7, 8, 9 oder 10 C-Atomen und 0, 1, 2 oder 3 N-, O- und/oder S-Atomen, der substituiert sein kann. Beispiele für geeignetes "Cyc" sind Phenyl, Pyridin, Pyrazin, Pyridazin, Pyrazolo[1,5-a]pyrimidinyl, oder Imidazo[1,2-b]pyridazinyl.

"Het²" bezeichnet im Sinne der Erfindung einen 3-5 gliedrigen aliphatischen Homo- oder Heterozyklus mit 0, 1, 2 oder 3 N-, O- oder S-Atomen. Beispiele für Het² sind Oxetan, Pyrrolidin oder Cyclopropyl.

In einer bevorzugten Ausgestaltung der vorliegenden Erfindung werden Arylchinazolin-Derivate der Formel (Ia) bereitgestellt 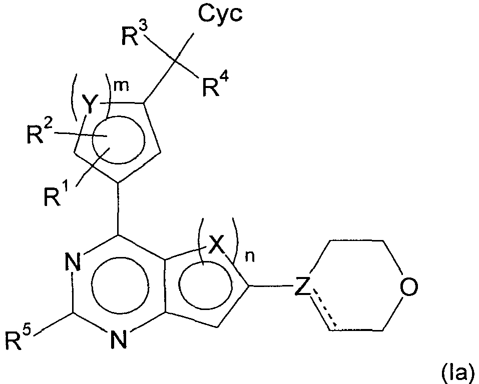 worin
- X, Y: unabhängig voneinander CH, S oder N sind,
- Z: C oder N ist,
- ----: bildet, wenn Z = C ist, zusammen mit der Einfachbindung eine Doppelbindung,
ist abwesend, wenn Z = N ist,
- n: 1 oder 2 ist, wobei
wenn n = 1 ist, ist X = S,
und wenn n = 2 ist, sind beide X = CH, oder das mit dem Pyrimidinring verknüpfte X ist CH und das nicht mit dem Pyrimidinring verknüpfte X ist N;
- m: 1 oder 2 ist, wobei
wenn m = 1 ist, ist Y = S,
und wenn m = 2 ist, sind beide Y = CH, oder ein Y ist CH und das andere Y ist N;
- R¹, R², R³, R⁴: unabhängig voneinander H, Hal, CN, OH, CONH₂ oder LA sind;
- R⁵: H, Hal, CN oder C=CH ist;
- Cyc: Phenyl ist, das unsubstituiert oder ein- oder zweifach unabhängig voneinander durch R⁶ substituiert sein kann, oder Het¹;
- Het¹: ein ein- oder zweikerniger, 5-10 gliedriger Heterozyklus ist, mit 1-3 N-, O- und/oder S-Atomen, der unsubstituiert oder ein- oder zweifach unabhängig voneinander durch R⁶ substituiert sein kann;
- R⁶: Hal, LA, Oxo, CN, NH₂ oder Het² ist;
- LA: unverzweigtes oder verzweigtes Alkyl mit 1-5 C-Atomen ist, das gesättigt oder partiell ungesättigt sein kann, worin 1-3 H-Atome durch Hal, und/oder ein H-Atom durch CN oder Het², und/oder ein oder zwei CH₂-Gruppen durch O, NH, NH₂, N(CH₃) oder CO ersetzt sein können;
- Het²: ein 3-5 gliedriger aliphatischer Homo- oder Heterozyklus mit 0, 1, 2 oder 3 N-, O- und/oder S-Atomen ist, der unsubstituiert ist;
- Hal: F, Cl, Br oder I ist;

Weiter bevorzugte Arylchinazolin-Derivate entsprechen der Formel (Ib) 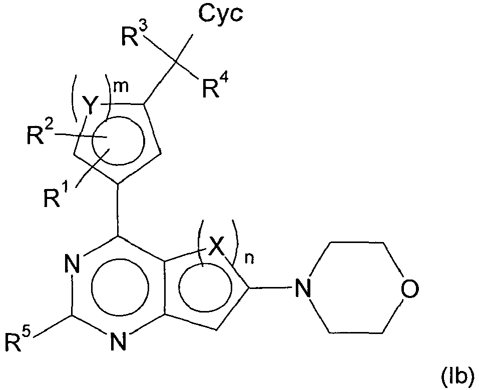 worin alle Substituenten die für die Formeln (I) oder (Ia) angegebene Bedeutung haben, und/oder ihre physiologisch unbedenklichen Salze, Tautomere und/oder Stereoisomere, einschließlich deren Mischungen in allen Verhältnissen.

In einer weiter bevorzugten Ausgestaltung der vorliegenden Erfindung werden Arylchinazolin-Derivate der Formel (II) bereitgestellt 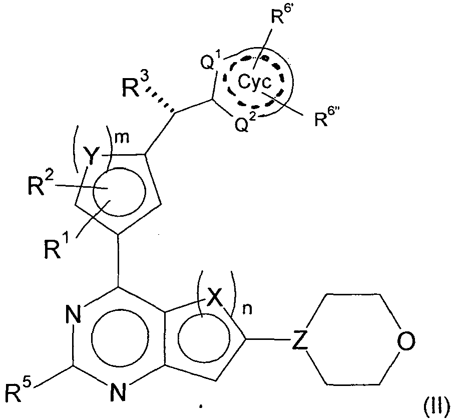 worin
- R³: Hal, CN, OH, CONH₂, CONH(LA) oder LA ist;
- R^{6'}, R^{6"}: unabhängig voneinander H, Hal, LA, Oxo, CN, NH₂ oder Het² sind;
- Q¹, Q²: unabhängig voneinander CH, N oder NH und in jedem Fall unsubstituiert sind;
- ----: die An- oder Abwesenheit von Doppelbindungen in Cyc bedeutet;
und die übrigen Substituenten die für die Formel (I) angegebene Bedeutung haben, und/oder ihre physiologisch unbedenklichen Salze, Tautomere und/oder Stereoisomere, einschließlich deren Mischungen in allen Verhältnissen.
Es wurde nämlich gefunden, daß die Aktivität der erfindungsgemäßen Verbindungen dann besonders hoch ist, wenn R³ wie in der Formel (II) dargestellt konfiguriert ist und Q keinen Substituenten trägt.

In einer weiterhin bevorzugten Ausgestaltung der vorliegenden Erfindung werden Arylchinazolin-Derivate der Formel (III) bereitgestellt 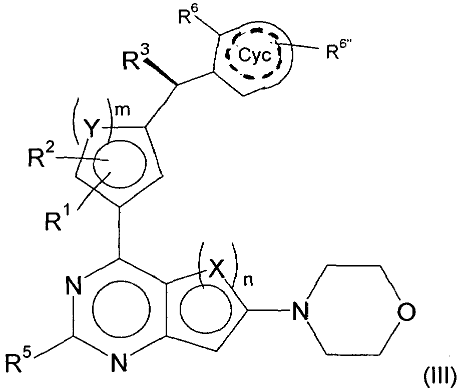 worin
- R³: Hal, CN, OH, CONH₂, CONH(LA) oder LA ist;
- R⁶: Hal, LA, Oxo, CN, NH₂ oder Het² ist;
- R^{6"}: H, Hal, LA, Oxo, CN, NH₂ oder Het² ist;
- ----: die An- oder Abwesenheit von Doppelbindungen in Cyc bedeutet;
und die übrigen Substituenten die für die Formel (I) angegebene Bedeutung haben, und/oder ihre physiologisch unbedenklichen Salze, Tautomere und/oder Stereoisomere, einschließlich deren Mischungen in allen Verhältnissen.
Es wurde nämlich gefunden, daß die Aktivität der erfindungsgemäßen Verbindungen dann besonders hoch ist, wenn R³ wie in der Formel (III) dargestellt konfiguriert ist und Cyc in Ortho-Stellung durch R⁶ substituiert ist.

Ganz besonders bevorzugt sind die Teilformeln (IIa), (IIb), (IIIa) und (IIIb) der Formeln (II) und (III): 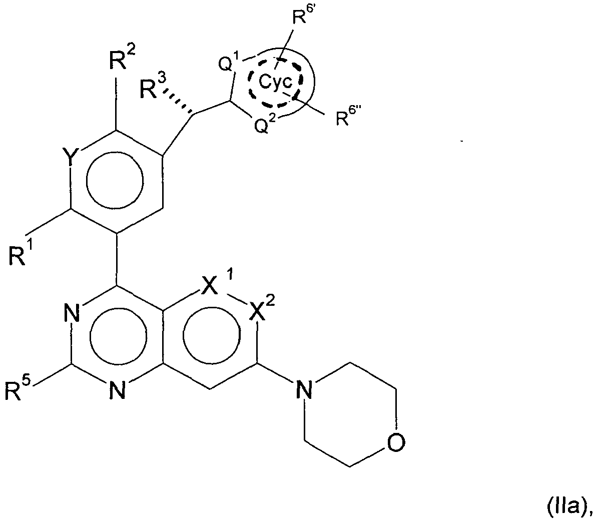 worin
- R², R³: unabhängig voneinander Hal, CN, OH, CONH₂, CON(LA) oder LA sind;
- R^{6'}, R^{6"}: unabhängig voneinander H, Hal, LA, Oxo, CN, NH₂ oder Het² sind;
- Q¹, Q²: unabhängig voneinander CH, N oder NH und in jedem Fall unsubstituiert sind;
- X¹: CH, CF oder N ist;
- X²: CH oder N ist,
wobei X¹, X² nicht gleichzeitig N sind;
- Y: CH oder N ist;
- ----: die An- oder Abwesenheit von Doppelbindungen in Cyc bedeutet;
und die übrigen Substituenten die für die Formel (I) angegebene Bedeutung haben, und/oder ihre physiologisch unbedenklichen Salze, Tautomere und/oder Stereoisomere, einschließlich deren Mischungen in allen Verhältnissen; 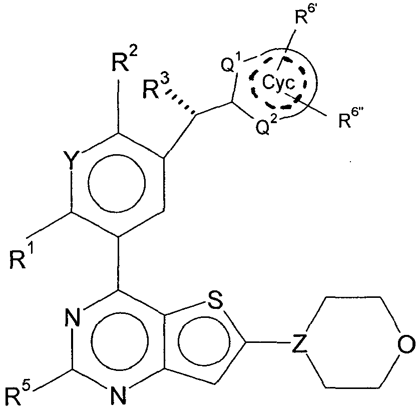 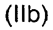 worin
- R², R³: unabhängig voneinander Hal, CN, OH, CONH₂, CON(LA) oder LA sind;
- R^{6'}, R^{6"}: unabhängig voneinander H, Hal, LA, Oxo, CN, NH₂ oder Het² sind;
- Q¹, Q²: unabhängig voneinander CH, N oder NH und in jedem Fall unsubstituiert sind;
- Y: CH oder N ist,
- ----: die An- oder Abwesenheit von Doppelbindungen in Cyc bedeutet;
und alle übrigen Substituenten die für die Formel (I) angegebene Bedeutung haben, und/oder ihre physiologisch unbedenklichen Salze, Tautomere und/oder Stereoisomere, einschließlich deren Mischungen in allen Verhältnissen; 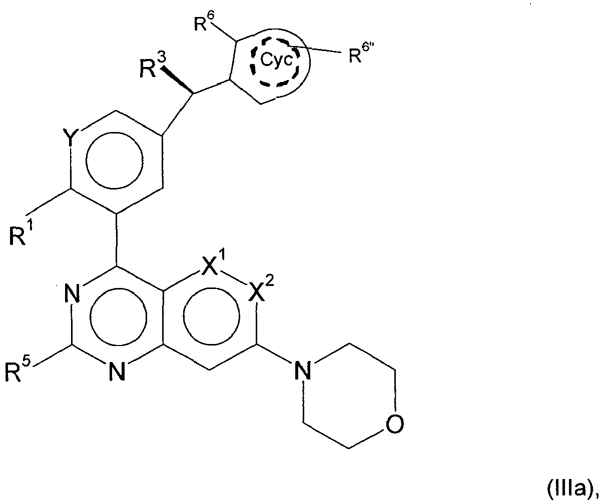 worin
- R³: Hal, CN, OH, CONH₂, CON(LA) oder LA ist;
- R⁶: Hal, LA, Oxo, CN, NH₂ oder Het² ist;
- R^{6"}: H, Hal, LA, Oxo, CN, NH₂ oder Het² ist;
- X¹: CH, CF oder N ist;
- X²: CH oder N ist,
wobei X¹, X² nicht gleichzeitig N sind;
- Y: CH oder N ist;
- ----: die An- oder Abwesenheit von Doppelbindungen in Cyc bedeutet;
und die übrigen Substituenten die für die Formel (I) angegebene Bedeutung haben, und/oder ihre physiologisch unbedenklichen Salze, Tautomere und/oder Stereoisomere, einschließlich deren Mischungen in allen Verhältnissen; 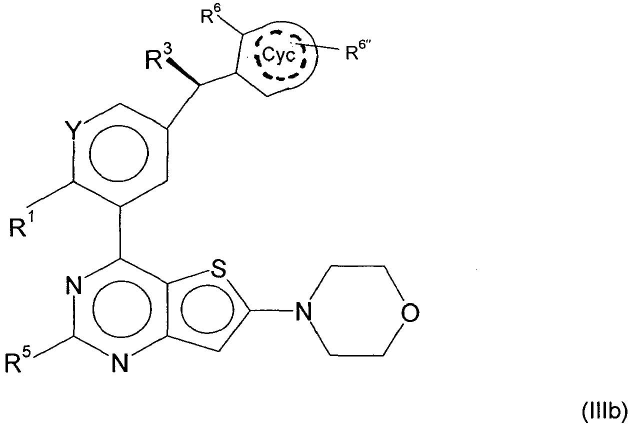 worin
- R³: Hal, CN, OH, CONH₂, CON(LA) oder LA ist;
- R⁶: Hal, LA, Oxo, CN, NH₂ oder Het² ist;
- R^{6"}: H, Hal, LA, Oxo, CN, NH₂ oder Het² ist;
- Y: CH oder N ist,
- ----: die An- oder Abwesenheit von Doppelbindungen in Cyc bedeutet;
und alle übrigen Substituenten die für die Formel (I) angegebene Bedeutung haben, und/oder ihre physiologisch unbedenklichen Salze, Tautomere und/oder Stereoisomere, einschließlich deren Mischungen in allen Verhältnissen.

Weiter bevorzugte Untergruppen von Verbindungen der Formel (IIa) können durch die folgenden Teilformeln (IIa-A) bis (IIa-O) ausgedrückt werden, die der Formel (IIa) entsprechen, worin jedoch
bei der Teilformel (IIa-A)
- X¹: CH,
- R¹: F oder Cl,
- R²: F oder Cl,
bei der Teilformel (IIa-B)
- R¹: F,
- R²: F oder Cl,
bei der Teilformel (IIa-C)
- X¹, X²: CH,
bei der Teilformel (IIa-D)
- X¹: CH,
- R⁵: H,
bei der Teilformel (IIa-E)
- R³: H, OH,
bei der Teilformel (IIa-F)
- X¹: CH,
- R³: OH,
bei der Teilformel (IIa-G)
- X¹: CH,
- Y: CH,
bei der Teilformel (IIa-H)
- X¹: CH,
- Cyc: Pyridin, Pyrazin oder Pyridazin, oder Pyrazolo[1,5-a]pyrimidinyl oder Imidazo[1,2-b]pyridazinyl,
bei der Teilformel (IIa-J)
- Cyc: Pyridin, Pyrazin, Pyridazin, Pyrazolo[1,5-a]pyrimidinyl, Imidazo[1,2-b]pyridazinyl, Furo[2,3-c]pyridinyl, Furo[2,3-d]pyridazinyl, Thieno[2,3-d]pyridazinyl, Thieno[2,3-d]pyrimidinyl oder Imidazo[4,5-c]pyridinyl, die jeweils unsubstituiert sein können, oder ein- oder zweifach mit Methoxy, Methyl, Oxo, Cl oder CHF₂O substituiert sein können,
bei der Teilformel (IIa-K)
- R¹: F oder Cl,
- R²: F oder Cl,
- R³: OH,
- R⁵: H,
- X¹, X²: CH,
bei der Teilformel (IIa-L)
- R¹: F,
- R²: F oder Cl,
- R³: H oder OH,
- R⁵: H,
bei der Teilformel (IIa-M)
- R¹: F oder Cl,
- R²: F oder Cl,
- R³: OH,
- R⁵: H,
- X¹, X²: CH,
- Cyc: Pyridin, Pyrazin oder Pyridazin, oder Pyrazolo[1,5-a]pyrimidinyl oder Imidazo[1,2-b]pyridazinyl,
bei der Teilformel (IIa-N)
- R¹: F,
- R²: F oder Cl,
- R³: H oder OH,
- R⁵: H,
- Cyc: Pyridin, Pyrazin, Pyridazin, Pyrazolo[1,5-a]pyrimidinyl, Imidazo[1,2-b]pyridazinyl, Furo[2,3-c]pyridinyl, Furo[2,3-d]pyridazinyl, Thieno[2,3-d]pyridazinyl, Thieno[2,3-d]pyrimidinyl oder Imidazo[4,5-c]pyridinyl, die jeweils unsubstituiert sein können, oder ein- oder zweifach mit Methoxy, Methyl, Oxo, Cl oder CHF₂O substituiert sein können,
bei der Teilformel (IIa-O)
- R¹: F,
- R²: F oder Cl,
- R³: H oder OH,
- R⁵: H,
- Cyc: 5-Methoxy-pyridazin-3-yl, Imidazo[1,2-b]pyridazin-6-yl, 3-Chloro-6-methoxy-pyrazin-2-yl, 3-Chloro-pyrazin-2-yl, Pyridazin-4-yl, 3-Methoxy-pyrazin-2-yl, 6-Methoxy-pyridazin-3-yl, 3-Difluoromethoxy-pyridin-2-yl, 3-Methyl-pyrazin-2-yl, Thieno[2,3-d]pyrimidin-4-yl, 1-Methyl-1H-pyridin-2-one-6-yl, 1H-Pyridazin-6-one-3-yl, Furo[2,3-d]pyridazin-7-yl, Thieno[2,3-d]pyridazin-7-yl, 3,5-Dimethyl-pyrazin-2-yl, Furo[2,3-d]pyrimidin-4-yl, 3-Methyl-3H-imidazo[4,5-c]pyridin-4-yl,
ist und/oder ihre physiologisch unbedenklichen Salze, Tautomere und/oder Stereoisomere, einschließlich deren Mischungen in allen Verhältnissen.

Weiter bevorzugte Untergruppen von Verbindungen der Formel (lila) können durch die folgenden Teilformeln (IIIa-B) bis (IIIa-O) ausgedrückt werden, die der Formel (IIIa) entsprechen, worin jedoch
bei der Teilformel (IIIa-B)
- R¹: F,
bei der Teilformel (IIIa-C)
- X¹, X²: CH,
bei der Teilformel (IIIa-D)
- X¹: CH,
- R⁵: H,
bei der Teilformel IIIa- (E)
- R³: H, OH,
bei der Teilformel (IIIa-F)
- X¹: CH,
- R³: OH,
bei der Teilformel (IIIa-G)
- X¹: CH,
- Y: CH,
bei der Teilformel (IIIa-H)
- X¹: CH,
- Cyc: Pyridin, Pyrazin oder Pyridazin, oder Pyrazolo[1,5-a]pyrimidinyl oder Imidazo[1,2-b]pyridazinyl,
bei der Teilformel (IIIa-J)
- Cyc: Pyridin, Pyrazin, Pyridazin, Pyrazolo[1,5-a]pyrimidinyl, Imidazo[1,2-b]pyridazinyl, Furo[2,3-c]pyridinyl, Furo[2,3-d]pyridazinyl, Thieno[2,3-d]pyridazinyl, Thieno[2,3-d]pyrimidinyl oder Imidazo[4,5-c]pyridinyl, die jeweils unsubstituiert sein können, oder ein- oder zweifach mit Methoxy, Methyl, Oxo, Cl oder CHF₂O substituiert sein können,
bei der Teilformel (IIIa-K)
- R¹: F oder Cl,
- R³: OH,
- R⁵: H,
- X¹, X²: CH,
bei der Teilformel (IIIa-L)
- R¹: F,
- R³: H oder OH,
- R⁵: H,
bei der Teilformel (IIIa-M)
- R¹: F oder Cl,
- R³: OH,
- R⁵: H,
- X¹, X²: CH,
- Cyc: Pyridin, Pyrazin oder Pyridazin, oder Pyrazolo[1,5-a]pyrimidinyl oder Imidazo[1,2-b]pyridazinyl,
bei der Teilformel (IIIa-N)
- R¹: F,
- R³: H oder OH,
- R⁵: H,
- Cyc: Pyridin, Pyrazin, Pyridazin, Pyrazolo[1,5-a]pyrimidinyl, Imidazo[1,2-b]pyridazinyl, Furo[2,3-c]pyridinyl, Furo[2,3-d]pyridazinyl, Thieno[2,3-d]pyridazinyl, Thieno[2,3-d]pyrimidinyl oder Imidazo[4,5-c]pyridinyl, die jeweils unsubstituiert sein können, oder ein- oder zweifach mit Methoxy, Methyl, Oxo, Cl oder CHF₂O substituiert sein können,
bei der Teilformel (IIIa-O)
- R¹: F,
- R³: H oder OH,
- R⁵: H,
- Cyc: 5-Methoxy-pyridazin-3-yl, Imidazo[1,2-b]pyridazin-6-yl, 3-Chloro-6-methoxy-pyrazin-2-yl, 3-Chloro-pyrazin-2-yl, Pyridazin-4-yl, 3-Methoxy-pyrazin-2-yl, 6-Methoxy-pyridazin-3-yl, 3-Difluoromethoxy-pyridin-2-yl, 3-Methyl-pyrazin-2-yl, Thieno[2,3-d]pyrimidin-4-yl, 1-Methyl-1H-pyridin-2-one-6-yl, 1H-Pyridazin-6-one-3-yl, Furo[2,3-d]pyridazin-7-yl, Thieno[2,3-d]pyridazin-7-yl, 3,5-Dimethyl-pyrazin-2-yl, Furo[2,3-d]pyrimidin-4-yl, 3-Methyl-3H-imidazo[4,5-c]pyridin-4-yl,
ist und/oder ihre physiologisch unbedenklichen Salze, Tautomere und/oder Stereoisomere, einschließlich deren Mischungen in allen Verhältnissen.

Weiter bevorzugte Untergruppen von Verbindungen der Formel (IIb) können durch die folgenden Teilformeln (IIb-Q) bis (IIb-U) ausgedrückt werden, die der Formel (IIb) entsprechen, worin jedoch
bei der Teilformel (IIb-Q)
- R¹: F oder Cl,
- R²: F oder Cl,
- R³: OH,
- R⁵: H,
- Y: CH,
bei der Teilformel (IIb-R)
- R¹: F,
- R²: F oder Cl,
- R³: OH,
- R⁵: H,
- Y: CH,
bei der Teilformel (IIb-S)
- Cyc: Pyridin, Pyrazin oder Pyridazin,
bei der Teilformel (IIb-T)
- R¹: F oder Cl,
- R²: F oder Cl,
- R³: OH,
- R⁵: H,
- Cyc: Pyridin, Pyrazin oder Pyridazin,
bei der Teilformel (IIb-U)
- R¹: F,
- R²: F oder Cl,
- R³: OH,
- R⁵: H,
- Cyc: Pyridin, Pyrazin, Pyridazin oder 3-Methyl-pyrazin-2-yl,
ist und/oder ihre physiologisch unbedenklichen Salze, Tautomere und/oder Stereoisomere, einschließlich deren Mischungen in allen Verhältnissen.

Weiter bevorzugte Untergruppen von Verbindungen der Formel (IIIb) können durch die folgenden Teilformeln (IIIb-Q) bis (IIIb-U) ausgedrückt werden, die der Formel (IIIb) entsprechen, worin jedoch
bei der Teilformel (IIIb-Q)
- R¹: F oder Cl,
- R³: OH,
- R⁵: H,
- Y: CH,
bei der Teilformel (IIIb-R)
- R¹: F,
- R³: OH,
- R⁵: H,
- Y: CH,
bei der Teilformel (IIIb-S)
- Cyc: Pyridin, Pyrazin oder Pyridazin,
bei der Teilformel (IIIb-T)
- R¹: F oder Cl,
- R³: OH,
- R⁵: H,
- Cyc: Pyridin, Pyrazin oder Pyridazin,
bei der Teilformel (IIIb-U)
- R¹: F,
- R³: OH,
- R⁵: H,
- Cyc: Pyridin, Pyrazin, Pyridazin oder 3-Methyl-pyrazin-2-yl,
ist und/oder ihre physiologisch unbedenklichen Salze, Tautomere und/oder Stereoisomere, einschließlich deren Mischungen in allen Verhältnissen.

Ganz besonders bevorzugt sind solche Verbindungen der Formeln (I) und deren Teilformeln, und/oder ihre physiologisch unbedenklichen Salze, Tautomere und/oder Stereoisomere, einschließlich deren Mischungen in allen Verhältnissen, die in den Tabellen 1-8 zusammengestellt sind.

Die Verbindungen der Formel (I) und auch die Ausgangsstoffe zu ihrer Herstellung werden nach an sich bekannten Methoden hergestellt, wie sie in der Literatur (z.B. in Standardwerken wie Houben-Weyl, Methoden der organischen Chemie, Georg-Thieme-Verlag, Stuttgart) beschrieben und/oder Fachmann bekannt sind, sowie unter Reaktionsbedingungen, die für die genannten Umsetzungen bekannt und geeignet sind. Dabei kann man auch von an sich bekannten, hier nicht näher erwähnten Varianten Gebrauch machen. Die Reaktionszeit liegt je nach angewendeten Bedingungen zwischen einigen min und 14 Tagen, die Reaktionstemperatur zwischen -70°C und 150°C, normalerweise zwischen -50°C und 100°C, besonders bevorzugt zwischen -10°C und 70°C.

Die Umsetzung erfolgt in einem inerten Lösungsmittel und in der Regel in Gegenwart eines säurebindenden Mittels, vorzugsweise einer organischen Base wie DIPEA, Triethylamin, Dimethylanilin, Pyridin, Chinolin, Piperidin oder Diethanolamin. Auch der Zusatz eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein. Als Basen eignen sich Metalloxide, wie z.B. Aluminiumoxid, Alkalimetallhydroxide (darunter Kaliumhydroxid, Natriumhydroxid und Lithiumhydroxid), Erdalkalimetallhydroxide (z.B. Bariumhydroxid und Calciumhydroxid) und Alkalimetallalkoholate (z.B. Kaliumethanolat und Natriumpropanolat).

Als inerte Lösungsmittel eignen sich u.a. Kohlenwasserstoffe wie Cyclohexan, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Methyl-tert.-butylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Essigsäureethylester oder Gemische der genannten Lösungsmittel. Besonders bevorzugt sind DMF, Methanol, Dichlormethan, THF, Essigsäure und Acetonitril.

Das Verfahren und die anschließende Aufarbeitung des Reaktionsgemisches können grundsätzlich als Batch-Reaktion oder in kontinuierlicher Reaktionsweise durchgeführt werden. Die kontinuierliche Reaktionsweise umfasst z.B. die Reaktion in einem kontinuierlichen Rührkesselreaktor, einer Rührkesselkaskade, einem Schlaufen- oder Querstromreaktor, einem Strömungsrohr oder in einem Mikroreaktor. Die Aufarbeitung der Reaktionsgemische erfolgt wahlweise, je nach Bedarf, durch Filtration über feste Phasen, Chromatographie, Separation zwischen unmischbaren Phasen (z. B. Extraktion), Adsorption an festen Trägern, Abdestillieren von Lösungsmitteln und/oder azeotropen Gemischen, selektive Destillation, Sublimation, Kristallisation, Co-Kristallisation oder durch Nanofiltration an Membranen.

Die Verbindungen der Formel (I) können vorzugsweise erhalten werden, indem man Verbindungen der Formeln (V) und (IV) umsetzt. Gegenstand der vorliegenden Erfindung ist damit auch ein Verfahren zum Herstellen von Verbindungen der Formel (I), Teilformeln davon und/oder ihrer physiologisch unbedenklichen Salze, Tautomere und/oder Stereoisomere, einschließlich deren Mischungen in allen Verhältnissen, mit den folgenden Schritten:
(a) Umsetzen einer Verbindung der Formel (V) 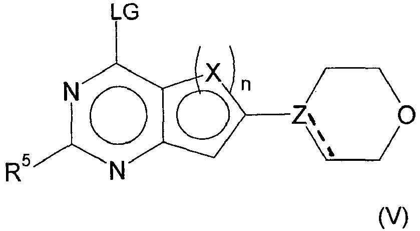 worin LG eine übliche Abgangsgruppe wie Hal ist,
   mit einer Verbindung der Formel (IV) 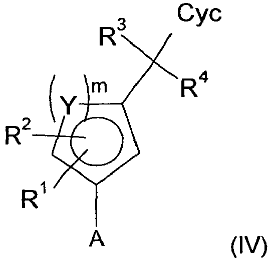 worin A Boronsäure oder ein Boronsäureester ist,
   unter Erhalt der Verbindungen der Formel (I), und gegebenenfalls
(b) Umwandeln einer Base oder Säure der Verbindungen der Formel (I) in eines ihrer Salze.

Die Ausgangsverbindungen sind in der Regel bekannt. Sind sie neu, so können sie nach an sich bekannten Methoden hergestellt werden. Die Verbindungen der Formeln (I), (Ia), (Ib), (II), (IIa), (IIb), (III), (IIIa), (IIIb), (IV) und (V) können nach bekannten Methoden bereitgestellt werden. Fall gewünscht, können die Ausgangsstoffe *in situ* gebildet werden, so dass man sie aus dem Reaktionsgemisch nicht isoliert, sondern sofort weiter zu den erfindungsgemäßen Verbindungen umsetzt. Es ist ebenso möglich, die Reaktion schrittweise durchzuführen.

Die genannten erfindungsgemäßen Verbindungen lassen sich in ihrer endgültigen Nichtsalzform verwenden. Andererseits umfasst die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel (I) sowie ihrer Teilformeln werden größtenteils konventionell hergestellt. Sofern die Verbindungen eine Carbonsäuregruppe enthalten, lässt sich eines ihrer geeigneten Salze dadurch bilden, dass man die Verbindung mit einer geeigneten Base zum entsprechenden Basenadditionssalz umsetzt. Solche Basen sind zum Beispiel Alkalimetallhydroxide (z.B. Kaliumhydroxid, Natriumhydroxid und Lithiumhydroxid), Erdalkalimetallhydroxide (z.B. Bariumhydroxid und Calciumhydroxid), Alkalimetallalkoholate (z.B. Kaliumethanolat und Natriumpropanolat) sowie verschiedene organische Basen wie Piperidin, Diethanolamin und N-Methylglutamin. Eine Base der Formel (I) sowie deren Teilformeln kann mit einer Säure in das zugehörige Säureadditionssalz übergeführt werden, beispielsweise durch Umsetzung äquivalenter Mengen der Base und der Säure in einem inerten Lösungsmittel, wie z.B. Ethanol, und Eindampfen im Anschluss. Für diese Umsetzung kommen insbesondere Säuren in Frage, die physiologisch unbedenkliche Salze liefern, wie z.B. Halogenwasserstoffe (z.B. Chlorwasserstoff, Bromwasserstoff oder Jodwasserstoff), andere mineralsäuren und ihre entsprechenden Salzen (z.B. Sulfat, Nitrat oder Phosphat und dergleichen), Alkyl- und Monoarylsulfonaten (z.B. Ethansulfonat, Toluolsulfonat und Benzolsulfonat) sowie andere organische Säuren und ihre entsprechenden Salze (z.B. Acetat, Trifluoracetat, Tartrat, Maleat, Succinat, Citrat, Benzoat, Salicylat, Ascorbat und dergleichen. Salze mit physiologisch bedenklichen Säuren, z.B. Pikrate, können zur Isolierung und/oder Aufreinigung der Verbindungen der Formel (I) verwendet werden.

Im Hinblick auf das oben Gesagte sieht man, dass unter dem Ausdruck "pharmazeutisch unbedenkliches Salz" im vorliegenden Zusammenhang ein Wirkstoff zu verstehen ist, der eine Verbindung der Formel (I) in der Form eines ihrer Salze enthält, insbesondere dann, wenn diese Salzform dem Wirkstoff im Vergleich zu der freien Form des Wirkstoffs verbesserte pharmakokinetische Eigenschaften verleiht. Die pharmazeutisch unbedenkliche Salzform des Wirkstoffs kann diesem Wirkstoff auch erst eine gewünschte pharmakokinetische Eigenschaft verleihen und kann sogar die Pharmakodynamik dieses Wirkstoffs in Bezug auf seine therapeutische Wirksamkeit im Körper positiv beeinflussen.

Erfindungsgemäße Verbindungen können aufgrund ihrer Molekülstruktur chiral sein und dementsprechend in verschiedenen enantiomeren Formen auftreten. Sie können daher in racemischer oder in optisch aktiver Form vorliegen. Da sich die pharmazeutische Wirksamkeit der Racemate bzw. der Stereoisomeren der Verbindungen der Formel (I) unterscheiden kann, mag es wünschenswert sein, die Enantiomere zu verwenden. In diesen Fällen kann das Endprodukt oder bereits das Zwischenprodukt in enantiomere Verbindungen, durch dem Fachmann bekannte chemische oder physikalische Maßnahmen, aufgetrennt oder bereits als solche bei der Synthese eingesetzt werden.

Es ist allgemein bekannt, dass Atome atomare Massen oder Massenzahlen haben können, die von den üblicherweise natürlich vorkommenden atomaren Massen oder Massenzahlen abweichen können. Beispiele für Isotope, die kommerziell verfügbar sind und die durch bekannte Methoden in eine erfindungsgemäße Verbindung inkorporiert werden können, sind Isotope von Wasserstoff, Kohlenstoff, Stickstoff, Sauerstoff, Phosphor, Fluor und Chor, bspw. ²H, ³H, ¹³C, ¹⁴C, ¹⁵N, ¹⁸O, ¹⁷O, ³¹P, ³²P, ³⁵S, ¹⁸F und ³⁶Cl. Der Einbau von schwereren Isotopen, insbesondere Deuterium (²H) in eine erfindungsgemäße Verbindung hat therapeutische Vorteile, die in der höheren metabolischen Stabilität dieser isotopenmarkierten Verbindung begründet liegen. Höhere metabolische Stabilität führt unmittelbar zu einer erhöhten *in vivo* Halbwertszeit, was eine geringere Dosierung ermöglicht.

Daher beziehen sich die Definitionen der Atome H, C, N usw., wie in den erfindungsgemäßen Verbindungen benutzt, auch auf die schwereren Isotope dieser Atome. Erfindungsgemäß besonders bevorzugt ist die Verwendung von D (Deuterium, ²H) anstelle von Wasserstoff (¹H).

Es wurde gefunden, dass die erfindungsgemäßen Verbindungen eine spezifische Hemmung von Serin-Threonin-Proteinkinasen bewirken. Ein weiterer Gegenstand der Erfindung betrifft deshalb die Verwendung von Verbindungen der Formel (I) bzw. Teilformeln davon und/oder ihrer physiologisch unbedenklichen Salze, Tautomere und/oder Stereoisomere, einschließlich deren Mischungen in allen Verhältnissen, zur Hemmung von Serin-Threonin-Proteinkinasen, vorzugsweise PIKK, besonders bevorzugt DNA-PK. Bevorzugt ist insbesondere die Hemmung vorgenannter Serin-Threonin-Proteinkinasen *ex vivo* oder *in vitro.* Der Begriff "Hemmung" bezieht sich auf jede Verringerung der Aktivität, die auf der Wirkung der spezifischen erfindungsgemäßen Verbindungen basiert, indem letztere in der Lage sind, mit dem Zielmolekül derart zu interagieren, dass eine Erkennung, Bindung und Blockierung ermöglicht wird. Die Verbindungen zeichnen sich durch hohe Affinität zu wenigstens einer Serin-Threonin-Proteinkinasen aus, wodurch eine verlässliche Bindung und vorzugsweise vollständige Blockade der Kinaseaktivität sichergestellt wird. Die Verbindungen sind besonders bevorzugt mono-spezifisch, um eine ausschließliche und unmittelbare Erkennung der ausgewählten Kinase zu garantieren. Der Begriff der "Erkennung" bezieht sich hierbei auf jede Art der Wechselwirkung zwischen der Verbindung und den genannten Zielmolekülen, insbesondere kovalente oder nicht-kovalente Bindungen, wie z.B. eine kovalente Bindung, hydrophobe/ hydrophile Wechselwirkungen, van der Waals'sche Kräfte, Ionenbeziehung, Wasserstoffbrücken, Ligand-Rezeptor-Wechselwirkungen, Basenpaarungen von Nukleotiden oder Wechselwirkungen zwischen Epitop und Antikörper-Bindungsstelle.

Die erfindungsgemäßen Verbindungen zeigen eine vorteilhafte biologische Aktivität, die in den hierin beschriebenen Tests nachweisbar ist, wie z.B. auf Enzymen basierenden Assays. Die Messung der Kinaseaktivität ist eine dem Fachmann wohlbekannte Technik. Generische Testsysteme zur Bestimmung der Kinaseaktivität mit Substraten, z.B. Histon (Alessi et al. (1996) FEBS Lett. 399(3): 333) oder dem basischen Myelinprotein sind in der Literatur beschrieben (Campos-González & Glenney (1992) JBC 267: 14535). Zur Identifikation von Kinase-Inhibitoren stehen verschiedene Assay-Systeme zur Verfügung. Beim Scintillation Proximity Assay (Sorg et al. (2002) J Biomolecular Screening 7: 11) und dem FlashPlate Assay werden die radioaktive Phosphorylierung eines Proteins oder Peptids als Substrat mit ATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations-(FP-) Technologien als Assay-Verfahren nützlich (Sills et al. (2002) J Biomolecular Screening 191). Andere nicht-radioaktive ELISA-Verfahren verwenden spezifische Phospho-Antikörper (Phospho-AK). Der Phospho-AK bindet nur das phosphorylierte Substrat. Diese Bindung ist mit einem zweiten Peroxidase-konjugierten Anti-Schaf-Antikörper durch Chemilumineszens nachweisbar.

Die vorgenannte Verwendung der Verbindungen kann in *in vitro* oder *in vivo* Modellen geschehen. Die Suszeptibilität einer bestimmten Zelle gegenüber der Behandlung mit den erfindungsgemäßen Verbindungen kann durch Testen *in vitro* bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und bis zu 9 Tagen. Zum Testen *in vitro* können kultivierte Zellen aus einer Biopsieprobe verwendet werden. Die Menge der nach der Behandlung zurückbleibenden Zellen wird dann bestimmt. Die Verwendung *in vitro* erfolgt insbesondere an Proben von Säugerspezies, die an Krebs, Tumoren bzw.Metastasen leiden. Der Wirt oder Patient kann jeglicher Säugerspezies angehören, z. B. einer Primatenspezies, insbesondere Menschen, aber auch Nagetieren (einschließlich Mäusen, Ratten und Hamstern), Kaninchen, Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Das Testen mehrerer spezifischer Verbindungen ermöglicht die Auswahl des Wirkstoffs, der am besten für die Behandlung des Patienten geeignet erscheint. Die *in vivo* Dosis der gewählten Verbindung wird vorteilhaft auf die Suszeptibilität der Kinase und/oder Schwere der Erkrankung des Patienten mit Blick auf die *in vitro* Daten abgestimmt, wodurch die therapeutische Wirksamkeit merklich erhöht ist. Die Dosis variiert abhängig von der verwendeten spezifischen Verbindung, der spezifischen Erkrankung, dem Patientenstatus usw. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die nachfolgende Lehre der Erfindung und deren Ausführungsformen betreffend die Verwendung von Verbindungen der Formel (I) für die Herstellung eines Arzneimittels zur Prophylaxe, Therapie und/oder Verlaufskontrolle ist gültig und ohne Einschränkungen auf die Verwendung der Verbindungen zur Hemmung der Kinaseaktivität anwendbar, sofern es sinnvoll erscheint.

Die Behandlung wird im Allgemeinen fortgesetzt, bis eine erhebliche Reduktion vorliegt, z.B. mindestens ca. 50 % Verminderung der Zelllast und kann fortgesetzt werden, bis im Wesentlichen keine unerwünschten Zellen mehr im Körper nachgewiesen werden. In derartigen Tests zeigen und bewirken die erfindungsgemäßen Verbindungen einen inhibierenden Effekt, der gewöhnlich durch IC₅₀-Werte in einem geeigneten Bereich, bevorzugt im mikromolaren Bereich und bevorzugter im nanomolaren bis picomolaren Bereich dokumentiert wird. Die Kinase wird insbesondere zu 50 % gehemmt, wenn die Konzentration der Verbindungen kleiner als 1 µM ist, vorzugsweise gleich oder kleiner als 0,5 µM, besonders bevorzugt kleiner als 0,1 µM. Diese Konzentration wird als IC₅₀-Wert bezeichnet.

Gegenstand der Erfindung ist auch ein Arzneimittel umfassend mindestens eine Verbindung der Formel (I) bzw. Teilformeln davon und/oder ihre physiologisch unbedenklichen Salze, Tautomere und/oder Stereoisomere, einschließlich deren Mischungen in allen Verhältnissen. Noch ein Gegenstand der Erfindung ist eine pharmazeutische Zusammensetzung umfassend als Wirkstoff eine wirksame Menge von mindestens einer Verbindung der Formel (I) bzw. Teilformeln davon und/oder ihrer physiologisch unbedenklichen Salze, Tautomere und/oder Stereoisomere, einschließlich deren Mischungen in allen Verhältnissen, zusammen mit pharmazeutisch verträglichen Hilfsstoffen.

Ein "Arzneimittel", "Medikament" sowie eine "pharmazeutische Zusammensetzung" oder "pharmazeutische Formulierung" ist hierbei jedes Mittel, welches in der Prophylaxe, Therapie, Verlaufskontrolle oder Nachbehandlung von Patienten eingesetzt werden kann, die zumindest zeitweise eine pathogene Modifikation des Gesamtzustandes bzw. des Zustandes einzelner Teile des Patientenorganismus zeigen, vorzugsweise infolge von Krebs, Tumoren bzw. Metastasen.

Um die protektive oder therapeutische Wirkung der erfindungsgemäßen Verbindungen zu erhöhen, können pharmazeutisch verträgliche Adjuvantien zugesetzt werden. Im Sinne der Erfindung ist jede Substanz, die mit den Verbindungen gemäß der Erfindung eine Wirkung ermöglicht, verstärkt oder modifiziert, ein "Adjuvans". Bekannte Adjuvantien sind z.B. Aluminiumverbindungen, wie z.B. Aluminiumhydroxid oder Aluminiumphosphat, Saponine, wie z.B. QS 21, Muramyldipeptid oder Muramyltripeptid, Proteine, wie z.B. Gammainterferon oder TNF, MF 59, Phosphatdibylcholin, Squalen oder Polyole. Ebenfalls kann die Co-Applikation von Ei-Albumin in komplettem Freund'schen Adjuvans eine gesteigerte zellvermittelte Immunität hervorrufen und somit die Wirkung gebildeter neutralisierender Antikörper unterstützen. Des Weiteren kann DNA, die eine immunstimulatorische Eigenschaft hat, oder die ein Protein mit Adjuvanseffekt kodiert, wie z.B. ein Cytokin, parallel oder in einem Konstrukt appliziert werden.

Das Einbringen des pharmazeutischen Mittels in eine Zelle respektive einen Organismus kann erfindungsgemäß auf jede Art und Weise geschehen, die es ermöglicht, dass die Kinasen mit den in der Zusammensetzung enthaltenen Verbindungen in Kontakt gebracht werden, infolgedessen eine Antwort induziert wird. Das pharmazeutische Mittel der vorliegenden Erfindung kann oral, transdermal, transmucosal, transurethal, vaginal, rektal, pulmonal, enteral und/oder parenteral angewendet werden. Die gewählte Art der Verabreichung richtet sich nach der Indikation, der zu verabreichenden Dosis, Individuumsspezifischen Parametern etc. Insbesondere ermöglichen die verschiedenen Arten der Verabreichung eine ortsspezifische Therapie, die Nebenwirkungen minimiert und die Wirkstoffdosis verringert. Ganz besonders bevorzugte Injektionen sind die intradermale, subkutane, intramuskuläre oder intravenöse Injektion. Die Applikation kann z.B. mit Hilfe so genannter Impfpistolen oder mittels Spritzen geschehen. Es ist auch möglich, die Substanz als Aerosol bereitzustellen, welches von dem Organismus, bevorzugt einem humanen Patienten, inhaliert wird.

Die Darreichungsformen des pharmazeutischen Mittels werden mit den üblichen festen oder flüssigen Trägerstoffen und/oder Verdünnungsmitteln und den üblicherweise eingesetzten Hilfsstoffen entsprechend der gewünschten Applikationsart in einer geeigneten Dosierung und in an sich bekannter Weise hergestellt. Grundsätzlich können also pharmazeutisch annehmbare und dem Fachkundigen bekannte Exzipienten einen Teil des erfindungsgemäßen pharmazeutischen Mittels bilden, wobei die Menge des Exzipientenmaterials, die mit dem Wirkstoff kombiniert wird, um eine Einzeldosierung herzustellen, in Abhängigkeit vom zu behandelnden Individuum und der Art der Verabreichung variiert. Diese pharmazeutisch verträglichen Zusätze umfassen Salze, Puffer, Füllstoffe, Stabilisatoren, Komplexbildner, Antioxidantien, Lösungsmittel, Bindemittel, Gleitmittel, Tablettenüberzüge, Geschmacksstoffe, Farbstoffe, Konservierungsmittel, Stellmittel und dergleichen. Beispiele für solche Exzipienten sind Wasser, pflanzliche Öle, Benzylalkohole, Alkylenglycol, Polyethylenglycol, Kolliphor, Glycerintriacetat, Gelatine, Hydroxypropylmethylcellulose (HPMC), Kohlenhydrate, wie z.B. Laktose oder Stärke, Magnesiumstearat, Talk und Vaseline.

Die pharmazeutische Formulierung kann als Tablette, Filmtablette, Dragee, Lutschtablette, Kapsel, Pille, Pulver, Granulat, Sirup, Saft, Tropfen, Lösung, Dispersion, Suspension, Suppositor, Emulsion, Extrudat, Implantat, Creme, Gel, Salbe, Paste, Lotion, Serum, Öl, Spray, Aerosol, Kleber, Pflaster oder Verband vorliegen. Als orale Darreichungsform werden vorzugsweise Tabletten, Filmtabletten, Dragees, Lutschtabletten, Kapseln, Pillen, Pulver, Granulate, Sirupe, Säfte, Tropfen, Lösungen, Dispersionen oder Suspensionen - auch als Depotform - zubereitet. Des Weiteren sind parenterale Arzneiformen, wie z.B. Suppositorien, Suspensionen, Emulsionen, Implantate oder Lösungen, in Betracht zu ziehen, vorzugsweise ölige oder wässrige Lösungen. Zur topischen Applikation wird der Arzneimittelwirkstoff mit mindestens einem pharmazeutisch annehmbaren Trägerstoff, wie z.B. mikrokristalliner Cellulose, und ggf. weiteren Hilfsstoffen, wie z.B. Feuchtigkeitsspendern, zu auf die Haut auftragbaren festen Formulierungen, wie z.B. Cremes, Gelen, Salben, Pasten, Pulver oder Emulsionen bzw. zu auf die Haut auftragbaren flüssigen Formulierungen, wie z.B. Lösungen, Suspensionen, Lotionen, Seren, Ölen, Sprays oder Aerosole in üblicher Weise formuliert. Vorzugsweise liegt das pharmazeutische Mittel als Injektionslösung vor. Für die Herstellung der Injektionslösung können wässrige Medien, wie z.B. eingeengtes Wasser oder physiologische Salzlösungen verwendet werden, wobei letztere saure und basische Additionssalze einschließen. Das pharmazeutische Mittel kann auch als feste Zusammensetzung, beispielsweise im lyophilisierten Zustand vorliegen, und dann durch Zugabe eines auflösenden Mittels, wie z.B. eingeengtes Wasser, vor der Verwendung bereitet werden. Die Grundlagen der Herstellung von Lyophilisaten sind dem Fachmann vertraut.

Die Konzentration der aktiven Verbindung in der Formulierung kann 0,1 bis 100 Gewichtsprozente betragen. Entscheidend ist, dass die pharmazeutische Zusammensetzung als Wirkstoff eine effektive Menge der Verbindung zusammen mit den pharmazeutisch verträglichen Hilfsstoffen umfasst. Die Begriffe "effektive Menge" oder "wirksame Dosis" werden hierin austauschbar verwendet und bezeichnen eine Menge des pharmazeutischen Wirkstoffs, die eine prophylaktisch oder therapeutisch relevante Wirkung auf eine Krankheit oder pathologische Veränderung in Zelle, Gewebe, Organ oder Säuger hat. Eine "prophylaktische Wirkung" verhindert das Ausbrechen einer Krankheit oder sogar die Infektion mit einem Pathogen nach dem Eindringen einzelner Vertreter derart, dass deren nachfolgende Ausbreitung stark verringert wird oder sie sogar vollständig inaktiviert werden. Eine "prophylaktische Wirkung" umfasst auch die Erhöhung der normalen physiologischen Funktion. Eine Prophylaxe ist insbesondere ratsam, wenn ein Individuum Veranlagungen für den Ausbruch der vorgenannten Krankheiten aufweist, wie z.B. eine familiäre Vorbelastung, einen Gendefekt oder eine kürzlich überstandene Krankheit. Eine "therapeutisch relevante Wirkung" befreit teilweise oder vollständig von einem, mehreren oder allen Krankheitssymptomen oder führt zur teilweisen oder vollständigen Rückkehr eines, mehrerer oder aller physiologischer oder biochemischer Parameter, die mit der Krankheit oder pathologischen Veränderung in Zusammenhang stehen oder ursächlich dafür sind, in den Normalzustand. Auch wird die Verlaufskontrolle als Art der therapeutischen Behandlung verstanden, wenn die Verbindungen in bestimmten Intervallen verabreicht werden, z.B. um die Symptome einer Krankheit vollständig zu beseitigen. Die jeweilige Dosis bzw. der Dosisbereich für die Gabe der erfindungsgemäßen Verbindungen ist groß genug, um den gewünschten prophylaktischen oder therapeutischen Effekt der Induktion einer biologischen oder medizinischen Antwort zu erreichen. Im Allgemeinen wird die Dosis mit dem Alter, der Konstitution und dem Geschlecht des Patienten variieren sowie die Schwere der Erkrankung berücksichtigen. Es versteht sich, dass die spezifische Dosis, Häufigkeit und Dauer der Verabreichung darüber hinaus von einer Vielzahl an Faktoren abhängen, wie z.B. der Zielsteuerungs- und Bindungsfähigkeit der Verbindungen, Ernährungsgewohnheiten des zu behandelnden Individuums, Art der Verabreichung, Ausscheidungsrate und Kombination mit anderen Medikamenten. Die individuelle Dosis kann sowohl in Bezug auf die primäre Erkrankung als auch in Bezug auf das Eintreten eventueller Komplikationen eingestellt werden. Die exakte Dosis ist durch einen Fachmann mit bekannten Mitteln und Methoden feststellbar. Diese Lehre der Erfindung ist gültig und ohne Einschränkungen auf die pharmazeutische Zusammensetzung umfassend die Verbindungen der Formel (I) anwendbar, sofern es sinnvoll erscheint.

In einer Ausführungsform der Erfindung werden die Verbindungen in einer Dosis von 0,01 mg bis 1 g pro Dosierungseinheit verabreicht, vorzugsweise zwischen 1 bis 700 mg, besonders bevorzugt 5 bis 200 mg. Die tägliche Dosierung liegt insbesondere zwischen 0,02 und 100 mg/kg Körpergewicht.

Zur Unterstützung der medizinischen Wirkung kann die pharmazeutische Zusammensetzung in einer Ausgestaltung der Erfindung auch einen oder mehrere weitere Wirkstoffe umfassen, wobei eine gleichzeitige oder aufeinander folgende Verabreichung denkbar ist. Die therapeutische Wirkung der erfindungsgemäßen pharmazeutischen Zusammensetzung kann beispielsweise darin bestehen, dass durch die Hemmung der DNA-PK als erwünschter Nebeneffekt bestimmte Antikrebsmittel besser wirken oder durch die Verminderung der Dosis die Anzahl der Nebenwirkungen dieser Medikamente reduziert wird.

In einer bevorzugten Ausführungsform der Erfindung wird die erfindungsgemäße pharmazeutische Zusammensetzung mit einem Antikrebsmittel kombiniert. Wie hier verwendet, betrifft der Begriff "Antikrebsmittel" jedes Mittel, das einem Patienten mit Krebs, Tumoren, bzw. Metastasen zum Zweck der Behandlung des Krebses verabreicht wird. Erfindungsgemäß bevorzugte Antikrebsmittel sind solche, die die DNA von Tumorzellen schädigen und damit in DNA-Replikation, DNA-Transkription oder die Genexpression eingreifen. Hierfür kommen insbesondere in Frage:
- Alkylierungsagentien, wie Altretamin, Bendamustin, Busulfan, Carmustin, Chlorambucil, Chlormethine, Cyclophosphamid, Dacarbazin, Ifosfamid, Improsulfantosylat, Lomustin, Melphalan, Mitobronitol, Mitolactol, Nimustin, Ranimustin, Temozolomid, Thiotepa, Treosulfan, Mechloretamin, Carboquon, Apaziquon, Fotemustin, Glufosfamid, Palifosfamid, Pipobroman, Trofosfamid, Uramustin;
- Platinverbindungen, wie Carboplatin, Cisplatin, Eptaplatin, Miriplatinhydrat, Oxaliplatin, Lobaplatin, Nedaplatin, Picoplatin, Satraplatin;
- Topoisomerase-Inhibitoren, wie Etoposid, Irinotecan, Razoxan, Sobuzoxan,
- DNA-verändernde Agentien, wie Amrubicin, Bisantren, Decitabin, Mitoxantron, Procarbazin, Trabectedin, Clofarabin, Amsacrin, Brostallicin, Pixantron, Laromustine;
- Antikrebs-Antibiotica, wie Bleomycin, Dactinomycin, Doxorubicin, Epirubicin, Idarubicin, Levamisol, Miltefosin, Mitomycin C,Romidepsin, Streptozocin, Valrubicin, Zinostatin, Zorubicin, Daunurobicin, Plicamycin, Aclarubicin, Peplomycin, Pirarubicin;
- Alpha-Emitters, wie Alpharadin (²²³Ra Dichlorid, Xofgio), ²¹¹At, ²¹³Bi, ²²⁵Ac, ²²⁷Th; Besonders bevorzugt sind Bleomycin und Alpharadin.

Die Erfindung kann auch als Kit praktiziert werden, der die erfindungsgemäßen Verbindungen enthält. Das Kit besteht aus getrennten Packungen von (a) einer wirksamen Menge einer Verbindung der Formel (I) und/oder ihrer physiologisch unbedenklichen Salze, Tautomere und/oder Stereoisomere, einschließlich deren Mischungen in allen Verhältnissen, und (b) einer wirksamen Menge eines Antikrebsmittels. Das Kit enthält geeignete Behälter, wie z.B. Schachteln oder Kartons, individuelle Flaschen, Beutel oder Ampullen. Das Kit kann z.B. separate Ampullen enthalten, in denen jeweils eine wirksame Menge an einer Verbindung der Formel (I) und/oder ihrer pharmazeutisch verwendbaren Salze, Tautomere und/oder Stereoisomere, einschließlich deren Mischungen in allen Verhältnissen, und einer wirksamen Menge eines Antikrebsmittelsgelöst oder in lyophilisierter Form vorliegt. Das Kit der Erfindung kann auch einen Artikel beinhalten, der schriftliche Anweisungen enthält oder den Anwender auf schriftliche Anweisungen hinweist, die den Umgang mit den Verbindungen der Erfindung erläutern.

Erfindungsgemäß werden die Verbindungen der Formel (I) bzw. deren Teilformeln und/oder ihrer physiologisch unbedenklichen Salze, Tautomere und/oder Stereoisomere, einschließlich deren Mischungen in allen Verhältnissen, zur Prophylaxe, Therapie und/oder Verlaufskontrolle von Erkrankungen verwendet, die durch die Aktivität von Serin-Threonin-Proteinkinasen hervorgerufen, vermittelt und/oder verbreitet werden. Gegenstand der vorliegenden Erfindung ist deshalb auch die Verwendung von Verbindungen der Formel (I) bzw. deren Teilformeln und/oder ihrer physiologisch unbedenklichen Salze, Tautomere und/oder Stereoisomere, einschließlich deren Mischungen in allen Verhältnissen, zur Herstellung eines Arzneimittels zur Prophylaxe, Therapie und/oder Verlaufskontrolle von Erkrankungen, die durch die Aktivität von Serin-Threonin-Proteinkinasen hervorgerufen, vermittelt und/oder verbreitet werden. Erfindungsgemäß sind Verbindungen der Formel (I) bzw. deren Teilformeln und/oder ihre physiologisch unbedenklichen Salze, Tautomere und/oder Stereoisomere, einschließlich deren Mischungen in allen Verhältnissen, zur Verwendung in der Prophylaxe, Therapie und/oder Verlaufskontrolle von Erkrankungen geeignet, die durch Aktivität von Serin-Threonin-Proteinkinasen hervorgerufen, vermittelt und/oder verbreitet werden. Zur Identifizierung eines entsprechenden Signalübertragungswegs und zum Nachweis von Wechselwirkungen zwischen verschiedenen Signalübertragungswegen sind geeignete Modelle oder Modellsysteme entwickelt worden, z.B. Zellkulturmodelle (Khwaja et al. (1997) EMBO 16: 2783) und Modelle transgener Tiere (White et al. (2001) Oncogene 20: 7064). Zur Bestimmung bestimmter Stufen in der Signalübertragungskaskade können wechselwirkende Verbindungen genutzt werden, um das Signal zu modulieren (Stephens et al. (2000) Biochemical J 351: 95). Darüber hinaus können die erfindungsgemäßen Verbindungen auch als Reagenzien zur Testung kinaseabhängiger Signalübertragungswege in Tieren und/oder Zellkulturmodellen oder in den in dieser Anmeldung genannten klinischen Erkrankungen verwendet werden. Wie hierin besprochen, sind diese Signalwege für verschiedene Erkrankungen relevant. Dementsprechend sind die erfindungsgemäßen Verbindungen nützlich bei der Prophylaxe, Therapie und/oder Verlaufskontrolle von Erkrankungen, die von Signalwegen mit Beteiligung von Serin-Threonin-Proteinkinasen abhängig sind.

Erfindungsgemäß sind die Verbindungen der Formel (I) bzw. deren Teilformeln und/oder ihre physiologisch unbedenklichen Salze, Tautomere und/oder Stereoisomere, einschließlich deren Mischungen in allen Verhältnissen, zur Verwendung in der Prophylaxe, Therapie und/oder Verlaufskontrolle von Krebs, Tumoren und/oder Metastasen geeignet.

Der Tumor ist insbesondere ausgewählt aus der Gruppe von malignen Erkrankungen von Blase, Magen, Nieren, Kopf, Hals, Ösophagus, Gebärmutterhals, Schilddrüse, Darm, Leber, Gehirn, Prostata, Urogenitaltrakts, lymphatisches System, Kehlkopf, Lunge, Haut, Blut, Knochen und Immunsystem, und/oder der Krebs ist ausgewählt aus der Gruppe von Monozytenleukämie, nicht-kleinzelliges Lungenkarzinom, kleinzelliges Lungenkarzinom, Bauchspeicheldrüsenkrebs, Glioblastom, kolorektales Karzinom, Brustkarzinom, akute myeloische Leukämie, chronische myeloische Leukämie, akute lymphatische Leukämie, chronische lymphatische Leukämie, Hodgkin-Lymphom und non-Hodgkin-Lymphom.

Eine weitere Ausgestaltung der vorliegenden Erfindung betrifft die erfindungsgemäßen Verbindungen in Kombination mit Radiotherapie und/oder mit mindestens einem weiteren Wirkstoff, vorzugsweise in Kombination mit Radiotherapie und/oder einem Antikrebsmittel. Technische Bestrahlungsmethoden, die klinische Verwendung finden, umfassen vorzugsweise Photonenbestrahlung (klassische, elektromagnetische Röntgen-/GammaStrahlung), Protonenbestrahlung, Schwerionenbestrahlung (ionisierter Kohlenstoff) sowie Neutronenbestrahlung, ohne hierauf beschränkt zu sein. Darüber hinaus findet die Brachytherapie mit Hilfe einer geeigneten Strahlenquelle (z.B. alpha emitters) in Form der Oberflächenapplikation sowie der intrakavitären und interstitiellen Anwendung klinisch Verwendung. Dem Fachmann sind diese Radiotherapien und weitere geeignete Bestrahlungstherapien im Sinne der Erfindung bekannt, wie z.B. aus Herrmann et al. (2006) Klinische Strahlenbiologie, Elsevier München, 4. Auflage, 67-68; Bhide & Nutting (2010) BMC Medicine 8: 25; Choi & Hung (2010) Current Urology Reports 11(3): 172. Als häufigste Anwendung ist die Photonenbestrahlung technisch durch die Verfahren IMRT (Intensitätsmodulierte Radiotherapie) sowie durch bildgebende Verfahren (dreidimensional konforme Radiotherapie) bei der Bestrahlungsplanung und -durchführung zur möglichst exakten Fokussierung verfeinert worden. Die erfindungsgemäßen Verbindungen erzielen bei existierenden Krebs-Therapien und Bestrahlungen synergistische Effekte und/oder stellen die Wirksamkeit existierender Krebs-Therapien und Bestrahlungen wieder her.

Noch eine weitere Ausgestaltung der Erfindung bezieht sich auf die Verwendung von mindestens einer Verbindung der Formel (I) und/oder ihrer physiologisch unbedenklichen Salze, Tautomere und/oder Stereoisomere, einschließlich deren Mischungen in allen Verhältnissen, zur Sensibilisierung von Krebszellen gegenüber einem Antikrebsmittel und/oder ionisierender Strahlung unter der Maßgabe, dass die Sensibilisierung nicht *in vivo* am menschlichen oder tierischen Körper erfolgt. Die Sensibilisierung erfolgt vorzugsweise *ex vivo* oder *in vitro,* indem die Verbindungen Zellen, Zellkulturen, Geweben oder Organen verabreicht werden, die Serin-Threonin-Proteinkinasen umfassen. Die *ex vivo* -Verwendung wird insbesondere bei tierischen Zellen angewandt, die aus einem tierischen Organismus stammen, der von einer Krankheit betroffen ist, die ausgewählt ist aus der Gruppe von Krebs, Tumoren bzw. Metastasen. Die *ex vivo* -behandelten Zellen können entweder für Folgeuntersuchungen weiter in Kultur gehalten oder in ein Tier überführt werden, wobei es sich um das Wirtstier oder ein anderes Tier handeln kann. Die erfindungsgemäße *ex vivo-*Sensibilisierung ist insbesondere von Vorteil, um die spezifische Wirkung der Verbindungen zu testen, so dass unter Auswertung dieser *ex vivo*-Daten die *in vivo*-Dosis entsprechend voreingestellt werden kann. Im Ergebnis dessen wird der therapeutische Effekt signifikant erhöht. Alternativ ist die Erfindung auch für die Anwendung *in vivo* ausgestaltet und betrifft mindestens eine Verbindung der Formel (I) und/oder ihre physiologisch unbedenklichen Salze, Tautomere und/oder Stereoisomere, einschließlich deren Mischungen in allen Verhältnissen, zur Verwendung zur Sensibilisierung von Krebszellen gegenüber einem Antikrebsmittel und/oder ionisierender Strahlung.

Die Erfindung lehrt ferner ein Verfahren zur Prophylaxe, Therapie und/oder Verlaufskontrolle von Krebs, Tumoren bzw. Metastasen, wobei eine wirksame Menge mindestens einer erfindungsgemäßen Verbindung und/oder ihrer physiologisch unbedenklichen Salze, Tautomere und/oder Stereoisomere, einschließlich deren Mischungen in allen Verhältnissen, einem zu behandelnden Probanden verabreicht wird. Bevorzugte Probanden im Sinne der Erfindung sind Mensch oder Tier, besonders bevorzugt der Mensch. Dem Fachmann ist hierbei bekannt, dass er die erfindungsgemäßen Verbindungen, die selbstverständlich auch als das erfindungsgemäße pharmazeutische Mittel verwendet werden können, in verschiedenen Dosierungen einem Organismus, insbesondere einem humanen Patienten, applizieren kann. Die wirksame Menge und die Art der Applikation können vom Fachmann durch Routineversuche bestimmt werden. Die vorherige Lehre der Erfindung und deren Ausführungsformen sind gültig und ohne Einschränkungen auf das Behandlungsverfahren anwendbar, sofern es sinnvoll erscheint.

Sämtliche genannte sowie weitere Bestandteile bzw. Komponenten sind dem Fachmann geläufig und können in Routineversuchen eine spezielle Ausgestaltung für die erfindungsgemäße Lehre erfahren. Sämtliche in der Beschreibung zitierten Dokumente sollen hiermit in ihrer Gesamtheit in die Offenbarung der vorliegenden Erfindung als Referenz einbezogen werden.

Im Rahmen der hier vorgestellten Erfindung wurden erstmals neuartige Arylchinazolin-Verbindungen der Formel (I) bereitgestellt. Die erfindungsgemäßen Verbindungen steuern affin und/oder selektiv Serin-Threonin-Proteinkinasen, insbesondere DNA-PK, an. Die Verbindungen aus Formel (I) und deren Derivate zeichnen sich durch eine hohe Spezifität und Stabilität, niedrige Herstellungskosten und leichte Handhabung aus. Auf diesen Eigenschaften basieren eine reproduzierbare Wirkungsweise und die verlässliche und sichere Wechselwirkung mit den entsprechenden Zielstrukturen. Die Erfindung beinhaltet auch die Verwendung der vorliegenden Arylchinazolin -Derivate zur Hemmung, Regulation und/oder Modulation der Signalkaskade von Serin-Threonin-Proteinkinasen, insbesondere DNA-PK, und bietet damit neuartige Werkzeuge für Forschung und/oder Diagnostik.

Arzneimittel und pharmazeutische Zusammensetzungen, die die genannten Verbindungen enthalten, und der Einsatz dieser Verbindungen zur Behandlung Kinase-vermittelter Störungen sind außerdem ein vielversprechenden Ansatz für ein breites Spektrum von Therapien, wodurch sich bei Mensch und Tier eine direkte und unmittelbare Linderung von Symptomen erreichen lässt. Dies ist besonders für die wirksame Bekämpfung schwerer Krankheiten wie Krebs von Vorteil, entweder als Monotherapie oder in Kombination mit anderen anti-neoplastischen Therapien. Die Schlüsselbeteiligung von DNA-PK an DNA-Reparaturprozessen und der Nachweis, dass der DNA-PK-Inhibitoren Säugerzellen strahlungsempfindlicher werden lässt, ermöglicht eine therapeutische Anwendung der DNA-PK spezifischen Inhibitoren im Rahmen der Behandlung von z.B. soliden Krebstumoren durch Bestrahlungs- und/oder einer auf DNA-DSBs abzielenden Chemotherapie.
Die Verbindungen der Formel (I), ihre Salze, Isomere, Tautomere, Enantiomere, Diastereomere, Racemate, Derivate, Prodrugs und/oder Metaboliten sind nicht nur bei den genannten klinischen Krankheitsbildern wirkungsvoll, sondern ebenso in der Diagnose und Therapie sämtlicher Erkrankungen in Zusammenhang mit der DNA-PK-Signalkaskade, vor allem im Hinblick auf die Hemmung von Zellproliferation und -migration. Darüber hinaus sind die erfindungsgemäßen Inhibitoren in der Behandlung von retroviralen Erkrankungen durch ein Zurückdrängen der retroviralen Integration nutzbar (R. Daniel (1999) Science 284: 644). Schließlich können die erfindungsgemäßen Hemmstoffe als Immunmodulatoren sowie Modulatoren der telomeren Wartung eingesetzt werden. Die niedermolekularen Inhibitoren werden einzeln und/oder in Kombination mit anderen Behandlungsmaßnahmen verwendet, wie z.B. chirurgischen Eingriffen, Immun-, Strahlen- und/oder Chemotherapie. Letztere beziehen sich auf eine zielgerichtete Therapie mit einem beliebigen NME (d.h. NCE und/oder NBE) als Mono- und/oder On-Target-/Off-Target-Kombinationstherapie.

Aufgrund ihrer überraschend starken und/oder selektiven Hemmung von solchen Enzymen, die über die Reparatur von dsDNA zelluläre Prozesse regeln, können die Verbindungen der Erfindung bei vorteilhaft niedriger Dosierung verabreicht werden, während sie im Vergleich mit den weniger potenten bzw. weniger selektiven Inhibitoren des Stands der Technik eine ähnliche oder sogar überlegene biologische Wirksamkeit erzielen. Die reduzierte Dosis geht auch mit verringerten oder keinen medizinischen Nebenwirkungen einher. Darüber hinaus wird die hoch selektive Hemmung durch die erfindungsgemäßen Verbindungen auch durch eine Verringerung unerwünschter Nebenwirkungen begleitet, die unabhängig von der Dosierung ist.

Insbesondere weisen die erfindungsgemäßen Verbindungen keine physiologisch relevanten Hemmungen bzw. Blockaden des Kv 11.1 hERG Kaliumionenkanals auf.

Es versteht sich, dass diese Erfindung nicht auf die spezifischen Verbindungen, pharmazeutischen Zusammensetzungen, Verwendungen und Verfahren beschränkt ist, wie sie hierin beschrieben sind, da solche Dinge variieren können. Es versteht sich des Weiteren, dass die vorliegend verwendete Terminologie ausschließlich dem Zweck der Beschreibung besonderer Ausführungsformen dient und nicht den Schutzumfang der Erfindung einschränken soll. Wie vorliegend in der Spezifikation einschließlich der anhängigen Ansprüche verwendet, schließen Wortformen im Singular, wie z. B. "ein", "eine", "einer", "der" oder "das" die Entsprechung im Plural ein, sofern der Kontext nicht eindeutig etwas anderes vorgibt. Beispielsweise enthält der Bezug auf "eine Verbindung" ein einzelne Verbindung oder mehrere Verbindungen, die wiederum identisch oder verschieden sein können, oder der Bezug auf "ein Verfahren" schließt äquivalente Schritte und Verfahren ein, die dem Fachmann bekannt sind.

Im Folgenden wird die Erfindung anhand nicht limitierender Beispiele für konkrete Ausführungsformen näher erläutert. Die Beispiele sind insbesondere dahingehend zu interpretieren, dass sie nicht auf die konkret veranschaulichten Merkmalskombinationen beschränkt sind, sondern die beispielhaften Merkmale wiederum frei kombiniert werden können, solange die Aufgabe der Erfindung gelöst wird.

### BEISPIELE

Eine Übersicht über die Ausführungsbeispiele liefern die Tabellen 1-7.
Für die darin wiedergegeben biologischen Daten gelten die folgenden Bereiche:
DNA-PK (enzymatisch):

| | |
|---|---|
| A: | IC₅₀ < 3 nM |
| B: | 3 nM ≤ IC₅₀ < 7 nM |
| C: | 7 nM ≤ IC₅₀ < 30 nM |
| D: | 30 nM ≤ IC₅₀ |

pDNA-PK (zellulär):

| | |
|---|---|
| A: | IC₅₀ < 0,5 µM |
| B: | 0,5 µM ≤ IC₅₀ < 5 µM |
| C: | 5 µM ≤ IC₅₀ < 10 µM |
| D: | 10 µM ≤ IC₅₀ < 30 µM |

Kv11.1 hERG:

| | |
|---|---|
| A: | Kᵢ > 25 µM |
| B: | 25 µM ≥ Kᵢ > 15 µM |
| C: | 15 µM ≥ Kᵢ > 10 µM |
| D: | 10 µM ≥ Kᵢ |

### Analytik

NMR (¹H) wurde mit den folgenden Parametern durchgeführt.
Geräte: Bruker Avance DRX 500, Bruker Avance 400, Bruker DPX 300
Referenz: TMS
TD (Time Domaine = Anzahl der Datenpunkte oder digitale Auflösung): 65536
Lösungsmittel: DMSO-d6
NS (Number of Scans = Häufigkeit der Abtastung): 32
SF (Spectrometer Frequence = Sendefrequenz): 400 oder 500 MHz
TE (Temperatur): 303 K, 363 K oder 393 K
Kopplungskonstanten (J) werden in Hertz (Hz) angegeben
HPLC: High Performance Chromatography mit UV-Detektor
LC-MS: High Performance Chromatography mit UV- und MS-Detektor
SFC: Supercritical Fluid Chromatography mit UV-Detektor

### Identifizierung von Synthesezwischen- und Syntheseendprodukten mittels LC-MS:

LC-MS Methode A:
Säule: Chromolith SpeedROD RP-18e 50-4.6 mm, Fluss: 2,4 ml/min., Wellenlänge: 220 nm, Eluent A: Wasser + 0,05 vol.% Ameisensäure, Eluent B: Acetonitril + 0,4 vol.%
Ameisensäure, Gradient: 4 vol.%-100 vol.% Eluent B in 2,8 min, dann 100% Eluent B für die Dauer von 0,5 min.

LC-MS Methode B:
Säule: Chromolith SpeedROD RP-18e 50-4.6 mm, Fluss: 2,4 ml/min., Wellenlänge: 220 nm,
Eluent A: Wasser + 0,1 vol.% Trifluoressigsäure, Eluent B: Acetonitril + 0,1vol.%
Trifluoressigsäure, Gradient: 4 vol.%-100 vol.% Eluent B in 2,8 min, dann 100 vol.-% Eluent B für die Dauer von 0,5 min.

### Auftrennung von Stereoisomerenmischunqen mittels HPLC und SFC:

HPLC: Zunächst wird für jede Stereoisomerenmischung ein Säulenscreening durchgeführt, mit den folgenden Säulen: Chiralpak AD-H, Chiralpak AS-H, Chiralpak IA, Chiralpak IB, Chiralpak IC, Chiralcel OD-H, Chiralcel OJ-H, Lux Cellulose-2, Lux-Amylose-2, alle Säulen: 250-4.6 mm. Die am besten geeignete Säule wird für die weiteren Messungen (z.B. Bestimmung des Enantiomerenverhältnis) verwendet. Fluss: 0,8 ml/min, Wellenlänge: variabel, wird entsprechend dem Extinktionsmaximum und den verwendeten Eluenten angepaßt. Eluent: folgende Lösungsmittel oder Lösungsmittelmischungen werden für die Eluenten verwendet: n-Heptan, n-Hexan, Ethanol, Methanol, 2-Propanol, Acetonitril, Essigsäureethylester, Dichlormethan; als Eluentenzusatz können verwendet werden: 0,01-0,5 vol.% Ameisensäure, 0,01-0,5 vol.% Diethylamin; je nach Bedarf werden Gradienten oder isokratische Messbedingungen verwendet.

SFC: Zunächst wird für jede Stereoisomerenmischung ein Säulenscreening durchgeführt, mit den folgenden Säulen: Chiralpak AD-H, Chiralpak AS-H, Chiralpak IA, Chiralpak IB, Chiralpak IC, Chiralcel OD-H, Chiralcel OJ-H, Lux Cellulose-2, Lux-Amylose-2, alle Säulen: 250-4.6 mm. Die am besten geeignete Säule wird für die weiteren Messungen (z.B. Bestimmung des Enantiomerenverhältnis) verwendet. Fluss: 5 ml/min, Wellenlänge: variabel, wird entsprechend dem Extinktionsmaximum und den verwendeten Eluenten angepaßt. Eluent: flüssiges Kohlendioxid (>70 bar), Co-Eluent: folgende Lösungsmittel oder Lösungsmittelmischungen werden für die Co-Eluenten verwendet: Ethanol, Methanol, Isopropanol, Acetonitril, Essigsäureethylester, Dichlormethan. Als Eluentenzusatz können verwendet werden: 0,01-0,5 vol.% Ameisensäure, 0.01-0.5 vol.% Diethylamin. Je nach Bedarf werden Gradienten oder isokratische Messbedingungen verwendet.

### Biologische Testung

### A) DNA-PK ASSAY (BIOCHEMISCH)

Der Kinase-Assay erfolgte in mit Streptavidin beschichteten 348-Well-Mikrotiter-FlashPlates. Dazu wurden 1,5 µg DNA-PK-Proteinkomplex und 100 ng biotinyliertes Substrat, wie z.B. PESQEAFADLWKK-Biotin-NH2 ("Biotin-DNA-PK-Peptid"), in einem Gesamtvolumen von 36,5 µl (34,25 mM HEPES/KOH; 7,85 mM Tris-HCl; 68,5 mM KCl;
5 µM ATP; 6,85 mM MgCl₂; 0,5 mM EDTA; 0,14 mM EGTA; 0,69 mM DTT; pH 7,4) mit 500 ng DNA aus Kälberthymus, 0,1 µCi 33P-ATP und 1,8 % DMSO pro Well mit bzw. ohne die Testverbindung 90 min bei Raumtemperatur inkubiert. Die Reaktion wurde mit 50 µl/Well 200 mM EDTA gestoppt. Nach Inkubieren für weitere 30 min bei Raumtemperatur wurde die Flüssigkeit entfernt. Jedes Well wurde dreimal mit 100 µl 0,9 %-iger Kochsalzlösung gewaschen. Eine unspezifische Reaktion (Leerwert) wurde mit 10 µM eines eigenen Kinase-Inhibitors ermittelt. Die Radioaktivitätsmessung erfolgte mit einem TopCount. IC₅₀-Werte wurden in RS1 berechnet.
Literatur: Kashishian et al. (2003) Molecular Cancer Therapeutics 1257.

### B) DNA-PK PHOSPHORYLIERUNG AN SERIN 2056 (ZELLULÄR)

HCT116 Zellen wurden in MEM alpha Medium mit 10% fötalem Kälberserum und 2 mM Glutamin bei 37°C und 10% CO2 kultiviert. Die Zellen wurden mithilfe von Trypsin/EDTA vom Boden der Kulturgefäße abgelöst, in Zentrifugenröhrchen abzentrifugiert, in frischem Medium aufgenommen und die Zelldichte wurde ermittelt. 100.000 Zellen wurden in 1 ml Kulturmedium pro Kavität einer 24-Well Zellkulturplatte ausgesät und über Nacht kultiviert. Am nächsten Tag wurde 10 µM Bleomycin (DNA-Interkalator und Induktor von DNA Doppelstrangbrüchen) und Testsubstanzen in frischem Kulturmedium zu den Zellen hinzugefügt und diese für weitere sechs Stunden kultiviert. Im Anschluss erfolgte eine Zelllyse und die Zelllysate wurden auf eine geblockte und mit DNA-PK spezifischen Antikörpern beschichtete 96-Well-ELISA-Platte gegeben (Sigma-Aldrich WH0005591M2: Gesamt-DNA-PK; Abcam ab18192 oder Epitomics EM09912: phospho-Serin 2056 DNA-PK) und bei 4°C über Nacht inkubiert. Anschließend wurden die 96-Well-ELISA-Platten mit einem Detektions-Antikörper (Abcam ab79444: Gesamt-DNA-PK) und einem Streptavidin-HRP-Konjugate behandelt. Die Entwicklung der enzymatischen Reaktion erfolgte mithilfe eines Chemilumineszenzreagenz, die Chemilumineszenz wurde mithilfe des Mithras LB940 gemessen. Die Signale mit dem phospho-DNA-PK spezifischen Antikörper wurden auf das Signal mit dem Antikörper gegen das gesamte Protein DNA-PKc normalisiert. Die Ermittlung von IC₅₀ Werten oder von Prozentwerten erfolgte durch Referenzierung auf das Signalniveau der Bleomycin-behandelten Vehikelkontrollgruppe (100 % der Kontrolle). Die DMSO-Kontrolle wurde als Blank verwendet.

### C) Kv11.1 (hERG) IONENKANALAKTIVITÄT (Patch Clamp Assay)

Methode zur Detektion und Charakterisierung von Prüfsubstanzen, die mit dem Kv11.1 (hERG) Kanal interferieren: Bei dem Kv11.1 (hERG, human Ether a-go-go related gene) handelt es sich um einen Kaliumkanal, der in den ventrikulären Kardiomyozyten eine zentrale Rolle für die Repolarisation der Zellen spielt.

Die Patch-Clamp Messung erfolgte bei Raumtemperatur in Ganzzellableitung (whole-cell Konfiguration) an humanen embryonalen Nierenzellen (HEK293), die stabil mit dem hERG Gen transfiziert wurden.

Die Ganzzellableitungen erfolgten mit einem automatisierten Patch Clamp Gerät (PatchlinerTM, Nanion Technologies, Munich). Dabei handelt es sich um ein Glaschipbasiertes System, mit dem automatisierte Whole-cell Messungen an bis zu 8 Zellen gleichzeitig möglich sind. Der Glaschip besitzt ein Loch definierter Größe, auf dem die Zelle durch Anlegung eines Unterdrucks in den Gigaseal und in die Whole-Cell-Konfiguration gebracht wird. Puffer, Zellsuspension und Prüfsubstanzen wurden mit einer teflonbeschichteten Pipette in Mikrokanäle des Chips zugegeben.

Die Zellen wurden auf ein Haltepotential von -80mV geklemmt. Zur Messung einer substanzvermittelten Inhibition des Kv11.1 Kanals wurde folgendes Spannungsprotokoll in 10-sekündigen Intervallen angelegt: 51 ms / -80 mV, 500 ms / +40 mV, 500 ms / -40 mV, 200 ms / -80 mV. Der Leckstrom wird mittels der P4 Methode subtrahiert. Die Zellen wurden in Extrazellularpuffer (EC) resuspendiert und in den Chip appliziert. Nachdem die Zelle gefangen worden war, wurde der Seal durch Zugabe eines Sealenhancer-Puffers verbessert. Sobald die Whole-cell Konfiguration erreicht war, wurde der Sealenhancer-Puffer ausgewaschen und durch Extrazellularpuffer ersetzt. Die Messung startete in EC für 1.5 min. Danach wurde DMSO (Vehikelkontrolle, 0.1% DMSO) appliziert und der Kontrollstrom für 3 min aufgezeichnet. Im Anschluss wurde die Prüfsubstanz zweimal in der gleichen Konzentration zugegeben und der Kaliumstrom für jeweils 3.5 min gemessen.

Sofern das Messergebnis einer Prüfsubstanz bei einer initialen Konzentration von 10µM kleiner als (-)50%Effekt (Schwellenwert) war (z.B. (-)60%Effekt), erfofgte zur Ermittlung einer Dosis-Wirkungsbeziehung die Zugabe der Prüfsubstanz kumulativ in aufsteigender Konzentration, wobei jede Konzentration für 5min gemessen wurde.

Als Referenzsubstanz diente der Kv11.1 (hERG) Ionenkanalblocker Quinidine. Die Effekte von Prüfsubstanzen und Quinidine wurden auf die zugehörige Vehikelkontrolle normalisiert. Der Effekt auf die Kv11.1 (hERG) Kanalaktivität wurde anhand des Kaliumstroms bei -40mV beurteilt. Für die Berechnung wurde der Strom des jeweiligen letzten trace ausgewertet. Eine Prüfsubstanz-bedingte Inhibition des Kv11.1 (hERG) Kanals wurde auf die Vehikel-Kontrolle (0,1% DMSO) normiert.

Während der Messung wurde ein Aliquot der Prüfsubstanz für eine Konzentrationsbestimmung entnommen. Die Probe wurde unmittelbar per HPLC vermessen und die finale Konzentration anhand einer Kalibrierungskurve ermittelt.

Sofern das Messergebnis einer Prüfsubstanz bei einer initialen Konzentration von 10µM größer oder gleich (-)50%Effekt (Schwellenwert) ist (z.B. (-)30%Effekt, d.h. 30% Inhibition bei 10µM), wird der Kᵢ nach folgender Formel berechnet: Kᵢ = 1,0E-5 x (100+%Effekt)/(- %Effekt), [M].

Das Messergebnis von (-)30%Effekt bei einer Prüfsubstanzkonzentration von 10µM ergibt einen Kᵢ von 23 µM.

### D) Kv11.1 lonenkanal-Bindungs Assay

Kv11.1 (hERG= human Ether a go-go related enzyme) ist ein Herz K⁺ Kanal, welcher möglichst nicht mit den Prüfsubstanzen interagieren sollte. Diese Interaktion wird mit Hilfe des Predictor™ hERG *Fluorescence Polarizations (FP) Assays* von *Life Technologies* quantitativ bestimmt. Bei diesem Assayprinzip werden Kardiomyozyten Zellmembranen mit einem bestimmen Anteil an Kv11.1 Kanälen isoliert. Ein Farbstoff markierter Kv11.1 Bindungspartner ergibt durch Interaktion mit dem Kv11.1 ein hohes Fluoreszenzpolarisationssignal. Im Falle einer Verdrängung des Farbstoffs kommt es zu einer Reduktion des Fluoreszenzpolarisationssignals.

Der Assay wird wie folgt automatisiert durchgeführt: Es werden 15 nL der Prüfsubstanzen (höchste Konzentration: 10mM, 10 Konzentrationen: Verdünnungsfaktoren 1:3.16 , DMSO) in eine leere Mikrotiterplatten mit 384 Kavitäten durch einen akustischen Pipettierer transferiert. Im Anschluss erfolgt die Zugabe von 3µL der isolierten Membranen. Membranen und Prüfsubstanzen werden für 15 min (+/-5min) bei 22°C inkubiert. Im nächsten Schritt erfolgt die Zugabe des Farbstoff markierten Bindungspartners, gefolgt von einer Inkubation bei 22°C. Nach zwei stündiger Inkubation erfolgt die Messung der Fluoreszenzpolarisation am Envision *multimode Reader.* Gemessene Rohdaten werden mit Hilfe von *Genedata Assay Analyzer* normalisiert. In *Genedata Condoseo* erfolgt die Berechnung der IC₅₀ und %Effekt Werte.

### Chemische Synthese

Vor- und nachstehend sind alle Temperaturen in °C angegeben.

Die stereochemischen Konfigurationszuordnungen der enantiomeren Beispiele 27, 72, 82, 83, 135, 136, 185, 234, 251, 455 und 456 wurden durch Röntgenstrukturanalysen bestätigt.

Für die Beispiele 234 und 251 erfolgte der Nachweis durch Kristallisation und Röntgenstrukturanalyse einer Vorstufe.

Die übrigen in den Tabellen als chiral gekennzeichneten Verbindungen (Stern am assymmetrischen C-Atom) wurden durch Chromatographie an chiraler fester Phase erhalten. Das unter den jeweiligen Bedingungen als erstes eluierte Enantiomer erhielt die Bezeichung "Ena1", das als zweites eluierte Enantiomer die Bezeichnung "Ena2".

### BEISPIELE 1 und 2:

### (3,5-Difluor-pyridin-4-yl)-[4-fluor-3-(7-morpholin-4-yl-chinazolin-4-yl)-phenyl]-methanol (1) (4-Chlor-5-fluor-pyridin-3-yl)-[4-fluor-3-(7-morpholin-4-yl-chinazolin-4-yl)-phenyl]-methanol (2)

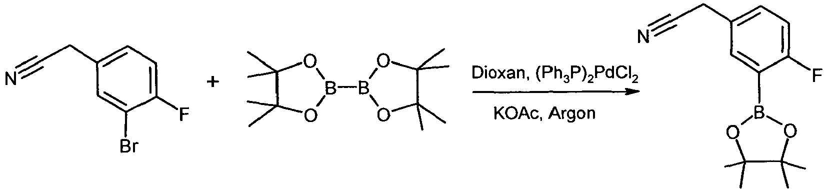

(3-Brom-4-fluor-phenyl)-acetonitril (4,00 g, 18,32 mmol), Bis(pinacolato)dibor (5,22 g, 20,15 mmol), Kaliumacetat (55,86 mmol) und Bis(triphenylphosphin)-palladium(II)-chlorid (15,2% Pd) (393,53 mg, 0,55 mmol) wurden in Sauerstoff-freiem 1,4-Dioxan (40 ml, max. 0,005% Wasser) unter Argon gelöst. Anschließend wurde das Reaktionsgemisch 90 min bei einer Temperatur von 130°C erhitzt. Nach vollständigem Reaktionsumsatz wurde über Kieselgur filtriert. Das Filtrat wurde mit Dichlormethan (200 ml) und Wasser (50 ml) verdünnt und extrahiert. Die organische Phase wurde über Natriumsulfat getrocknet, anschließend filtriert und im Vakuum zur Trockne eingeengt, wobei [4-Fluor-3-(4,4,5,5-tetramethyl-[1,3,2]dioxaborolan-2-yl)-phenyl]-acetonitril als Öl (7,59 g, Reinheit 81%, MS: 262.2 [M+H⁺]) anfällt, dass ohne weitere Aufarbeitung weiter umgesetzt wurde.

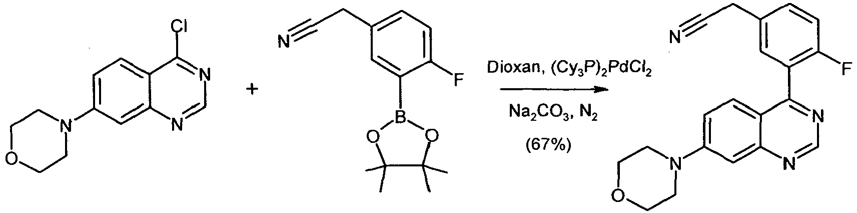

4-Fluor-3-(4,4,5,5-tetramethyl-[1,3,2]dioxaborolan-2-yl)-phenyl]-acetonitril (7,60 g, 23,53 mmol), 1,4-Dioxan (85,6 ml, max. 0,005% Wasser), 4-Chlor-7-morpholin-4-yl-chinazolin (5,00 g, 20,02 mmol), Bis(tricyclohexylphosphin)-palladium-(II)-dichlorid (597,24 mg, 0,80 mmol) und Natriumcarbonat-Lösung (2,0 M, 30 ml, 60,07 mmol) wurden in einem Dreihalskolben vorgelegt. Unter einer Stickstoffatmosphäre wurde die erhaltene Suspension bei einer Temperatur von 140°C für die Dauer von 2.5 h unter Rühren erhitzt. Nach beendeter Reaktionsumsetzung wurde auf Raumtemperatur abgekühlt und über Kieselgur filtriert. Das Filtrat wurde mit Essigsäureethylester (250 ml) und Wasser (100 ml) verdünnt und extrahiert. Die wässrige Phase wurde zweimal mit Essigsäureethylester (je 75 ml) nachgewaschen. Die vereinten organischen Phasen wurden über Natriumsulfat getrocknet, anschließend filitriert und im Vakuum zur Trockne eingeengt. Zur weiteren Aufarbeitung wurde in Methyl-tert.-butylether suspendiert, abfiltriert und zweimal mit weiterem Methyl-tert.-butylether (je 30 ml) nachgewaschen. Der Filterkuchen wurde über Nacht im Vakuum getrocknet, wobei [4-Fluor-3-(7-morpholin-4-yl-chinazolin-4-yl)-phenyl]-acetonitril (4,91 g, 13,49 mmol, MS: 349.1 [M+H⁺], 67% Ausbeute) als gelber Feststoff anfiel.

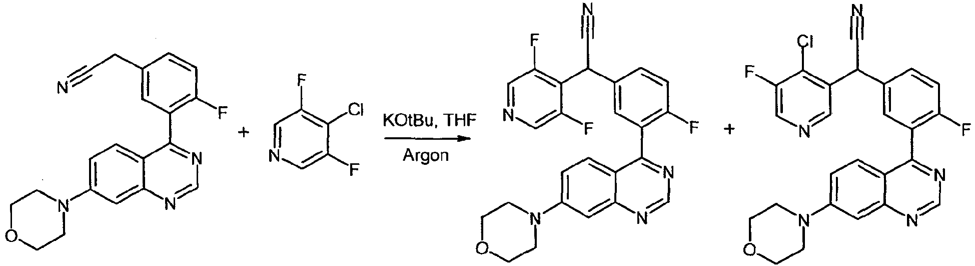

[4-Fluor-3-(7-morpholin-4-yl-chinazolin-4-yl)-phenyl]-acetonitril (400 mg, 1,12 mmol), 4-Chlor-3,5-difluor-pyridin (189,9 mg, 1,23 mmol) wurden in Sauerstoff-freien, entgastem Tetrahydrofuran (8 ml, max. 0,0075 % Wasser) unter einer trockenen Argonatmosphäre gelöst. Anschließend wurde Kalium-tert.-butylat (263,9 mg, 2,35 mmol) zur Reaktionsmischung zugefügt, wobei eine dunkelrote Lösung entstand, die bei Raumtemperatur für weitere 30 min gerührt wurde. Nach beendeter Reaktion wurde mit gesättigter Ammoniumchlorid-Lösung (20 ml) und Wasser (50 ml) verdünnt. Anschließend wurde die wässrige Phase zweimal mit Dichlormethan (je 60 ml) extrahiert. Die organische Phase wurde über Natriumsulfat getrocknet, abfiltiert und im Vakuum zur Trockne einrotiert. Der Rückstand wurde durch Flash-Säulenchromatographie (Gradient: Dichlormethan / 0-4 vol.% Ethanol) aufgereinigt, wobei ein Gemisch (420 mg, ca. 5:3) der Öle (3,5-Difluor-pyridin-4-yl)-[4-fluor-3-(7-morpholin-4-yl-chinazolin-4-yl)-phenyl]-acetonitril (263 mg, 0,57 mmol, MS: 462,1 [M+H⁺], 50% Ausbeute) und (4-Chlor-5-fluor-pyridin-3-yl)-[4-fluor-3-(7-morpholin-4-yl-chinazolin-4-yl)-phenyl]-acetonitril (157 mg, 0,33 mmol, MS: 478,1/480,1 [M+H⁺], 29% Ausbeute) erhalten wurde.

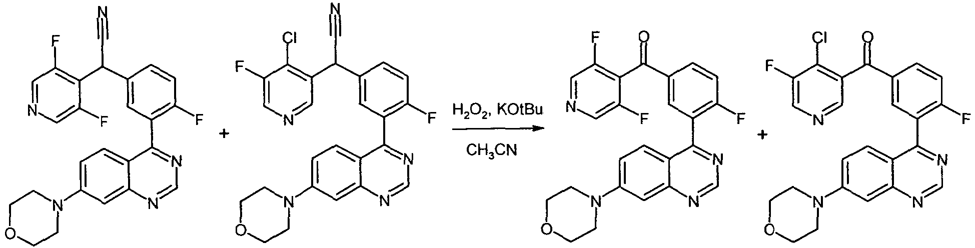

Das Gemisch (420 mg, ca. 5:3) aus (3,5-Difluor-pyridin-4-yl)-[4-fluor-3-(7-morpholin-4-yl-chinazolin-4-yl)-phenyl)-acetonitril und (4-Chlor-5-fluor-pyridin-3-yl)-[4-fluor-3-(7-morpholin-4-yl-chinazolin-4-yl)-phenyl]-acetonitril wurde in Acetonitril (12,7 ml) gelöst. Unter Rühren wurde Kalium-tert.-butylat (80,90 mg, 0,72 mmol) zur Reaktionslösung zugefügt, wobei eine dunkelrote Lösung entstand. Nach 15 min rühren wurde auf 0°C gekühlt und anschließend 30%iger Wasserstoffperoxid-Lösung (276 µl, 2,70 mmol) zugetropft. Das Kältebad wurde nach 5 min rühren bei 0° entfernt. Die Reaktionslösung wurde eine weitere 1 h bei Raumtemperatur gerührt. Nach vollständigem Reaktionsumsatz wurde mit 10%iger Natriumthiosulfat-Lösung (5 ml) versetzt und mit Wasser (25 ml) verdünnt. Die wässrige Lösung wurde mit Dichlormethan zweimal (je 50 ml) extrahiert. Die vereinten organischen Phasen wurden über Natriumsulfat getrocknet, abfilitiert und im Vakuum zur Trockne eingeengt. Der Rückstand wurde durch Flash-Säulenchromatographie (Gradient: Dichlormethan / 0-4 vol.% Ethanol) aufgereinigt, wobei ein Gemsich (310 mg, ca. 3:1) der Öle (3,5-Difluor-pyridin-4-yl)-[4-fluor-3-(7-morpholin-4-yl-chinazolin-4-yl)-phenyl]-methanon (233 mg, 0,50 mmol, MS: 451,1 [M+H⁺]) und (4-Chlor-5-fluor-pyridin-3-yl)-[4-fluor-3-(7-morpholin-4-yl-chinazolin-4-yl)-phenyl]-methanon (77 mg, 0,16 mmol, MS: 467,1/469,1 [M+H⁺]) erhalten wurde.

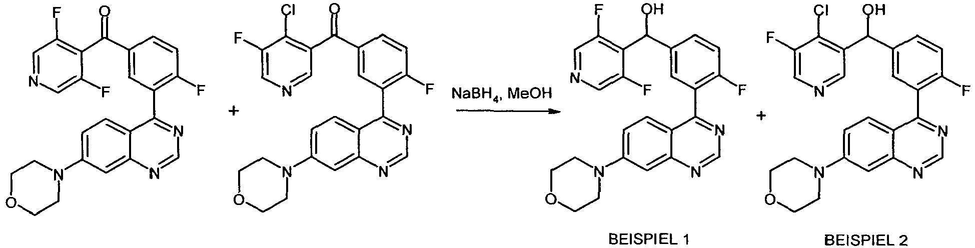

Das Gemisch (310 mg, ca. 3:1) aus (3,5-Difluor-pyridin-4-yl)-[4-fluor-3-(7-morpholin-4-yl-chinazolin-4-yl)-phenyl]-methanon und (4-Chlor-5-fluor-pyridin-3-yl)-[4-fluor-3-(7-morpholin-4-yl-chinazolin-4-yl)-phenyl]-methanon wurde in Methanol (15 ml) gelöst. Anschließend wurde Natriumborhydrid (30,4 mg, 0,80 mmol) zugegeben (Gasentwicklung). Die Reaktionslösung wurde 45 min bei Raumtemperatur gerührt. Nach beendeter Reaktion wurde mit gesättigter Ammoniumchlorid-Lösung (5 ml) und Wasser (15 ml) verdünnt. Anschließend wurde die wässrige Phase dreimal mit Dichlormethan (je 20 ml) extrahiert. Die vereinigten organischen Phasen wurden über Natriumsulfat getrocknet, abfiltiert und im Vakuum zur Trockne einrotiert. Der Rückstand wurde in Dimethylsulfoxid (4,8 ml) gelöst und mittels präparativer HPLC (Gradient: Wasser/1-50 vol.% Acetonitril über 21 min, Flussgeschwindigkeit 50 ml/min) chromatographisch aufgereinigt. Die Produktfraktionen wurden vereinigt, mit gesättigter Natriumhydrogencarbonat-Lösung (je 5 ml) verdünnt und zweimal mit Dichlormethan (je 40 ml) extrahiert. Die organischen Phasen wurden im Vakuum eingeengt, die Rückstände anschließend in 1,4-Dioxan (5 ml) und Wasser (30 ml) aufgenommen und gefriergetrocknet, wobei reines (3,5-Difluor-pyridin-4-yl)-[4-fluor-3-(7-morpholin-4-yl-chinazolin-4-yl)-phenyl]-methanol (BEISPIEL 1, 42,50 mg, 0,09 mmol, MS: 453,1 [M+H⁺]) und (4-Chlor-5-fluor-pyridin-3-yl)-[4-fluor-3-(7-morpholin-4-yl-chinazolin-4-yl)-phenyl]-methanol (BEISPIEL 2, 28,40 mg, 0,06 mmol, MS: 469,1/471,1 [M+H⁺]) als Feststoffe erhalten wurden.

### BEISPIEL 37

### (3-Chlor-pyrazin-2-yl)-[4-fluor-3-(6-morpholin-4-yl-thieno[3,2-d]pyrimidin-4-yl)-phenyl]-methanol (37)

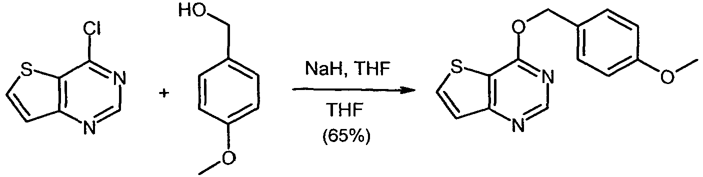

In einem Glasgefäß wurde Natriumhydrid (60%ige Suspension in Paraffinlöl, 1,41 g, 35,0 mmol) in trockenem Tetrahydrofuran (25 ml) unter Argon suspendiert. Anschließend wurde 4-Methoxy-phenyl-methanol (4,21 g, 30,0 mmol), gelöst in trockenen Tetrahydrofuran (5 ml), unter Rühren langsam zugetropft und 1 h bei Raumtemperatur gerüht. Danach wurde eine Suspension aus 4-Chlor-thieno[3,2-d]pyrimidin (4,00 g, 23,4 mmol) in trockenen Tetrahydrofuran (20 ml) langsam zugefügt und eine weitere Stunde gerührt. Nach vollständigem Reaktionsumsatz wurde vorsichtig mit Methanol (15 ml) versetzt, anschließend im Vakuum eingeengt und mit einer Mischung aus Wasser (100 ml) und Essigsäureethylester (150 ml) verdünnt. Die wässrige Phase wurde dreimal mit Essigsäureethylester (je 100 ml) extrahiert, über Natriumsulfat getrocknet, abgesaugt und das Filtrat im Vakuum eingeengt. Der lösemittelfreie Rückstand wurde in Ethanol (40 ml) aufgenommen und 16 h bei ca. 5°C vorsichtig gerührt. Die über Nacht ausgefallenen Kristalle wurden abgesaugt, mit etwas kaltem Ethanol nachgewaschen und bei Raumtemperatur getrocknet. Man erhielt 4-(4-Methoxy-benzyloxy)-thieno[3,2-d]pyrimidin 4,15 g, 15,24 mmol, MS: 273,0 [M+H⁺]), 65% Ausbeute) als kristallinen Feststoff.

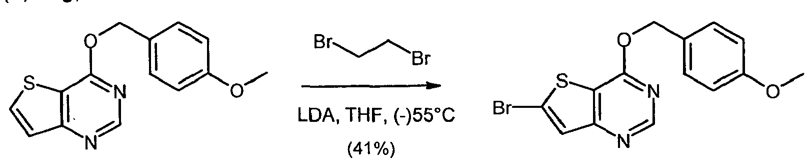

4-(4-Methoxy-benzyloxy)-thieno[3,2-d]pyrimidin (2,60 g, 9,55 mmol) wurde in trockenem Tetrahydrofuran (35 ml) gelöst und auf (-)55°C gekühlt. Eine frisch präparierte Lithiumdiisopropylamid-Lösung (21 mmol, hergestellt aus Diisopropylamin [2.13 g, 21 mmol] und n-BuLi [15%ige Lösung aus n-Hexan, 13,13 ml, 21 mmol in trockenem Tetrahydrofuran [35 ml] bei [-]10°C) wurde über 10 min bei (-)55°C zugetropft. Die erhaltene Suspension wurde weiter gerührt. Anschießend wurde 1,2-Dibromethan (10,76 g, 6,0 mmol) zugegeben. Nach weiteren 10 min wurde auf (-)20°C erwärmt und 1 h gerührt. Mit beendeter Reaktion wurde die Reaktionslösung in eine 50%ige wässrige Natriumhydrogencarbonat-/Natriumthiosulfat-Lösung (Volumenverhältnis 1:1, 120 ml) gegeben. Die wässrige Phase wurde dreimal mit Essigsäureethylester extrahiert. Die vereinigten organischen Phasen wurden mit gesättigter Natriumchlorid-Lösung gewaschen, anschießend über Natriumsulfat getrocknet, filtriert und am Rotationsverdampfer zur Trockne eingeengt. Der Rückstand wurde mittels Flash-Säulenchromatographie aufgereinigt (Gradient Cyclohexan / 0-18 vol.% Essigsäure-ethylester, CombiFlash Rf 200, 80 g Silica-Säule, Fluss = 50 ml/min., λ = 220 nm), wobei 6-Brom-4-(4-methoxy-benzyloxy)-thieno[3,2-d]pyrimidin (1.39 g, 3.95 mmol, MS: 351,0/353,0 [M+H⁺]), 41% Ausbeute) als Feststoff erhalten wurde.

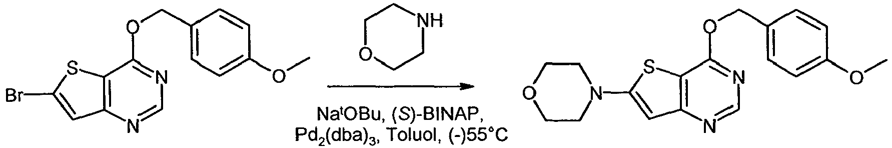

In einem Mikrowellengefäß wurde 6-Brom-4-(4-methoxy-benzyloxy)-thieno[3,2-d]pyrimidin (1,38 g, 3,93 mmol), Morpholin (1,03 g, 11,79 mmol), Natrium-tert.-butylat (1,13 g, 11,79 mmol), (S)-(-)-2,2'-Bis(diphenylphosphino)-1,1'-binaphtyl (S-BINAP, 122,3 mg, 0,196 mmol) und Tris(dibenzylidenaceton)dipalladium (179,9 mg, 0,196 mmol) unter Stickstoff in Toluol (20 ml) gelöst. Die Reaktionslösung wurde 4 h bei 95°C erhitzt. Anschließend wurde mit Wasser (60 ml) und Dichlormethan (60 ml) verdünnt. Die wässrige Phase wurde dreimal mit Dichlormethan extrahiert. Die vereinigten organischen Phasen wurden über Natriumsulfat getrocknet und am Rotationsverdampfer eingeengt. Der Rückstand wurde mittels FlashChromatographie aufgereinigt (Gradient Dichlormethan / 5-25 vol.% [Dichlormethan/Ethanol 9:1], CombiFlash Rf 200, 80 g Silica-Säule, λ = 220 nm). Die geeigneten Produktfraktionen wurden vereinigt und die Lösungsmittel am Rotationsverdampfer entfernt, wobei 4-(4-Methoxy-benzyloxy)-6-morpholin-4-yl-thieno[3,2-d]pyrimidin (756.0 mg, 2.12 mmol, MS: 358,2 [M+H⁺]) 54% Ausbeute) als Feststoff erhalten wurde.

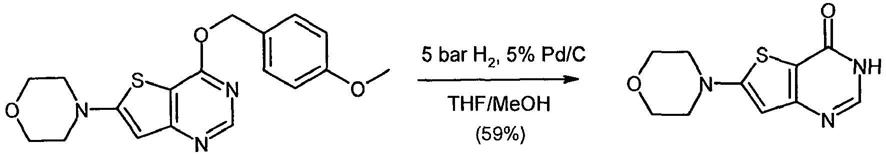

4-(4-Methoxy-benzyloxy)-6-morpholin-4-yl-thieno[3,2-d]pyrimidin (923 mg, 2,58 mmol) wurde in Tetrahydrofuran (5 ml) und Methanol (5 ml) gelöst. Pd-C (5%, 1,9 g) wurde portionsweise (zum Reaktionsbeginn, nach weiteren 7 h und 24 h) zugegeben und bei maximal 5 bar Wasserstoffdruck (H₂, Reinheit 3.0, 57,9 g) für 36 h hydriert. Die erhaltene Lösung wurde über Kieselgur filtriert und am Rotationsverdampfer eingeengt. Der Rückstand wurde mittels Flash-Säulenchromatographie (Gradient: Dichlormethan/10-20 vol.% [Methanol/Ammoniak 10:1], CombiFlash Rf 200, 24 g Silica-Säule, λ = 220 nm) aufgereinigt. Die geeigneten Produktfraktionen wurden vereinigt und die Lösungsmittel am Rotationsverdampfer entfernt. Man erhielt 6-Morpholin-4-yl-3H-thieno[3,2-d]pyrimidin-4-on (361.0 mg, 1.521 mmol, MS: 238,0 [M+H⁺], 59% Ausbeute) als Feststoff.

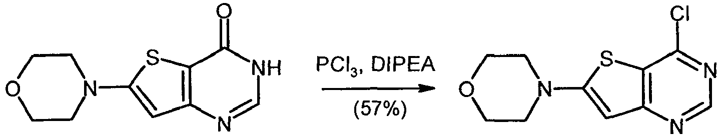

6-Morpholin-4-yl-3H-thieno[3,2-d]pyrimidin-4-on (206 mg, 0,87 mmol) wurde in Phosphorylchlorid (1,67 g, 10,89 mmol) suspendiert. Der Suspension wurde anschließend N-Ethyldiisopropylamin (56,1 mg, 0,43 mmol) zugefügt. Die Reaktionsmischung wurde über Nacht bei Raumtemperatur gerührt. Zur Aufarbeitung wurde ein Gemisch aus gesättigter Natriumhydrogencarbonat-Lösung (30 ml) und Dichlormethan (20 ml) zugegeben. Die resultierende Lösung wurde dreimal mit Dichlormethan extrahiert. Die vereinigten organischen Phasen wurden über Natriumsulfat getrocknet, filtriert und im Vakuum zur Trockne eingeengt, wobei 4-Chlor-6-morpholin-4-yl-thieno[3,2-d]pyrimidin (127 mg, 0,497 mmol, MS: 256.0/258,0 [M+H⁺], 57% Ausbeute) als Feststoff erhalten wurden.

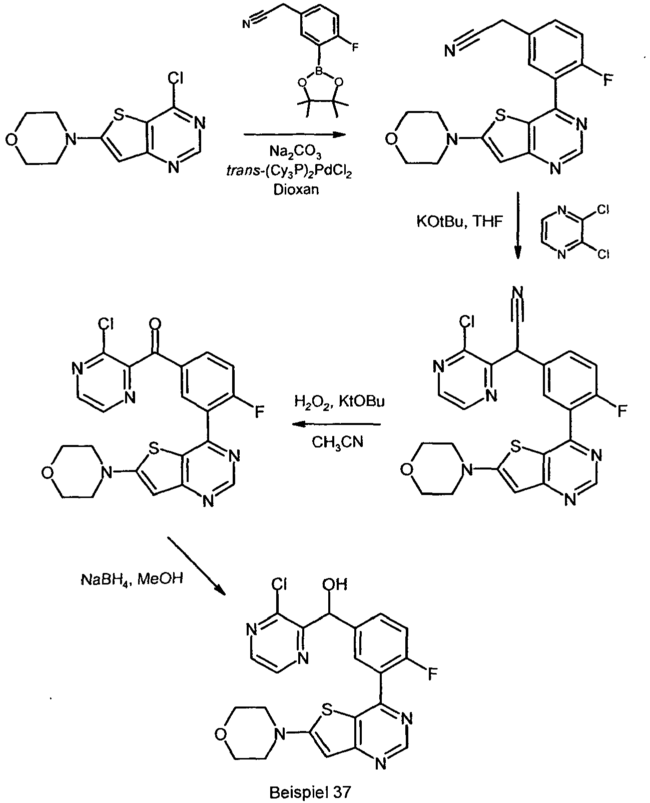

Ausgehend von 4-Chlor-6-morpholin-4-yl-thieno[3,2-d]pyrimidin wurde (3-Chlor-pyrazin-2-yl)-[4-fluor-3-(6-morpholin-4-yl-thieno[3,2-d]pyrimidin-4-yl)-phenyl]-methanol (BEISPIEL 37) analog der für die Beispiele 1 und 2 beschriebenen Synthesesequenz hergestellt.

Verbindungen die entsprechend den Beispielen 1,2 und 37 hergestellt wurden, finden sich in der nachfolgenden Tabelle 1.

**Tabelle 1 Verbindungen der Formel (I)**

| Beispiel | Strukturformel | Name | IC₅₀ DNA-PK | IC₅₀ pDNA-PK | Kᵢ [Kv1.11 hERG] |
|---|---|---|---|---|---|
| **1** | 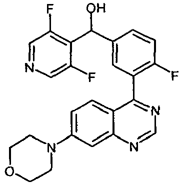 | (3,5-Difluoro-pyridin-4-yl)-[4-fluoro-3-(7-morpholin-4-yl-quinazolin-4-yl)-phenyl]-methanol | B | B | B |
| | MS: 453.1 (M+H⁺) | 1H NMR (500 MHz, DMSO-d6) ppm = 9.10 (s, 1H), 8.50 (s, 2H), 7.65 - 7.60 (m, 2H), 7.55 - 7.48 (m, 2H), 7.45 - 7.40 (m, 1H), 7.20 (d, J=2.1, 1H), 6.62 (d, J=4.6, 1H), 6.23 (d, J=4.6, 1H), 3.80 - 3.75 (m, 4H), 3.47 - 3.42 (m, 4H) | | | |
| **2** | 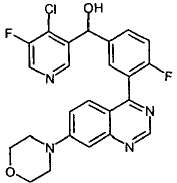 | (4-Chloro-5-fluoro-pyridin-3-yl)-[4-fluoro-3-(7-morpholin-4-yl-quinazolin-4-yl)-phenyl]-methanol | B | A | C |
| | MS: 469.1/471.1 (M+H⁺) (Cl Isotopie, rel. Peakintensitäts-Verhältnis [%] 100:37) | 1H NMR (500 MHz, DMSO-d6) ppm = 9.10 (s, 1H), 8.70 (s, 1H), 8.66 (s, 1H), 7.64 - 7.59 (m, 2H), 7.55 - 7.48 (m, 2H), 7.44 - 7.39 (m, 1H), 7.20 (d, J=2.2, 1H), 6.56 (d, J=4.4, 1H), 6.12 (d, J=4.5, 1H), 3.81 - 3.74 (m, 4H), 3.47 - 3.40 (m, 4H) | | | |
| **3** | 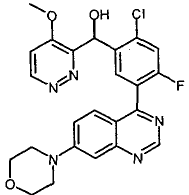 | [2-Chloro-4-fluoro-5-(7-morpholin-4-yl-quinazolin-4-yl)-phenyl]-(4-methoxy-pyridazin-3-yl)-methanol | B | B | A |
| | MS: 504.1/506.1 (M+Na⁺) (Cl Isotopie, rel. Peakintensitäts-Verhältnis [%] 100:35) | 1H NMR (500 MHz, DMSO-d6) ppm = 9.13 (s, 1H), 8.96 (d, J=6.0, 1H), 8.04 - 8.01 (m, 1H), 7.65 - 7.60 (m, 2H), 7.56 (dd, J=9.4, 2.5, 1H), 7.32 (d, J=6.0, 1H), 7.22 (d, J=2.5, 1H), 6.46 (d, J=6.2, 1H), 6.39 (d, J=6.3, 1H), 3.96 (s, 3H), 3.81 - 3.75 (m, 4H), 3.50 - 3.43 (m, 4H) | | | |
| **4** | 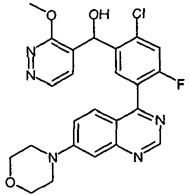 | [2-Chloro-4-fluoro-5-(7-morpholin-4-yl-quinazolin-4-yl)-phenyl]-(5-methoxy-pyridazin-3-yl)-methanol | A | A | A |
| | MS: 482.1/484.1 (M+H⁺) (Cl Isotopie, rel. Peakintensitäts-Verhältnis [%] 100:34) | 1H NMR (500 MHz, DMSO-d6) ppm = 9.07 (s, 1H), 8.88 (d, J=4.7, 1H), 7.71 (d, J=9.5, 1H), 7.59 (dd, J=4.7, 0.9, 1H), 7.54 (d, J=7.6, 1H), 7.52 - 7.50 (m, 2H), 7.21 - 7.17 (m, 1H), 6.46 (d, J=5.1, 1H), 6.15 (d, J=5.2, 1H), 4.01 (s, 3H), 3.81 - 3.74 (m, 4H), 3.48 - 3.41 (m, 4H) | | | |
| **5** | 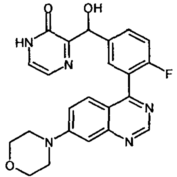 | 3-{[4-Fluoro-3-(7-morpholin-4-yl-quinazolin-4-yl)-phenyl]-hydroxy-methyl}-1H-pyrazin-2-one | D | D | B |
| | MS: 434.2 (M+H⁺) | 1H NMR (500 MHz, DMSO-d6) ppm = 9.09 (s, 1H), 7.69 - 7.59 (m, 2H), 7.56 - 7.50 (m, 2H), 7.39 - 7.28 (m, 3H), 7.20 (d, J=2.1, 1H), 6.00 (s, 1H), 5.87 - 5.75 (m, 1H), 3.81 - 3.74 (m, 4H), 3.44 (t, J=5.0, 4H) | | | |
| **6** | 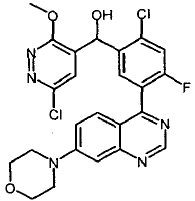 | [2-Chloro-4-fluoro-5-(7-morpholin-4-yl-quinazolin-4-yl)-phenyl]-(6-chloro-5-methoxy-pyridazin-3-yl)-methanol | A | A | C |
| | MS: 516.1/518.1/520.1 (M+H⁺) (Cl₂ Isotopie, rel. Peakintensitäts-Verhältnis [%] 100:71:14) | 1H NMR (500 MHz, DMSO-d6) ppm = 9.07 (s, 1H), 7.75 (d, J=1.0, 1H), 7.72 (d, J=9.5, 1H), 7.56 (d, J=7.6, 1H), 7.51 (dd, J=9.5, 2.5, 1H), 7.47 (dd, J=9.4, 2.9, 1H), 7.19 (d, J=2.4, 1H), 6.59 (d, J=5.0, 1H), 6.13 - 6.10 (m, 1H), 3.98 (s, 3H), 3.80 - 3.74 (m, 4H), 3.48 - 3.41 (m, 4H) | | | |
| **7** | 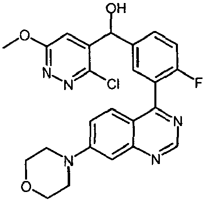 | (3-Chloro-6-methoxy-pyridazin-4-yl)-[4-fluoro-3-(7-morpholin-4-yl-quinazolin-4-yl)-phenyl]-methanol | B | A | B |
| | MS: 482.1/484.1 (M+H⁺) (Cl Isotopie, rel. Peakintensitäts-Verhältnis [%] 100:39) | 1H NMR (500 MHz, DMSO-d6) ppm = 9.09 (s, 1H), 7.62 - 7.57 (m, 2H), 7.55 - 7.47 (m, 3H), 7.43 - 7.39 (m, 1H), 7.20 (d, J=2.4 1H), 6.58 (d, J=4.5, 1H), 5.88 (d, J=4.4, 1H), 4.04 (s, 3H), 3.80 - 3.75 (m, 4H), 3.46 - 3.42 (m, 4H) | | | |
| **8** | 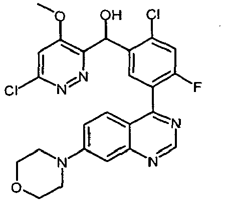 | [2-Chloro-4-fluoro-5-(7-morpholin-4-yl-quinazolin-4-yl)-phenyl]-(6-chloro-4-methoxy-pyridazin-3-yl)-methanol | C | B | C |
| | MS: 516.1/518.1/520.1 (M+H⁺) (Cl₂ Isotopie, rel. Peakintensitäts-Verhältnis [%] 100:72:16) | 1H NMR (500 MHz, DMSO-d6) ppm = 9.13 (s, 1H), 8.01 - 7.99 (m, 1H), 7.65 - 7.59 (m, 3H), 7.56 (dd, J=9.5, 2.5, 1H), 7.22 (d, J=2.5, 1H), 6.52 (d, J=6.2, 1H), 6.39 (d, J=6.2, 1H), 4.02 (s, 3H), 3.80 - 3.76 (m, 4H), 3.48 - 3.44 (m, 4H) | | | |
| **9** | 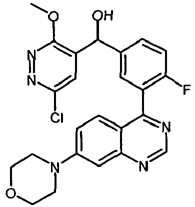 | (6-Chloro-5-methoxy-pyridazin-3-yl)-[4-fluoro-3-(7-morpholin-4-yl-quinazolin-4-yl)-phenyl]-methanol | B | A | D |
| | MS: 482.1/484.1 (M+H⁺) (Cl Isotopie, rel. Peakintensitäts-Verhältnis [%] 100:38) | 1H NMR (500 MHz, DMSO-d6) ppm = 9.10 (s, 1H), 7.88 (d, J=1.0, 1H), 7.63 - 7.58 (m, 2H), 7.55 - 7.47 (m, 2H), 7.42 - 7.35 (m, 1H), 7.20 (d, J=2.3, 1H), 6.46 (d, J=4.3, 1H), 5.85 - 5.82 (m, 1H), 4.00 (s, 3H), 3.81 - 3.74 (m, 4H), 3.48 - 3.41 (m, 4H) | | | |
| **10** | 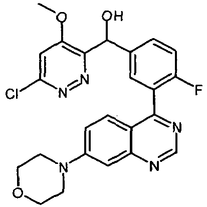 | (6-Chloro-4-methoxy-pyridazin-3-yl)-[4-fluoro-3-(7-morpholin-4-yl-quinazolin-4-yl)-phenyl]-methanol | B | A | A |
| | MS: 482.1/484.1 (M+H⁺) (Cl Isotopie, rel. Peakintensitäts-Verhältnis [%] 100:38) | 1H NMR (500 MHz, DMSO-d6) ppm = 9.09 (s, 1H), 7.64 - 7.60 (m, 2H), 7.53 - 7.51 (m, 3H), 7.40 - 7.35 (m, 1H), 7.21 - 7.19 (m, 1H), 6.29 (d, J=5.3, 1H), 618 (d, J=4.3, 1H), 3.93 (s, 3H), 3.80 - 3.75 (m, 4H), 3.46 - 3.42 (m, 4H) | | | |
| **11** | 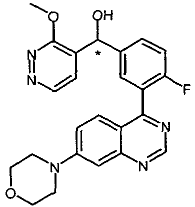 | [4-Fluoro-3-(7-morpholin-4-yl-quinazolin-4-yl)-phenyl]-(5-methoxy-pyridazin-3-yl)-methanol (*Ena 1*) | B | A | C |
| | MS: 448.2 (M+H⁺); Rₜ 6.1min (SFC, Chiracel OJ-H, CO₂/15 vol.% Methanol, 0.5 vol.% Diethylamin) | s. Racemat | | | |
| **12** | 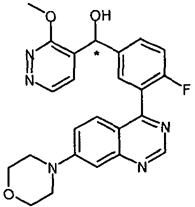 | [4-Fluoro-3-(7-morpholin-4-yl-quinazolin-4-yl)-phenyl]-(5-methoxy-pyridazin-3-yl)-methanol (*Ena 2*) | A | B | B |
| | MS: 448.2 (M+H⁺); Rₜ 8.72min (SFC, Chiracel OJ-H, CO₂/15 vol.% Methanol, 0.5 vol.% Diethylamin) | s. Racemat | | | |
| **13** | 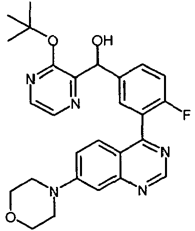 | (3-tert-Butoxy-pyrazin-2-yl)-[4-fluoro-3-(7-morpholin-4-yl-quinazolin-4-yl)-phenyl]-methanol | D | D | C |
| | MS: 490.2 (M+H⁺) | 1H NMR (500 MHz, DMSO-d6) ppm = 9.09 (s, 1H), 8.14 (d, J=2.7, 1H), 8.08 (d, J=2.7, 1H), 7.63 - 7.56 (m, 2H), 7.54 - 7.47 (m, 2H), 7.40 - 7.35 (m, 1H), 7.21 - 7.18 (m, 1H), 5.97 (d, J=6.0, 1H), 5.91 (d, J=6.1, 1H), 3.81 - 3.75 (m, 4H), 3.47 - 3.41 (m, 4H), 1.47 (s, 9H) | | | |
| **14** | 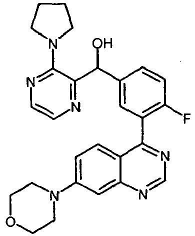 | [4-Fluoro-3-(7-morpholin-4-yl-quinazolin-4-yl)-phenyl]-(3-pyrrolidin-1-yl-pyrazin-2-yl)-methanol | B | B | A |
| | MS: 487.2 (M+H⁺) | 1H NMR (500 MHz, DMSO-d6) ppm = 9.09 (s, 1H), 7.99 (d, J=2.4, 1H), 7.80 (d, J=2.5, 1H), 7.58 - 7.52 (m, 4H), 7.38 - 7.32 (m, 1H), 7.21 - 7.17 (m, 1H), 6.18 - 6.13 (m, 1H), 6.09 - 6.01 (m, 1H), 3.78 (t, J=4.9, 4H), 3.68 - 3.56 (m, 4H), 3.44 (t, J=4.9, 4H), 1.96 - 1.81 (m, 4H) | | | |
| **15** | 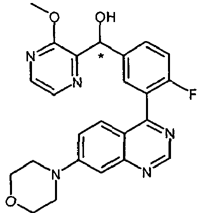 | [4-Fluoro-3-(7-morpholin-4-yl-quinazolin-4-yl)-phenyl]-(3-methoxy-pyrazin-2-yl)-methanol (*Ena 2*) | A | A | B |
| | MS: 448.2 (M+H⁺); Rₜ 19.73min, (HPLC, Chiracel OJ-H, Methanol) | s. Racemat | | | |
| **16** | 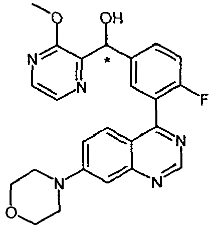 | [4-Fluoro-3-(7-morpholin-4-yl-quinazolin-4-yl)-phenyl]-(3-methoxy-pyrazin-2-yl)-methanol (*Ena 1*) | C | B | A |
| | MS: 448.2 (M+H⁺); Rₜ 7.55min, (HPLC, Chiracel OJ-H, Methanol) | s. Racemat | | | |
| **17** | 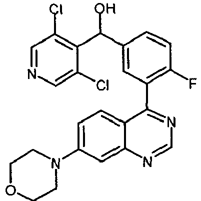 | (3,5-Dichloro-pyridin-4-yl)-[4-fluoro-3-(7-morpholin-4-yl-quinazolin-4-yl)-phenyl]-methanol | B | B | C |
| | MS: 485.1/487.1/489.1 (M+H⁺) (Cl₂ Isotopie, rel. Peakintensitäts-Verhältnis [%] 100:64:11) | 1H NMR (500 MHz, DMSO-d6) ppm = 9.09 (s, 1H), 8.62 (s, 2H), 7.58 - 7.48 (m, 4H), 7.43 - 7.37 (m, 1H), 7.19 (d, J=2.2, 1H), 6.70 - 6.65 (m, 1H), 6.52 (d, J=4.7, 1H), 3.81 - 3.74 (m, 4H), 3.48 - 3.40 (m, 4H) | | | |
| **18** | 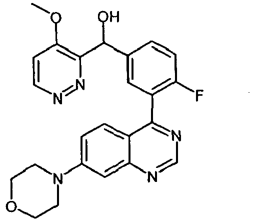 | [4-Fluoro-3-(7-morpholin-4-yl-quinazolin-4-yl)-phenyl]-(4-methoxy-pyridazin-3-yl)-methanol | A | B | B |
| | MS: 448.1 (M+H⁺) | 1H NMR (500 MHz, DMSO-d6) ppm = 9.11 - 9.08 (m, 1H), 8.99 - 8.95 (m, 1H), 7.65 - 7.59 (m, 2H), 7.55 - 7.50 (m, 2H), 7.40 - 7.34 (m, 1H), 7.27 (d, J=5.9, 1H), 7.22 - 7.18 (m, 1H), 6.24 - 6.18 (m, 2H), 3.88 (s, 3H), 3.81 - 3.74 (m, 4H), 3.48 - 3.40 (m, 4H) | | | |
| **19** | 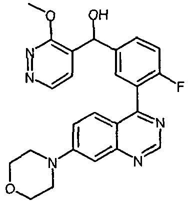 | [4-Fluoro-3-(7-morpholin-4-yl-quinazolin-4-yl)-phenyl]-(5-methoxy-pyridazin-3-yl)-methanol | A | A | A |
| | MS: 448.2 (M+H⁺) | 1H NMR (500 MHz, DMSO-d6) ppm = 9.11 - 9.08 (m, 1H), 8.94 - 8.90 (m, 1H), 7.80 - 7.77 (m, 1H), 7.59 - 7.55 (m, 2H), 7.51 (qd, J=9.4, 2.5, 2H), 7.41 - 7.35 (m, 1H), 7.20 (d, J=2.3, 1H), 6.34 - 6.31 (m, 1H), 5.87 (d, J=4.2, 1H), 4.01 (s, 3H), 3.82 - 3.74 (m, 4H), 3.48 - 3.40 (m, 4H) | | | |
| **20** | 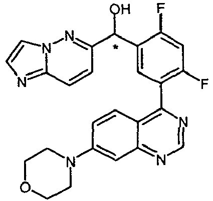 | [2,4-Difluoro-5-(7-morpholin-4-yl-quinazolin-4-yl)-phenyl]-imidazo[1,2-b]pyridazin-6-yl-methanol (*Ena 2*) | C | C | A |
| | MS: 475.2 (M+H⁺); Rₜ 15.12 min (HPLC, Chiralpak AD-H, n-Heptan/2-Propanol, 1:9 vol./vol.) | s. Racemat | | | |
| **21** | 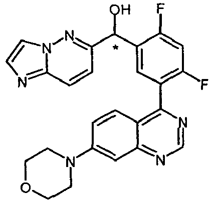 | [2,4-Difluoro-5-(7-morpholin-4-yl-quinazolin-4-yl)-phenyl]-imidazo[1,2-b]pyridazin-6-yl-methanol (*Ena 1*) | A | A | A |
| | MS: 475.2 (M+H⁺); Rₜ 8.05 min (HPLC, Chiralpak AD-H, n-Heptan/2-Propanol, 1:9 vol./vol.) | - s. Racemat | | | |
| **22** | 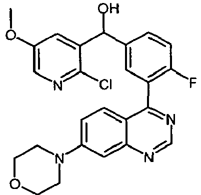 | (2-Chloro-5-methoxy-pyridin-3-yl)-[4-fluoro-3-(7-morpholin-4-yl-quinazolin-4-yl)-phenyl]-methanol | A | A | D |
| | MS: 481.2/483.2 (M+H⁺) (Cl Isotopie, rel. Peakintensitäts-Verhältnis [%] 100:30) | 1H NMR (500 MHz, DMSO-d6) ppm = 9.09 (s, 1H), 8.28 (d, J=2.6, 1H), 7.62 - 7.55 (m, 3H), 7.55 - 7.49 (m, 2H), 7.41 - 7.32 (m, 1H), 7.21 - 7.18 (m, 1H), 6.16 (d, J=6.1, 1H), 6.03 (d, J=6.1, 1H), 3.85 (s, 3H), 3.80 - 3.75 (m, 4H), 3.47 - 3.42 (m, 4H) | | | |
| **23** | 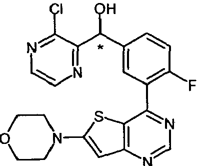 | (3-Chloro-pyrazin-2-yl)-[4-fluoro-3-(6-morpholin-4-yl-thieno[3,2-d]pyrimidin-4-yl)-phenyl]-methanol (*Ena 1*) | C | C | A |
| | MS: 458.1/460.1 (M+H⁺) (Cl Isotopie, rel. Peakintensitäts-Verhältnis [%] 100:41); Rₜ 4.96min (SFC, Chiracel OD-H, CO₂ /30 vol.% Methanol) | identisch mit Enantiomer (24) | | | |
| **24** | 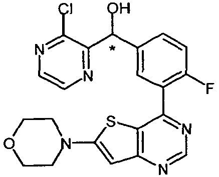 | (3-Chloro-pyrazin-2-yl)-[4-fluoro-3-(6-morpholin-4-yl-thieno[3,2-d]pyrimidin-4-yl)-phenyl]-methanol (*Ena 2*) | A | A | B |
| | MS: 458.1/460.1 (M+H⁺) (Cl Isotopie, rel. Peakintensitäts-Verhältnis [%] 100:40); Rₜ 7.13min (SFC, Chiracel OD-H, CO₂ /30vol.% Methanol) | 1H NMR (500 MHz, DMSO-d6) ppm = 8.88 (s, 1H), 8.68 (d, J=2.4, 1H), 8.47 (d, J=2.4, 1H), 7.73 (dd, J=7.0, 2.3, 1H), 7.66 - 7.61 (m, 1H), 7.39 (dd, J=10.3, 8.6, 1H), 6.52 (s, 1H), 6.39 (d, J=5.7, 1H), 6.21 (d, J=5.7, 1H), 3.78 - 3.71 (m, 4H), 3.44 - 3.37 (m, 4H). | | | |
| **25** | 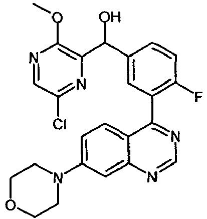 | (6-Chloro-3-methoxy-pyrazin-2-yl)-[4-fluoro-3-(7-morpholin-4-yl-quinazolin-4-yl)-phenyl]-methanol | B | A | D |
| | MS: 482.1/484.1 (M+H⁺) (Cl Isotopie, rel. Peakintensitäts-Verhältnis [%] 100:36) | 1H NMR (500 MHz, DMSO-d6) ppm = 9.11 (s, 1H), 8.30 (s, 1H), 7.65 - 7.60 (m, 2H), 7.57 - 7.51 (m, 2H), 7.43 - 7.36 (m, 1H), 7.22 - 7.19 (m, 1H), 6.18 (s, 1H), 6.06 - 6.02 (m, 1H), 3.94 (s, 3H), 3.81 - 3.75 (m, 4H), 3.47 - 3.43 (m, 4H) | | | |
| **26** | 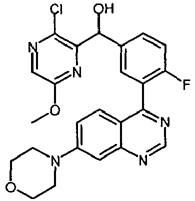 | (3-Chloro-6-methoxy-pyrazin-2-yl)-[4-fluoro-3-(7-morpholin-4-yl-quinazolin-4-yl)-phenyl]-methanol | A | A | A |
| | MS: 482.1/484.1 (M+H⁺) (Cl Isotopie, rel. Peakintensitäts-Verhältnis [%] 100:35) | 1H NMR (500 MHz, DMSO-d6) ppm = 9.10 (s, 1H), 8.11 (s, 1H), 7.73 - 7.68 (m, 2H), 7.54 - 7.50 (m, 2H), 7.44 - 7.38 (m, 1H), 7.21 - 7.19 (m, 1H), 6.31 (d, J=5.7, 1H), 6.11 (d, J=5.8, 1H), 3.89 (s, 3H), 3.80 - 3.75 (m, 4H), 3.47 - 3.41 (m, 4H) | | | |
| **27** | 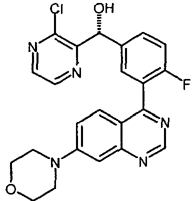 | (R)-(3-Chloro-pyrazin-2-yl)-[4-fluoro-3-(7-morpholin-4-yl-quinazolin-4-yl)-phenyl]-methanol | A | A | A |
| | MS: 452.1/454.1 (M+H⁺) (Cl Isotopie, rel. Peakintensitäts-Verhältnis [%] 100:40); Rₜ 67.12 min, (HPLC, ChiralPak AD-H, Ethanol) | s. Racemat | | | |
| **28** | 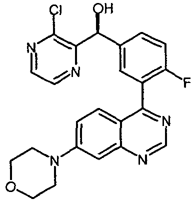 | (S)-(3-Chloro-pyrazin-2-yl)-[4-fluoro-3-(7-morpholin-4-yl-quinazolin-4-yl)-phenyl]-methanol | C | C | B |
| | MS: 452.1/454.1 (M+H⁺) (Cl Isotopie, rel. Peakintensitäts-Verhältnis [%] 100:40); Rₜ 37.09 min, (HPLC, ChiralPak AD-H, Ethanol) | s. Racemat | | | |
| **29** | 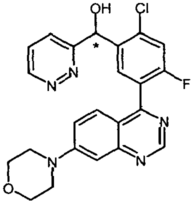 | [2-Chloro-4-fluoro-5-(7-morpholin-4-yl-quinazolin-4-yl)-phenyl]-pyridazin-3-yl-methanol (*Ena 2*) | C | D | B |
| | MS: 452.1/454.0 (M+H⁺) (Cl Isotopie, rel. Peakintensitäts-Verhältnis [%] 100:35); Rₜ 5.61min (SFC, ChiralPak IA, / 40 vol.% 2-Propanol, 0.5 vol.% Diethylamin) | 1H NMR (500 MHz, DMSO-d6) ppm = 9.14 (dd, J=4.9, 1.7, 1H), 9.11 (s, 1H), 7.88 (d, J=7.7, 1H), 7.80 (dd, J=8.6, 1.7, 1H), 7.72 - 7.66 (m, 2H), 7.58 (dd, J=9.4, 3.3, 1H), 7.53 (dd, J=9.5, 2.5, 1H), 7.21 (d, J=2.4, 1H), 6.71 (d, J=5.0, 1H), 6.32 (d, J=4.9, 1H), 3.80 - 3.75 (m, 4H), 3.47 - 3.43 (m, 4H) | | | |
| **30** | 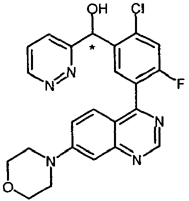 | [2-Chloro-4-fluoro-5-(7-morpholin-4-yl-quinazolin-4-yl)-phenyl]-pyridazin-3-yl-methanol (*Ena 1*) | A | A | B |
| | MS: 474.1/476.1 (M+Na⁺) (Cl Isotopie, rel. Peakintensitäts-Verhältnis [%] 100:34); Rₜ 2.87min (SFC, ChiralPak IA, CO₂/ 40 vol.% 2-Propanol, 0.5 vol.% Diethylamin) | 1H NMR (500 MHz, DMSO-d6) ppm = 9.14 (dd, J=4.9, 1.7, 1H), 9.11 (s, 1H), 7.88 (d, J=7.7, 1H) 7.80 (dd, J=8.6, 1.7, 1H), 7.73 - 7.66 (m, 2H), 7.58 (dd, J=9.4, 3.3, 1H), 7.53 (dd, J=9.5, 2.5, 1H), 7.21 (d. J=2.4, 1H), 6.71 (d, J=5.0, 1H), 6.32 (d, J=4.9, 1H), 3.80 - 3.75 (m, 4H), 3.48 - 3.42 (m, 4H) | | | |
| **31** | 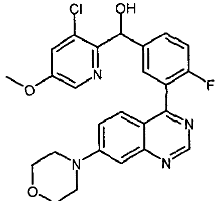 | (3-Chloro-5-methoxy-pyridin-2-yl)-[4-fluoro-3-(7-morpholin-4-yl-quinazolin-4-yl)-phenyl]-methanol | A | A | C |
| | MS: 481:1/482.1 (M+H⁺) (Cl Isotopie, rel. Peakintensitäts-Verhältnis [%] 100:30) | 1H NMR (500 MHz, DMSO-d6) ppm = 9.09 (s, 1H), 8.28 (d, J=2.6, 1H), 7.62 - 7.55 (m, 3H), 7.55 - 7.49 (m, 2H), 7.41 - 7.32 (m, 1H), 7.21 - 7.18 (m, 1H), 6.16 (d, J=6.1, 1H), 6.03 (d, J=6.1, 1H), 3.85 (s, 3H), 3.80 - 3.75 (m, 4H), 3.47 - 3.42 (m, 4H) | | | |
| **32** | 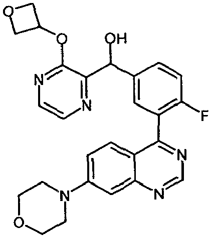 | [4-Fluoro-3-(7-morpholin-4-yl-quinazolin-4-yl)-phenyl]-[3-(oxetan-3-yloxy)-pyrazin-2-yl]-methanol | C | C | B |
| | MS: 490.2 (M+H⁺) | 1H NMR (500 MHz, DMSO-d6) ppm = 9.09 (s, 1H), 8.24 (d, J=2.7, 1H), 8.09 (d, J=2.8, 1H), 7.71 - 7.64 (m, 2H), 7.54 - 7.51 (m, 2H), 7.42 - 7.36 (m, 1H), 7.22 - 7.19 (m, 1H), 6.15 - 6.11 (m, 2H), 5.63 - 5.58 (m, 1H), 4.88 - 4.83 (m, 2H), 4.58 - 4.52 (m, 2H), 3.80 - 3.75 (m, 4H), 3.47 - 3.41 (m, 4H) | | | |
| **33** | 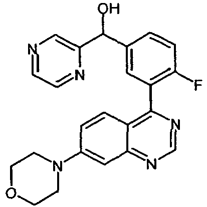 | [4-Fluoro-3-(7-morpholin-4-yl-quinazolin-4-yl)-phenyl]-pyrazin-2-yl-methanol | C | D | D |
| | MS: 418.1 (M+H⁺) | 1H NMR (400 MHz, DMSO-d6) ppm = 9.11 (s, 1H), 8.89 (d, J=1.5, 1H), 8.59 - 8.53 (m, 2H), 7.71 - 7.65 (m, 2H), 7.57 - 7.50 (m, 2H), 7.44 - 7.37 (m, 1H), 7.21 - 7.19 (m, 1H), 5.94 (s, 1H), 3.81 - 3.75 (m, 4H), 3.49 - 3.44 (m, 4H) | | | |
| **34** | 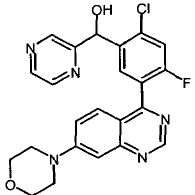 | [2-Chloro-4-fluoro-5-(7-morpholin-4-yl-quinazolin-4-yl)-phenyl]-pyrazin-2-yl-methanol | C | C | B |
| | MS: 452.1/454.1 (M+H⁺) (Cl Isotopie, rel. Peakintensitäts-Verhältnis [%] 100:36) | 1H NMR (500 MHz, DMSO-d6) ppm = 9.11 (s, 1H), 8.86 (d, J=1.3, 1H), 8.58 - 8.55 (m, 2H), 7.88 (d, J=7.7, 1H), 7.66 (d, J=9.5, 1H), 7.60 - 7.52 (m, 2H), 7.21 (d, J=2.3, 1H), 6.62 (d, J=4.9, 1H), 6.19 (d, J=4.8, 1H), 3.80 - 3.75 (m, 4H), 3.49 - 3.42 (m, 4H) | | | |
| **35** | 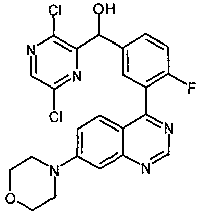 | (3,6-Dichloro-pyrazin-2-yl)-[4-fluoro-3-(7-morpholin-4-yl-quinazolin-4-yl)-phenyl]-methanol | A | A | C |
| | MS: 486.1/488-1/490.0 (M+H⁺) (Cl₂ Isotopie, rel. Peakintensitäts-Verhältnis [%] 100:65:15) | 1H NMR (500 MHz, DMSO-d6) ppm = 9.10 (s, 1H), 8.63 (s, 1H), 7.68 - 7.64 (m, 2H), 7.55 - 7.51 (m, 2H), 7.45 - 7.40 (m, 1H) 7.20 (d, J=2.1, 1H) 6.51 (d, J=5.5, 1H), 6.18 (d, J=5.5, 1H), 3.81 - 3.75 (m, 4H), 3.48 - 3.41 (m, 4H) | | | |
| **36** | 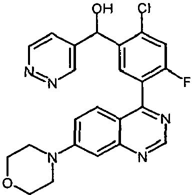 | [2-Chloro-4-fluoro-5-(7-morpholin-4-yl-quinazolin-4-yl)-phenyl]-pyridazin-4-yl-methanol | A | A | A |
| | MS: 452.1/454.1 (M+H⁺) (Cl Isotopie, rel. Peakintensitäts-Verhältnis [%] 100:33) | 1H NMR (500 MHz, DMSO-d6) ppm = 9.27 - 9.25 (m, 1H), 9.19 (dd, J=5.3, 1.2, 1H), 9.11 (s, 1H), 7.89 (d, J=7.6, 1H), 7.72 (d, J=9.5, 1H), 7.62 - 7.56 (m, 2H), 7.53 (dd, J=9.4, 2.5, 1H), 7.21 (d, J=2.4, 1H), 6.72 (s, 1H), 6.13 (s, 1H), 3.81 - 3.75 (m, 4H), 3.48 - 3.43 (m, 4H) | | | |
| **37** | 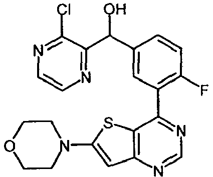 | (3-Chloro-pyrazin-2-yl)-[4-fluoro-3-(6-morpholin-4-yl-thieno[3,2-d]pyrimidin-4-yl)-phenyl]-methanol | A | A | C |
| | MS: 458.1/460.1 (M+H⁺) (Cl Isotopie, rel. Peakintensitäts-Verhältnis [%] 100:39) | 1H NMR (500 MHz, DMSO-d6) ppm = 8.88 (s, 1H), 8.68 (d, J=2.4, 1H), 8.47 (d, J=2.4, 1H), 7.73 (dd, J=7.0, 2.3, 1H), 7.66 - 7.61 (m, 1H), 7.43 - 7.35 (m, 1H), 6.52 (s, 1H), 6.39 (d, J=5.7, 1H), 6.21 (d, J=5.6, 1H), 3.77 - 3.71 (m, 4H), 3.44 - 3.37 (m, 4H) | | | |
| **38** | 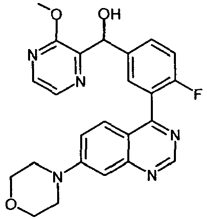 | [4-Fluoro-3-(7-morpholin-4-yl-quinazolin-4-yl)-phenyl]-(3-methoxy-pyrazin-2-yl)-methanol | A | A | A |
| | MS: 448.2 (M+H⁺) | 1H NMR (500 MHz, DMSO-d6) ppm = 9.09 (s, 1H), 8.19 (d, J=2.7, 1H), 8.15 (d, J=2.7, 1H), 7.64 - 7.59 (m, 2H), 7.55 - 7.50 (m, 2H), 7.39 - 7.34 (m, 1H), 7.21 - 7.18 (m, 1H), 6.08 (d, J=5.9, 1H), 6.04 (d, J=5.9, 1H), 3.93 (s, 3H), 3.80 - 3.75 (m, 4H), 3.47 - 3.42 (m, 4H) | | | |
| **39** | 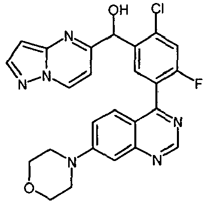 | [2-Chloro-4-fluoro-5-(7-morpholin-4-yl-quinazolin-4-yl)-phenyl]-pyrazolo[1,5-a]pyrimidin-5-yl-methanol | B | A | B |
| | MS: 491.1/493.1 (M+H⁺) (Cl Isotopie, rel. Peakintensitäts-Verhältnis [%] 100:38) | 1H NMR (500 MHz, DMSO-d6) ppm = 9.11 (s, 1H), 9.08 (dd, J=7.2, 0.9, 1H), 8.19 (d, J=2.4, 1H), 7.87 (d, J=7.7, 1H), 7.69 (d, J=9.5, 1H), 7.58 (dd, J=9.4, 3.2, 1H), 7.52 (dd, J=9.5, 2.5, 1H), 7.21 (d, J=2.5, 1H), 7.17 (d, J=7.3, 1H), 6.71 (d, J=5.0, 1H), 6.67 (dd, J=2.3, 0.9, 1H), 6.11 (d, J=4.8, 1H), 3.82 - 3.74 (m, 4H), 3.49 - 3.41 (m, 4H) | | | |
| **40** | 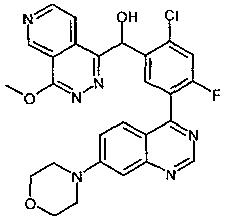 | [2-Chloro-4-fluoro-5-(7-morpholin-4-yl-quinazolin-4-yl)-phenyl]-(4-methoxy-pyrido[3,4-d]pyridazin-1-yl)-methanol | C | B | C |
| | MS: 533.2/535.2 (M+H⁺) (Cl Isotopie, rel. Peakintensitäts-Verhältnis [%] 100:38) | 1H NMR (400 MHz, DMSO-d6) ppm = 9.97 (s, 1H), 9.15 (s, 1H) 9.11 (d, J=5.5, 1H), 8.10 - 8.02 (m, 2H), 7.73 - 7.62 (m, 2H), 7.57 (dd, J=9.5, 2.5, 1H), 7.23 (d, J=2.4, 1H), 6.95 - 6.87 (m, 2H), 4.18 (s, 3H), 3.84 - 3.74 (m, 4H), 3.52 - 3.41 (m, 4H) | | | |
| **41** | 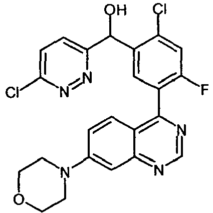 | [2-Chloro-4-fluoro-5-(7-morpholin-4-yl-quinazolin-4-yl)-phenyl]-(6-chloro-pyridazin-3-yl)-methanol | B | A | B |
| | MS: 486.0/488.1/490.0 (M+H⁺) (Cl₂ Isotopie, rel. Peakintensitäts-Verhältnis [%] 100:67:17) | 1H NMR (400 MHz, DMSO-d6) ppm = 9.11 (s, 1H), 7.92 - 7.90 (m, 2H), 7.90 - 7.87 (m, 1H), 7.69 (d, J=9.5, 1H), 7.58 (dd, J=9.4, 3.2, 1H), 7.53 (dd, J=9.5, 2.4, 1H), 7.20 (d, J=2.3, 1H), 6.86 (d, J=5.0, 1H), 6.32 (d, J=4.5, 1H), 3.81 - 3.74 (m, 4H), 3.48 - 3.43 (m, 4H) | | | |
| **42** | 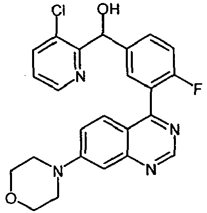 | (3-Chloro-pyridin-2-yl)-[4-fluoro-3-(7-morpholin-4-yl-quinazolin-4-yl)-phenyl]-methanol | A | A | B |
| | MS: 451.1/452.1 (M+H⁺) (Cl Isotopie, rel. Peakintensitäts-Verhältnis [%] 100:35) | 1H NMR (500 MHz, DMSO-d6) ppm = 9.09 (s, 1H), 8.56 (dd, J=4.6, 1.4, 1H), 7.92 (dd, J=8.1, 1.4, 1H), 7.64 - 7.59 (m, 2H), 7.54 - 7.49 (m, 2H), 7.40 - 7.34 (m, 2H), 7.22-7.17 (m, 1H), 6.21 (s, 1H), 6.18 - 6.09 (m, 1H), 3.81 - 3.73 (m, 4H), 3.48 - 3.40 (m, 4H) | | | |
| **43** | 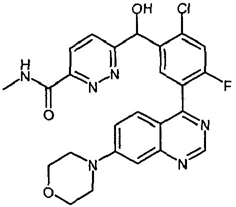 | 6-{[2-Chloro-4-fluoro-5-(7-morpholin-4-yl-quinazolin-4-yl)-phenyl]-hydroxy-methyl}-pyridazine-3-carboxylic acid methylamide | B | B | A |
| | MS: 531.2/533.2 (M+Na⁺) (Cl Isotopie, rel. Peakintensitäts-Verhältnis [%] 100:39) | 1H NMR (500 MHz, DMSO-d6) ppm = 9.14 (q, J=4.7, 1H), 9.11 (s, 1H), 8.19 (d, J=8.7, 1H), 7.99 (d, J=8.7, 1H), 7.87 (d, J=7.7, 1H), 7.69 (d, J=9.5, 1H), 7.58 - 7.51 (m, 2H), 7.22 - 7.19 (m, 1H), 6.84 (s, 1H), 6.42 (s, 1H), 3.80 - 3.76 (m, 4H), 3.47 - 3.42 (m, 4H), 2.85 (d, J=4.7, 3H) | | | |
| **44** | 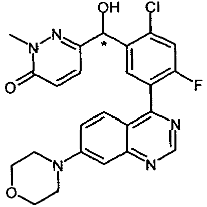 | 6-{[2-Chloro-4-fluoro-5-(7-morpholin-4-yl-quinazolin-4-yl)-phenyl]-hydroxy-methyl}-2-methyl-2H-pyridazin-3-one (*Ena 2*) | C | C | B |
| | MS: 482.0/484.1 (M+H⁺) (Cl Isotopie, rel. Peakintensitäts-Verhältnis [%] 100:35) Rₜ 5.91min, (SFC, Chiralcel OJ-H, CO₂ /20 vol.% Methanol, 0.5 vol.% Diethylamin) | 1H NMR (500 MHz, DMSO-d6) ppm = 9.12 (s, 1H), 7.93 (d, J=7.7, 1H), 7.68 (d, J=9.5, 1H), 7.61 - 7.52 (m, 2H), 7.48 (d, J=9.6, 1H), 7.21 (d, J=2.4, 1H), 6.93 (d, J=9.6, 1H), 6.60 (d, J=4.8, 1H), 5.89 (d, J=4.8, 1H), 3.80 - 3.75 (m, 4H), 3.59 (s, 3H), 3.48 - 3.43 (m, 4H) | | | |
| **45** | 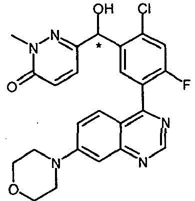 | 6-{[2-Ch loro-4-fluoro-5-(7-morpholin-4-yl-quinazolin-4-yl)-phenyl]-hydroxy-methyl}-2-methyl-2H-pyridazin-3-one (*Ena 1*) | A | A | B |
| | MS: 482.1/484.1 (M+H⁺) (Cl Isotopie, rel. Peakintensitäts-Verhältnis [%]100:35) ) Rₜ 4.10min, (SFC, Chiralcel OJ-H, CO₂ / 20 vol.% Methanol, 0.5 vol.% Diethylamin) | 1H NMR (500 MHz, DMSO-d6) ppm = 9.12 (s, 1H), 7.93 (d, J=7.7, 1H), 7.68 (d, J=9.5, 1H), 7.60 - 7.52 (m, 2H), 7.48 (d, J=9.6, 1H), 7.21 (d, J=2.4, 1H), 6.93 (d, J=9.6, 1H), 6.60 (d, J=4.9, 1H), 5.89 (d, J=4.8, 1H), 3.81 - 3.75 (m, 4H), 3.59 (s, 3H), 3.48 - 3.42 (m, 4H) | | | |
| **46** | 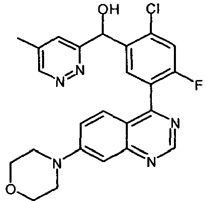 | [2-Chloro-4-fluoro-5-(7-morpholin-4-yl-quinazolin-4-yl)-phenyl]-(5-methyl-pyridazin-3-yl)-methanol | C | C | B |
| | MS: 488.1/490.2 (M+Na⁺) (Cl Isotopie, rel. Peakintensitäts-Verhältnis [%] 100:32) | 1H NMR (500 MHz, DMSO-d6) ppm = 9.11 (s, 1H), 9.00 (d, J=2.0, 1H), 7.87 (d, J=7.7, 1H), 7.67 (d, J=9.5, 1H), 7.63 - 7.61 (m, 1H), 7.58 (dd, J=9.4, 3.3, 1H), 7.53 (dd, J=9.5, 2.5, 1H), 7.21 (d, J=2.4, 1H), 6.65 (d, J=4.9, 1H), 6.28 (d, J=4.9, 1H), 3.80 - 3.75 (m, 4H), 3.48 - 3.42 (m, 4H), 2.32 (s, | | | |
| **47** | 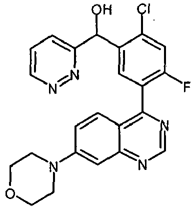 | [2-Chloro-4-fluoro-5-(7-morpholin-4-yl-quinazolin-4-yl)-phenyl]-pyridazin-3-yl-methanol | B | A | A |
| | MS: 452.1/454.1 (M+H⁺) (Cl Isotopie, rel. Peakintensitäts-Verhältnis %1100:39) | 1H NMR (500 MHz, DMSO-d6) ppm = 9.16 - 9.12 (m, 1H), 9.12 - 9.09 (m, 1H), 7.91 - 7.86 (m, 1H), 7.81 - 7.77 (m, 1H), 7.73 - 7.65 (m, 2H), 7.61 - 7.56 (m, 1H), 7.55 - 7.51 (m, 1H), 7.23 - 7.19 (m, 1H), 6.72 - 6.69 (m, 1H), 6.34 - 6.30 (m, 1H), 3.81 - 3.75 (m, 4H), 3.48 - 3.42 (m, 4H) | | | |
| **48** | 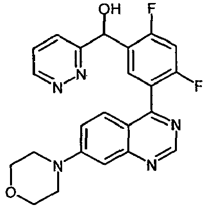 | [2,4-Difluoro-5-(7-morpholin-4-yl-quinazolin-4-yl)-phenyl]-pyridazin-3-yl-methanol | B | B | B |
| | MS: 436.2 (M+H⁺) | 1H NMR (500 MHz, DMSO-d6) ppm = 9.14 (dd, J=4.9, 1.6, 1H), 9.10 (s, 1H), 7.86 (dd, J=8.5, 1.6, 1H), 7.81 (t, J=8.1, 1H), 7.72 (dd, J=8.5, 4.9, 1H), 7.58 - 7.51 (m, 2H), 7.46 (t, J=10.1, 1H), 7.20 (d, J=2.3, 1H), 6.66 (d, J=4.9, 1H), 6.26 (d, J=4.9, 1H), 3.81 - 3.75 (m, 4H), 3.45 (t, J=4.9 4H) | | | |
| **49** | 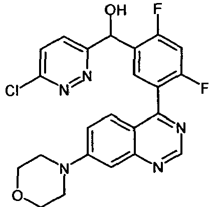 | (6-Chloro-pyridazin-3-yl)-[2,4-difluoro-5-(7-morpholin-4-yl-quinazolin-4-yl)-phenyl]-methanol | A | B | A |
| | MS: 470.1/471.1 (M+H⁺) (Cl Isotopie, rel. Peakintensitäts-Verhältnis [%] 100:35) | 1H NMR (500 MHz, DMSO-d6) ppm = 9.10 (s, 1H), 7.98 - 7.91 (m, 2H), 7.82 (t, J=8.1, 1H), 7.58 - 7.51 (m, 2H), 7.47 (t, J=10.1, 1H), 7.20 (d, J=2.3, 1H), 6.78 (d, J=4.9, 1H), 6.26 (d, J=4.6, 1H), 3.82 - 3.74 (m, 4H), 3.48 - 3.41 (m, 4H). | | | |
| **50** | 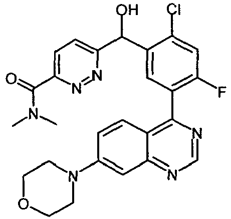 | 6-{[2-Chloro-4-fluoro-5-(7-morpholin-4-yl-quinazolin-4-yl)-phenyl]-hydroxy-methyl}-pyridazine-3-carboxylic acid dimethylamide | C | D | A |
| | MS: 523.2/525.2 (M+H⁺) (Cl Isotopie, rel. Peakintensitäts-Verhältnis [%] 100:35) | 1H NMR (500 MHz, Chloroform-d) ppm = 9.15 (s, 1H), 7.85 (d, J=8.7, 1H), 7.79 (d, J=7.4, 1H), 7.65 (d, J=8.7, 1H), 7.56 (dd, J=9.3, 3.3, 1H), 7.36 (d, J=9.0, 1H), 7.27 (d, J=2.5, 1H), 7.22 (d, J=2.5, 1H), 6.54 (s, 1H), 4.99 (s, 1H), 3.92 - 3.87 (m, 4H), 3.46 - 3.42 (m, 4H), 3.22 (s, 3H), 3.19 (s, 3H) | | | |
| **51** | 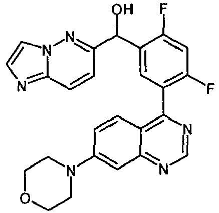 | [2,4-Difluoro-5-(7-morpholin-4-yl-quinazolin-4-yl)-phenyl]-imidazo[1,2-b]pyridazin-6-yl-methanol | A | A | B |
| | MS: 475.2 (M+H⁺) | 1H NMR (500 MHz, DMSO-d6) ppm = 9.12 (s, 1H), 8.26 (s, 1H), 8.12 (d, J=9.5, 1H), 7.92 (t, J=8.1, 1H), 7.77 (s, 1H), 7.61 - 7.51 (m, 2H), 7.48 (t, J=10.1, 1H), 7.35 (d, J=9.5, 1H), 7.21 (d, J=2.4, 1H), 6.75 (d, J=4.9, 1H), 6.10 (d, J=4.7, 1H), 3.78 (t, J=4.9, 4H), 3.45 (t, J=4.9, 4H) | | | |
| **52** | 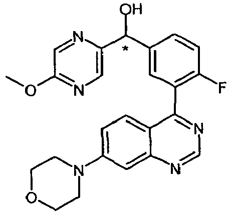 | [4-Fluoro-3-(7-morpholin-4-yl-quinazolin-4-yl)-phenyl]-(5-methoxy-pyrazin-2-yl)-methanol (*Ena 1*) | B | C | A |
| | MS: 448.2 (M+H⁺); Rₜ 16.15 min, (HPLC, 2 x Chiralcel OJ-H, Methanol) | 1H NMR (500 MHz, DMSO-d6) ppm = 9.09 (s, 1H), 8.39 - 8.38 (m, 1H), 8.23 (d, J=1.3, 1H), 7.66 - 7.61 (m, 2H), 7.55 - 7.50 (m, 2H), 7.40 - 7.35 (m, 1H), 7.21 - 7.18 (m, 1H), 6.30 (d, J=4.4, 1H), 5.88 (d, J=4.4, 1H), 3.89 (s, 3H), 3.80 - 3.75 (m, 4H), 3.46-3.42 (m, 4H) | | | |
| **53** | 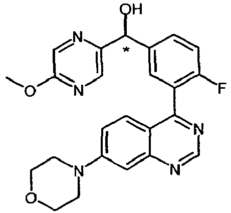 | [4-Fluoro-3-(7-morpholin-4-yl-quinazolin-4-yl)-phenyl]-(5-methoxy-pyrazin-2-yl)-methanol (*Ena 2*) | B | C | A |
| | MS: 448.2 (M+H⁺); Rₜ 19.06 min, (HPLC, 2 x Chiralcel OJ-H, Methanol) | 1H NMR (500 MHz, DMSO-d6) ppm = 9.09 (s, 1H), 8.39 - 8.38 (m, 1H), 8.23 (d, J=1.3, 1H), 7.66 - 7.61 (m, 2H), 7.55 - 7.50 (m, 2H), 7.40 - 7.35 (m, 1H), 7.21 - 7.18 (m, 1H), 6.30 (d, J=4.4, 1H), 5.88 (d, J=4.4, 1H), 3.89 (s, 3H), 3.80 - 3.75 (m, 4H), 3.46 - 3.42 (m, 4H) | | | |
| **54** | 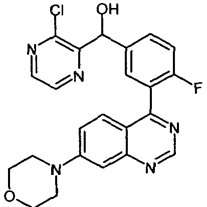 | (3-Chloro-pyrazin-2-yl)-[4-fluoro-3-(7-morpholin-4-yl-quinazolin-4-yl)-phenyl]-methanol | A | A | B |
| | MS: 452.1/453.1 (M+H⁺) (Cl Isotopie, rel. Peakintensitäts-Verhältnis [%] 100:35) | 1H NMR (500 MHz, DMSO-d6) ppm = 9.09 (s, 1H), 8.69 (d, J=2.4, 1H), 8.46 (d, J=2.4, 1H), 7.67 - 7.63 (m, 2H), 7.54 - 7.52 (m, 2H), 7.43 - 7.37 (m, 1H), 7.20 - 7.19 (m, 1H), 6.39 (d, J=5.7, 1H), 6.23 (d, J=5.7, 1H), 3.79 - 3.76 (m, 4H), 3.46 - 3.42 (m, 4H) | | | |
| **55** | 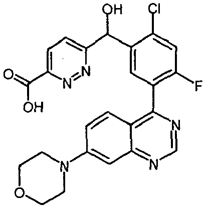 | 6-{[2-Chloro-4-fluoro-5-(7-morpholin-4-yl-quinazolin-4-yl)-phenyl]-hydroxy-methyl}-pyridazine-3-carboxylic acid | B | D | A |
| | MS: 496.1/498.0 (M+H⁺) (Cl Isotopie, rel. Peakintensitäts-Verhältnis [%] 100:36) | 1H NMR (500 MHz, DMSO-d6) ppm = 13.30 (s, 1H), 9.11 (s, 1H), 8.21 (d, J=8.7, 1H), 7.99 (d, J=8.7, 1H), 7.87 (d, J=7.6, 1H), 7.71 (d, J=9.5, 1H), 7.55 (qd, J=9.4, 2.7, 2H), 7.20 (d, J=2.3, 1H), 7.08 - 6.66 (m, 1H), 6.42 (s, 1H), 3.80 - 3.75 (m, 4H), 3.49 - 3.44 (m, 4H) | | | |
| **56** | 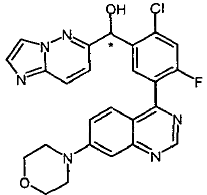 | [2-Chloro-4-fluoro-5-(7-morpholin-4-yl-quinazolin-4-yl)-phenyl]-imidazo[1,2-b]pyridazin-6-yl-methanol (*Ena 2*) | B | B | C |
| | MS: 491.1/493.1 (M+H⁺) (Cl Isotopie, rel. Peakintensitäts-Verhältnis [%] 100:34); Rₜ 13.59min (SFC, Chiralpak AD-H, CO₂/ 40 vol.% Methanol, 0.5 vol.% Diethylamin) | s. Racemat | | | |
| **57** | 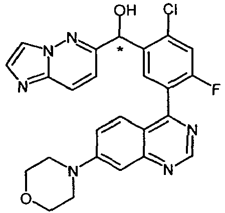 | [2-Chloro-4-fluoro-5-(7-morpholin-4-yl-quinazolin-4-yl)-phenyl]-imidazo[1,2-b]pyridazin-6-yl-methanol (*Ena 1*) | A | A | C |
| | MS: 491.1/493.1 (M+H⁺) (Cl Isotopie, rel. Peakintensitäts-Verhältnis [%] 100:36); Rₜ 3.87min (SFC, Chiralpak AD-H, CO₂/ 40 vol.% Methanol, 0.5 vol.% Diethylamin) | s. Racemat | | | |
| **58** | 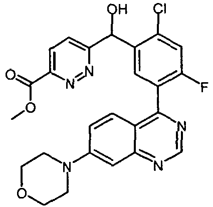 | 6-{[2-Ch loro-4-fluoro-5-(7-morpholin-4-yl-quinazolin-4-yl)-phenyl]-hydroxy-methyl}-pyridazine-3-carboxylic acid methyl ester | B | C | A |
| | MS: 510.1/512.1 (M+H⁺) (Cl Isotopie, rel. Peakintensitäts-Verhältnis [%] 100:33) | 1H NMR (500 MHz, DMSO-d6) ppm = 9.11 (s, 1H), 8.23 (d, J=8.7, 1H), 8.01 (d, J=8.7, 1H), 7.86 (d, J=7.6, 1H), 7.70 (d, J=9.5, 1H), 7.55 (qd, J=9.4, 2.8, 2H), 7.20 (d, J=2.4, 1H), 6.94 - 6.81 (m, 1H), 6.42 (s, 1H), 3.94 (s, 3H), 3.81 - 3.75 (m, 4H), 3.48 - 3.42 (m, 4H) | | | |
| **59** | 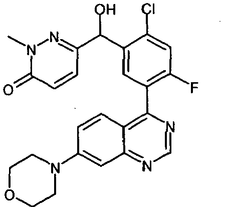 | 6-{[2-Chloro-4-fluoro-5-(7-morpholin-4-yl-quinazolin-4-yl)-phenyl]-hydroxy-methyl}-2-methyl-2H-pyridazin-3-one | A | B | B |
| | MS: 482.1/484.1 (M+H⁺) (Cl Isotopie, rel. Peakintensitäts-Verhältnis [%] 100:45) | 1H NMR (500 MHz, DMSO-d6) ppm = 9.12 (s, 1H), 7.92 (d, J=7.7, 1H), 7.68 (d, J=9.5, 1H), 7.59 (dd, J=9.4, 3.1, 1H), 7.55 (dd, J=9.4, 2.5, 1H), 7.49 (d, J=9.6, 1H), 7.21 (d, J=2.4, 1H), 6.95 (d, J=9.6, 1H), 6.68 (d, J=4.8, 1H), 5.90 (d, J=3.6, 1H), 3.80 - 3.77 (m, 4H), 3.61 - 3.58 (m, 3H), 3.45 (s, 4H) | | | |
| **60** | 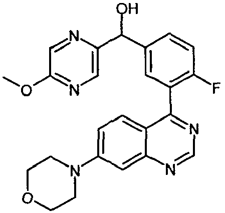 | [4-Fluoro-3-(7-morpholin-4-yl-quinazolin-4-yl)-phenyl]-(5-methoxy-pyrazin-2-yl)-methanol | C | B | B |
| | MS: 448.2 (M+H⁺) | 1H NMR (500 MHz, DMSO-d6) ppm = 9.09 (s, 1H), 8.39 - 8.38 (m, 1H), 8.23 (d, J=1.3, 1H), 7.66 - 7.61 (m, 2H), 7.55 - 7.50 (m, 2H), 7.40 - 7.35 (m, 1H), 7.21 - 7.18 (m, 1H), 6.30 (d, J=4.4, 1H), 5.88 (d, J=4.4, 1H), 3.89 (s, 3H), 3.80 - 3.75 (m, 4H), 3.46 - 3.42 (m, 4H) | | | |
| **61** | 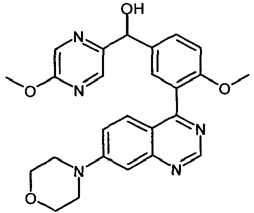 | [4-Methoxy-3-(7-morpholin-4-yl-quinazolin-4-yl)-phenyl]-(5-methoxy-pyrazin-2-yl)-methanol | D | D | A |
| | MS: 460.2 (M+H⁺) | 1H NMR (500 MHz, DMSO-d6) ppm = 9.04 (s, 1H), 8.36 - 8.35 (m, 1H), 8.20 (d, J=1.4, 1H), 7.53 (dd, J=8.6, 2.3, 1H), 7.44 (dd, J=9.4, 2.5, 1H), 7.38 (d, J=9.4, 1H), 7.35 (d, J=2.2, 1H), 7.17 (d, J=8.6, 1H), 7.14 (d, J=2.5, 1H), 6.13 (d, J=4.4, 1H), 5.79 (d, J=4.4, 1H), 3.88 (s, 3H), 3.79 - 3.75 (m, 4H), 3.66 (s, 3H), 3.43 - 3.38 (m, 4H) | | | |
| 62 | 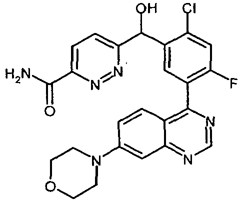 | 6-{[2-Chloro-4-fluoro-5-(7-morpholin-4-yl-quinazolin-4-yl)-phenyl]-hydroxy-methyl}-pyridazine-3-carboxylic acid amide | B | B | A |
| | MS: 495.1/497.1 (M+H⁺) (Cl Isotopie, rel. Peakintensitäts-Verhältnis [%] 100:35) | 1H NMR (500 MHz, DMSO-d6) ppm = 9.11 (s, 1H), 8.49 (s, 1H), 8.20 (d, J=8.7, 1H), 8.00 (d, J=8.7, 1H), 7.91 - 7.85 (m, 2H), 7.69 (d, J=9.5, 1H), 7.59 - 7.51 (m, 2H), 7.21 (d, J=2.2, 1H), 6.84 (d, J=5.0, 1H), 6.42 (d, J=5.0, 1H), 3.80 - 3.75 (m, 4H), 3.48 - 3.42 (m, 4H) | | | |
| **63** | 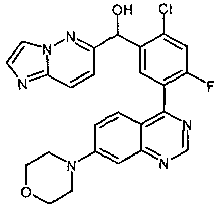 | [2-Chloro-4-fluoro-5-(7-morpholin-4-yl-quinazolin-4-yl)-phenyl]-imidazo[1,2-b]pyridazin-6-yl-methanol | A | B | C |
| | MS: 491.1/493.1 (M+H⁺) (Cl Isotopie, rel. Peakintensitäts-Verhältnis | 1H NMR (500 MHz, DMSO-d6) ppm = 9.13 (s, 1H), 8.27 - 8.25 (m, 1H), 8.11 (dd, J=9.4, 0.7, 1H), 8.00 (d, J=7.7, 1H), 7.77 (d, J=1.2, 1H), 7.70 (d, J=9.5, | | | |
| | [%] 100:38) | 1H), 7.61 (dd, J=9.4, 3.3, 1H), 7.55 (dd, J=9.4, 2.5, 1H), 7.30 (d, J=9.5, 1H), 7.22 (d, J=2.5, 1H), 6.81 (d, J=4.5, 1H), 6.15 (d, J=4.2, 1H), 3.81 - 3.75 (m, 4H), 3.48 - 3.44 (m, 4H) | | | |
| **64** | 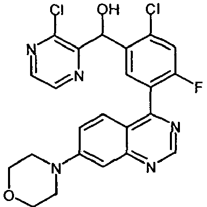 | [2-Chloro-4-fluoro-5-(7-morpholin-4-yl-quinazolin-4-yl)-phenyl]-(3-chloro-pyrazin-2-yl)-methanol | B | A | C |
| | MS: 486.1/488.1/490.0 (M+H⁺) (Cl₂ Isotopie, rel. Peakintensitäts-Verhältnis [%] 100:63:11) | 1H NMR (500 MHz, DMSO-d6) ppm = 9.14 (s, 1H), 8.61 (d, J=2.4, 1H), 8.49 (d, J=2.4, 1H), 8.00 (d, J=7.7, 1H), 7.66 (d, J=9.5, 1H), 7.64 - 7.55 (m, 2H), 7.22 (d, J=2.4, 1H), 6.70 (d, J=6.0, 1H), 6.42 (d, J=6.0, 1H), 3.81 - 3.76 (rr 4H), 349 - 3.44 (m, 4H) | | | |
| **65** | 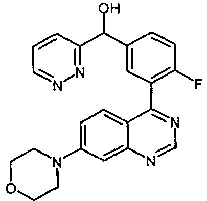 | [4-Fluoro-3-(7-morpholin-4-yl-quinazolin-4-yl)-phenyl]-pyridazin-3-yl-methanol | C | B | A |
| | MS: 418.3 (M+H⁺) | 1H NMR (500 MHz, DMSO-d6) ppm = 9.12 (dd, J=4.9, 1.7, 1H), 9.09 (s, 1H), 7.83 (dd, J=8.6, 1.7, 1H), 7.72 - 7.65 (m, 3H), 7.52 - 7.49 (m, 2H), 7.42 - 7.37 (m, 1H), 7.21 - 7.18 (m, 1H), 6.56 (d, J=4.3, 1H), 6.12 - 6.09 (m, 1H), 3.80 - 3.75 (m, 4H), 3.46 - 3.41 (m, 4H) | | | |
| **66** | 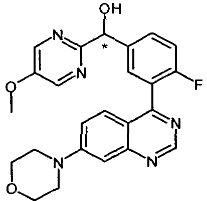 | [4-Fluoro-3-(7-morpholin-4-yl-quinazolin-4-yl)-phenyl]-(5-methoxy-pyrimidin-2-yl)-methanol (*Ena 2*) | C | D | A |
| | MS: 448.1 (M+H⁺); Rₜ 53.23 min, (HPLC, Chiralpak AD-H, Ethanol) | 1H NMR (500 MHz, DMSO-d6) ppm = 9.09 (s, 1H), 8.53 (s, 2H), 7.70 - 7.63 (m, 2H), 7.52 (s, 2H), 7.40 - 7.32 (m, 1H), 7.22 - 7.15 (m, 1H), 6.06 (d, J=5.5, 1H), 5.86 (d, J=5.5, 1H), 3.89 (s, 3H), 3.79 - 3.76 (m, 4H), 3.45 - 3.43 (m, 4H) | | | |
| **67** | 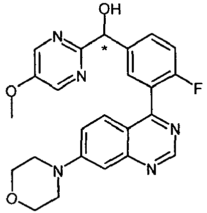 | [4-Fluoro-3-(7-morpholin-4-yl-quinazolin-4-yl)-phenyl]-(5-methoxy-pyrimidin-2-yl)-methanol (*Ena 1*) | C | C | A |
| | MS: 448.1 (M+H⁺); Rₜ 45.79 min, (HPLC, Chiralpak AD-H, Ethanol) | 1H NMR (500 MHz, DMSO-d6) ppm = 9.09 (s, 1H), 8.53 (s, 2H), 7.70 - 7.63 (m, 2H), 7.52 (s, 2H), 7.40 - 7.32 (m, 1H), 7.22 - 7.15 (m, 1H), 6.06 (d, J=5.5, 1H), 5.86 (d, J=5.5, 1H), 3.89 (s, 3H), 3.79 - 3.76 (m, 4H), 3.45 - 3.43 (m, 4H) | | | |
| **68** | 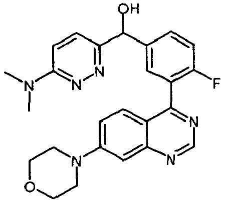 | (6-Dimethylamino-pyridazin-3-yl)-[4-fluoro-3-(7-morpholin-4-yl-quinazolin-4-yl)-phenyl]-methanol | B | C | A |
| | MS: 461.2 (M+H⁺) | 1H NMR (500 MHz, DMSO-d6) ppm = 9.09 (s, 1H), 7.65 - 7.56 (m, 2H), 7.55 - 7.48 (m, 2H), 7.43 - 7.34 (m, 2H), 7.24 - 7.17 (m, 1H), 7.07 (d, J=9.4, 1H), 6.27 (d, J=4.4, 1H), 5.93 (d, J=4.4, 1H), 3.81 - 3.75 (m, 4H), 3.47 - 3.41 (m, 4H), 3.06 (s, 6H) | | | |
| **69** | 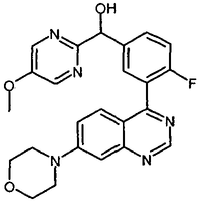 | [4-Fluoro-3-(7-morpholin-4-yl-quinazolin-4-yl)-phenyl]-(5-methoxy-pyrimidin-2-yl)-methanol | B | C | A |
| | MS: 448.1 (M+H⁺) | 1H NMR (500 MHz, DMSO-d6) ppm = 9.09 (s, 1H), 8.53 (s, 2H), 7.70 - 7.63 (m, 2H), 7.52 (s, 2H), 7.40 - 7.32 (m, 1H), 7.22 - 7.15 (m, 1H), 6.06 (d, J=5.5, 1H), 5.86 (d, J=5.5, 1H), 3.89 (s, 3H), 3.79 - 3.76 (m, 4H), 3.45 - 3.43 (m, 4H) | | | |
| **70** | 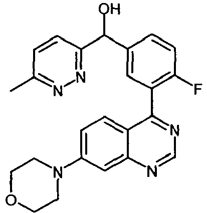 | [4-Fluoro-3-(7-morpholin-4-yl-quinazolin-4-yl)-phenyl]-(6-methyl-pyridazin-3-yl)-methanol | B | B | A |
| | MS: 432.1 (M+H⁺) | 1H NMR (500 MHz, DMSO-d6) ppm = 9.09 (s, 1H), 7.72 - 7.61 (m, 3H), 7.58 - 7.47 (m, 3H), 7.43 - 7.33 (m, 1H), 7.22 - 7.16 (m, 1H), 6.50 (d, J=4.0, 1H), 6.07 (d, J=3.8, 1H), 3.77 (t, J=5.9, 3.9, 4H), 3.44 (t, J=4.9, 4H), 2.58 (s, 3H) | | | |
| **71** | 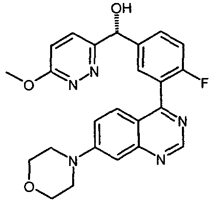 | (R)-[4-Fluoro-3-(7-morpholin-4-yl-quinazolin-4-yl)-phenyl]-(6-methoxy-pyridazin-3-yl)-methanol | A | B | A |
| | MS: 448.1 (M+H⁺); Rₜ 24.02 min, (SFC, Chiralcel OJ-H, CO₂/ 15 vol.% 2-Propanol, 0.5 vol.% Diethylamin) | 1H NMR (500 MHz, DMSO-d6) ppm = 9.09 (s, 1H), 7.70 (d, J=9.2, 1H), 7.66 - 7.61 (m, 2H), 7.53 - 7.50 (m, 2H), 7.42 - 7.37 (m, 1H), 7.22 - 7.19 (m, 2H), 6.48 (d, J=4.4, 1H), 6.02 (d, J=4.4, 1H), 4.00 (s, 3H), 3.80 - 3.75 (m, 4H), 3.46 - 3.41 (m, 4H) | | | |
| **72** | 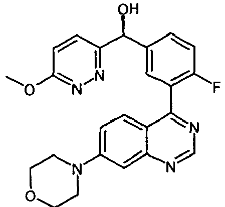 | (S)-[4-Fluoro-3-(7-morpholin-4-yl-quinazolin-4-yl)-phenyl]-(6-methoxy-pyridazin-3-yl)-methanol | A | B | A |
| | MS: 448.1 (M+H⁺); Rₜ 19.10 min, (SFC, Chiralcel OJ-H, CO₂/ 15 vol.% 2-Propanol, 0.5 vol.% Diethylamin) | 1H NMR (500 MHz, DMSO-d6) ppm = 9.09 (s, 1H), 7.69 (d, J=9.2, 1H), 7.66 - 7.61 (m, 2H), 7.53 - 7.50 (m, 2H), 7.41 - 7.37 (m, 1H), 7.21 - 7.19 (m, 2H), 6.48 (d, J=4.4, 1H), 6.02 (d, J=4.4, 1H), 4.00 (s, 3H), 3.79 - 3.76 (m, 4H), 3.46 - 3.42 (m, 4H) | | | |
| **73** | 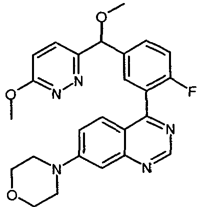 | 4-{2-Fluoro-5-[methoxy-(6-methoxy-pyridazin-3-yl)-methyl]-phenyl}-7-morpholin-4-yl-quinazoline | B | B | A |
| | MS: 462.1 (M+H⁺) | 1H NMR (500 MHz, DMSO-d6) ppm = 9.10 (s, 1H), 7.69 (d, J=9.1, 1H), 7.65 - 7.61 (m, 2H), 7.54 - 7.48 (m, 2H), 7.45 - 7.40 (m, 1H), 7.23 (d, J=9.2, 1H), 7.21 - 7.19 (m, 1H), 5.71 (s, 1H), 4.01 (s, 3H), 3.80 - 3.75 (m, 4H), 3.47 - 3.41 (m, 4H), 3.36 (s, 3H) | | | |
| **74** | 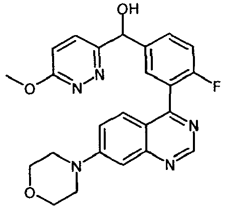 | [4-Fluoro-3-(7-morpholin-4-yl-quinazolin-4-yl)-phenyl]-(6-methoxy-pyridazin-3-yl)-methanol | A | A | A |
| | MS: 448.1 (M+H⁺) | 1H NMR (400 MHz, DMSO-d6) ppm = 9.09 (s, 1H), 7.69 (d, J=9.2, 1H), 7.67 - 7.61 (m, 2H), 7.54 - 7.48 (m, 2H), 7.42 - 7.36 (m, 1H), 7.22 - 7.17 (m, 2H), 6.51 - 6.45 (m, 1H), 6.04 - 6.00 (m, 1H), 4.00 (s, 3H), 3.80 - 3.74 (m, 4H), 3.47 - 3.41 (m, 4H) | | | |
| **75** | 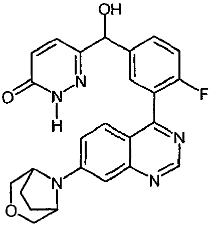 | 6-({4-Fluoro-3-[7-(3-oxa-8-aza-bicyclo[3.2.1]oct-8-yl)-quinazolin-4-yl]-phenyl}-hydroxy-methyl)-2H-pyridazin-3-one | D | | A |
| | MS: 460.1 (M+H⁺) | 1H NMR (500 MHz, DMSO-d6) ppm = 12.88 - 12.84 (m, 1H), 9.04 (s, 1H), 7.63 - 7.57 (m, 2H), 7.50 - 7.45 (m, 2H), 7.44 - 7.38 (m, 2H), 7.14 (d, J=2.4, 1H), 6.87 (dd, J=9.8, 2.2, 1H), 6.42 (d, J=4.4, 1H), 5.68 - 5.64 (m, 1H), 4.55 - 4.46 (m, 2H), 3.69 (d, J=10.9, 2H), 3.54 (d, J=10.4, 2H), 2.09 - 1.94 (m, 4H) | | | |
| **76** | 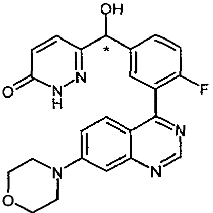 | 6-{[4-Fluoro-3-(7-morpholin-4-yl-quinazolin-4-yl)-phenyl]-hydroxy-methyl}-2H-pyridazin-3-one (*Ena 2*) | A | B | A |
| | MS: 434.1 (M+H⁺); Rₜ 16.74 min, (SFC, Chiralcel OJ-H, CO₂/ 15 vol.% Methanol, 0.5 vol.% Diethylamin) | 1H NMR (400 MHz, DMSO-d6) ppm = 12.86 (s, 1H), 9.10 (s, 1H), 7.64 - 7.58 (m, 2H), 7.55 - 7.50 (m, 2H), 7.48 (d, J=9.8, 1H), 7.45 - 7.38 (m, 1H), 7.23 - 7.16 (m, 1H), 6.87 (d, J=9.8, 1H), 6.44 (d, J=4.3, 1H), 5.70 - 5.62 (m, 1H), 3.81 - 3.74 (m, 4H), 3.47 - 3.41 (m, 4H) | | | |
| | *Ena 1 zu dieser Verbindung: Beispiel 367* | | | | |
| **77** | 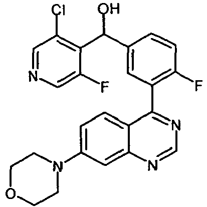 | (3-Chloro-5-fluoro-pyridin-4-yl)-[4-fluoro-3-(7-morpholin-4-yl-quinazolin-4-yl)-phenyl]-methanol | B | A | C |
| | MS: 469.1/471.1 (M+H⁺) (Cl Isotopie rel. Peakintensitäts-Verhältnis [%] 100:31) | 1H NMR (500 MHz, DMSO-d6) ppm = 9.10 (s, 1H), 8.58 - 8.53 (m, 2H), 7.65 - 7.56 (m, 2H), 7.56 - 7.47 (m, 2H), 7.46 - 7.38 (m, 1H), 7.23 - 7.17 (m, 1H), 6.65 (d, J=4.7, 1H), 6.33 (d, J=4.7, 1H), 3.80 - 3.75 (m, 4H), 3.47 - 3.41 (m, 4H) | | | |
| **78** | 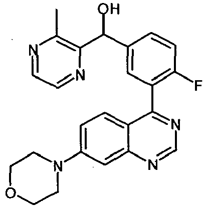 | [4-Fluoro-3-(7-morpholin-4-yl-quinazolin-4-yl)-phenyl]-(3-methyl-pyrazin-2-yl)-methanol | B | A | B |
| | MS: 432.2 (M+H⁺) | 1H NMR (400 MHz, DMSO-d6) ppm = 9.09 (s, 1H), 8.43 (s, 2H), 7.63 - 7.55 (m, 2H), 7.55 - 7.50 (m, 2H), 7.42 - 7.34 (m, 1H), 7.22 - 7.17 (m, 1H), 6.28 (d, J=5.5, 1H), 6.08 (d, J=5.4, 1H), 3.81 - 3.74 (m, 4H), 3.48 - 3.40 (m, 4H), 2.58 - 2.53 (m, 3H) | | | |
| **79** | 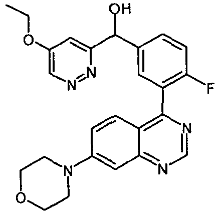 | (5-Ethoxy-pyridazin-3-yl)-[4-fluoro-3-(7-morpholin-4-yl-quinazolin-4-yl)-phenyl]-methanol | D | C | B |
| | MS: 462.1 (M+H⁺) | 1H NMR (500 MHz, DMSO-d6) ppm = 9.09 (s, 1H), 8.84 (d, J=2.9, 1H), 7.72 - 7.66 (m, 2H), 7.52 - 7.49 (m, 2H), 7.41 - 7.36 (m, 1H), 7.30 (d, J=2.9, 1H), 7.21 - 7.18 (m, 1H), 6.52 (d, J=4.5, 1H), 6.04 (d, J=4.5, 1H), 4.26 - 4.16 (m, 2H), 3.81 - 3.74 (m, 4H), 3.48 - 3.40 (m, 4H), 1.34 (t, J=6.9, 3H). | | | |
| **80** | 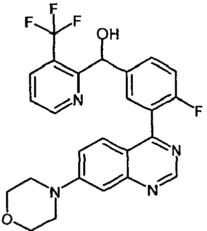 | [4-Fluoro-3-(7-morpholin-4-yl-quinazolin-4-yl)-phenyl]-(3-trifluoromethyl-pyridin-2-yl)-methanol | B | B | A |
| | MS: 485.2 (M+H⁺) | 1H NMR (500 MHz, DMSO-d6) ppm = 9.10 (s, 1H), 8.90 - 8.85 (m, 1H), 8.20 (dd, J=8.1, 1.6, 1H), 7.63 (dd, J=6.9, 2.3, 1H), 7.60 - 7.51 (m, 4H), 7.38 (dd, J=9.9, 8.6, 1H), 7.21 - 7.18 (m, 1H), 6.32 (d, J=6.3, 1H), 6.12 (d, J=6.0, 1H), 3.81 - 3.74 (m, 4H), 3.47 - 3.41 (m, 4H). | | | |
| **81** | 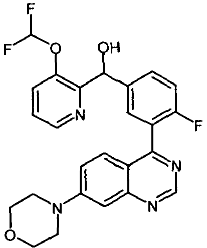 | (3-Difluoromethoxy-pyridin-2-yl)-[4-fluoro-3-(7-morpholin-4-yl-quinazolin-4-yl)-phenyl]-methanol | A | A | A |
| | MS: 483.2 (M+H⁺) | 1H NMR (500 MHz, DMSO-d6) ppm = 9.09 (s, 1H), 8.46 (dd, J=4.6, 1.3, 1H), 7.67 - 7.64 (m, 1H), 7.62 - 7.57 (m, 2H), 7.54 - 7.49 (m, 2H), 7.42 (dd, J=8.3, 4.6, 1H), 7.40 - 7.10 (m, 3H), 6.14 - 6.07 (m, 2H), 3.81 - 3.75 (m, 4H), 3.47 - 3.41 (m, 4H). | | | |
| **82** | 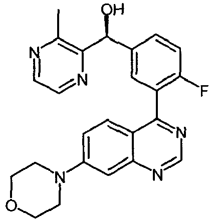 | (S)-[4-Fluoro-3-(7-morpholin-4-yl-quinazolin-4-yl)-phenyl]-(3-methyl-pyrazin-2-yl)-methanol | C | B | A |
| | MS: 432.2 (M+H⁺);); Rₜ 12.50min (SFC, ChiralPak AD-H, CO₂/ 25 vol.% 2-Propanol, 0.5 vol.% Diethylamin) | 1H NMR (500 MHz, DMSO-d6) ppm = 9.10 (s, 1H), 8.44 (s, 2H), 7.64 - 7.51 (m, 4H), 7.44 - 7.35 (m, 1H), 7.20 (s, 1H), 6.31 (d, J=5.5, 1H), 6.09 (d, J=5.4, 1H), 3.81 - 3.74 (m, 4H), 3.48 - 3.40 (m, 4H), 2.56 (s, 3H). | | | |
| **83** | 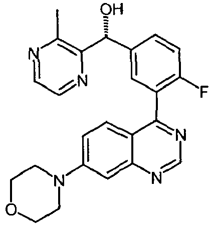 | (R)-[4-Fluoro-3-(7-morpholin-4-yl-quinazolin-4-yl)-phenyl]-(3-methyl-pyrazin-2-yl)-methanol | A | A | A |
| | MS: 432.1 (M+H⁺); Rₜ 19.51min (SFC, ChiralPak | 1H NMR (400 MHz, DMSO-d6) ppm = 9.09 (s, 1H), 8.43 (s, 2H), 7.63 - 7.49 (m, 4H), 7.43 - 7.34 (m, | | | |
| | AD-H, CO₂/ 25 vol.% 2-Propanol, 0.5 vol.% Diethylamin) | 1H), 7.19 (s, 1H), 6.28 (d, J=5.5, 1H), 6.08 (d, J=5.4, 1H), 3.82 - 3.73 (m, 4H), 3.48 - 3.40 (m, 4H), 2.55 (s, 3H). | | | |
| **84** | 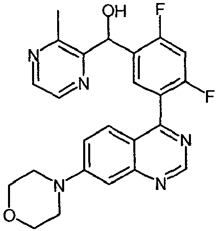 | [2,4-Difluoro-5-(7-morpholin-4-yl-quinazolin-4-yl)-phenyl]-(3-methyl-pyrazin-2-yl)-methanol | B | A | B |
| | MS: 450.2 (M+H⁺) | 1H NMR (500 MHz, DMSO-d6) ppm = 9.12 (s, 1H), 8.45 (d, J=2.5, 1H), 8.39 (dd, J=2.5, 0.8, 1H), 7.89 (t, J=8.2, 1H), 7.61 (dd, J=9.4, 3.2, 1H), 7.56 (dd, J=9.4, 2.5, 1H), 7.40 (t, J=10.1, 1H), 7.21 (d, J=2.4, 1H), 6.39 - 6.35 (m, 1H), 6.27 (s, 1H), 3.81 - 3.75 (m, 4H), 3.49 - 3.43 (m, 4H), 2.67 (s, 3H). | | | |
| **85** | 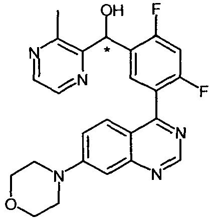 | [2,4-Difluoro-5-(7-morpholin-4-yl-quinazolin-4-yl)-phenyl]-(3-methyl-pyrazin-2-yl)-methanol (*Ena 1*) | A | B | B |
| | MS: 450.2 (M+H⁺) ; Rₜ 3.45min (SFC, ChiralPak AD-H, CO₂/ 30 vol.% Methanol, 0.5 vol.% Diethylamin) | s. Racemat | | | |
| **86** | 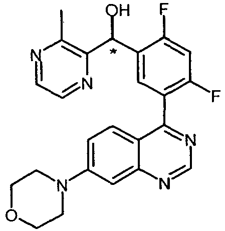 | [2,4-Difluoro-5-(7-morpholin-4-yl-quinazolin-4-yl)-phenyl]-(3-methyl-pyrazin-2-yl)-methanol (*Ena 2*) | A | A | A |
| | MS: 450.2 (M+H⁺) ; Rₜ 5.60min (SFC, ChiralPak AD-H, CO₂/ 25 vol.% Methanol, 0.5 vol.% Diethylamin) | s. Racemat | | | |
| **87** | 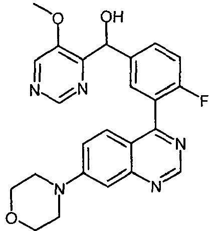 | [4-Fluoro-3-(7-morpholin-4-yl-quinazolin-4-yl)-phenyl]-(5-methoxy-pyrimidin-4-yl)-methanol | A | A | B |
| | MS: 448.1 (M+H⁺) | 1H NMR (400 MHz, DMSO-d6) ppm = 9.11 (s, 1H), 8.82 (s, 1H), 8.58 (s, 1H), 7.67 - 7.61 (m, 2H), 7.55 - 7.52 (m, 2H), 7.41 - 7.34 (m, 1H), 7.23 - 7.19 (m, 1H), 6.13 (d, J=6.1, 1H), 6.07 (d, J=6.2, 1H), 3.95 (s, 3H), 3.83 - 3.76 (m, 4H), 3.49 - 3.42 (m, 4H). | | | |
| **88** | 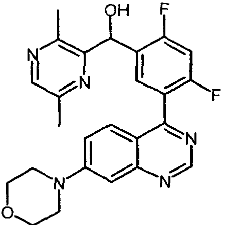 | [2,4-Difluoro-5-(7-morpholin-4-yl-quinazolin-4-yl)-phenyl]-(3,6-dimethyl-pyrazin-2-yl)-methanol | A | A | B |
| | MS: 464.2 (M+H⁺) | 1H NMR (400 MHz, DMSO-d6) ppm = 9.13 (s, 1H), 8.35 (s, 1H), 7.88 (t, J=8.2, 1H), 7.61 (dd, J=9.4, 3.0, 1H), 7.56 (dd, J=9.5, 2.5, 1H), 7.42 (t, J=10.2, 1H), 7.23 (d, J=2.4, 1H), 6.31 (d, J=5.9, 1H), 6.22 (d, J=5.6, 1H), 3.85 - 3.75 (m, 4H), 3.51 - 3.43 (m, 4H), 2.58 (s, 3H), 2.40 (s, 3H). | | | |
| **89** | 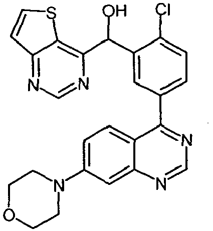 | [2-Chloro-5-(7-morpholin-4-yl-quinazolin-4-yl)-phenyl]-thieno[3,2-d]pyrimidin-4-yl-methanol | B | A | C |
| | MS: 490.1/492.1 (M+H⁺) (Cl Isotopie, rel. Peakintensitäts-Verhältnis [%] 100:43) | 1H NMR (500 MHz, DMSO-d6) ppm = 9.06 (s, 1H), 9.04 (s, 1H), 8.47 (d, J=5.6, 1H), 7.89 (d, J=2.1, 1H), 7.76 (d, J=9.4, 1H), 7.73 (dd, J=8.2, 2.2, 1H), 7.69 (d, J=8.2, 1H), 7.62 (d, J=5.6, 1H), 7.47 (dd, J=9.5, 2.6, 1H), 7.20 - 7.16 (m, 2H), 6.47 (d, J=4.9, 1H), 3.81 - 3.74 (m, 4H), 3.47 - 3.39 (m, 4H). | | | |
| **90** | 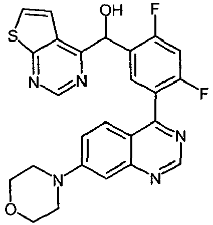 | [2,4-Difluoro-5-(7-morpholin-4-yl-quinazolin-4-yl)-phenyl]-thieno[2,3-d]pyrimidin-4-yl-methanol | A | A | A |
| | MS: 492.2 (M+H⁺) | 1H NMR (500 MHz, DMSO-d6) ppm = 9.07 (s, 1H), 9.05 (s, 1H), 8.47 (d, J=5.6, 1H), 7.76 (t, J=8.0, 1H), 7.61 (d, J=5.6, 1H), 7.55 - 7.46 (m, 3H), 7.20 - 7.17 (m, 2H), 6.32 (d, J=4.7, 1H), 3.81 - 3.74 (m, 4H), 3.47 - 3.41 (m, 4H). | | | |
| **91** | 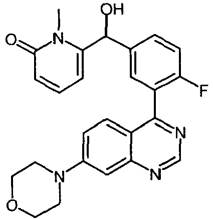 | 6-{[4-Fluoro-3-(7-morpholin-4-yl-quinazolin-4-yl)-phenyl]-hydroxy-methyl}-1-methyl-1H-pyridin-2-one | A | A | A |
| | MS: 447.2 (M+H⁺ ) | 1H NMR (500 MHz, DMSO-d6) ppm = 9.10 (s, 1H), 7.60 - 7.56 (m, 2H), 7.55 - 7.51 (m, 2H), 7.48 - 7.43 (m, 1H), 7.40 (dd, J=9.1, 6.9, 1H), 7.21 - 7.19 (m, 1H), 6.52 (d, J=5.3, 1H), 6.36 (dd, J=9.1, 1.4, 1H), 6.25 (dd, J=7.0, 1.4, 1H), 5.94 (d, J=5.1, 1H), 3.80 - 3.75 (m, 4H), 3.47 - 3.41 (m, 4H), 3.38 (s, 3H). | | | |

### BEISPIELE 92 und 93:

### 3-[[2-Chlor-4-fluor-5-(7-morpholin-chinazolin-4-yl)phenyl]-hydroxy-methyl]-1H-pyridazin-6-on (92)

### 6-{[2-Chlor-4-fluor-5-(7-morpholin-4-yl-chinazolin-4-yl)-phenyl]-hydroxy-methyl}-2-ethyl-2H-pyridazin-3-on (93)

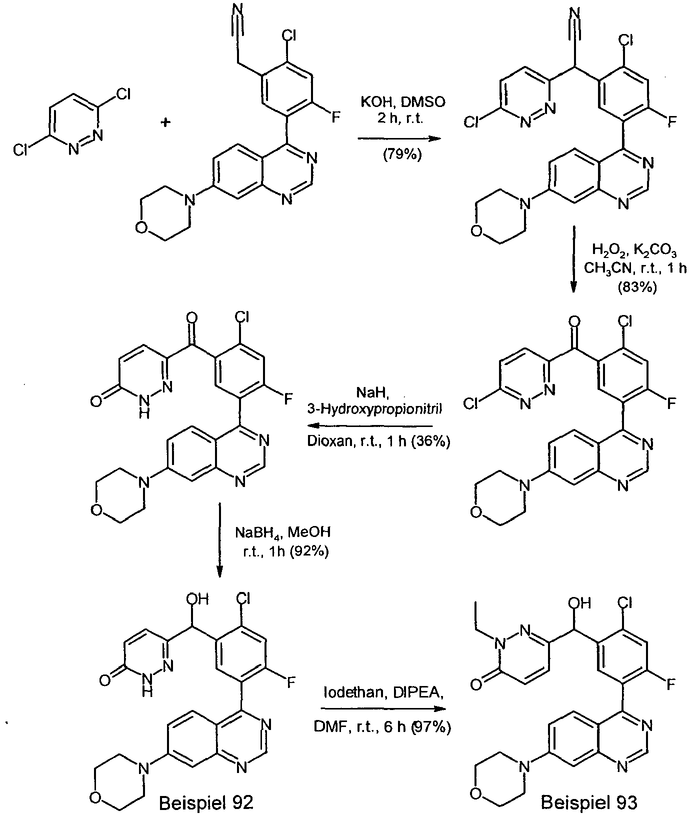

[2-Chlor-4-fluor-5-(7-morpholin-chinazolin-4-yl)phenyl]-(6-chlorpyridazin-3-yl)methanon, ausgehend von 2,6-Dichlorpyridazin und 2-[2-Chlor-4-fluor-5-(7-morpholin-chinazolin-4-yl)phenyl]acetonitril, sowie 3-[[2-Chlor-4-fluor-5-(7-morpholin-chinazolin-4-yl)phenyl]-hydroxy-methyl]-1H-pyridazin-6-on (BEISPIEL 92) wurden in analoger Weise zu den unter den BEISPIELEN 1 und 2 beschriebenen Syntheseverfahren hergestellt.

### Herstellung von 3-[2-Chlor-4-fluor-5-(7-morpholin-chinazolin-4-yl)benzoyl]-1H-pyridazin-6-on aus [2-Chlor-4-fluor-5-(7-morpholin-chinazolin-4-yl)phenyl]-(6-chlorpyridazin-3-yl)methanon:

[2-Chlor-4-fluor-5-(7-morpholin-chinazolin-4-yl)phenyl]-(6-chlorpyridazin-3-yl)methanon (2,0 g, 4,13 mmol) wurde in 1,4-Dioxan (80 ml, max. 0,005% Wasser) unter einer Argonatmosphäre gelöst. Anschließend wurden 3-Hydroxypropionitril (570 µl ml, 8,27 mmol) und Natriumhydrid (60% Dispersion in Paraffinöl) (215 mg; 5,37 mmol) zugefügt (Gasentwicklung). Die Reaktionsmischung wurde 2 h bei Raumtemperatur gerührt. Nach Beendigung der Reaktion wurde vorsichtig mit Wasser (100 ml) verdünnt und mit Salzsäure (1,0 M) neutralisiert. Die wässrige Phase wurde anschließend zweimal mit Essigsäureethylester (je 200 ml) extrahiert. Die vereinigten organischen Phasen wurden mit gesättigter Natriumchlorid-Lösung gewaschen, anschießend über Natriumsulfat getrocknet, filtriert und am Rotationsverdampfer zur Trockne eingeengt. Der Rückstand wurde mittels Flash-Säulenchromatographie aufgereinigt (Dichlormethan / 0-10 vol.% Ethanol, CombiFlash Rf 200), wobei 3-[2-Chlor-4-fluor-5-(7-morpholin-chinazolin-4-yl)benzoyl]-1H-pyridazin-6-on (695 mg, 1,47 mmol, MS: 466,1/468,1 [M+H⁺]), 36% Ausbeute) als Feststoff erhalten wurde.

### Herstellung von 6-{[2-Chlor-4-fluor-5-(7-morpholin-4-yl-chinazolin-4-yl)-phenyl]-hydroxy-methyl}-2-ethyl-2H-pyridazin-3-on (BEISPIEL 93) aus 3-[[2-Chlor-4-fluor-5-(7-morpholin-chinazolin-4-yl)phenyl]-hydroxy-methyl]-1H-pyridazin-6-on (BEISPIEL 92):

3-[[2-Chlor-4-fluor-5-(7-morpholin-chinazolin-4-yl)phenyl]-hydroxy-methyl]-1H-pyridazin-6-on (150 mg; 0,316 mmol) wurde in N,N-Dimethylformamid (5,0 ml) gelöst. Anschließend wurden lodethan (52 µl, 0,632 mmol) und Kaliumcarbonat (132 mg, 0,947 mmol) zugefügt. Die Reaktionsmischung wurde 6 h bei Raumtemperatur gerührt. Nach Beendigung der Reaktion wurde auf Wasser (100 ml) abdekantiert. Die wässrige Phase wurde anschließend zweimal mit Essigsäureethylester (je 100 ml) extrahiert. Die vereinigten organischen Phasen wurden mit Wasser (40 ml) nachgewaschen, anschießend über Natriumsulfat getrocknet, filtriert uns im Vakuum zur Trockne eingeengt. Der Rückstand wurde in Aceton suspendiert und abgesaugt. Der Filterkuchen wurde im Hochvakuum bei Raumtemperatur getrocknet, wobei 6-{[2-Chlor-4-fluor-5-(7-morpholin-4-yl-chinazolin-4-yl)-phenyl]-hydroxy-methyl}-2-ethyl-2H-pyridazin-3-on (BEISPIEL 93, 157 mg, 0,31 mmol, MS: 496.1/498.1 [M+H⁺], 97 % Ausbeute) als Feststoff erhalten wurde.

Verbindungen die entsprechend BEISPIEL 93 hergestellt wurden, finden sich in der nachfolgenden Tabelle 2.

**Tabelle 2 Verbindungen der Formel (I)**

| Nr. | Strukturformel | Name | IC₅₀ DNA-PK | IC₅₀ pDNA-PK | Kᵢ [Kv1.11 hERG] [ |
|---|---|---|---|---|---|
| **92** | 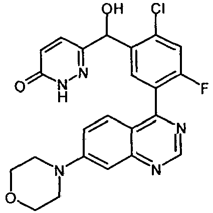 | 3-[[2-Chloro-4-fluoro-5-(7-morpholino-quinazolin-4-yl)phenyl]-hydroxy-methyl]-1H-pyridazin-6-one | A | A | A |
| | MS: 468.1/470.0 (M+H⁺) (Cl Isotopie, rel. Peakintensitäts-Verhältnis [%] 100:38) | 1H NMR (400 MHz, DMSO-d6) ppm = 12.89 (d, J=2.4, 1H), 9.12 (s, 1H), 7.92 (d, J=7.7, 1H), 7.68 (d, J=9.5, 1H), 7.59 - 7.55 (m, 2H), 7.53 (d, J=9.8, 1H), 7.21 (d, J=2.2, 1H), 6.90 (dd, J=9.8, 2.3, 1H), 6.61 (d, J=5.1, 1H), 5.89 (d, J=5.1, 1H), 3.81 - 3.76 (m, 4H), 3.48 - 3.44 (m, 4H) | | | |
| **93** | 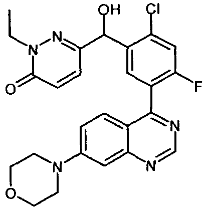 | 6-{[2-Chloro-4-fluoro-5-(7-morpholin-4-yl-quinazolin-4-yl)-phenyl]-hydroxy-methyl}-2-ethyl-2H-pyridazin-3-one | B | A | C |
| | MS: 496.1/498.1 (M+H⁺) (Cl Isotopie, rel. Peakintensitäts-Verhältnis [%] 100:41) | 1H NMR (500 MHz, DMSO-d6) ppm = 9.13 (s, 1H), 7.94 (d, J=7.7, 1H), 7.68 (d, J=9.5, 1H), 7.59 - 7.52 (m, 2H), 7.46 (d, J=9.6, 1H), 7.22 (d, J=2.3, 1H), 6.92 (d, J=9.6, 1H), 6.61 (d, J=4.8, 1H), 5.90 (d, J=4.8, 1H), 4.08 - 3.95 (m, 2H), 3.81 - 3.74 (m, 4H), 3.49 - 3.41 (m, 4H), 1.20 (t, J=7.2, 3H) | | | |
| **94** | 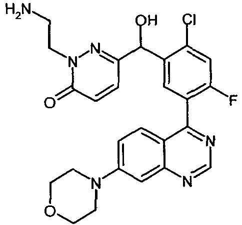 | 2-(2-Amino-ethyl)-6-{[2-chloro-4-fluoro-5-(7-morpholin-4-yl-quinazolin-4-yl)-phenyl]-hydroxy-methyl}-2H-pyridazin-3-one | C | D | A |
| | MS: 511.1/513.2 (M+H⁺) (Cl Isotopie, rel. Peakintensitäts-Verhältnis [%] 100:38) | 1H NMR (500 MHz, DMSO-d6) ppm = 9.13 (s, 1H), 7.94 (d, J=7.7, 1H), 7.68 (d, J=9.5, 1H), 7.60 - 7.52 (m, 2H), 7.48 (d, J=9.7, 1H), 7.21 (d, J=2.2, 1H), 6.94 (d, J=9.6, 1H), 6.72 - 6.57 (m, 1H), 5.91 (s, 1H), 4.10 - 3.96 (m, 2H), 3.82 - 3.73 (m, 4H), 3.48 - 3.43 (m, 4H), 2.88 (t, J=6.6, 2H) | | | |
| **95** | 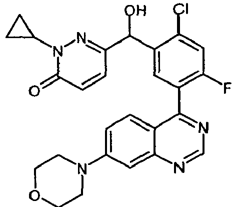 | 6-{[2-Chloro-4-fluoro-5-(7-morpholin-4-yl-quinazolin-4-yl)-phenyl]-hydroxy-methyl}-2-cyclopropyl-2H-pyridazin-3-one | C | B | A |
| | MS: 508.1/510.1 (M+H⁺) (Cl Isotopie, rel. Peakintensitäts-Verhältnis [%] 100:38) | 1H NMR (400 MHz, Methylenchlorid-d2) ppm = 9.04 (s, 1H), 7.71 (d, J=7.6, 1H), 7.49 (dd, J=9.3, 3.5, 1H), 7.26 - 7.20 (m, 2H), 7.18 - 7.14 (m, 2H), 6.74 (d, J=9.6, 1H), 5.97 (s, 1H), 4.03 - 3.94 (m, 1H), 3.82 - 3.74 (m, 4H), 3.39 - 3.31 (m, 4H), 0.98 - 0.82 (m, 4H) | | | |
| **96** | 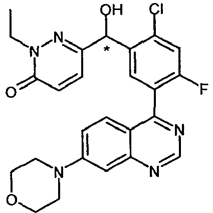 | 6{[2-Chloro-4-fluoro-5-(7-morpholin-4-yl-quinazolin-4-yl)-phenyl]-hydroxy-methyl}-2-ethyl-2H-pyridazin-3-one (*Ena 2*) | C | B | A |
| | MS: 496.2/498.1 (M+H⁺) (Cl Isotopie, rel. Peakintensitäts-Verhältnis [%] 100:34); Rₜ 4.59min (SFC, Chiracel OJ-H, CO₂/ 20 vol.% Methanol, 0.5 vol.% Diethylamin) | s. Racemat | | | |
| **97** | 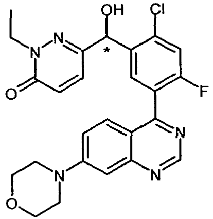 | 6-{[2-Chloro-4-fluoro-5-(7-morpholin-4-yl-quinazolin-4-yl)-phenyl]-hydroxy-methyl}-2-ethyl-2H-pyridazin-3-one (*Ena 1*) | A | B | A |
| | MS: 496.2/498.1 (M+H⁺) (Cl Isotopie, rel. Peakintensitäts-Verhältnis [%] 100:36); Rₜ 3.00min (SFC, Chiracel OJ-H, CO₂/ 20 vol.% Methanol, 0.5 vol.% Diethylamin) | s. Racemat | | | |
| **98** | 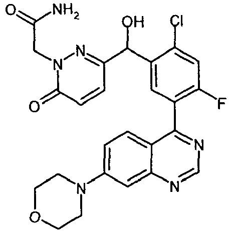 | 2-(3-{[2-Chloro-4-fluoro-5-(7-morpholin-4-yl-quinazolin-4-yl)-phenyl]-hydroxy-methyl}-6-oxo-6H-pyridazin-1-yl)-acetamide | B | D | A |
| | MS: 525.1/527.1 (M+H⁺) (Cl Isotopie, rel. Peakintensitäts-Verhältnis [%] 100:36) | 1H NMR (500 MHz, DMSO-d6) ppm = 9.12 (s, 1H), 7.93 (d, J=7.7, 1H), 7.67 (d, J=9.5, 1H), 7.58 (dd, J=9.4, 3.0, 1H), 7.53 (dd, J=9.5, 2.5, 1H), 7.51 - 7.44 (m, 2H), 7.21 (d, J=2.4, 1H), 7.15 (s, 1H), 6.94 (d, J=9.6, 1H), 6.62 (d, J=4.8, 1H), 5.89 (d, J=4.8, 1H), 4.61 - 4.51 (m, 2H), 3.81 - 3.75 (m, 4H), 3.48 - 3.42 (m, 4H) | | | |
| **99** | 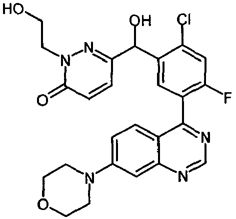 | 6-{[2-Chloro-4-fluoro-5-(7-morpholin-4-yl-quinazolin-4-yl)-phenyl]-hydroxy-methyl}-2-(2-hydroxy-ethyl)-2H-pyridazin-3-one | B | B | A |
| | MS: 512.2/514.2 (M+H⁺) (Cl Isotopie, rel. Peakintensitäts-Verhältnis [%] 100:38) | 1H NMR (500 MHz, DMSO-d6) ppm = 9.13 (s, 1H), 7.94 (d, J=7.7, 1H), 7.68 (d, J=9.5, 1H), 7.60 - 7.52 (m, 2H), 7.46 (d, J=9.6, 1H), 7.22 (d, J=2.3, 1H), 6.92 (d, J=9.6, 1H), 6.61 (d, J=4.8, 1H), 5.90 (d, J=4.8, 1H), 4.75 (t, J=5.8, 1H), 4.11 - 4.00 (m, 2H), 3.82 - 3.74 (m, 4H), 3.65 (q, J=6.2, 2H), 3.49 - 3.42 (m, 4H) | | | |

### BEISPIEL 100:

### [2-Chlor-4-fluor-5-(7-morpholin-4-yl-chinazolin-4-yl)-phenyl]-[6-(oxetan-3-yloxy)-pyridazin-3-yl]-methanol (100)

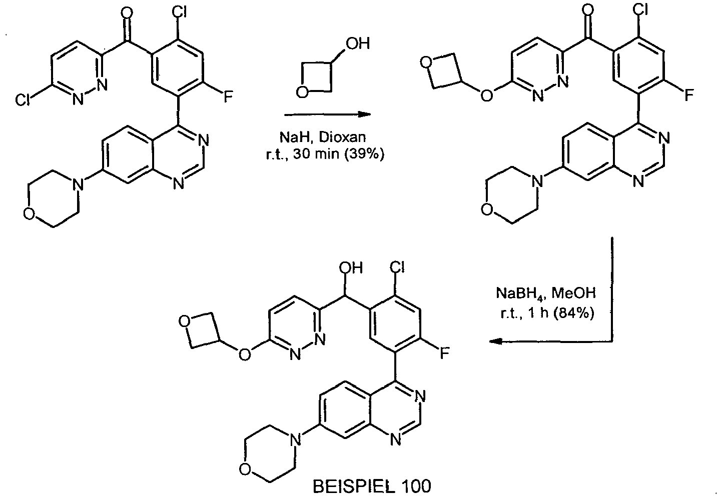

[2-Chlor-4-fluor-5-(7-morpholin-4-yl-chinazolin-4-yl)-phenyl]-(6-chlorpyridazin-3-yl)-methanon (700 mg, 1,30 mmol) und Oxetan-3-ol(112 mg, 1,43 mmol) wurden in 1,4-Dioxan (25 ml, max. 0,005% Wasser) unter einer Argonatmoshäre gelöst vorgelegt. Anschließend wurde Natriumhydrid (60% Dispersion in Paraffinöl, 62 mg, 1,56 mmol) zugefügt (Gasentwicklung).

Die Reaktionsmischung wurde bei Raumtemperatur 30 min gerührt. Nach Beendigung der Reaktion wurde vorsichtig mit Wasser (80 ml) verdünnt und mit Salzsäure (1,0 M) neutralisiert. Die wässrige Phase wurde anschließend zweimal mit Essigsäureethylester (je 80 ml) extrahiert. Die vereinigten organischen Phasen wurden mit Wasser (20 ml) nachgewaschen, anschießend über Natriumsulfat getrocknet, filtriert und am Rotationsverdampfer zur Trockne eingeengt. Der Rückstand wurde mittels Flash-Säulenchromatographie aufgereinigt (Dichlormethan / 0-10 vol.% Ethanol, CombiFlash Rf 200), wobei [2-Chlor-4-fluor-5-(7-morpholin-4-yl-chinazolin-4-yl)phenyl]-[6-(oxetan-3-yloxy)pyridazin-3-yl]methanon (264 mg, 0,506 mmol, 522,2 [M+H⁺]), 39% Ausbeute) als Feststoff erhalten wurde.

[2-Chlor-4-fluor-5-(7-morpholin-4-yl-chinazolin-4-yl)-phenyl]-[6-(oxetan-3-yloxy)-pyridazin-3-yl]-methanol (BEISPIEL 100) wurde ausgehend von [2-Chlor-4-fluor-5-(7-morpholin-4-yl-chinazolin-4-yl)phenyl]-[6-(oxetan-3-yloxy)pyridazin-3-yl]methanon in analoger Weise zu dem unter den BEISPIELEN 1 und 2 beschriebenen Syntheseverfahren hergestellt.

Verbindungen die entsprechend BEISPIEL 100 hergestellt wurden, finden sich in der nachfolgenden Tabelle 3.

**Tabelle 3: Verbindungen der Formel (I)**

| Nr. | Strukturformel | Name | IC₅₀ DNA-PK | IC₅₀ pDNA-PK | Kᵢ [Kv1.11 hERG] |
|---|---|---|---|---|---|
| **100** | 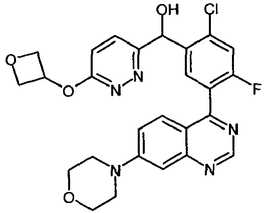 | [2-Chloro-4-fluoro-5-(7-morpholin-4-yl-quinazolin-4-yl)-phenyl]-[6-(oxetan-3-yloxy)-pyridazin-3-yl]-methanol | A | A | C |
| | MS: 524.2/526.1 (M+H⁺) (Cl Isotopie, rel. Peakintensitäts-Verhältnis [%] 100:39) | 1H NMR (500 MHz, DMSO-d6) ppm = 9.11 (s, 1H), 7.91 (d, J=7.7, 1H), 7.77 (d, J=9.2, 1H), 7.66 (d, J=9.4, 1H), 7.61 - 7.51 (m, 2H), 7.32 (d, J=9.2, 1H), 7.21 (d, J=2.3, 1H), 6.63 (d, J=4.8, 1H), 6.22 (d, J=4.8, 1H), 5.69 (p, J=5.7, 1H), 4.94 - 4.87 (m, 2H), 4.62 - 4.56 (m, 2H), 3.80 - 3.76 (m, 4H), 3.48 - 3.43 (m, 4H) | | | |
| **101** | 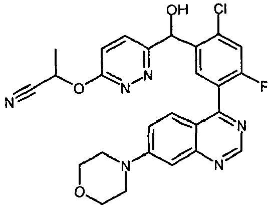 | 2-(6-{[2-Chloro-4-fluoro-5-(7-morpholin-4-yl-quinazolin-4-yl)-phenyl]-hydroxy-methyl}-pyridazin-3-yloxy)-propionitrile | A | A | C |
| | MS: 521.2/523.2 (M+H⁺) (Cl Isotopie, rel. Peakintensitäts-Verhältnis [%] 100:39) | 1H NMR (500 MHz, DMSO-d6) ppm = 9.12 (d, J=2.7, 1H), 7.93 (dd, J=11.2, 7.7, 1H), 7.84 (dd, J=16.3, 9.2, 1H), 7.70 - 7.65 (m, 1H), 7.63 - 7.57 (m, 1H), 7.57 - 7.51 (m, 1H), 7.38 (dd, J=9.1, 3.5, 1H), 7.21 (d, J=2.4, 1H), 6.71 (dd, J=10.3, 5.0, 1H), 6.28 (dd, J=9.5, 5.0, 1H), 5.95 - 5.87 (m, 1H), 3.81 - 3.74 (m, 4H), 3.48 - 3.42 (m, 4H), 1.76 - 1.70 (m, 3H) | | | |
| **102** | 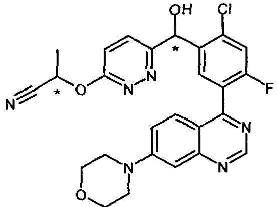 | 2-(6-{[2-Chloro-4-fluoro-5-(7-morpholin-4-yl-quinazolin-4-yl)-phenyl]-hydroxy-methyl}-pyridazin-3-yloxy)-propionitrile (*eluate 1*) | A | A | B |
| | MS: 543.0/545.0 (M+Na⁺) (Cl Isotopie, rel. Peakintensitäts-Verhältnis [%] 100:35); Rₜ 4.09min (SFC, Chiralpak AS-H, CO₂/20 vol.% Methanol, 0.5 vol.% Diethylamin) | s.a. Diastereomerengemisch | | | |
| **103** | 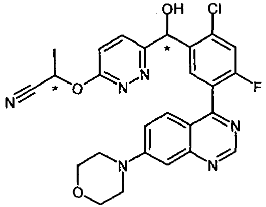 | 2-(6-{[2-Chloro-4-fluoro-5-(7-morpholin-4-yl-quinazolin-4-yl)-phenyl]-hydroxy-methyl}-pyridazin-3-yloxy)-propionitrile (*eluate 3*) | D | D | A |
| | MS: 521.1/523.1 (M+H⁺) (Cl Isotopie, rel. Peakintensitäts-Verhältnis [%] 100:38) ; Rₜ 6.68min (SFC, Chiralpak AS-H, CO₂/20 vol.% Methanol, 0.5 vol.% Diethylamin) | s.a. Diastereomerengemisch | | | |
| **104** | 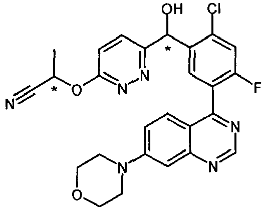 | 2-(6-{[2-Chloro-4-fluoro-5-(7-morpholin-4-yl-quinazolin-4-yl)-phenyl]-hydroxy-methyl}-pyridazin-3-yloxy)-propionitrile (*eluate* 2) | C | B | B |
| | MS: 521.1/523.1 (M+H⁺) (Cl Isotopie, rel. Peakintensitäts-Verhältnis [%] 100:35); Rₜ | s.a. Diastereomerengemisch | | | |
| | 5.12min (SFC, Chiralpak AS-H, CO₂/ 20 vol.% Methanol, 0.5 vol.% Diethylamin) | | | | |
| **105** | 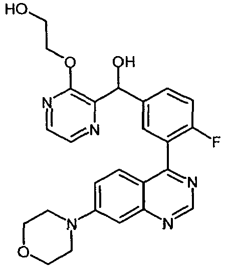 | 2-(3-{[4-Fluoro-3-(7-morpholin-4-yl-quinazolin-4-yl)-phenyl]-hydroxy-methyl}-pyrazin-2-yloxy)-ethanol | C | D | A |
| | MS: 478.2 (M+H⁺) | 1H NMR (500 MHz, DMSO-d6) ppm = 9.09 (s, 1H), 8.19 (d, J=2.8, 1H), 8.12 (d, J=2.8, 1H), 7.70 - 7.64 (m, 2H), 7.54 - 7.51 (m, 2H), 7.38 - 7.33 (m, 1H), 7.21 - 7.19 (m, 1H), 6.14 (d, J=5.9, 1H), 6.00 (d, J=6.0, 1H), 4.86 (t, J=5.7, 1H), 4.34 - 4.30 (m, 2H), 3.80 - 3.75 (m, 4H), 3.74 - 3.69 (m, 2H), 3.46 - 3.42 (m, 4H) | | | |
| **106** | 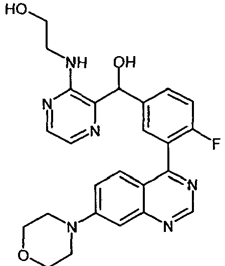 | 2-(3-{[4-Fluoro-3-(7-morpholin-4-yl-quinazolin-4-yl)-phenyl]-hydroxy-methyl}-pyrazin-2-ylamino)-ethanol | C | C | B |
| | MS: 477.1 (M+H⁺) | 1H NMR (500 MHz, DMSO-d6) ppm = 9.09 (s, 1H), 7.91 (d, J=2.7, 1H), 7.67 (d, J=2.8, 1H), 7.63 - 7.58 (m, 2H), 7.55 - 7.49 (m, 2H), 7.41 - 7.35 (m, 1H), 7.20 (d, J=2.0, 1H), 6.82 - 6.75 (m, 2H), 5.91 (d, J=4.2, 1H), 4.74 (t, J=5.1, 1H), 3.82 - 3.73 (m, 4H), 3.60 - 3.45 (m, 8H) | | | |
| **107** | 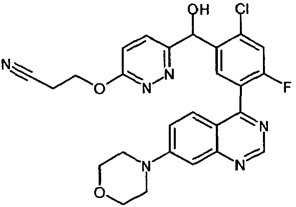 | 3-(6-{[2-Chloro-4-fluoro-5-(7-morpholin-4-yl-quinazolin-4-yl)-phenyl]-hydroxy-methyl}-pyridazin-3-yloxy)-propionitrile | B | B | A |
| | MS: 521.2/523.2 (M+H⁺) (Cl Isotopie, rel. Peakintensitäts-Verhältnis [%] 100:39) | 1H NMR (500 MHz, DMSO-d6) ppm = 9.12 (s, 1H), 7.95 (d, J=7.7, 1H), 7.67 (d, J=9.5, 1H), 7.59 (dd, J=9.4, 3.2, 1H), 7.55 - 7.49 (m, 2H), 7.21 (d, J=2.4, 1H), 6.99 (d, J=9.6, 1H), 6.67 (d, J=4.9, 1H), 5.92 (d, J=4.9, 1H), 4.31 -4.17 (m, 2H), 3.81 - 3.75 (m, 4H), 3.49 - 3.42 (m, 4H), 2.99 - 2.88 (m, 2H) | | | |
| **108** | 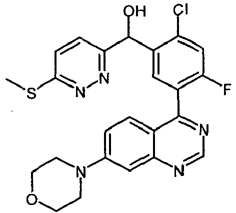 | [2-Chloro-4-fluoro-5-(7-morpholin-4-yl-quinazolin-4-yl)-phenyl]-(6-methylsulfanyl-pyridazi n-3-yl)-methanol | B | A | B |
| | MS: 498.1/500.1 (M+H⁺) (Cl Isotopie, rel. Peakintensitäts-Verhältnis [%] 100:47) | 1H NMR (500 MHz, DMSO-d6) ppm = 9.12 (s, 1H), 7.90 (d, J=7.7, 1H), 7.68 (d, J=9.5, 1H), 7.66 - 7.60 (m, 2H), 7.60 - 7.56 (m, 1H), 7.54 (dd, J=9.4, 2.5, 1H), 7.21 (d, J=2.4, 1H), 6.68 (s, 1H), 6.25 (s, 1H), 3.80 - 3.76 (m, 4H), 3.47 - 3.43 (m, 4H), 2.61 (s, 3H) | | | |
| **109** | 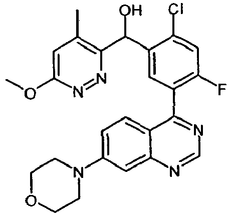 | [2-Chloro-4-fluoro-5-(7-morpholin-4-yl-quinazolin-4-yl)-phenyl]-(6-methoxy-4-methyl-pyridazin-3-yl)-methanol | D | C | A |
| | MS: 518.2/520.2 (M+Na⁺) (Cl Isotopie, rel. Peakintensitäts-Verhältnis [%] 100:38) | 1H NMR (500 MHz, DMSO-d6) ppm = 9.14 (s, 1H), 7.99 (d, J=7.8, 1H), 7.67 - 7.61 (m, 2H), 7.57 (dd, J=9.4, 2.6, 1H), 7.23 (d, J=2.5, 1H), 7.10 (d, J=1.1, 1H), 6.42 (d, J=6.4, 1H), 6.25 (d, J=6.3, 1H), 3.96 (s, 3H), 3.82 - 3.75 (m, 4H), 3.50 - 3.42 (m, 4H), 2.49 - 2.46 (m, 3H) | | | |
| **110** | 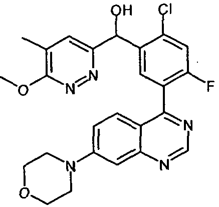 | [2-Chloro-4-fluoro-5-(7-morpholin-4-yl-quinazolin-4-yl)-phenyl]-(6-methoxy-5-methyl-pyridazin-3-yl)-methanol | C | B | C |
| | MS: 496.1/498.1 (M+H⁺) (CI Isotopie, rel. Peakintensitäts-Verhältnis [%] 100:38) | 1H NMR (500 MHz, DMSO-d6) ppm = 9.12 (s, 1H), 7.91 (d, J=7.8, 1H), 7.66 (d, J=9.6, 1H), 7.59 (dd, J=9.4, 3.3, 1H), 7.56 - 7.52 (m, 2H), 7.21 (d, J=2.4, 1H), 6.54 (d, J=4.9, 1H), 6.18 (d, J=4.9, 1H), 4.02 (s, 3H), 3.80 - 3.75 (m, 4H), 3.48 - 3.43 (m, 4H), 2.17 (d, J=1.0, 3H) | | | |
| **111** | 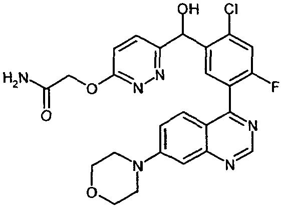 | 2-(6-{[2-Chloro-4-fluoro-5-(7-morpholin-4-yl-quinazolin-4-yl)-phenyl]-hydroxy-methyl}-pyridazin-3-yloxy)-acetamide | B | C | A |
| | MS: 525.2/527.2 (M+H⁺) (Cl Isotopie, rel. Peakintensitäts-Verhältnis [%] 100:34) | 1H NMR (500 MHz, DMSO-d6) ppm = 9.12 (s, 1H), 7.92 (d, J=7.7, 1H), 7.74 (d, J=9.1, 1H), 7.66 (d, J=9.5, 1H), 7.59 (dd, J=9.4, 3.3, 1H), 7.57 - 7.49 (m, 2H), 7.27 (d, J=9.1, 1H), 7.23 - 7.16 (m, 2H), 6.63 (d, J=5.0, 1H), 6.23 (d, J=5.0, 1H), 4.81 (s, 2H), 3.81 - 3.75 (m, 4H), 3.49 - 3.43 (m, 4H) | | | |
| **112** | 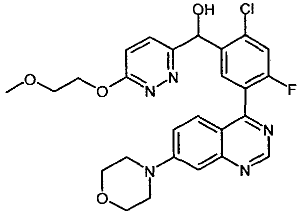 | [2-Ch loro-4-fluoro-5-(7-morpholin-4-yl-quinazolin-4-yl)-phenyl]-[6-(2-methoxy-ethoxy)-pyridazin-3-yl]-methanol | A | B | B |
| | MS: 526.2/528.3 (M+H⁺) (Cl Isotopie, rel. Peakintensitäts-Verhältnis [%] 100:38) | 1H NMR (500 MHz, DMSO-d6) ppm = 9.12 (s, 1H), 7.91 (d, J=7.7, 1H), 7.69 (d, J=9.2, 1H), 7.67 (d, J=9.5, 1H), 7.59 (dd, J=9.4, 3.3, 1H), 7.54 (dd, J=9.5, 2.5, 1H), 7.25 - 7.19 (m, 2H), 6.61 (d, J=5.0, 1H), 6.23 (d, J=5.0, 1H), 4.54 - 4.50 (m, 2H), 3.80 - 3.75 (m, 4H), 3.71 - 3.67 (m, 2H), 3.48 - 3.43 (m, 4H), 3.29 (s, 3H) | | | |
| **113** | 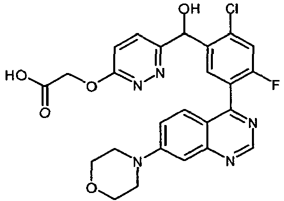 | (6-{[2-Chloro-4-fluoro-5-(7-morpholin-4-yl-quinazolin-4-yl)-phenyl]-hydroxy-methyl}-pyridazin-3-yloxy)-acetic acid | B | D | A |
| | MS: 526.2/528.1 (M+H⁺) (Cl Isotopie, rel. Peakintensitäts-Verhältnis [%] 100:39) | 1H NMR (500 MHz, DMSO-d6) ppm = 12.91 (s, 1H), 9.12 (s, 1H), 7.92 (d, J=7.7, 1H), 7.75 (d, J=9.1, 1H), 7.66 (d, J=9.5, 1H), 7.59 (dd, J=9.4, 3.3, 1H), 7.54 (dd, J=9.5, 2.5, 1H), 7.31 (d, J=9.1, 1H), 7.21 (d, J=2.4, 1H), 6.66 (s, 1H), 6.23 (s, 1H), 4.97 (s, 2H), 3.81 - 3.75 (m, 4H), 3.48 - 3.43 (m, 4H) | | | |
| **114** | 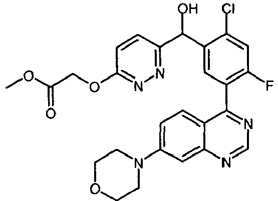 | (6-{[2-Chloro-4-fluoro-5-(7-morpholin-4-yl-quinazolin-4-yl)-phenyl]-hydroxy-methyl}-pyridazin-3-yloxy)-acetic acid methyl ester | B | D | A |
| | MS: 540.2/542.2 (M+H⁺) (Cl Isotopie, rel. Peakintensitäts-Verhältnis [%] 100:38) | 1H NMR (500 MHz, DMSO-d6) ppm = 9.12 (s, 1H), 7.92 (d, J=7.7, 1H), 7.77 (d, J=9.1, 1H), 7.67 (d, J=9.5, 1H), 7.59 (dd, J=9.3, 3.2, 1H), 7.57 - 7.51 (m, 1H), 7.35 (d, J=9.1, 1H), 7.23 - 7.19 (m, 1H), 6.62 (s, 1H), 6.23 (s, 1H), 5.07 (s, 2H), 3.81 - 3.75 (m, 4H), 3.67 (s, 3H), 3.49 - 3.44 (m, 4H) | | | |
| **115** | 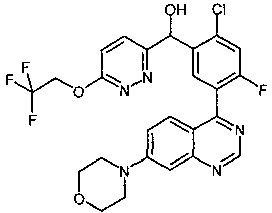 | [2-Chloro-4-fluoro-5-(7-morpholin-4-yl-quinazolin-4-yl)-phenyl]-[6-(2,2,2-trifluoro-ethoxy)-pyridazin-3-yl]-methanol | B | B | C |
| | MS: 550.2/552.1 (M+H⁺) (Cl Isotopie, rel. Peakintensitäts-Verhältnis [%] 100:40) | 1H NMR (500 MHz, DMSO-d6) ppm = 9.11 (s, 1H), 7.91 (d, J=7.7, 1H), 7.81 (d, J=9.2, 1H), 7.66 (d, J=9.5, 1H), 7.60 - 7.52 (m, 2H), 7.41 (d, J=9.1, 1H), 7.20 (d, J=2.3, 1H), 6.72 (d, J=5.0, 1H), 6.26 (d, J=4.5, 1H), 5.22 - 5.08 (m, 2H), 3.80 - 3.75 (m, 4H), 3.47 - 3.43 (m, 4H) | | | |
| **116** | 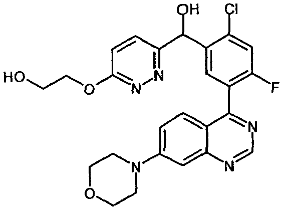 | 2-(6-{[2-Chloro-4-fluoro-5-(7-morpholin-4-yl-quinazolin-4-yl)-phenyl]-hydroxy-methyl}-pyridazin-3-yloxy)-ethanol | A | B | A |
| | MS: 512.2/514.1 (M+H⁺) (Cl Isotopie, rel. Peakintensitäts-Verhältnis [%] 100:38) | 1H NMR (500 MHz, DMSO-d6) ppm = 9.12 (s, 1H), 7.91 (d, J=7.7, 1H), 7.71 - 7.64 (m, 2H), 7.61 - 7.51 (m, 2H), 7.23 - 7.17 (m, 2H), 6.60 (d, J=5.0, 1H), 6.22 (d J=5.0, 1H), 4.84 (t, J=5.5, 1H), 4.44 - 4.39 (m, 2H), 3.81 - 3.76 (m, 4H), 3.74 (q, J=5.4, 2H), 3.48 - 3.43 4H) | | | |
| **117** | 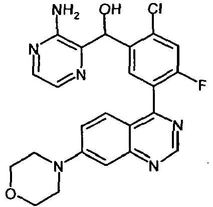 | (3-Amino-pyrazin-2-yl)-[2-chloro-4-fluoro-5-(7-morpholin-4-yl-quinazolin-4-yl)-phenyl]-methanol | B | B | B |
| | MS: 467.1/469.1 (M+H⁺) (Cl Isotopie, rel. Peakintensitäts-Verhältnis [%] 100:34) | 1H NMR (400 MHz, DMSO-d6) ppm = 9.14 (s, 1H), 7.91 (d, J=7.8, 1H), 7.87 (d, J=2.7, 1H), 7.67 - 7.55 (m, 4H), 7.21 (d, J=2.5, 1H), 6.41 (s, 3H), 6.09 (s, 1H), 3.81 - 3.75 (m, 4H), 3.50 - 3.45 (m, 4H) | | | |
| **118** | 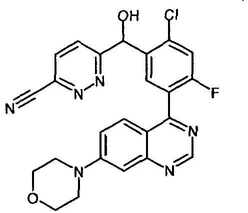 | 6-{[2-Chloro-4-fluoro-5-(7-morpholin-4-yl-quinazolin-4-yl)-phenyl]-hydroxy-methyl}-pyridazine-3-carbonitrile | B | B | B |
| | MS: 477.0/479.1 (M+H⁺) (Cl Isotopie, rel. Peakintensitäts-Verhältnis [%] 100:41) | 1H NMR (500 MHz, DMSO-d6) ppm = 9.11 (s, 1H), 8.36 (d, J=8.7, 1H), 8.13 (d, J=8.7, 1H), 7.86 (d, J=7.6, 1H), 7.71 (d, J=9.5, 1H), 7.55 (qd, J=9.4, 2.7, 2H), 7.21 (d, J=2.3, 1H), 6.96 (d, J=4.9, 1H), 6.43 (d, J=4.9, 1H), 3.80 - 3.76 (m, 4H), 3.48 - 3.43 (m, 4H) | | | |
| **119** | 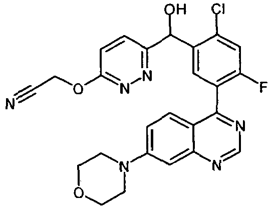 | (6-{[2-Chloro-4-fluoro-5-(7-morpholin-4-yl-quinazolin-4-yl)-phenyl]-hydroxy-methyl}-pyridazin-3-yloxy)-acetonitrile | A | A | B |
| | MS: 507.1/509.1 (M+H⁺) (Cl Isotopie, rel. Peakintensitäts-Verhältnis [%] 100:35) | 1H NMR (400 MHz, DMSO-d6) ppm = 9.12 (s, 1H), 7.93 (d, J=7.7, 1H), 7.85 (d, J=9.2, 1H), 7.67 (d, J=9.5, 1H), 7.60 (dd, J=9.4, 3.3, 1H), 7.54 (dd, J=9.4, 2.5, 1H), 7.40 (d, J=9.1, 1H), 7.21 (d, J=2.4, 1H), 6.72 (d, J=5.0, 1H), 6.28 (d, J=4.8, 1H), 5.38 (s, 2H), 3.81 - 3.75 (m, 4H), 3.48 - 3.42 (m, 4H) | | | |

### BEISPIELE 120, 121 und 122:

### [2,4-Difluor-5-(7-morpholin-4-yl-chinazolin-4-yl)-phenyl]-(4-methoxy-phenyl)-methanol (BEISPIEL 120)

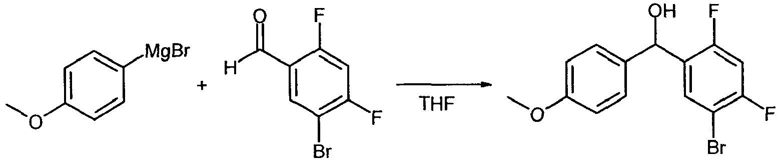

In einem ausgeheizten Dreihalskolben mit Innenthermometer, Schutzgaseinlass, Septum und Rührfisch wurde 5-Brom-2,4-difluor-benzaldehyd (280 mg, 1.27 mmol) in trockenen Tetrahydrofuran (10 ml) vorgelegt. 4-Methoxyphenylmagnesiumbromid (1 M in THF, 1.39 ml, 1.39 mmol) wurde bei 5°C langsam zugetropft und die Reaktionslösung 18 h bei Raumtemperatur gerührt. Anschließend wurde mit Wasser (20 ml) zur Reaktionslösung gegeben. Die Phasen wurden separiert und die wässrige Phase zweimal mit Essigsäureethylester (20 ml) extrahiert. Die vereinigten organischen Phasen wurden mit Wasser gewaschen, über Natriumsulfat getrocknet, filtriert und am Rotationsverdampfer zur Trocken eingengt. Man erhält (5-Brom-2,4-difluor-phenyl)-(4-methoxy-phenyl)-methanol (530 mg, 1.61 mmol, MS: 353 [M+H⁺]) als öliges Rohprodukt, das ohne weitere Reinigung für den nächsten Syntheseschritt verwendet wurde.

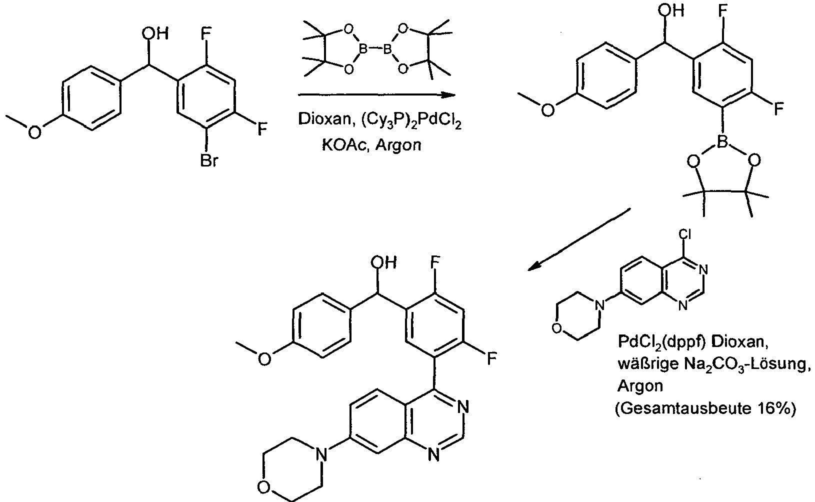

### BEISPIEL 120

Ausgehend von (5-Brom-2,4-difluor-phenyl)-(4-methoxy-phenyl)-methanol wurde [2,4-Difluor-5-(7-morpholin-4-yl-chinazolin-4-yl)-phenyl]-(4-methoxy-phenyl)-methanol (BEISPIEL 120) in analoger Weise zu den unter den BEISPIELEN 1 und 2 beschriebenen Syntheseverfahren hergestellt.

### (6-Difluoromethoxy-pyridazin-3-yl)-[4-fluor-3-(7-morpholin-4-yl-chinazolin-4-yl)-phenyl]-methanol (BEISPIEL 121)

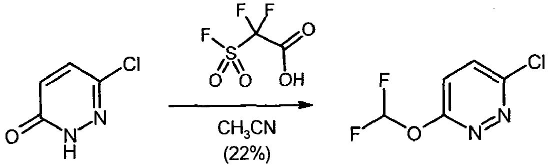

In einem Gefäß mit Rührfisch wurden 6-Chlor-2H-pyridazin-3-on (944 mg, 7,23 mmol) und Difluor-fluorsulfonyl-essigsäure (1,42 g, 7,96 mmol) in Acetonitril (19 ml) gelöst und für 40 h bei Raumtemperatur gerührt. Danach wurde die Reaktionslösung mit Essigsäureethylester (150 ml) verdünnt und nacheinander mit Wasser, gesättigter NatriumhydrogencarbonatLösung und wiederum mit Wasser gewaschen. Die organische Phase wurde mit Natriumsulfat getrocknet, filtriert und am Rotationsverdampfer zur Trockene eingeengt. Der Rückstand wurde in Cyclohexan aufgenommen, erneut filtriert und das Lösungsmittel am Rotationsverdampfer entfernt. Der erhaltene Rückstand wurde mittels Flash-Säulenchromatographie gereinigt (Gradient Cyclohexan/0-50vol.% Essigsäureethylester, CombiFlash Rf 200). Die geeigneten Produktfraktionen wurden vereinigt und die Lösungsmittel am Rotationsverdampfer entfernt. Man erhielt 3-Chlor-6-(difluormethoxy)pyridazin (285 mg, 1,58 mmol, MS: 181,0/183,1[M+H⁺]), 22% Ausbeute) als farblose Flüssigkeit.

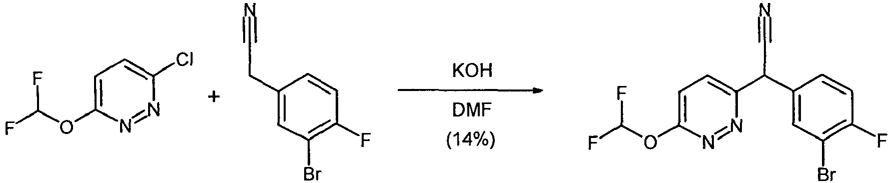

In einem Glasgefäß mit Rührfisch wurde Kaliumhydroxidpulver (603 mg, 10,75 mmol) in trockenen N,N-Dimethylformamid (2 ml) suspendiert und 30 min bei Raumtemperatur gerührt. Anschließend wurde (3-Brom-4-fluor-phenyl)-acetonitril (1,0 g, 4,67 mmol), gelöst in N,N'-Dimethylformamid (1,3 ml), tropfenweise zugegeben. Die Reaktionsmischung wurde weitere 30 min bei Raumtemperatur gerührt. Danach wurde (5-Brom-2,4-difluor-phenyl)-(4-methoxy-phenyl)-methanol (506 mg, 2,80 mmol) portionsweise zur Reaktionsmischung gegeben und 2 h unter einer Sauerstoff-freien Argonschutzgasatmosphäre bei 50°C gerührt. Die Reaktionsmischung wurde in ein Gemisch aus Wasser (50 ml) und gesättigter Natriumchloridlösung (35 ml) gegeben und zweimal mit Essigsäureethylester extrahiert. Die vereinigten organischen Phasen wurden über Natriumsulfat getrocknet, filtriert und am Rotationsverdampfer zur Trockene eingeengt. Der Rückstand wurde mittels RP-Säulenchromatographie gereinigt (Gradient Wasser/Acetonitril mit 0,1 vol.% Ameisensäure, CombiFlash Rf 200). Die geeigneten Produktfraktionen wurden vereinigt und die Lösungsmittel am Rotationsverdampfer entfernt. Man erhält (3-Brom-4-fluor-phenyl)-(6-difluormethoxy-pyridazin-3-yl)-acetonitril (146 mg, 0,41 mmol, MS: 358,0/360,0[M+H⁺], 14% Ausbeute) als Flüssigkeit. Als Nebenprodukt fällt 2-(3-Brom-4-fluor-phenyl)-2-(6-chlorpyridazin-3-yl)acetonitril an.

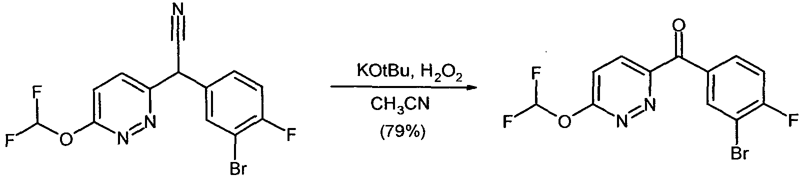

(3-Brom-4-fluor-phenyl)-(6-difluormethoxy-pyridazin-3-yl)-acetonitril (146 mg, 0,41 mmol) wurde in trockenen Acetonitril (4 ml) gelöst. Anschließend wurde Kalium-tert.-butylat (43,6 mg, 0,388 mmol) zugegeben und die Reaktionsmischung für 25 min bei Raumtemperatur gerührt. Danach wurde die Reaktionslösung im Eisbad auf 0°C gekühlt, Wasserstoffperoxid (30% in Wasser, 92 µL, 0.90 mmol) tropfenweise zugegeben und die Reaktionsmischung zunächst weitere 25 min bei 0°C und dann 1 h bei Raumtemperatur gerührt. Zur Aufarbeitung wurde die Reaktionsmischung in Wasser (40 ml) gegeben und zweimal mit Essigsäureethylester extrahiert. Die vereinigten organischen Phasen wurden über Natriumsulfat getrocknet, filtriert und am Rotationsverdampfer zur Trockene eingeengt. Man erhielt (3-Brom-4-fluor-phenyl)-(6-difluormethoxy-pyridazin-3-yl)-methanon (113 mg, 0,32 mmol, MS: 346,9/349,0[M+H⁺], 79% Ausbeute) als Feststoff.

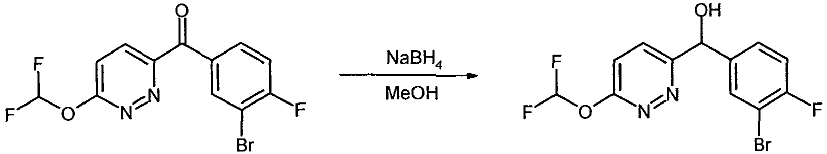

(3-Brom-4-fluor-phenyl)-(6-difluormethoxy-pyridazin-3-yl)-methanon (126 mg, 0,36 mmol) wurde in Methanol (4 ml) gelöst. Anschließend wurde Natriumborhydrid (60,4 mg, 1,60 mmol) portionsweise zugegeben und die Reaktionsmischung 1 h bei Raumtemperatur gerührt. Nach Beendigung der Reaktion wurde mit gesättigter Ammoniumchlorid-Lösung (5 ml) verdünnt und anschließend mit Essigsäureethylester (30 ml) zweimal extrahiert. Die vereinigten organischen Phasen wurden mit Wasser gewaschen, über Natriumsulfat getrocknet, filtriert und am Rotationsverdampfer zur Trockene eingeengt, wobei (3-Bromo-4-fluor-phenyl)-(6-difluormethoxy-pyridazin-3-yl)-methanol (127 mg, MS: 349/351[M+H⁺]) als Rohprodukt in Form eines Feststoffes erhalten wurde, der ohne weitere Aufreinigung für weitere Synthesestufen verwendet wurde.

(6-Difluormethoxy-pyridazin-3-yl)-[4-fluor-3-(7-morpholin-4-yl-chinazolin-4-yl)-phenyl]-methanol (BEISPIEL 121) wurde analog nach dem für [2,4-Difluor-5-(7-morpholin-4-yl-chinazolin-4-yl)-phenyl]-(4-methoxy-phenyl)-methanol (BEISPIEL 120) beschriebenen Syntheseverfahren erhalten.

### 1-[2-Chlor-4-fluor-5-(7-morpholin-4-yl-chinazolin-4-yl)-phenyl]-1-(6-methoxy-pyridazin-3-yl)-prop-2-yn-1-ol (BEISPIEL 122)

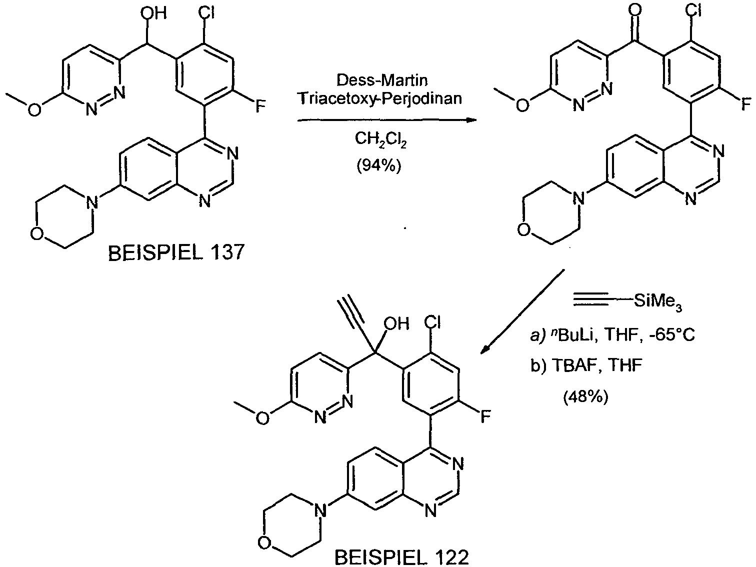

([2-Chlor-4-fluor-5-(7-morpholin-4-yl-chinazolin-4-yl)-phenyl]-(6-methoxy-pyridazin-3-yl)-methanol (BEISPIEL 137, 898 mg, 1,75 mmol) wurde in Diochlormethan (15 ml) gelöst. Anschließend wurde Dess-Martin Triacetoxy-perjodinan (15%ig in Dichlormethan, 7,23 ml, 3,50 mmol) zugegeben. Die Reaktionsuspension wurde 1 h bei Raumtemperatur gerührt. Zur Aufarbeitung wurde Wasser (60 ml) und eine 10%ige, wässrige Natriumthiosulfat-Lösung zugefügt. Die wässrige Phase wurde zweimal mit Essigsäureethylester (je 80 ml) extrahiert. Die vereinigten organischen Phasen wurden mit gesättigter Natriumchloridlösung (30 ml) gewaschen, über Natriumsulfat getrocknet, filtriert und das Filtrat im Vakuum zur Trockne eingeengt, wobei 2,1 g eines Rohproduktes im Form eines Öls erhalten wurden. Der Rückstand wurde mittels Flash-Säulechromatographie gereinigt (Gradient: Dichlormethan / 0-25vol.% Dichlormethan-Ethanol 9:1, CombiFlash Rf 200), wobei [2-Chlor-4-fluor-5-(7-morpholin-4-yl-chinazolin-4-yl)-phenyl]-(6-methoxy-pyridazin-3-yl)-methanon (792 mg, 1,65 mmol, MS: 480,1/482,1 [M+H⁺], 94% Ausbeute) als Schaum erhalten wurde.

In einem Glasgefäß mit Rührfisch und Innenthermometer wurde unter Argon Trimethylsilylacetylen (179 µL, 125 mg, 1,25 mmol) in trockenem Tetrahydrofuran (3 ml) gelöst vorgelegt. Die Reaktionslösung wurde auf (-)20°C gekühlt und n-Butyllithium (1,6 M in n-Hexan, 781 µl, 1,25 mmol) langsam zugetropft. Die Reaktionsmischung wurde für weitere 30 min bei (-)20°C gerührt. Danach wurde die Reaktionslösung auf (-)70°C gekühlt und anschließend [2-Chlor-4-fluor-5-(7-morpholin-4-yl-chinazolin-4-yl)-phenyl]-(6-methoxy-pyridazin-3-yl)-methanon (200 mg, 0.417 mmol) gelöst in trockenem Tetrahydrofuran (6 ml) zugetropft. Über die Dauer von 1 h wurde die Temperatur des Reaktionsgemisches auf (-)40°C erhöht. Anschließend wurde Wasser (40 ml) zugegeben und die Phasen separiert. Die organische Phase wurde zweimal mit Dichlormethan extrahiert. Die vereinigten organischen Phasen wurden mit Natriumsulfat getrocknet, filtriert und im Vakuum zur Trockne eingeengt. Der Rückstand wurde in trockenem Tetrahydrofuran (4 ml) gelöst und Tetra-n-butylammoniumfluorid-trihydrat (109 mg, 0,42 mmol) zugegeben. Anschließend wurde 18 h bei Raumtemperatur gerührt. Danach wurden die flüchtigen Reaktionsbestandteile am Rotationsverdampfer entfernt. Der Rückstand wurde mittels Flash-Säulechromatographie vorgereinigt (Gradient: Dichlormethan / 0-34vol.% Dichlormethan-Ethanol 1:1, CombiFlash Rf 200). Die Produktfraktionen wurden vereinigt und die Lösungsmittel im Vakuum am Rotationsverdampfer entfernt. Der Rückstand wurde mittels präparativer RP-Chromatographie endgereinigt (Chromolith RP-18e 21.2x100 mm, Fluss: 50 ml/min., Wellenlänge: 220 nm). Die flüchtigen Lösungsmittelbestandteile der geeigneten Fraktionen wurden per Vakuumzentrifuge (Genevac HT-12) entfernt und das Produkt aus Acetonitril/Wasser (1:3 Volumenanteile) gefriergetrocknet. Man erhielt 1-[2-Chlor-4-fluor-5-(7-morpholin-4-yl-chinazolin-4-yl)-phenyl]-1-(6-methoxy-pyridazin-3-yl)-prop-2-yn-1-ol (BEISPIEL 122, 102 mg, 0.20 mmol, MS: 506,1/508,1 [M+H⁺], 48% Ausbeute) als Feststoff.

Verbindungen die entsprechend der BEISPIELE 120, 121 und 122 hergestellt wurden, finden sich in der nachfolgenden Tabelle 4.

**Tabelle 4.: Verbindungen der Formel (I)**

| **Nr.** | Strukturformel | Name | IC₅₀ DNA-PK | IC₅₀ pDNA-PK | Kᵢ [Kv1.11 hERG] |
|---|---|---|---|---|---|
| **120** | 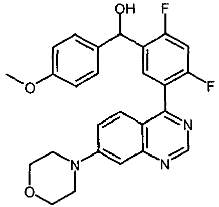 | [2,4-Difluoro-5-(7-morpholin-4-yl-quinazolin-4-yl)-phenyl]-(4-methoxy-phenyl)-methanol | C | B | D |
| | MS: 464.2 (M+H⁺) | 1H NMR (400 MHz, DMSO-d6) ppm = 9.09 (s, 1H), 7.61 - 7.47 (m, 3H), 7.36 - 7.30 (m, 2H), 7.27 (t, J=9.1, 1H), 7.20 (d, J=2.3, 1H), 6.93 - 6.87 (m, 2H), 6.18 (d, J=4.6, 1H), 6.14 (d, J=4.6, 1H), 3.81 - 3.75 (m, 4H), 3.73 (s, 3H), 3.48 - 3.41 (m, 4H) | | | |
| **121** | 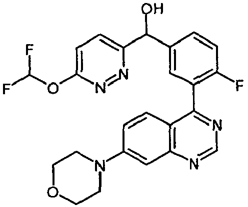 | (6-Difluoromethoxy-pyridazin-3-yl)-[4-fluoro-3-(7-morpholin-4-yl-quinazolin-4-yl)-phenyl]-methanol | B | B | B |
| | MS: 484.1 (M+H⁺) | 1H NMR (500 MHz, DMSO-d6) ppm = 9.10 (s, 1H), 7.96 (d, J=9.1, 1H), 7.73 - 7.63 (m, 2H), 7.57 - 7.47 (m, 3H), 7.46 - 7.31 (m, 1H), 7.22 - 7.16 (m, 1H), 6.63 (d, J=4.4, 1H), 6.10 (d, J=4.4, 1H), 3.81 - 3.75 (m, 4H), 3.47 - 3.41 (m, 4H) | | | |
| **122** | 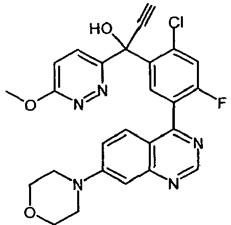 | 1-[2-Chloro-4-fluoro-5-(7-morpholin-4-yl-quinazolin-4-yl)-phenyl]-1-(6-methoxy-pyridazin-3-yl)-prop-2-yn-1-ol | C | B | C |
| | MS: 506.1/508.1 (M+H⁺) (Cl Isotopie, rel. Peakintensitäts-Verhältnis [%] 100:35) | 1H NMR (500 MHz, DMSO-d6) ppm = 9.16 (s, 1H), 8.28 (d, J=7.7, 1H), 7.96 (d, J=9.2, 1H), 7.69 - 7.63 (m, 2H), 7.59 (dd, J=9.5, 2.5, 1H), 7.50 (s, 1H), 7.30 (d, J=9.2, 1H), 7.23 (d, J=2.5, 1H), 4.02 (s, 3H), 3.94 (s, 1H), 3.81 - 3.76 (m, 4H), 3.50 - 3.44 (m, 4H) | | | |
| **123** | 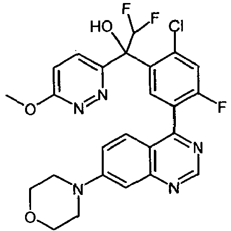 | 1-[2-Chloro-4-fluoro-5-(7-morpholin-4-yl-quinazolin-4-yl)-phenyl]-2,2-difluoro-1-(6-methoxy-pyridazin-3-yl)-ethanol | C | D | A |
| | MS: 532.1/534.1 (M+H⁺) (Cl Isotopie, rel. Peakintensitäts-Verhältnis [%] 100:40) | 1H NMR (500 MHz, DMSO-d6) ppm = 9.15 (s, 1H), 8.17 (d, J=7.7, 1H), 7.67 - 7.61 (m, 3H), 7.57 (dd, J=9.5, 2.5, 1H), 7.32 (s, 1H), 7.26 (d, 1H), 7.25 - 7.22 (m, 1H), 7.22 - 6.96 (m, 2H), 4.05 (s, 3H), 3.81 - 3.75 (m, 4H), 3.50 - 3.45 (m, 4H). | | | |
| **124** | 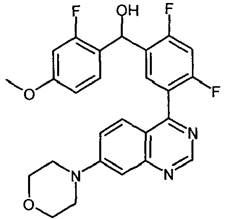 | [2,4-Difluoro-5-(7-morpholin-4-yl-quinazolin-4-yl)-phenyl]-(2-fluoro-4-methoxy-phenyl)-methanol | C | B | D |
| | MS: 582.1 (M+H⁺) | 1H NMR (400 MHz, DMSO-d6) ppm = 9.09 (s, 1H), 7.70 (t, J=8.8, 1H), 7.62 - 7.44 (m, 3H), 7.25 (t, J=9.2, 1H), 7.20 (d, J=2.4, 1H), 6.82 (dd, J=8.6, 2.5, 1H), 6.74 (dd, J=12.6, 2.5, 1H), 6.34 (d, J=4.6, 1H), 6.28 (d, J=4.6, 1H), 3.82 - 3.71 (m, 7H), 3.49 - 3.41 (m, 4H) | | | |
| **125** | 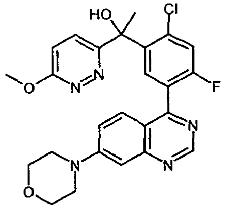 | 1-[2-Chloro-4-fluoro-5-(7-morpholin-4-yl-quinazolin-4-yl)-phenyl]-1-(6-methoxy-pyridazin-3-yl)-ethanol | B | D | B |
| | MS: 496.1/498.1 (M+H⁺) (Cl Isotopie, rel. Peakintensitäts-Verhältnis [%] 100:35) | 1H NMR (500 MHz, DMSO-d6) ppm = 9.15 (s, 1H), 8.20 (d, J=7.9, 1H), 7.67 (dd, J=9.4, 3.4, 1H), 7.60 - 7.54 (m, 3H), 7.23 (d, J=2.5, 1H), 7.18 (d, J=9.2, 1H), 6.39 (s, 1H), 4.03 (s, 3H), 3.82 - 3.75 (m, 4H), 3.49 - 3.45 (m, 4H), 2.02 (s, 3H) | | | |
| **126** | 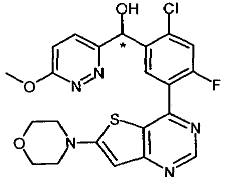 | [2-Chloro-4-fluoro-5-(6-morpholin-4-yl-thieno[3,2-d]pyrimidin-4-yl)-phenyl]-(6-methoxy-pyridazin-3-yl)-methanol (*Ena 2*) | A | A | C |
| | MS: 488.1/490.1 (M+H⁺) (Cl Isotopie, rel. Peakintensitäts-Verhältnis [%] 100:39); Rₜ 16.85min (SFC, Chiracel OD-H, CO₂/15 vol.% Methanol, 0.5 vol.% Diethylamin) | 1H NMR (500 MHz, DMSO-d6) ppm = 8.90 (s, 1H), 8.00 (d, J=7.9, 1H), 7.69 (d, J=9.2, 1H), 7.66 (d, J=10.0, 1H), 7.22 (d, J=9.2, 1H), 6.62 (d, J=4.9, 1H), 6.54 (s, 1H), 6.22 (d, J=4.9, 1H), 3.99 (s, 3H), 3.77 - 3.72 (m, 4H), 3.45 - 3.39 (m, 4H) | | | |
| **127** | 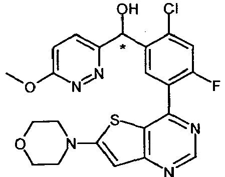 | [2-Chloro-4-fluoro-5-(6-morpholin-4-yl-thieno[3,2-d]pyrimidin-4-yl)-phenyl]-(6-methoxy-pyridazin-3-yl)-methanol (*Ena 1*) | B | C | C |
| | MS: 488.1/490.1 (M+H⁺) (Cl Isotopie, rel. Peakintensitäts-Verhältnis [%] 100:40); Rₜ 14.73min (SFC, Chiracel OD-H, CO₂/15 vol.% Methanol, 0.5 vol.% Diethylamin) | 1H NMR (500 MHz, DMSO-d6) ppm = 8.90 (s, 1H), 8.00(d, J=7.9, 1H), 7.69 (d, J=9.2, 1H), 7.66 (d, J=10.0, 1H), 7.22 (d, J=9.1, 1H), 6.61 (d, J=5.0, 1H), 6.54 (s, 1H), 6.21 (d, J=4.9, 1H), 3.99 (s, 3H), 3.77 - 3.72 (m, 4H), 3.45 - 3.40 (m, 4H) | | | |
| **128** | 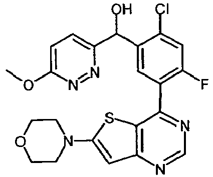 | [2-Chloro-4-fluoro-5-(6-morpholin-4-yl-thieno[3,2-d]pyrimidin-4-yl)-phenyl]-(6-methoxy-pyridazin-3-yl)-methanol | A | B | B |
| | MS: 488.1/490.1 (M+H⁺) (Cl Isotopie, rel. Peakintensitäts-Verhältnis [%] 100:41) | 1H NMR (500 MHz, DMSO-d6) ppm = 8.90 (s, 1H), 8.00 (d, J=7.9, 1H), 7.69 (d, J=9.2, 1H), 7.66 (d, J=10.0, 1H), 7.22 (d, J=9.1, 1H), 6.61 (d, J=4.9, 1H), 6.54 (s, 1H), 6.22 (d, J=4.9, 1H), 3.99 (s, 3H), 3.77 - 3.73 (m, 4H), 3.44 - 3.40 (m, 4H) | | | |
| **129** | 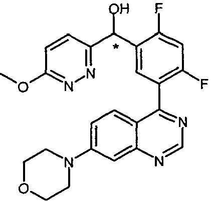 | [2,4-Difluoro-5-(7-morpholin-4-yl-quinazolin-4-yl)-phenyl]-(6-methoxy-pyridazin-3-yl)-methanol (*Ena 2*) | C | C | B |
| | MS: 466.2 (M+H⁺); Rₜ 4.13 min, (SFC, Chiralpak AS-H, CO₂/18 vol.% Methanol, 0.5 vol.% Diethylamin) | s. Racemat | | | |
| **130** | 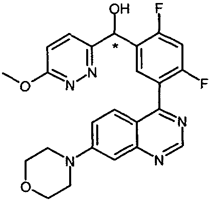 | [2,4-Difluoro-5-(7-morpholin-4-yl-quinazolin-4-yl)-phenyl]-(6-methoxy-pyridazin-3-yl)-methanol (*Ena 1*) | A | A | A |
| | MS: 466.2 (M+H⁺); Rₜ 2.79 min, (SFC, Chiralpak AS-H, CO₂/ 18 vol.% Methanol, 0.5 vol.% Diethylamin) | s. Racemat | | | |
| **131** | 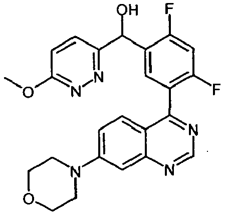 | [2,4-Difluoro-5-(7-morpholin-4-yl-quinazolin-4-yl)-phenyl]-(6-methoxy-pyridazin-3-yl)-methanol | B | B | B |
| | MS: 466.2 (M+H⁺) | 1H NMR (500 MHz, DMSO-d6) ppm = 9.11 (s, 1H), 7.83 (t, J=8.1, 1H), 7.75 (d, J=9.2, 1H), 7.56 (qd, J=9.4, 2.8, 2H), 7.45 (t, J=10.1, 1H), 7.25 - 7.19 (m, 2H), 6.59 - 6.57 (m, 1H), 6.20 - 6.16 (m, 1H), 4.00 (s, 3H), 3.81 - 3.75 (m, 4H), 3.48 - 3.42 (m, 4H) | | | |
| **132** | 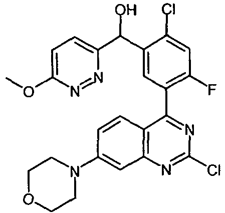 | [2-Chloro-5-(2-chloro-7-morpholin-4-yl-quinazolin-4-yl)-4-fluoro-phenyl]-(6-methoxy-pyridazin-3-yl)-methanol | B | B | A |
| | MS: 516.1/518.1/520.0 (M+H⁺) (Cl₂ Isotopie, rel. Peakintensitäts-Verhältnis [%] 100:69:12) | 1H NMR (500 MHz, DMSO-d6) ppm = 7.94 (d, J=7.7, 1H), 7.72 - 7.65 (m, 2H), 7.63 - 7.49 (m, 2H), 7.21 (d, J=9.1, 1H), 7.15 (d, J=2.4, 1H), 6.22 (s, 1H), 4.00 (s, 3H), 3.79 - 3.73 (m, 4H), 3.53 - 3.47 (m, 4H) | | | |
| **133** | 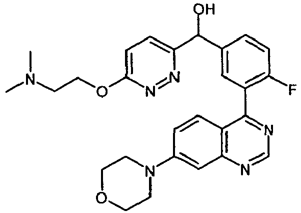 | [6-(2-Dimethylamino-ethoxy)-pyridazin-3-yl]-[4-fluoro-3-(7-morpholin-4-yl-quinazolin-4-yl)-phenyl]-methanol | C | C | A |
| | MS: 505.3 (M+H⁺) | 1H NMR (500 MHz, DMSO-d6) ppm = 9.09 (s, 1H), 7.69 (d, J=9.2, 1H), 7.67 - 7.61 (m, 2H), 7.55 - 7.47 (m, 2H), 7.44 - 7.36 (m, 1H), 7.24 - 7.15 (m, 2H), 6.49 (d, J=4.0, 1H), 6.04 - 5.98 (m, 1H), 4.56 - 4.41 (m, 2H), 3.80 - 3.74 (m, 4H), 3.47 - 3.41 (m, 4H), 2.64 (t, J=5.8, 2H), 2.19 (s, 6H) | | | |
| **134** | 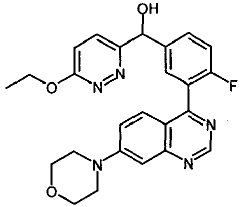 | (6-Ethoxy-pyridazin-3-yl)-[4-fluoro-3-(7-morpholin-4-yl-quinazolin-4-yl)-phenyl]-methanol | C | B | C |
| | MS: 462.1 (M+H⁺) | 1H NMR (500 MHz, DMSO-d6) ppm = 9.09 (s, 1H), 7.68 (d, J=9.2, 1H), 7.66 - 7.61 (m, 2H), 7.53 - 7.50 (m, 2H), 7.43 - 7.35 (m, 1H), 7.21 -7.19 (m, 1H), 7.17 (d, J=9.1, 1H), 6.47 (d, J=4.4, 1H), 6.01 (d, J=4.3, 1H), 4.52 - 4.38 (m, 2H), 3.82 - 3.73 (m, 4H), 3.46 - 3.41 (m, 4H), 1.36 (t, J=7.0, 3H) | | | |
| **135** | 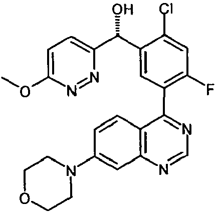 | (R)-[2-Chloro-4-fluoro-5-(7-morpholin-4-yl-quinazolin-4-yl)-phenyl]-(6-methoxy-pyridazin-3-yl)-methanol | C | D | A |
| | MS: 482.1/484.1 (M+H⁺) (Cl Isotopie, rel. Peakintensitäts-Verhältnis [%] 100:36); Rₜ 5.34min (SFC, Chiralpak AD-H, CO₂/ 40 vol.% Methanol, 0.5 vol.% Diethylamin) | 1H NMR (500 MHz, DMSO-d6) ppm = 9.12 (s, 1H), 7.91 (d, J=7.7, 1H), 7.69 (d, J=9.2, 1H), 7.67 (d, J=9.5, 1H), 7.62 - 7.51 (m, 2H), 7.24 - 7.18 (m, 2H), 6.61 (d, J=4.8, 1H), 6.23 (d, J=4.8, 1H), 4.00 (s, 3H), 3.81 - 3.75 (m, 4H), 3.48 - 3.42 (m, 4H) | | | |
| **136** | 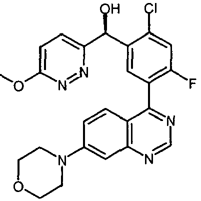 | (S)-[2-Chloro-4-fluoro-5-(7-morpholin-4-yl-quinazolin-4-yl)-phenyl]-(6-methoxy-pyridazin-3-yl)-methanol | A | A | A |
| | MS: 482.1/484.1 (M+H⁺) (Cl Isotopie, rel. Peakintensitäts-Verhältnis [%] 100:36); Rₜ 3.38min (SFC, Chiralpak AD-H, CO₂/ 40 vol.% Methanol, 0.5 vol.% Diethylamin) | 1H NMR (500 MHz, DMSO-d6) ppm = 9.12 (s, 1H), 7.91 (d, J=7.7, 1H), 7.69 (d, J=9.2, 1H), 7.66 (d, J=9.5, 1H), 7.61 - 7.52 (m, 2H), 7.24 - 7.19 (m, 2H), 6.61 (d, J=5.0, 1H), 6.23 (d, J=4.9, 1H), 4.00 (s, 3H), 3.81 - 3.75 (m, 4H), 3.48 - 3.43 (m, 4H) | | | |
| **137** | 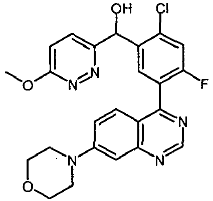 | [2-Chloro-4-fluoro-5-(7-morpholin-4-yl-quinazolin-4-yl)-phenyl]-(6-methoxy-pyridazin-3-yl)-methanol | A | B | C |
| | MS: 482.1/484.1 (M+H⁺) (Cl Isotopie, rel. Peakintensitäts-Verhältnis [%] 100:36) | 1H NMR (400 MHz, DMSO-d6) ppm = 9.12 (s, 1H), 7.91 (d, J=7.7, 1H), 7.69 (d, J=9.1, 1H), 7.66 (d, J=9.5, 1H), 7.62 - 7.51 (m, 2H), 7.24 - 7.17 (m, 2H), 6.60 (d, J=4.9, 1H), 6.23 (d, J=3.5, 1H), 4.00 (s, 3H), 3.82 - 3.74 (m, 4H), 3.49 - 3.42 (m, 4H) | | | |

### BEISPIEL 138

### 1-[5-(7-Morpholin-4-yl-chinazolin-4-yl)-pyridin-3-yl]-1-thiazol-2-yl-ethanol (BEISPIEL 138)

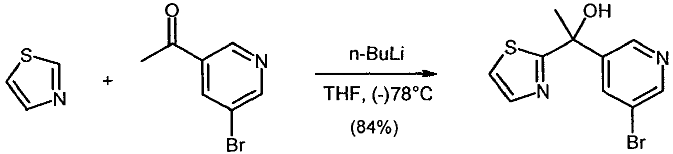

In einem ausgeheizten Dreihalskolben wurde Thiazol (143 µl, 2,0 mmol) in trockenem Tetrahydrofuran (10 ml) vorgelegt. Die Reaktionslösung wurde mittels Aceton/Trockeneis-Bad auf (-)78°C gekühlt. n-Butyllithium (15%ige Lösung in n-Hexan, 1,63 ml, 2,6 mmol) wurde über die Dauer von 10 min bei konstanter Temperatur zugetropft. Die Reaktionsmischung wurde weitere 10 min gerührt. Anschließend wurde die Suspension wurde auf (-)30°C erwärmt und wieder auf (-)55°C eingekühlt und 1-(5-Brom-pyridin-3-yl)-ethanon (380 mg, 1,90 mmol), gelöst in trockenem Tetrahydrofuran (6 ml), bei (-)40°C zugetropft. Über 1,5 h lässt man die Reaktionstemperatur auf (-)10°C ansteigen. Nach Beendigung der Reaktion (HPLC Kontrolle) wurde gesättigte Ammoniumchlorid-Lösung zugegeben und für 30 min bei Raumtemperatur gerührt. Die Reaktionsmischung wurde in eine zweiphasige Lösung aus Wasser (60 ml) und Essigsäureethylester (80 ml) gegeben und dreimal mit Essigsäureethylester extrahiert. Die vereinigten organischen Phasen wurden mit gesättigter Natriumchloridlösung gewaschen, über Natriumsulfat getrocknet, filtriert und am Rotationsverdampfer eingeengt. Das ölige Rohprodukt wurde mittels Flash-Säulenchromatographie gereinigt (Lösemittel: Dichlormethan/2,0vol.% Methanol, dann Dichlormethan/3,0vol.% Methanol + 1,0vol.% Ammoniak, Flashkieselgelmenge 30g). Die Produktfraktionen wurden vereinigt und die Lösungsmittel im Vakuum am Rotationsverdampfer entfernt. Man erhielt 1-(5-Brom-pyridin-3-yl)-1-thiazol-2-yl-ethanol (479 mg, 1,68 mmol, MS: 285,0/287,0 [M+H⁺], 84% Ausbeute) als Öl.

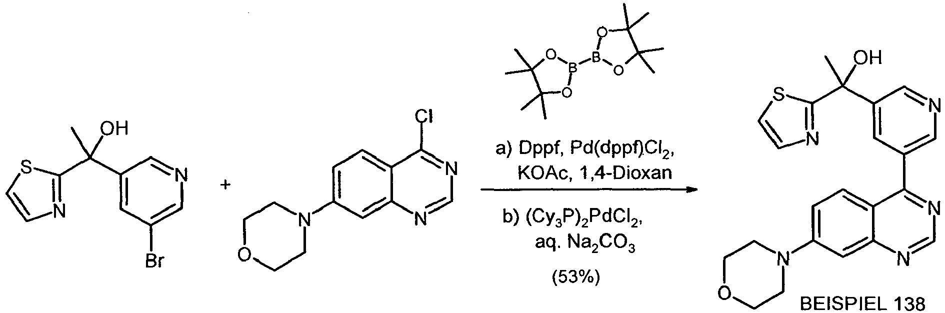

In ein Glasgefäß mit Rührfisch wurden 1-(5-Brom-pyridin-3-yl)-1-thiazol-2-yl-ethanol (162 mg, 0,55 mmol), Bis(pinacolato)dibor (140 mg, 0,55 mmol), 1,1'-Bis(diphenylphosphino)-ferrocen (Dppf, 7,1 mg, 0,013 mmol), 1,1'-Bis(diphenylphosphino)ferrocene-palladium(II)dichloride [Pd(dppf)Cl₂, 10,4 mg, 0,013 mmol] und Kaliumacetat (167 mg, 1,7 mmol) in trockenem, Sauerstoff-freiem 1,4-Dioxan suspendiert. Das Glasgefäß wurde mit einem Septum verschlossen. Die Reaktionslösung wurde gerührt und für 2.5 h auf 115°C erhitzt. Die Reaktionskontrolle erfolgt per HPLC. Der Reaktionslösung wurden 4-Chlor-7-morpholin-4-yl-chinazoline (106 mg, 0,43 mmol), Bis(tricyclohexylphosphin)-palladium-(II)-dichlorid (9,4 mg, 0,013 mmol) und 2,0 M Natriumcarbonat-Lösung (531 µl) zugefügt. Anschließend wurde die Reaktionsmischung bei einer Temperatur von 125°C für 1,5 h gerührt. Die Mischung wurde in Wasser/Dichlormethan (1:1 Volumenanteile, 40 ml) dekantiert und die resultierende Lösung dreimal mit Dichlormethan extrahiert. Die vereinigten organischen Phasen wurden über Natriumsulfat getrocknet, filtriert und am Rotationsverdampfer eingeengt. Der Rückstand wurde mittels Flash-Chromatographie [Gradient: Dichlormethan / 20-58vol.% eines Lösemittelgemisches aus Dichlormethan/Methanol 9:1 (Volumenanteile), CombiFlash Rf 200] aufgereinigt. Die geeigneten Produktfraktionen wurden vereinigt und die Lösungsmittel am Rotationsverdampfer entfernt, wobei 1-[5-(7-Morpholin-4-yl-chinazolin-4-yl)-pyridin-3-yl]-1-thiazol-2-yl-ethanol (BEISPIEL 138, 95 mg, 0.23 mmol, MS: 420,2 [M+H⁺], 53% Ausbeute) als Öl erhalten wurde.

### BEISPIEL 139

### {3-[7-(3,6-Dihydro-2H-pyran-4-yl)-chinazolin-4-yl]-4-fluor-phenyl}-thiazol-2-yl-methanol (139)

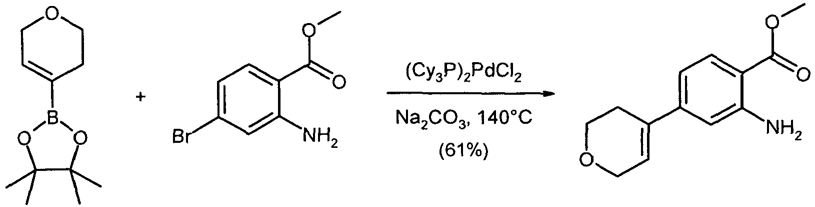

In einem Mikrowellenglasgefäß mit Rührfisch wurden 4-(4,4,5,5-Tetramethyl-[1,3,2]dioxaborolan-2-yl)-3,6-dihydro-2H-pyran (575 mg, 2,74 mmol), 2-Amino-4-brom-benzoesäuremethylester (600 mg, 2,61 mmol), Bis(tricyclohexylphosphin)-palladium-(II)-dichlorid (57,8 mg, 0.078 mmol) und Sauerstoff-freie 2,0 M Natriumcarbonat-Lösung (3,26 ml, 6,52 mmol) in entgastem, Sauerstoff-freiem 1,4-Dioxan (12 ml) vorgelegt. Die Substanzmischung wurde für die Dauer von 55 min in einen *Personal Chemistry Microwave Synthesizer* bei 100 Watt auf 135°C erhitzt. Anschließend wurde die Reaktionslösung in ein Gemisch aus Wasser (40 ml) und Essigsäureethylester (30 ml) Gemisch abdekantiert. Die resultierende Lösung wurde dreimal mit Essigsäureethylester extrahiert. Die vereinigten organischen Phasen wurden mit gesättigter Natriumchloridlösung gewaschen, über Natriumsulfat getrocknet, filtriert und im Vakuum am Rotationsverdampfer eingeengt. Der Rückstand wurde mittels Flash-Chromatographie [Gradient: Dichlormethan / 0-10vol.% eines Lösemittelgemisches aus Dichlormethan/Methanol 10:1 (Volumenanteile), CombiFlash Rf 200] aufgereinigt. Die geeigneten Produktfraktionen wurden vereinigt und die Lösungsmittel am Rotationsverdampfer entfernt, wobei 2-Amino-4-(3,6-dihydro-2H-pyran-4-yl)-benzoesäuremethylester (371,1 mg, 1,59 mmol, MS: 234,2 [M+H⁺], 61% Ausbeute) als Feststoff erhalten wurde

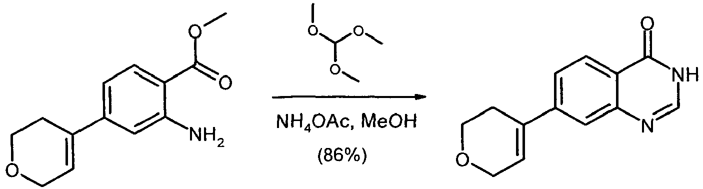

In einem Glasgefäß mit Rührfisch wurde 2-Amino-4-(3,6-dihydro-2H-pyran-4-yl)-benzoesäuremethylester (620 mg, 2,66 mmol), Trimethylorthoformiat (564,1 mg, 5,32 mmol) und Ammoniumacetat (410 mg, 5,32 mmol) in Methanol (20 ml) gelöst vorgelegt. Die Substanzmischung wurde bei 80°C über Nacht gerührt. Anschließend wurde Wasser (10 ml) hinzugeben und der ausgefallene Feststoff abgesaugt, mit wenig Wasser gewaschen und anschließend im Vakuum getrocknet. Man erhielt 7-(3,6-Dihydro-2H-pyran-4-yl)-3H-chinazolin-4-on (520 mg, 2,28 mmol, MS: 229,1 [M+H⁺], 86% Ausbeute) als Feststoff.

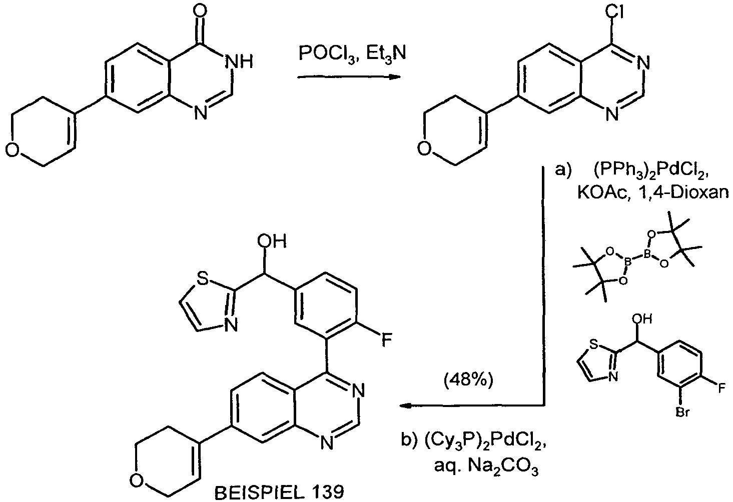

{3-[7-(3,6-Dihydro-2H-pyran-4-yl)-chinazolin-4-yl]-4-fluor-phenyl}-thiazol-2-yl-methanol (BEISPIEL 139) wurde analog der Syntheseverfahren zur Herstellung von 1-[5-(7-Morpholin-4-yl-chinazolin-4-yl)-pyridin-3-yl]-1-thiazol-2-yl-ethanol (BEISPIEL 138) erhalten.

### BEISPIEL 140

### [4-Fluor-3-(7-morpholin-4-yl-pyrido[4,3-d]pyrimidin-4-yl)-phenyl]-thiazol-2-yl-methanol (140)

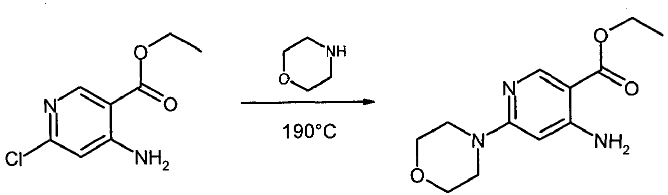

4-Amino-6-chlor-nicotinsäureethylester (8,38 g, 39,7 mmol) wurden in Morpholin (40 ml) gelöst. Die Substanzmischung wurde für 4 h auf 120°C erhitzt. Nach beendeter Reaktion wurde die abgekühlte Reaktionslösung auf Wasser (400 ml) dekantiert. Die wässrige Suspension wurde 10 min gerührt und der Niederschlag anschließend abfiltiert. Der Filterkuchen wurde mit etwas Wasser nachgewaschen und im Vakuum über Nacht bei 60°C getrocknet, wobei reiner 4-Amino-6-morpholin-4-yl-nicotinsäureethylester (8,55 g, 34,03 mmol, MS: 252,2 [M+H⁺], 85% Ausbeute) als farbloser Feststoff erhalten wurden.

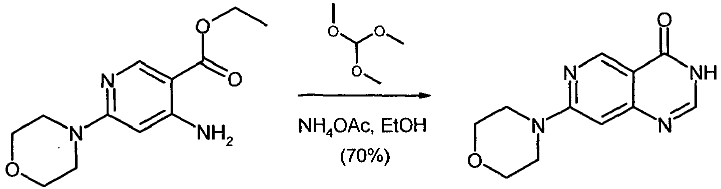

Aus 4-Amino-6-morpholin-4-yl-nicotinsäureethylester (3,54 g, 14.1 mmol) wurde analog dem zu BEISPIEL 139 beschriebenen Syntheseverfahren 7-Morpholin-4-yl-3H-pyrido[4,3-d]pyrimidin-4-one (2,29 g, 9,86 mmol, MS: 233,1 [M+H⁺] 70% Ausbeute) als Feststoff erhalten.

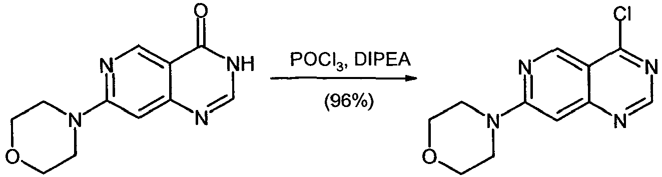

7-Morpholin-4-yl-3H-pyrido[4,3-d]pyrimidin-4-on (600 mg, 2,58 mmol) wurde in 1,4-Dioxan (10 ml) suspendiert. Zur Reaktionsmischung wurden Phosphorylchlorid (POCl₃, 546 µL, 5.9 mmol) und Hünigs Base (N-Ethyldiisopropylamin, 220 µL, 1,29 mmol) zugefügt. Anschließend wurde für 3 h bei einer Temperatur von 100°C gerührt. Nach Beendigung der Reaktion wurde die Reaktionslösung in eine halbgesättigte Natriumhydrogencarbonat-Lösung (80 ml) dekantiert. Die wässrige Phase wurde dreimal mit Dichlormethan (je 40 ml) extrahiert. Die vereinigten organischen Phasen wurden über Natriumsulfat getrocknet, filtriert und am Rotationsverdampfer im Vakuum eingeengt, wobei 4-Chlor-7-morpholin-4-yl-pyrido[4,3-d]pyrimidin (627 mg, 2,50 mmol, MS: 251,0/253,0 [M+H⁺], 96% Ausbeute) als Feststoff erhalten wurde.

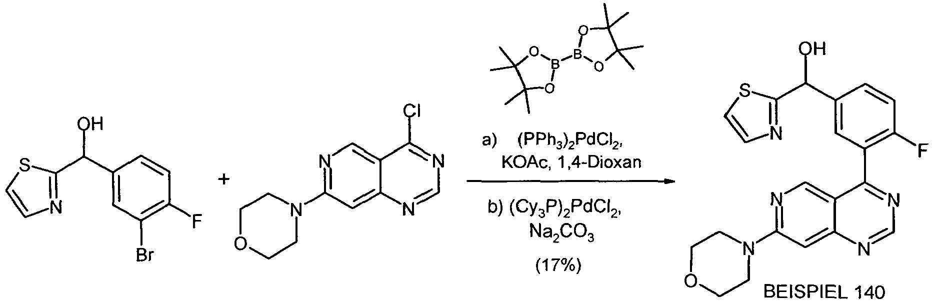

[4-Fluor-3-(7-morpholin-4-yl-pyrido[4,3-d]pyrimidin-4-yl)-phenyl]-thiazol-2-yl-methanol (BEISPIEL 140) wurde analog der Syntheseverfahren zur Herstellung von 1-[5-(7-Morpholin-4-yl-chinazolin-4-yl)-pyridin-3-yl]-1-thiazol-2-yl-ethanol (BEISPIEL 138) erhalten.

### BEISPIEL 141

### [2-Chlor-4-fluor-5-(7-morpholin-4-yl-pyrido[4,3-d]pyrimidin-4-yl)-phenyl]-(4-hydroxymethyl-thiazol-2-yl)-methanol (141)

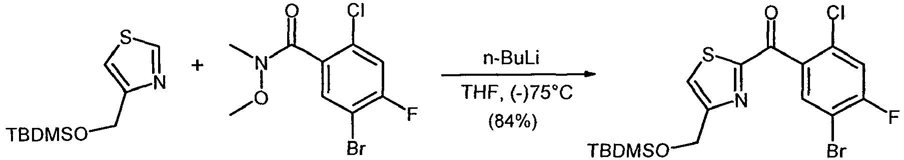

In einem Zweihalskolben mit Rührfisch, Innenthermometer und Septum wurde 4-(tert-Butyl-dimethyl-silanyloxymethyl)-thiazol (10,15 g, 43,5 mmol) unter Argon in trockenem Tetrahydrofuran (78 ml) gelöst vorgelegt. Die Reaktionslösung wurde mittels Aceton/Trockeneis-Bad auf (-)75°C gekühlt. Anschließend wurde n-Butyllithium (15%ige Lösung in n-Hexan, 29,3 ml, 46,6 mmol) bei konstanter Temperatur langsam zu der Reaktionslösung zugetropft. Die Reaktionslösung wurde weitere 30 min bei (-)75°C gerührt, anschließend wurde auf 0°C erwärmt. Danach wurde erneut auf (-)50°C gekühlt. In die Reaktionslösung wurde auf (-)50°C vorgekühlte Lösung von 5-Brom-2-chlor-4-fluor-N-methoxy-N-methyl-benzamid (5,58 g, 12,4 mmol), gelöst in trockenem Tetrahydrofuran (21 ml) bei (-)50°C langsam über die Dauer von 1.5 h zugetropft. Die Reaktionslösung wurde weitere 30 min bei (-)50°C gerührt. Nach beendeter Reaktion wurde Wasser (20 ml) zur Reaktionslösung gegeben. Danach ließ man die Reaktionslösung unter Rühren auf Raumtemperatur erwärmen. Die Reaktionslösung wurde mit Essigsäureethylester (400 ml) und gesättigter Natriumchloridlösung (100 ml) verdünnt. Die Phasen wurden getrennt und die wässrige Phase mit Essigsäureethylester extrahiert. Die vereinigten organischen Phasen wurden über Natriumsulfat getrocknet, filtriert und im Vakuum am Rotationsverdampfer eingeengt. Der Rückstand wurde mittels Flash-Chromatographie gereinigt (Gradient: Cyclohexan/0-7vol.% Essigsäureethylester, CombiFlash Rf 200). Die geeigneten Produktfraktionen wurden vereinigt und die organischen Lösungsmittel am Rotationsverdampfer entfernt. Man erhielt (5-Brom-2-chlor-4-fluor-phenyl)-[4-(tert.-butyl-dimethyl-silanyloxymethyl)-thiazol-2-yl]-methanon (4,94 g, 10,39 mmol, MS: Hauptpeak 466 [M+H⁺], 84% Ausbeute) als Öl.

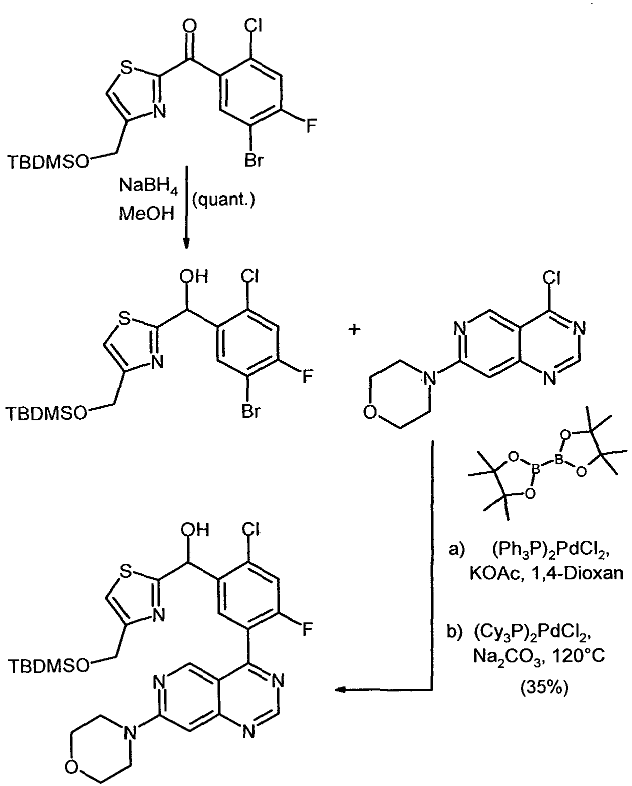

[4-[[tert.-Butyl(dimethyl)silyl]oxymethyl]thiazol-2-yl]-[2-chlor-4-fluor-5-(7-morpholinopyrido[4,3-d]pyrimidin-4-yl)phenyl]methanol wurde analog der Syntheseverfahren der BEISPIELE 1 und 2 sowie 138 aus (5-Brom-2-chlor-4-fluor-phenyl)-[4-(tert.-butyl-dimethyl-silanyloxymethyl)-thiazol-2-yl]-methanon und 4-Chlor-7-morpholin-4-yl-pyrido[4,3-d]pyrimidin hergestellt.

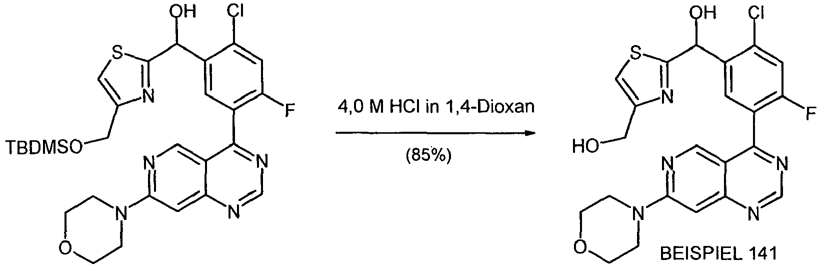

[4-(tert.-Butyl-dimethyl-silanyloxymethyl)-thiazol-2-yl]-[2-chlor-4-fluor-5-(7-morpholin-4-yl-pyrido[4,3-d]pyrimidin-4-yl)-phenyl]-methanol (333 mg, 0,55 mmol) wurde in 1,4-Dioxan (7 ml) gelöst. 4,0 M HCl gelöst in 1,4-Dioxan (1,38 ml, 5,53 mmol) wurde zugegeben und die Reaktionslösung anschließend für 30 min bei Raumtemperatur gerührt. Nach beendeter Reaktion wurde die Reaktionslösung filtriert und Lösemittel am Rotationsverdampfer entfernt. Der Rückstand wurde mittels Flash-Chromatographie gereinigt (Gradient: Dichlormethan/0-15vol.%Ethanol, CombiFlash Rf 200). Die geeigneten Produktfraktionen wurden vereinigt und die Lösungsmittel im Vakuum entfernt. Der Rückstand wurde in Dichlormethan aufgenommen, mit gesättigter Natriumhydrogencarbonat-Lösung extrahiert, über Natriumsulfat getrocknet, filtriert und das Filtrat zur Trockne eingeengt, wobei [2-Chlor-4-fluor-5-(7-morpholin-4-yl-pyrido[4,3-d]pyrimidin-4-yl)-phenyl]-(4-hydroxymethyl-thiazol-2-yl)-methanol (BEISPIEL 141, 229 mg, 0,47 mmol, MS: 488,0/490,0 [M+H⁺], 85% Ausbeute) als Feststoff.

### BEISPIELE 142 und 143

### 2-Chlor-4-fluor-5-(7-morpholin-4-yl-chinazolin-4-yl)-phenyl]-(4-hydroxymethyl-thiazol-2-yl)-methanol (142)

### [2-Chlor-4-fluor-5-(7-morpholin-4-yl-chinazolin-4-yl)-phenyl]-(4-methylaminomethyl-thiazol-2-yl)-methanol (143)

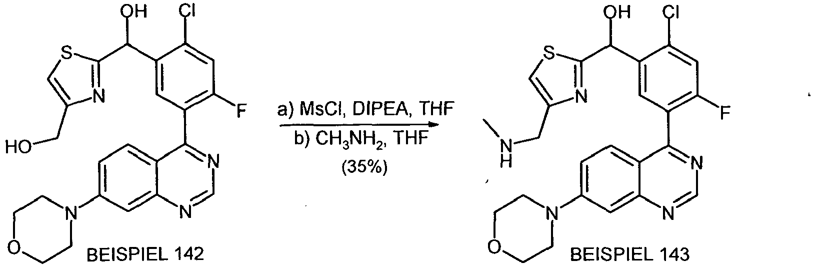

Unter Argon wurde [2-Chlor-4-fluor-5-(7-morpholin-4-yl-pyrido[4,3-d]pyrimidin-4-yl)-phenyl]-(4-hydroxymethyl-thiazol-2-yl)-methanol (46,6 mg, 96 µmol) in trockenem Tetrahydrofuran (3,1 ml) gelöst. N-Ethyldiisopropylamin (98 µL, 57,4 µmol) und Methansulfonylchlorid (14,8 µL, 191 µmol) wurden zugegeben. Die Reaktionslösung wurde für 30 min bei Raumtemperatur gerührt. Anschließend wurde Methylamin (40%ige Lösung in Wasser, 183 µl, 1,91 mol) zugegeben und für weitere 2 h bei Raumtemperatur gerührt. Nach Beendigung der Reaktion wurde die Reaktionslösung mit Essigsäureethylester (15 ml) und gesättigter Natriumchloridlösung (10 ml) versetzt. Die Phasen wurden separiert und die wässrige Phase mit Essigsäureethylester extrahiert. Die vereinigten organischen Phasen wurden über Natriumsulfat getrocknet und filtriert. Das Filtrat wurde am Rotationsverdampfer eingeengt und der Rückstand mittels präparativer RP-HPLC aufgereinigt (Gradient Wasser + 0.1% Trifluoressigsäure/Acetonitril + 0.1% Trifluoressigsäure, Sunfire Prep C-18 150-21 mm, Fluss: 50 ml/min., λ = 220 nm). Die geeigneten Produktfraktionen wurden vereinigt und die Lösungsmittel im Vakuum am Rotationsverdampfer entfernt und der Rückstand aus Dioxan/Wasser gefriergetrocknet, wobei [2-Chlor-4-fluor-5-(7-morpholin-4-yl-chinazolin-4-yl)-phenyl]-(4-methylaminomethyl-thiazol-2-yl)-methanol (BEISPIEL 143, 14,8 mg, 0.030 mmol, MS: 500,1/502,0 [M+H⁺], 31% Ausbeute) als Feststoff.

### BEISPIELE 144, 145 und 146

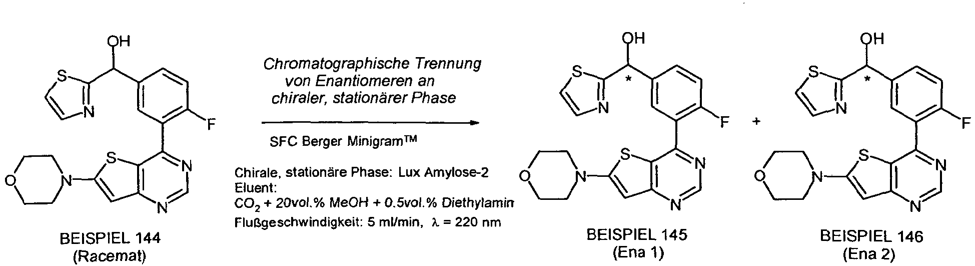

Racemisches [4-Fluor-3-(6-morpholin-4-yl-thieno[3,2-d]pyrimidin-4-yl)-phenyl]-thiazol-2-yl-methanol (BEISPIEL 144, 35 mg, 0,082 mmol) wurde an chiraler stationärer Phase unter Verwendung von präparativer SFC in seine Enantiomere chromatographisch aufgetrennt: Nach einem analytischen Säulenscreening zur Identifikation der geeignetsten chiralen Phase mit höchster Selektivität wurde die Lux Amylose-2 Phase der Firma Phenomex ausgewählt. SFC-Bedingungen: Gerät: SFC Berger minigram; Säule: Lux Amylose-2, 250x4.6 mm; Eluent: Kohlendioxid + 20vol.%Methanol + 0.5vol.% Diethylamin, Flussgeschwindigkeit: 5 ml/min, Wellenlänge: 220 nm. Unter den gleichen Bedingungen wurde die präparative Trennung in die Enantiomere im *SFC Berger minigram Stacked Injection Mode* durchgeführt. Die geeigneten Fraktionen wurden gesammelt und die Lösungsmittel am im Vakuum am Rotationsverdampfer entfernt. Man erhielt die enantiomerenreinen [4-Fluor-3-(6-morpholin-4-yl-thieno[3,2-d]pyrimidin-4-yl)-phenyl]-thiazol-2-yl-methanol (BEISPIEL 145, Rₜ = 7,85 min 12 mg, 0,028 mmol, > 99% ee *Ena 1*) und [4-Fluor-3-(6-morpholin-4-yl-thieno[3,2-d]pyrimidin-4-yl)-phenyl]-thiazol-2-yl-methanol (BEISPIEL 146, Rₜ = 8.82 min, 12,0 mg, 0,028 mmol, 92% ee, *Ena 2*) als Feststoffe.

Verbindungen die analog der BEISPIELE 138-146 hergestellt wurden, finden sich in der nachfolgenden Tabelle 5.

**Tabelle 5: Verbindungen der Formel (I)**

| Nr. | Strukturformel | Name | IC₅₀ DNA-PK | IC₅₀ pDNA-PK | Kᵢ [Kv1.11 hERG] |
|---|---|---|---|---|---|
| **138** | 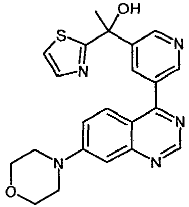 | 1-[5-(7-Morpholin-4-yl-quinazolin-4-yl)-pyridin-3-yl]-1-thiazol-2-yl-ethanol | D | | B |
| | MS: 420.2 (M+H⁺) | 1H NMR (400 MHz, DMSO-d6) ppm = 9.11 (s, 1H), 8.97 (d, J=2.2, 1H), 8.83 (d, J=2.1, 1H), 8.30 (t, J=2.2, 1H), 7.82 (d, J=9.5, 1H), 7.79 (d, J=3.2, 1H), 7.65 (d, J=3.3, 1H), 7.57 (dd, J=9.5, 2.6, 1H), 7.22 (d, J=2.5, 1H), 7.09 (s, 1H), 3.82 - 3.75 (m, 4H), 3.50 - 3.43 (m, 4H), 2.02 (s, 3H) | | | |
| **139** | 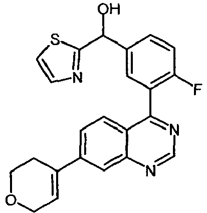 | {3-[7-(3,6-Dihydro-2H-pyran-4-yl)-quinazolin-4-yl]-4-fluoro-phenyl}-thiazol-2-yl-methanol | C | D | A |
| | MS: 420.0 (M+H⁺) | 1H NMR (500 MHz, DMSO-d6) ppm = 9.36 (s, 1H), 8.02 (d, J=1.8, 1H), 7.94 (dd, J=8.9, 1.9, 1H), 7.76 - 7.69 (m, 4H), 7.65 (d, J=3.2, 1H), 7.49 - 7.43 (m, 1H), 7.00 - 6.96 (m, 1H), 6.71 - 6.67 (m, 1H), 6.11 - 6.08 (m, 1H), 4.33 - 4.30 (m, 2H), 3.89 (t, J=5.5, 2H), 2.65 - 2.60 (m, 2H) | | | |
| **140** | 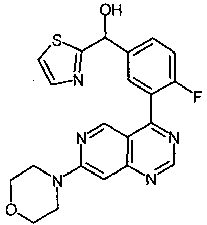 | [4-Fluoro-3-(7-morpholin-4-yl-pyrido[4,3-d]pyrimidin-4-yl)-phenyl]-thiazol-2-yl-methanol | D | D | B |
| | MS: 424.2 (M+H⁺) | 1H NMR (400 MHz, DMSO-d6) ppm = 9.16 (s, 1H), 8.80 (d, J=3.6, 1H), 7.77 - 7.70 (m, 3H), 7.65 (d, J=3.2, 1H), 7.48 - 7.42 (m, 1H), 7.08 - 6.94 (m, 2H), 6.10 (s 1H), 3.78 - 3.66 (m, 8H) | | | |
| **141** | 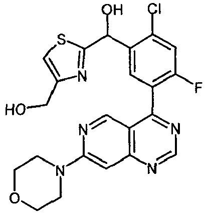 | [2-Chloro-4-fluoro-5-(7-morpholin-4-yl-pyrido[4,3-d]pyrimidin-4-yl)-phenyl]-(4-hydroxymethyl-thiazol-2-yl)-methanol | D | D | B |
| | MS: 488.0/490.0 (M+H⁺) (Cl Isotopie, rel. Peakintensitäts-Verhältnis [%] 100:38) | 1H NMR (400 MHz, DMSO-d6) ppm = 9.15 (s, 1H), 8.84 (d, J=3.4, 1H), 7.83 (d, J=7.6, 1H), 7.74 (d, J=9.7, 1H), 7.39 - 7.35 (m, 1H), 7.04 (s, 1H), 6.99 (s, 1H), 6.28 (s, 1H), 5.24 (s, 1H), 4.50 (s, 2H), 3.77 - 3.69 (m, 8H) | | | |
| **142** | 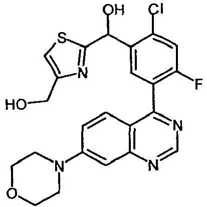 | [2-Chloro-4-fluoro-5-(7-morpholin-4-yl-quinazolin-4-yl)-phenyl]-(4-hydroxymethyl-thiazol-2-yl)-methanol | A | B | D |
| | MS: 487.1/489.0 (M+H⁺) (Cl Isotopie, rel. Peakintensitäts-Verhältnis [%] 100:41) | 1H NMR (400 MHz, DMSO-d6) ppm = 9.10 (s, 1H), 7.75 - 7.69 (m, 2H), 7.59 - 7.49 (m, 2H), 7.36 (t, J=1.1, 1H), 7.23 -7.17 (m, 1H), 7.00 (d, J=5.1, 1H), 6.27 (d, J=5.0, 1H), 5.24 (t, J=5.7, 1H), 4.52 - 4.45 (m, 2H), 3.81 - 3.74 (m, 4H), 3.49 - 3.41 (m, 4H) | | | |
| **143** | 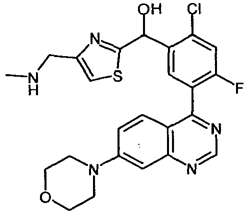 | [2-Chloro-4-fluoro-5-(7-morpholin-4-yl-quinazolin-4-yl)-phenyl]-(4-methylaminomethyl-thiazol-2-yl)-methanol | C | C | C |
| | MS: 500.1/502.0 (M+H⁺) (Cl Isotopie, rel. Peakintensitäts-Verhältnis [%] 100:34) | 1H NMR (500 MHz, DMSO-d6) ppm = 9.10 (s, 1H), 8.26 (s, 1H), 7.76 - 7.69 (m, 2H), 7.58 - 7.50 (m, 2H), 7.48 - 7.43 (m, 1H), 7.24 - 7.16 (m, 1H), 6.97 (s, 1H), 6.28 (s, 1H), 3.80 (s, 2H), 3.79 - 3.74 (m, 4H), 3.48 - 3.42 (m, 4H), 2.33 (s, 3H) | | | |
| **144** | 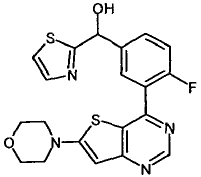 | [4-Fluoro-3-(6-morpholin-4-yl-thieno[3,2-d]pyrimidin-4-yl)-phenyl]-thiazol-2-yl-methanol | A | B | B |
| | MS: 429.0 (M+H⁺) | 1H NMR (500 MHz, DMSO-d6) ppm = 8.89 (s, 1H), 7.76 (dd, J=7.0, 2.3, 1H), 7.73 (d, J=3.2, 1H), 7.69 - 7.64 (m, 2H), 7.43 - 7.38 (m, 1H), 6.96 (d, J=4.6, 1H), 6.53 (s, 1H), 6.07 (d, J=4.6, 1H), 3.77 - 3.71 (m, 4H), 3.44 - 3.38 (m, 4H) | | | |
| **145** | 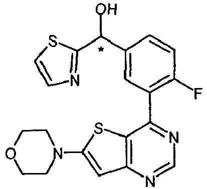 | [4-Fluoro-3-(6-morpholin-4-yl-thieno[3,2-d]pyrimidin-4-yl)-phenyl]-thiazol-2-yl-methanol (*Ena 1*) | A | B | A |
| | MS: 429.0 (M+H⁺); Rₜ 7.85 min, (SFC, Lux Amylose, CO2 / 0.5 vol.% Methanol) | s. Racemat | | | |
| **146** | 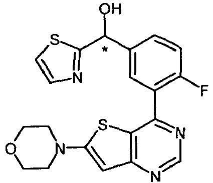 | [4-Fluoro-3-(6-morpholin-4-yl-thieno[3,2-d]pyrimidin-4-yl)-phenyl]-thiazol-2-yl-methanol(*Ena* 2) | A | B | A |
| | MS: 429.0 (M+H⁺); Rₜ 8.82 min, (SFC, Lux Amylose-2, CO2 / 0.5 vol.% Methanol) | s. Racemat | | | |
| **147** | 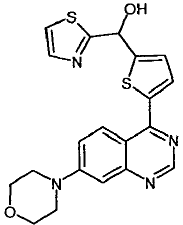 | [5-(7-Morpholin-4-yl-quinazolin-4-yl)-thiophen-2-yl]-thiazol-2-yl-methanol | D | | C |
| | MS: 411.0 (M+H⁺) | 1H NMR (500 MHz, DMSO-d6) ppm = 8.93 (s, 1H), 8.34 (d, J=9.5, 1H), 7.83 (d, J=3.9, 1H), 7.79 (d, J=3.2, 1H), 7.71 (d, J=3.2, 1H), 7.56 (dd, J=9.5, 2.7, 1H), 7.32 (d, J=4.8, 1H), 7.26 - 7.24 (m, 1H), 7.16 (d, J=2.6, 1H), 6.31 - 6.29 (m, 1H), 3.81 - 3.75 (m, 4H), 3.47 - 3.41 (m, 4H) | | | |
| **148** | 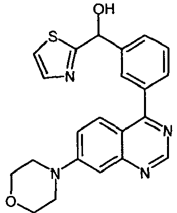 | [3-(7-Morpholin-4-yl-quinazolin-4-yl)-phenyl]-thiazol-2-yl-methanol | C | D | C |
| | MS: 405.2 (M+H⁺) | 1H NMR (500 MHz, DMSO-d6) ppm = 9.07 (s, 1H), 7.86 (d, J=9.4, 1H), 7.83 - 7.81 (m, 1H), 7.74 (d, J=3.2, 1H), 7.68 - 7.64 (m, 3H), 7.59 - 7.55 (m, 1H), 7.52 (dd, J=9.5, 2.6, 1H), 7.20 (d, J=2.6, 1H), 6.93 (d, J=4.5, 1H), 6.10 (d, J=4.0, 1H), 3.80 - 3.76 (m, 4H), 3.46 - 3.42 (m, 4H) | | | |
| **149** | 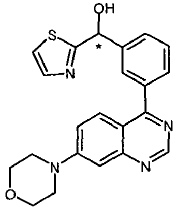 | [3-(7-Morpholin-4-yl-quinazolin-4-yl)-phenyl]-thiazol-2-yl-methanol (*Ena 1*) | D | | A |
| | MS: 405.2 (M+H⁺); Rₜ 10.57min, (HPLC, Chiralpak AD-H, Heptan/Ethanol 40/60) | s. Racemat | | | |
| **150** | 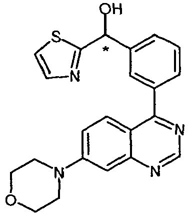 | [3-(7-Morpholin-4-yl-quinazolin-4-yl)-phenyl]-thiazol-2-yl-methanol (*Ena 2*) | C | C | A |
| | MS: 405.2 (M+H⁺); Rₜ 13.55 min, (HPLC, Chiralpak AD-H, Heptan/Ethanol 40/60) | s. Racemat | | | |
| **151** | 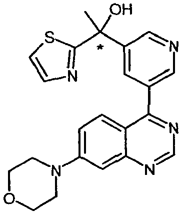 | 1-[5-(7-Morpholin-4-yl-quinazolin-4-yl)-pyridin-3-yl]-1-thiazol-2-yl-ethanol (*Ena 1*) | D | | |
| | MS: 420.2 (M+H⁺); Rₜ 8.95 min, (SFC, Chiralpak AS-H, CO2 / 10 vol.% Methanol, 0.5 vol.% Diethylamin) | s. Racemat | | | |
| **152** | 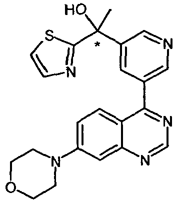 | 1-[5-(7-Morpholin-4-yl-quinazolin-4-yl)-pyridin-3-yl]-1-thiazol-2-yl-ethanol (*Ena 2*) | C | D | A |
| | MS: 420.2 (M+H⁺), Rₜ 10.45min, (SFC, Chiralpak AS-H, CO2 / 10 vol.% Methanol, 0.5 vol.% Diethylamin) | s. Racemat | | | |
| **153** | 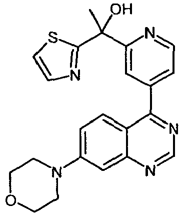 | 1-[4-(7-Morpholin-4-yl-quinazolin-4-yl)-pyridin-2-yl]-1-thiazol-2-yl-ethanol | D | | |
| | MS: 420.2 (M+H⁺) | 1H NMR (400 MHz, DMSO-d6) ppm = 9.11 (s, 1H), 8.73 (dd, J=5.0, 0.8, 1H), 7.99 - 7.97 (m, 1H), 7.79 (d, J=9.4, 1H), 7.72 (d, J=3.2, 1H), 7.64 (dd, J=5.0, 1.6, 1H), 7.62 (d, J=3.2, 1H), 7.56 (dd, J=9.5, 2.6, 1H), 7.23 (d, J=2.5, 1H), 6.84 (s, 1H), 3.81 - 3.75 (m, 4H), 3.49 - 3.44 (m, 4H), 2.04 (s, 3H) | | | |
| **154** | 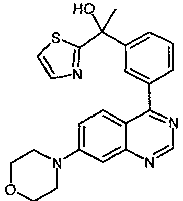 | 1-[3-(7-Morpholin-4-yl-quinazolin-4-yl)-phenyl]-1-thiazol-2-yl-ethanol | D | | B |
| | MS: 419.2 (M+H⁺) | 1H NMR (500 MHz, DMSO-d6) ppm = 9.07 (s, 1H), 7.96 - 7.93 (m, 1H), 7.83 (d, J=9.4, 1H), 7.79 - 7.76 (m, 1H), 7.74 (d, J=3.2, 1H), 7.63 - 7.61 (m, 1H), 7.60 (d, J=3.3, 1H), 7.55 (d, J=7.7, 1H), 7.53 - 7.50 (m, 1H), 7.20 (d, J=2.6, 1H), 6.82 (s, 1H), 3.81 - 3.75 (m, 4H), 3.47 - 3.42 (m, 4H), 1.98 (s 3H) | | | |
| **155** | 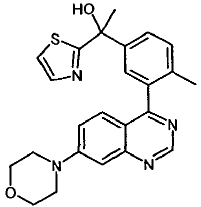 | 1-[4-Methyl-3-(7-morpholin-4-yl-quinazolin-4-yl)-phenyl]-1-thiazol-2-yl-ethanol | D | | |
| | MS: 433.2 (M+H⁺) | 1H NMR (500 MHz, DMSO-d6) ppm = 9.07 (s, 1H), 7.70 (d, J=3.2, 1H), 7.61 (dd, J=8.0, 2.1, 1H), 7.57 (d, J=3.2, 1H), 7.48 - 7.44 (m, 2H), 7.34 (d, J=8.1, 1H), 7.32 (d, J=9.3, 1H), 7.19 (d, J=2.5, 1H), 6.70 (s, 1H), 3.79 - 3.76 (m, 4H), 3.44 - 3.41 (m, 4H), 2.03 (s, 3H), 1.92 (s, 3H) | | | |
| **156** | 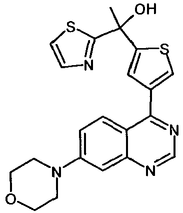 | 1-[4-(7-Morpholin-4-yl-quinazolin-4-yl)-thiophen-2-yl]-1-thiazol-2-yl-ethanol | D | | |
| | MS: 425.0 (M+H⁺) | 1H NMR (400 MHz, DMSO-d6) ppm = 8.99 (s, 1H), 8.13 (d, J=9.5, 1H), 8.04 (d, J=1.5, 1H), 7.76 (d, J=3.2, 1H), 7.64 (d, J=3.2, 1H), 7.54 (dd, J=9.5, 2.6, 1H), 7.52 (d, J=1.5, 1H), 7.20 (s, 1H), 7.16 (d, J=2.6, 1H), 3.82 - 3.75 (m, 4H), 3.47 - 3.40 (m, 4H), 2.04 (s, 3H) | | | |
| **157** | 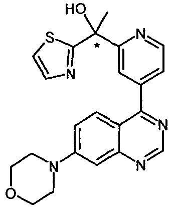 | 1-[4-(7-Morpholin-4-yl-quinazolin-4-yl)-pyridin-2-yl]-1-thiazol-2-yl-ethanol (*Ena 1*) | D | | |
| | MS: 420.2 (M+H⁺); Rₜ 22.14min, (HPLC, Chiralpak AD-H, Heptan/Ethanol 70/30,0.5 vol.% Diethylamin) | s. Racemat | | | |
| **158** | 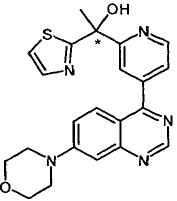 | 1-[4-(7-Morpholin-4-yl-quinazolin-4-yl)-pyridin-2-yl]-1-thiazol-2-yl-ethanol (*Ena 2*) | D | | |
| | MS: 420.2 (M+H⁺); Rₜ 27.88min, (HPLC, Chiralpak AD-H, Heptan/Ethanol 70/30,0.5 vol.% Diethylamin) | s. Racemat | | | |
| **159** | 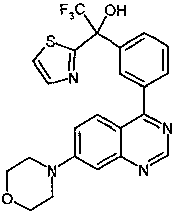 | 2,2,2-Trifluoro-1-[3-(7-morpholin-4-yl-quinazolin-4-yl)-phenyl]-1-thiazol-2-yl-ethanol | C | D | D |
| | MS: 473.0 (M+H⁺) | 1H NMR (500 MHz, DMSO-d6) ppm = 9.08 (s, 1H), 8.54 (s, 1H), 8.15 - 8.13 (m, 1H), 7.98 - 7.95 (m, 1H), 7.94 (d, J=3.3, 1H), 7.87 (d, J=3.2, 1H), 7.83 - 7.79 (m, 2H), 7.67 (t, J=7.8, 1H), 7.53 (dd, J=9.5, 2.6, 1H), 7.21 (d, J=2.6, 1H), 3.80 - 3.76 (m, 4H), 3.47 - 3.43 (m, 4H) | | | |
| **160** | 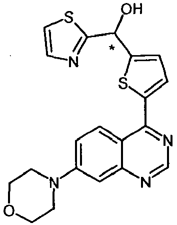 | [5-(7-Morpholin-4-yl-quinazolin-4-yl)-thiophen-2-yl]-thiazol-2-yl-methanol (*Ena 1*) | D | | |
| | MS: 409.0 (M-H⁺); O-TBDPS-Etherderivat: Rₜ 4.78min (SFC, Chiralpak AD-H, CO₂/30 vol.% 2-Propanol, 0.5 vol.% Diethylamin | s. Racemat | | | |
| **161** | 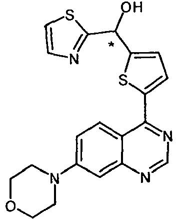 | [5-(7-Morpholin-4-yl-quinazolin-4-yl)-thiophen-2-yl]-thiazol-2-yl-methanol (*Ena 2*) | D | | |
| | MS: 409.0 (M-H⁺); *O-*TBDPS-Etherderivat: Rₜ 7.20 min (SFC, Chiralpak AD-H, CO₂/30 vol.% 2-Propanol, 0.5 vol.% Diethylamin | s. Racemat | | | |
| **162** | 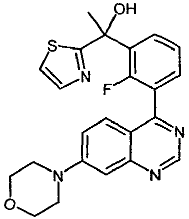 | 1-[2-Fluoro-3-(7-morpholin-4-yl-quinazolin-4-yl)-phenyl]-1-thiazol-2-yl-ethanol | D | | |
| | MS: 437.2 (M+H⁺) | 1H NMR (500 MHz, DMSO-d6) ppm = 9.07 (s, 1H), 7.94 - 7.87 (m, 1H), 7.71 (d, J=3.2, 1H), 7.63 (d, J=3.3, 1H), 7.55 - 7.40 (m, 4H), 7.17 (d, J=2.4, 1H), 6.75 (s, 1H), 3.79 - 3.74 (m, 4H), 3.46 - 3.40 (m, 4H), 2.01 (s, 3H) | | | |
| **163** | 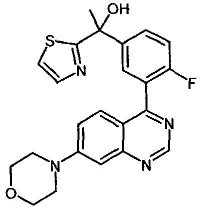 | 1-[4-Fluoro-3-(7-morpholin-4-yl-quinazolin-4-yl)-phenyl]-1-thiazol-2-yl-ethanol | C | D | C |
| | MS: 437.2 (M+H⁺) | 1H NMR (400 MHz, DMSO-d6) ppm = 9.11 (s, 1H), 7.82 - 7.75 (m, 2H), 7.73 (d, J=3.2, 1H), 7.61 (d, J=3.2, 1H), 7.53 - 7.50 (m, 2H), 7.41 - 7.36 (m, 1H), 7.20 (d, J=1.9, 1H), 6.90 (s, 1H), 3.80 - 3.75 (m, 4H), 3.46 - 3.41 (m, 4H), 1.95 (s, 3H) | | | |
| **164** | 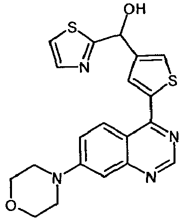 | [5-(7-Morpholin-4-yl-quinazolin-4-yl)-thiophen-3-yl]-thiazol-2-yl-methanol | D | | |
| | MS: 411.0 (M+H⁺) | 1H NMR (500 MHz, DMSO-d6) ppm = 8.95 (s, 1H), 8.30 (d, J=9.5, 1H), 7.93 (d, J=1.3, 1H), 7.80 - 7.78 (m, 1H), 7.76 (d, J=3.2, 1H), 7.67 (d, J=3.2, 1H), 7.62 (dd, J=9.5, 2.7, 1H), 7.17 (d, J=2.6, 1H), 6.93 (d, J=5.0, 1H), 6.14 - 6.11 (m, 1H), 3.81 - 3.76 (m, 4H), 3.48 - 3.43 (m, 4H) | | | |
| **165** | 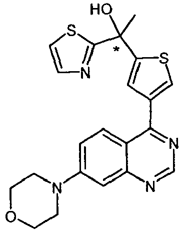 | 1-[4-(7-Morpholin-4-yl-quinazolin-4-yl)-thiophen-2-yl]-1-thiazol-2-yl-ethanol (*Ena 1*) | D | | |
| | MS: 425.0 (M+H⁺); Rₜ 5.32min, (SFC, Chiralcel OJ-H, CO2 / 25 vol.% 2-Propanol, 0.5 vol.% Diethylamin) | 1H NMR (500 MHz, DMSO-d6) ppm = 8.99 (s, 1H), 8.13 (d, J=9.4, 1H), 8.05 (d, J=1.5, 1H), 7.76 (d, J=3.2, 1H), 7.64 (d, J=3.2, 1H), 7.54 (dd, J=9.5, 2.6, 1H), 7.52 (d, J=1.5, 1H), 7.20 (s, 1H), 7.16 (d, J=2.6, 1H), 3.81 - 3.74 (m, 4H), 3.46 - 3.39 (m, 4H), 2.04 (s, 3H) | | | |
| **166** | 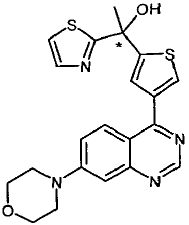 | 1-[4-(7-Morpholin-4-yl-quinazolin-4-yl)-thiophen-2-yl]-1-thiazol-2-yl-ethanol (*Ena 2*) | D | | |
| | MS: 425.0 (M+H⁺); Rₜ 7.18min, (SFC, Chiralcel OJ-H, CO2 / 25 vol.% 2-Propanol, 0.5 vol.% Diethylamin) | 1H NMR (500 MHz, DMSO-d6) ppm = 8.99 (s, 1H), 8.13 (d, J=9.4, 1H), 8.05 (d, J=1.5, 1H), 7.76 (d, J=3.2, 1H), 7.64 (d, J=3.2, 1H), 7.54 (dd, J=9.5, 2.7, 1H), 7.52 (d, J=1.5, 1H), 7.20 (s, 1H), 7.16 (d, J=2.6, 1H), 3.81 - 3.75 (m, 4H), 3.46 - 3.40 (m, 4H), 2.03 (s, 3H) | | | |
| **167** | 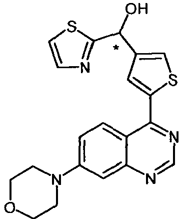 | [5-(7-Morpholin-4-yl-quinazolin-4-yl)-thiophen-3-yl]-thiazol-2-yl-methanol (Ena *1*) | D | | |
| | MS: 411.0 (M+H⁺); *O-*TBDPS-Etherderivat: Rₜ 13.11min (SFC, Chiralpak IA, CO₂ / 20 vol.% 2-Methanol, 0.5 vol.% Diethylamin | 1H NMR (500 MHz, DMSO-d6) ppm = 8.95 (s, 1H), 8.30 (d, J=9.5, 1H), 7.94 - 7.92 (m, 1H), 7.79 - 7.78 (m, 1H), 7.76 (d, J=3.2, 1H), 7.67 (d, J=3.2, 1H), 7.62 (dd, J=9.5, 2.7, 1H), 7.17 (d, J=2.6, 1H), 6.93 (d, J=5.0, 1H), 6.12 (d, J=4.9, 1H), 3.81 - 3.77 (m, 4H), 3.47 - 3.44 (m, 4H) | | | |
| **168** | 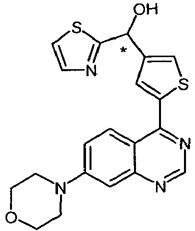 | [5-(7-Morpholin-4-yl-quinazolin-4-yl)-thiophen-3-yl]-thiazol-2-yl-methanol (*Ena 2*) | D | | |
| | MS: 411.0 (M+H⁺); *O-*TBDPS-Etherderivat: Rₜ 16.82min (SFC, Chiralpak IA, CO₂/ 20 vol.% 2-Methanol, 0.5 vol.% Diethylamin | 1H NMR (500 MHz, DMSO-d6) ppm = 8.95 (s, 1H), 8.30 (d, J=9.5, 1H), 7.94 - 7.92 (m, 1H), 7.79 - 7.78 (m, 1H), 7.76 (d, J=3.2, 1H), 7.67 (d, J=3.2, 1H), 7.62 (dd, J=9.5, 2.7, 1H), 7.17 (d, J=2.6, 1H), 6.93 (s, 1H), 6.12 (s, 1H), 3.82 - 3.74 (m, 4H), 3.50 - 3.42 (m, 4H) | | | |
| **169** | 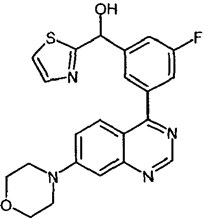 | [3-Fluoro-5-(7-morpholin-4-yl-quinazolin-4-yl)-phenyl]-thiazol-2-yl-methanol | C | D | |
| | MS: 423.0 (M+H⁺) | 1H NMR (500 MHz, DMSO-d6) ppm = 9.08 (s, 1H), 7.86 (d, J=9.4, 1H), 7.77 (d, J=3.2, 1H), 7.68 - 7.66 (m, 2H), 7.54 (dd, J=9.4, 2.6, 1H), 7.51 - 7.46 (m, 2H), 7.21 (d, J=2.6, 1H), 7.08 (d, J=4.7, 1H), 6.14 (d, J=4.7, 1H), 3.80 - 3.76 (m, 4H), 3.47 - 3.43 (m, 4H) | | | |
| **170** | 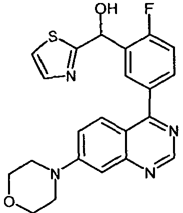 | [2-Fluoro-5-(7-morpholin-4-yl-quinazolin-4-yl)-phenyl]-thiazol-2-yl-methanol | C | C | |
| | MS: 423.0 (M+H⁺) | 1H NMR (500 MHz, DMSO-d6) ppm = 9.04 (s, 1H), 7.85 - 7.81 (m, 2H), 7.77 - 7.73 (m, 1H), 7.72 (d, J=3.2, 1H), 7.66 (d, J=3.2, 1H), 7.52 (dd, J=9.5, 2.6, 1H), 7.44 - 7.39 (m, 1H), 7.18 (d, J=2.5, 1H), 7.05 (d, J=4.9, 1H), 6.28 (d, J=3.8, 1H), 3.79 - 3.75 (m, 4H), 3.33 - 3.29 (m, 4H) | | | |
| **171** | 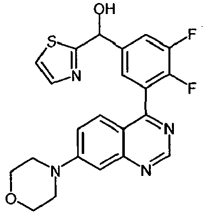 | [3,4-Difluoro-5-(7-morpholin-4-yl-quinazolin-4-yl)-phenyl]-thiazol-2-yl-methanol | A | B | D |
| | MS: 441.0 (M+H⁺) | 1H NMR (500 MHz, DMSO-d6) ppm = 9.11 (s, 1H), 7.75 (d, J=3.2, 1H), 7.72 - 7.66 (m, 2H), 7.61 - 7.57 (m, 1H), 7.56 - 7.49 (m, 2H), 7.21 (d, J=2.4, 1H), 7.10(d, J=4.8, 1H), 6.10 (d, J=4.8, 1H), 3.81 - 3.75 (m, 4H), 3.48 - 3.43 (m, 4H) | | | |
| **172** | 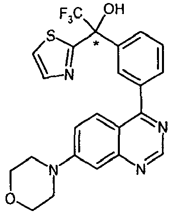 | 2,2,2-Trifluoro-1-[3-(7-morpholin-4-yl-quinazolin-4-yl)-phenyl]-1-thiazol-2-yl-ethanol (*Ena 1*) | C | D | |
| | MS: 473.0 (M+H⁺); Rₜ 12.21min, (HPLC, Chiralpak AD-H, n-Heptan/Ethanol, 70:30, vol.:vol) | s. Racemat | | | |
| **173** | 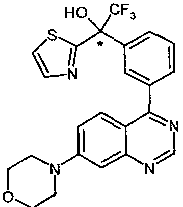 | 2,2,2-Trifluoro-1-[3-(7-morpholin-4-yl-quinazolin-4-yl)-phenyl]-1-thiazol-2-yl-ethanol (*Ena 2*) | A | C | D |
| | MS: 473.0 (M+H⁺); Rₜ 16.91min, (HPLC, Chiralpak AD-H, n-Heptan/Ethanol, 70:30, vol.:vol.) | s. Racemat | | | |
| **174** | 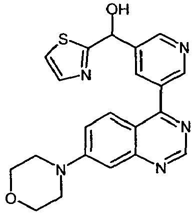 | 7-Morpholin-4-yl-4-piperidin-1-yl-thieno[3,2-d]pyrimidine | C | D | |
| | MS: 305.2 (M+H⁺) | 1H NMR (500 MHz, DMSO-d6) ppm = 9.10 (s, 1H), 8.88 - 8.85 (m, 2H), 8.19 - 8.17 (m, 1H), 7.84 (d, J=9.4, 1H), 7.78 (d, J=3.2, 1H), 7.69 (d, J=3.2, 1H), 7.56 (dd, J=9.5, 2.6, 1H), 7.22 (d, J=2.6, 1H), 7.14 (d, J=4.8, 1H), 6.23 (d, J=4.8, 1H), 3.81 - 3.76 (m, 4H), 3.48 - 3.43 (m, 4H) | | | |
| **175** | 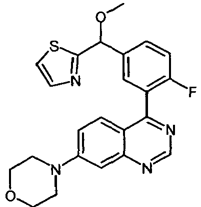 | 4-[2-Fluoro-5-(methoxy-thiazol-2-yl-methyl)-phenyl]-7-morpholin-4-yl-quinazoline | D | | |
| | MS: 337.2 (M+H⁺) | 1H NMR (500 MHz, DMSO-d6) ppm = 9.11 (s, 1H), 7.78 (d, J=3.2, 1H), 7.73 (d, J=3.2, 1H), 7.67 - 7.62 (m, 2H), 7.54 - 7.52 (m, 2H), 7.49 - 7.44 (m, 1H), 7.21 - 7.19 (m, 1H), 5.84 (s, 1H), 3.80 - 3.75 (m, 4H), 3.46 - 3.43 (m, 4H), 3.43 (s, 3H) | | | |
| **176** | 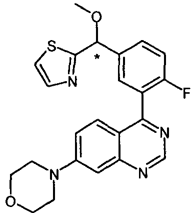 | 4-[2-Fluoro-5-(-methoxy-th iazol-2-yl-methyl)-phenyl]-7-morpholin-4-yl-quinazoline (*Ena 1*) | C | | |
| | MS: 437.2 (M+H⁺); Rₜ 8.47 min, (SFC, Chiralcel OD-H, CO2 / 20 vol.% 2-Propanol, 0.5 vol.% Diethylamin) | s. Racemat | | | |
| **177** | 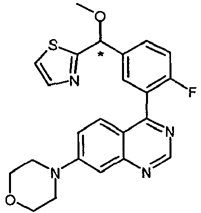 | 4-[2-Fluoro-5-(-methoxy-thiazol-2-yl-methyl)-phenyl]-7-morpholin-4-yl-quinazoline (*Ena 2*) | B | D | A |
| | MS: 437.2 (M+H⁺); Rₜ 9.90 min, (SFC, Chiralcel OD-H, CO2 / 20 vol.% 2-Propanol, 0.5 vol.% Diethylamin) | s. Racemat | | | |
| **178** | 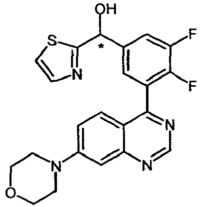 | [3,4-Difluoro-5-(7-morpholin-4-yl-quinazolin-4-yl)-phenyl]-thiazol-2-yl-methanol (*Ena 1*) | A | B | A |
| | MS: 441.0 (M+H⁺); Rₜ 8.34 min, (SFC, Chiralcel OD-H, CO2 / 20 vol.% 2-Propanol, 0.5 vol.% Diethylamin) | s. Racemat | | | |
| **179** | 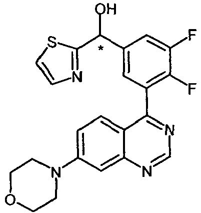 | [3,4-Difluoro-5-(7-morpholin-4-yl-quinazolin-4-yl)-phenyl]-thiazol-2-yl-methanol (*Ena 2*) | B | B | D |
| | MS: 441.0 (M+H⁺); Rₜ 9.68 min, (SFC, Chiralcel OD-H, CO2 / 20 vol.% 2-Propanol, 0.5 vol.% Diethylamin) | s. Racemat | | | |
| **180** | 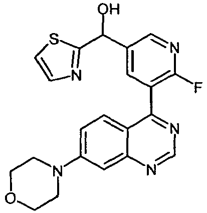 | [6-Fluoro-5-(7-morpholin-4-yl-quinazolin-4-yl)-pyridin-3-yl]-thiazol-2-yl-methanol | A | C | D |
| | MS: 424.2 (M+H⁺) | 1H NMR (400 MHz, DMSO-d6) ppm = 9.11 (s, 1H), 8.56 - 8.52 (m, 1H), 8.21 (dd, J=9.0, 2.3, 1H), 7.77 (d, J=3.2, 1H), 7.69 (d, J=3.2, 1H), 7.59 - 7.52 (m, 2H), 7.23 - 7.20 (m, 1H), 7.18 (d, J=4.7, 1H), 6.23 (d, J=4.5, 1H), 3.82 - 3.73 (m, 4H), 3.50 - 3.42 (m, 4H) | | | |
| **181** | 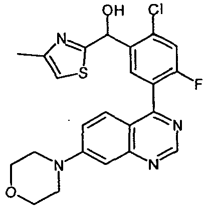 | [2-Chloro-4-fluoro-5-(7-morpholin-4-yl-quinazolin-4-yl)-phenyl]-(4-methyl-thiazol-2-yl)-methanol | A | B | D |
| | MS: 471.1/473.1 (M+H⁺) (Cl Isotopie, rel. Peakintensitäts-Verhältnis [%] 100:42) | 1H NMR (500 MHz, DMSO-d6) ppm = 9.10 (s, 1H), 7.73 (dd, J=10.5, 8.6, 2H), 7.57 - 7.51 (m, 2H), 7.22 - 7.18 (m, 2H), 6.97 (s, 1H), 6.26 (s, 1H), 3.80 - 3.74 (m, 4H), 3.49 - 3.41 (m, 4H), 2.30 (d, J=1.0, 3H) | | | |
| **182** | 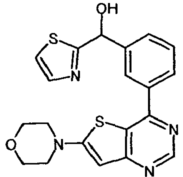 | [3-(6-Morpholin-4-yl-thieno[3,2-d]pyrimidin-4-yl)-phenyl]-thiazol-2-yl-methanol | C | C | |
| | MS: 411.0 (M+H⁺) | 1H NMR (400 MHz, DMSO-d6) ppm = 8.90 (s, 1H), 8.14 (t, J=1.8, 1H), 7.97 - 7.93 (m, 1H), 7.73 (d, J=3.2, 1H), 7.68 - 7.64 (m, 2H), 7.61 - 7.54 (m, 1H), 6.93 (d, J=4.4, 1H), 6.54 (s, 1H), 6.09 (d, J=4.4, 1H), 3.81 - 3.74 (m, 4H), 3.47 - 3.41 (m, 4H) | | | |
| **183** | 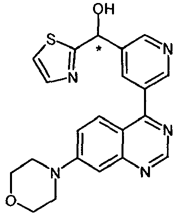 | [5-(7-Morpholin-4-yl-quinazolin-4-yl)-pyridin-3-yl]-thiazol-2-yl-methanol (*Ena 1*) | C | | |
| | MS: 406.0 (M+H⁺); Rₜ 20.67min, (HPLC, Chiralcel OD-H, 2-Propanol) | s. Racemat | | | |
| **184** | 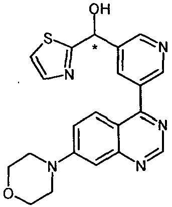 | [5-(7-Morpholin-4-yl-quinazolin-4-yl)-pyridin-3-yl]-thiazol-2-yl-methanol (*Ena 2*) | C | | |
| | MS: 406.0 (M+H⁺); Rt 24.19 min, (HPLC, Chiralcel OD-H, 2-Propanol) | s. Racemat | | | |
| **185** | 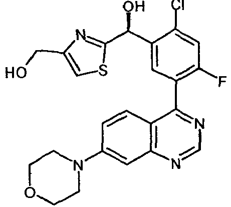 | (S)-[2-Chloro-4-fluoro-5-(7-morpholin-4-yl-quinazolin-4-yl)-phenyl]-(4-hydroxymethyl-thiazol-2-yl)-methanol | A | B | A |
| | MS: 487.1/489.1 (M+H⁺) (Cl Isotopie, rel. Peakintensitäts-Verhältnis [%] 100:41); Rₜ 2.83min (SFC, Chiralpak AD-H, CO₂ / 40 vol.% Methanol, 0.5 vol.% Diethylamin) | 1H NMR (500 MHz, DMSO-d6) ppm = 9.10 (s, 1H), 7.73 (dd, J=8.6, 4.8, 2H), 7.58 - 7.50 (m, 2H), 7.39 - 7.34 (m, 1H), 7.22 - 7.18 (m, 1H), 7.02 (d, J=5.0, 1H), 6.27 (d, J=5.0, 1H), 5.27 (t, J=5.7, 1H), 4.51 - 4.47 (m, 2H), 3.80 - 3.74 (m, 4H), 3.48 - 3.40 (m, 4H) | | | |
| **186** | 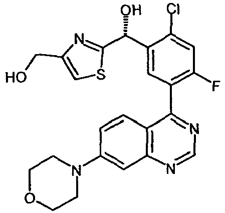 | (R)-[2-Chloro-4-fluoro-5-(7-morpholin-4-yl-quinazolin-4-yl)-phenyl]-(4-hydroxymethyl-thiazol-2-yl)-methanol | C | C | D |
| | MS: 487.1/489.1 (M+H⁺) (Cl Isotopie, rel. Peakintensitäts-Verhältnis [%] 100:41); Rₜ 5.77min (SFC, Chiralpak AD-H, CO₂ / 40 vol.% Methanol, 0.5 vol.% Diethylamin) | 1H NMR (500 MHz, DMSO-d6) ppm = 9.10 (s, 1H), 7.73 (dd, J=8.6, 4.5, 2H), 7.57 - 7.52 (m, 2H), 7.38 - 7.35 (m, 1H), 7.22 - 7.19 (m, 1H), 7.03 (d, J=5.0, 1H), 6.27 (d, J=5.0, 1H), 5.27 (t, J=5.7, 1H), 4.50 - 4.46 (m, 2H), 3.80 - 3.74 (m, 4H), 3.48 - 3.42 (m, 4H) | | | |
| **187** | 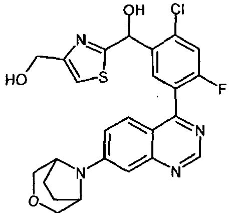 | {2-Chloro-4-fluoro-5-[7-(3-oxa-8-aza-bicyclo[3.2.1]oct-8-yl)-quinazolin-4-yl]-phenyl}-(4-hydroxymethyl-thiazol-2-yl)-methanol | D | D | B |
| | MS: 513.1/515.0 (M+H⁺) (Cl Isotopie, rel. Peakintensitäts-Verhältnis [%] 100:41) | 1H NMR (500 MHz, DMSO-d6) ppm = 9.03 (s, 1H), 7.71 (t, J=8.5, 2H), 7.50 (dd, J=9.3, 3.2, 1H), 7.40 (dd, J=9.4, 2.5, 1H), 7.37 - 7.35 (m, 1H), 7.13 (d, J=2.4, 1H), 7.01 (d, J=5.1, 1H), 6.27 (d, J=5.0, 1H), 5.26 (t, J=5.7, 1H), 4.52 - 4.50 (m, 2H), 4.50 - 4.47 (m, 2H), 3.69 (d, J=10.9, 2H), 3.57 - 3.50 (m, 2H), 2.09 - 1.94 (m, 4H) | | | |
| **188** | 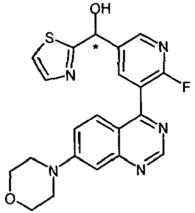 | [6-Fluoro-5-(7-morpholin-4-yl-quinazolin-4-yl)-pyridin-3-yl]-thiazol-2-yl-methanol (*Ena 1*) | A | C | A |
| | MS: 424.0 (M+H⁺); Rₜ 44.51min, (HPLC, Chiralcel OD-H, Hexan/2-Propanol 80/20) | s. Racemat | | | |
| **189** | 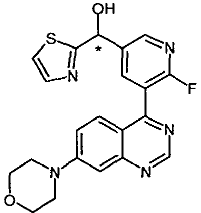 | [6-Fluoro-5-(7-morpholin-4-yl-quinazolin-4-yl)-pyridi n-3-yl]-thiazol-2-yl-methanol (*Ena 2*) | B | B | B |
| | MS: 424.0 (M+H⁺); Rₜ 49.66 min, (HPLC, Chiralcel OD-H, Hexan/2-Propanol 80/20) | s. Racemat | | | |
| **190** | 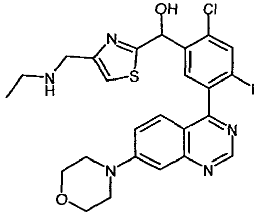 | [2-Chloro-4-fluoro-5-(7-morpholin-4-yl-quinazolin-4-yl)-phenyl]-(4-ethylaminomethyl-thiazol-2-yl)-methanol | C | B | B |
| | MS: 514.2/516.1 (M+H⁺) (Cl Isotopie, rel. Peakintensitäts-Verhältnis [%] 100:41) | 1H NMR (500 MHz, DMSO-d6) ppm = 9.09 (s, 1H), 8.25 (s, 1H), 7.73 (d, J=1.6, 1H), 7.71 (s, 1H), 7.58 - 7.49 (m, 2H), 7.44 - 7.40 (m, 1H), 7.23 - 7.18 (m, 1H), 7.05 (s, 1H), 6.27 (s, 1H), 3.80 - 3.76 (m, 6H), 3.48 - 3.40 (m, 4H), 2.60 (q, J=7.1, 2H), 1.01 (t, J=7.1, 3H) | | | |
| **190** | 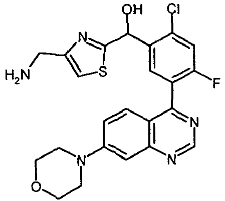 | (4-Aminomethyl-thiazol-2-yl)-[2-chloro-4-fluoro-5-(7-morpholin-4-yl-quinazolin-4-yl)-phenyl]-methanol | C | B | B |
| | MS: 486.0/488.1 (M+H⁺) (Cl Isotopie, rel. Peakintensitäts-Verhältnis [%] 100:38) | 1H NMR (400 MHz, DMSO-d6) ppm = 9.09 (s, 1H), 8.30 (s, 1H), 7.76 - 7.69 (m, 2H), 7.57 - 7.50 (m, 2H), 7.48 - 7.43 (m, 1H), 7.23 - 7.17 (m, 1H), 6.28 (s, 1H), 3.86 (s, 2H), 3.81 - 3.74 (m, 4H), 3.47 - 3.44 (m, 4H) | | | |
| **191** | 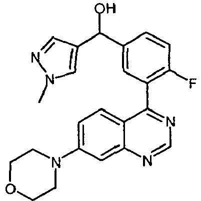 | [4-Fluoro-3-(7-morpholin-4-yl-quinazolin-4-yl)-phenyl]-(1-methyl-1H-pyrazol-4-yl)-methanol | A | D | A |
| | MS: 420.1 (M+H⁺) | 1H NMR (400 MHz, DMSO-d6) ppm = 9.10 (s, 1H), 7.68 - 7.33 (m, 6H), 7.31 - 7.15 (m, 2H), 5.76 (s, 2H), 3.90 - 3.64 (m, 7H), 3.51 - 3.39 (m, 4H) | | | |
| **192** | 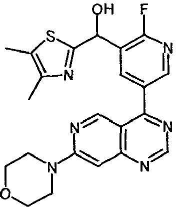 | (4,5-Dimethylthiazol-2-yl)-[2-fluoro-5-(7-morpholin-4-yl-pyrido[4,3-d]pyrimidin-4-yl)-pyridin-3-yl]-methanol | D | | C |
| | MS: 453.0 (M+H⁺) | 1H NMR (400 MHz, DMSO-d6) ppm = 9.14 (s, 1H), 9.09 (s, 1H), 8.67 - 8.65 (m, 1H), 8.46 (dd, J=9.0, 2.4, 1H), 7.03 - 6.99 (m, 2H), 6.11 (d, J=5.2, 1H), 3.77 - 3.68 (m, 8H), 2.31 (s, 3H), 2.19 (s, 3H) | | | |
| **194** | 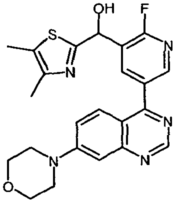 | (4,5-Dimethylthiazol-2-yl)-[2-fluoro-5-(7-morpholin-4-yl-quinazolin-4-yl)-pyridin-3-yl]-methanol | C | | A |
| | MS: 452.2 (M+H⁺) | 1H NMR (400 MHz, DMSO-d6) ppm = 9.10 (s, 1H), 8.58 - 8.56 (m, 1H), 8.37 (dd, J=9.0, 2.4, 1H), 7.87 (d, J=9.4, 1H), 7.57 (dd, J=9.4, 2.6, 1H), 7.22 (d, J=2.5, 1H), 6.99 (d, J=5.1, 1H), 6.11 (d, J=5.1, 1H), 3.80 - 3.76 (m, 4H), 3.49 - 3.43 (m, 4H), 2.30 (s, 3H), 2.19 (s, 3H) | | | |
| **195** | 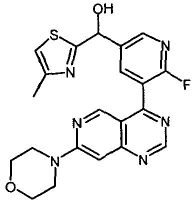 | [6-Fluoro-5-(7-morpholin-4-yl-pyrido[4,3-d]pyrimidin-4-yl)-pyridin-3-yl]-(4-methyl-thiazol-2-yl)-methanol | D | | B |
| | MS: 439.0 (M+H⁺) | 1H NMR (400 MHz, DMSO-d6) ppm = 9.17 (s, 1H), 8.90 (d, J=2.5, 1H), 8.57 - 8.53 (m, 1H), 8.29 (dd, J=9.1, 2.4, 1H), 7.24 - 7.21 (m, 1H), 7.10 (d, J=4.9, 1H), 7.00 (s, 1H), 6.16 (d, J=4.9, 1H), 3.79 - 3.67 (m, 8H), 2.33 (d, J=1.0, 3H) | | | |
| **196** | 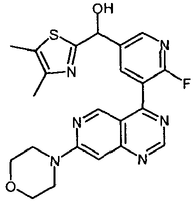 | (4,5-Dimethylthiazol-2-yl)-[6-fluoro-5-(7-morpholin-4-yl-pyrido[4,3-d]pyrimidin-4-yl)-pyridin-3-yl]-methanol | C | D | A |
| | MS: 407.2 (M+H⁺) | 1H NMR (500 MHz, DMSO-d6) ppm = 9.16 (s, 1H), 8.90 (d, J=2.4, 1H), 8.53 (d, J=2.3, 1H), 8.27 (dd, J=9.1, 2.4, 1H), 7.06 (d, J=4.9, 1H), 6.99 (s, 1H), 6.08 (d, J=4.9, 1H), 3.76 - 3.69 (m, 8H), 2.30 (s, 3H), 2.21 (s, 3H) | | | |
| **197** | 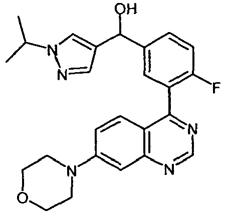 | [4-Fluoro-3-(7-morpholin-4-yl-quinazolin-4-yl)-phenyl]-(1-isopropyl-1H-pyrazol-4-yl)-methanol | B | C | D |
| | MS: 448.2 (M+H⁺) | 1H NMR (500 MHz, DMSO-d6) ppm = 9.10 (s, 1H), 7.64 - 7.60 (m, 1H), 7.60 - 7.57 (m, 2H), 7.56 - 7.50 (m, 2H), 7.41 - 7.35 (m, 1H), 7.28 (s, 1H), 7.20 (d, J=2.2, 1H), 5.76 (s, 2H), 4.42 (h, J=6.6, 1H), 3.80 - 3.76 (m, 4H), 3.46 - 3.42 (m, 4H), 1.36 (d, J=6.7, 6H) | | | |
| **198** | 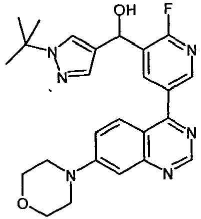 | (1-tert-Butyl-1H-pyrazol-4-yl)-[2-fluoro-5-(7-morpholin-4-yl-quinazolin-4-yl)-pyridin-3-yl]-methanol | C | | A |
| | MS: 463.2 (M+H⁺) | 1H NMR (500 MHz, DMSO-d6) ppm = 9.10 (s, 1H), 8.54 - 8.49 (m, 1H), 8.46 (dd, J=9.4, 2.6, 1H), 7.89 (d, J=9.4, 1H), 7.73 (s, 1H), 7.57 (dd, J=9.5, 2.6, 1H), 7.36 (s, 1H), 7.23 (d, J=2.5, 1H), 6.02 (d, J=4.8, 1H), 5.95 (d, J=4.6, 1H), 3.82 - 3.75 (m, 4H), 3.50 - 3.42 (m, 4H), 1.48 (s, 9H) | | | |
| **199** | 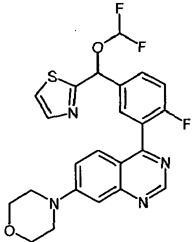 | 4-[5-(Difluoromethoxy-thiazol-2-yl-methyl)-2-fluoro-phenyl]-7-morpholin-4-yl-quinazoline | B | D | D |
| | MS: 473.0 (M+H⁺) | 1H NMR (400 MHz, DMSO-d6) ppm = 9.11 (s, 1H), 7.85 (d, J=3.2, 1H), 7.81 (d, J=3.2, 1H), 7.73 - 7.71 (m, 2H), 7.55 - 7.52 (m, 2H), 7.52 - 7.48 (m, 1H), 7.22 - 6.82 (m, 3H), 3.80 - 3.75 (m, 4H), 3.48 - 3.42 (m, 4H) | | | |
| **200** | 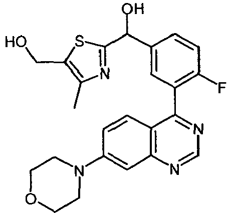 | [4-Fluoro-3-(7-morpholin-4-yl-quinazolin-4-yl)-phenyl]-(5-hydroxymethyl-4-methyl-thiazol-2-yl)-methanol | B | C | A |
| | MS: 467.2 (M+H⁺) | 1H NMR (500 MHz, DMSO-d6) ppm = 9.11 (s, 1H), 7.70 - 7.63 (m, 2H), 7.55 - 7.50 (m, 2H), 7.44 - 7.39 (m, 1H), 7.22 - 7.19 (m, 1H), 6.81 (d, J=4.5, 1H), 5.95 (d, J=4.3, 1H), 5.35 (t, J=5.6, 1H), 4.54 (d, J=5.4, 2H), 3.80 - 3.75 (m, 4H), 3.47 - 3.42 (m, 4H), 2.22 (s, 3H) | | | |
| **201** | 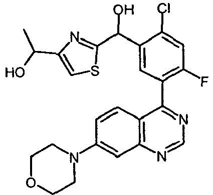 | 1-(2-{[2-Chloro-4-fluoro-5-(7-morpholin-4-yl-quinazolin-4-yl)-phenyl]-hydroxymethyl}-thiazol-4-yl)-ethanol | B | B | B |
| | MS: 501.0/503.0 (M+H⁺) (Cl Isotopie, rel. Peakintensitäts-Verhältnis [%] 100:39) | 1H NMR (500 MHz, DMSO-d6) ppm = 9.11 - 9.09 (m, 1H), 7.75 - 7.70 (m, 2H), 7.55 - 7.52 (m, 2H), 7.34 - 7.32 (m, 1H), 7.21 - 7.19 (m, 1H), 7.00 - 6.96 (m, 1H), 6.28 - 6.25 (m, 1H), 5.26 - 5.18 (m, 1H), 4.77 - 4.70 (m, 1H), 3.79 - 3.75 (m, 4H), 3.47 - 3.43 (m, 4H), 1.36 - 1.30 (m, 3H) | | | |
| **202** | 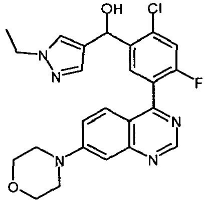 | [2-Chloro-4-fluoro-5-(7-morpholin-4 -yl-quinazolin-4-yl)-phenyl]-(1-eth yl-1H-pyrazol-4-yl)-methanol | C | B | B |
| | MS: 468.0/470.0 (M+H⁺) (Cl Isotopie, rel. Peakintensitäts-Verhältnis [%] 100:35) | 1H NMR (500 MHz, DMSO-d6) ppm = 9.12 (s, 1H), 7.91 - 7.88 (m, 1H), 7.64 (d, J=9.6, 1H), 7.59 (dd, J=9.4, 3.2, 1H), 7.57 - 7.52 (m, 2H), 7.29 (s, 1H), 7.22 - 7.20 (m, 1H), 5.98 (d, J=4.8, 1H), 5.93 (d, J=4.8, 1H), 4.08 - 4.02 (m, 2H), 3.80 - 3.76 (m, 4H), 3.48 - 3.43 (m, 4H), 1.34 - 1.28 (m, 3H) | | | |
| **203** | 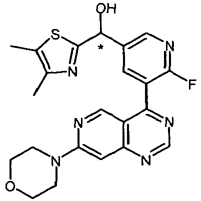 | (4,5-Dimethylthiazol-2-yl)-[6-fluoro-5-(7-morpholin-4-yl-pyrido[4,3-d]pyrimidin-4-yl)-pyridin-3-yl]-methanol (*Ena 2*) | D | D | A |
| | MS: 453.2 (M+H⁺); Rₜ 73.58min, (HPLC, Chiralcel OD-H, Hexan/2-Propanol 90/10) | s. Racemat | | | |
| **204** | 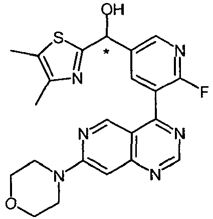 | (R)-(4,5-Dimethylthiazol-2-yl)-[6-fluoro-5-(7-morpholin-4-yl-pyrido[4,3-d]pyrimidin-4-yl)-pyridin-3-yl]-methanol (*Ena 1*) | D | D | C |
| | MS: 453.2 (M+H⁺); Rₜ 68.00min, (HPLC, Chiralcel OD-H, Hexan/2-Propanol 90/10) | s. Racemat | | | |
| **205** | 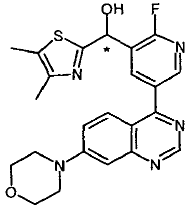 | (4,5-Dimethylthiazol-2-yl)-[2-fluoro-5-(7-morpholin-4-yl-quinazolin-4-yl)-pyridin-3-yl]-methanol (*Ena 1*) | C | D | C |
| | MS: 452.2 (M+H⁺); Rt 24.50min, (HPLC, Chiralpak AD-H, Ethanol) | s. Racemat | | | |
| **206** | 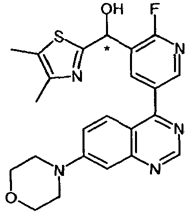 | (4,5-Dimethylthiazol-2-yl)-[2-fluoro-5-(7-morpholin-4-yl-quinazolin-4-yl)-pyridin-3-yl]-methanol (*Ena 2*) | D | | B |
| | MS: 452.2 (M+H⁺); Rt 27.44min, (HPLC, Chiralpak AD-H, Ethanol) | s. Racemat | | | |

### BEISPIEL 207

### [4-Fluor-2-methyl-5-(7-morpholinyl-chinazolin-4-yl)phenyl]-thiazol-2-yl-methanol (BEISPIEL 207)

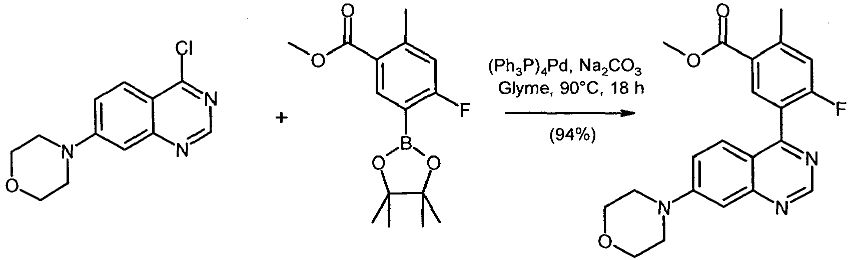

Ausgehend von 4-Chlor-7-morpholin-4-yl-chinazolin und 4-Fluor-2-methyl-5-(4,4,5,5-tetramethyl-1,3,2-dioxaborolan-2-yl)-benzoesäuremethylester wurde 4-Fluor-2-methyl-5-(7-morpholinyl-chinazolin-4-yl)benzoesäuremethylester in analoger Weise zu den unter den BEISPIELEN 1 und 2 beschriebenen Syntheseverfahren hergestellt.

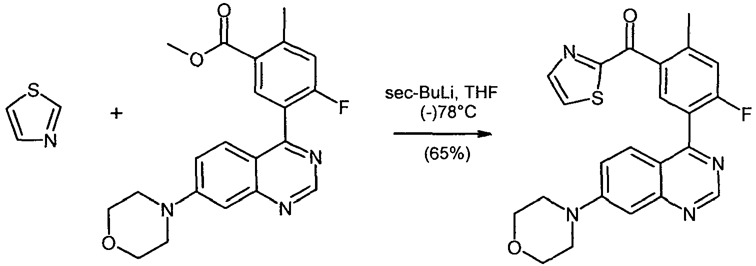

Ausgehend von Thiazol und 4-Fluor-2-methyl-5-(7-morpholinyl-chinazolin-4-yl)benzoesäuremethylester wurde [4-Fluor-2-methyl-5-(7-morpholinyl-chinazolin-4-yl)phenyl]-thiazol-2-yl-methanon in analoger Weise zu den unter dem BEISPIEL 138 beschriebenen Syntheseverfahren hergestellt.

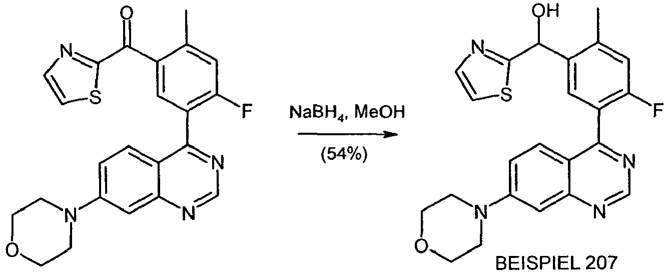

Ausgehend von [4-Fluor-2-methyl-5-(7-morpholinyl-chinazolin-4-yl)phenyl]-thiazol-2-yl-methanon wurde [4-Fluor-2-methyl-5-(7-morpholinyl-chinazolin-4-yl)phenyl]-thiazol-2-yl-methanol (BEISPIEL 207) in analoger Weise zu den unter den BEISPIELEN 1 und 2 beschriebenen Syntheseverfahren hergestellt.

### BEISPIEL 208

### [3-(2-Ethinyl-7-morpholinyl-chinazolin-4-yl)-4-fluor-phenyl]-thiazol-2-yl-methanol (BEISPIEL 208)

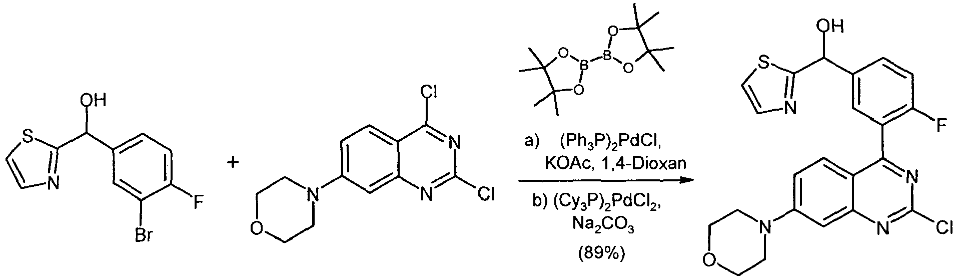

Ausgehend von (3-Brom-4-fluor-phenyl)-thiazol-2-yl-methanol und 4-(2,4-Dichlor-chinazolin-7-yl)morpholin wurde [3-(2-Chlor-7-morpholinyl-chinazolin-4-yl)-4-fluor-phenyl]-thiazol-2-yl-methanol in analoger Weise zu den unter dem BEISPIEL 138 beschriebenen Syntheseverfahren hergestellt.

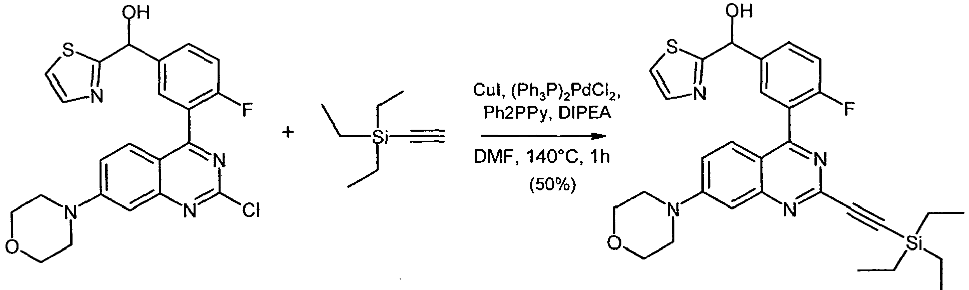

[3-(2-Chlor-7-morpholinyl-chinazolin-4-yl)-4-fluor-phenyl]-thiazol-2-yl-methanol (102 mg, 0,225 mmol) wurde in Sauerstoff-freiem N,N-Dimethylformamid (4 ml) in einer Argonatmosphäre gelöst. Anschließend wurden Cul (17 mg, 90 µmol), (Ph₃P)₂PdCl₂ (63 mg, 90 µmol), 2-Diphenylphosphanyl-pyridin (95 mg, 0,359 mmol), DIPEA (765 µl, 4,49 mmol) und Triethylethinylsilan (275 µl, 1,48 mmol) zugegeben. Danach wurde die Reaktionsmischung 1 h bei einer Temperatur von 140°C erhitzt. Zur Aufarbeitung wurden Essigsäureethylester (50 ml), Wasser (10 ml) und gesättigter Natriumchlorid-Lösung (15 ml) zugefügt. Die wässrige Phase wurde abgetrennt und mit Essigsäureethylester (20 ml) extrahiert. Die vereinigten organischen Phasen wurden über Natriumsulfat getrocknet, filtriert und das Filtrat im Vakuum zu Trockne eingeengt. Der Rückstand wurde in Dimethylsulfoxid (2 ml) gelöst mittels RP-Chromatographie (Lösemittel: Acetonitril/Wasser/0,1 vol.% HCOOH, CombiFlash Rf 200) aufgereinigt. Die geeigneten Produktfraktionen wurden vereinigt und die Lösungsmittel am Rotationsverdampfer entfernt, wobei [4-Fluor-3-[7-morpholinyl-2-(2-triethylsilylethinyl)chinazolin-4-yl]phenyl]-thiazol-2-yl-methanol (64 mg, 0.114 mmol, MS: 561,2 [M+H⁺], 50% Ausbeute) als wachsartiger Feststoff erhalten wurde.

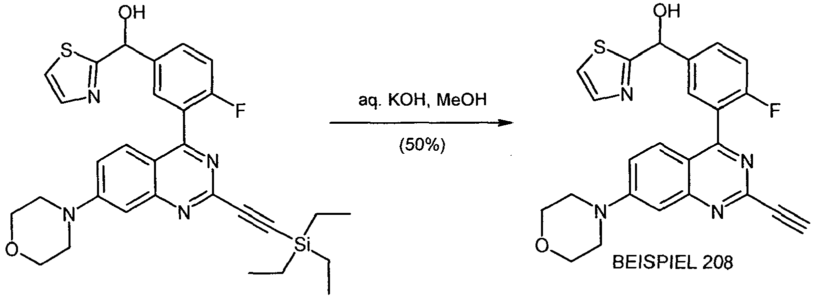

[4-Fluor-3-[7-morpholinyl-2-(2-triethylsilylethinyl)chinazolin-4-yl]phenyl]-thiazol-2-yl-methanol (552 mg, 0.985 mmol) wurde in Methanol (102 ml) gelöst. Anschließend wurde Kaliumhydroxid-Lösung (1,0 M, 15 ml, 15 mmol) zugegeben und 90 min bei Raumtemperatur gerührt. Nach beendeter Reaktion wurde mit Salzsäure (1,0 M, 15 ml, 15 mmol) vorsichtig neutralisiert. Anschließend wurden Essigsäureethylester (500 ml), Wasser (100 ml) und gesättigte Natriumchlorid-Lösung (150 ml) zugefügt. Die Phasen wurden getrennt und die wässrige Phase mit Essigsäureethylester (100 ml) extrahiert. Die vereinigten organischen Phasen wurden über Natriumsulfat getrocknet, filtriert und das Filtrat im Vakuum zu Trockne eingeengt. Der Rückstand wurde in Dimethylsulfoxid (8 ml) gelöst mittels Flash-Säulenchromatographie (Gradient: Dichlormethan/0-5vol.% Ethanol, CombiFlash Rf 200) aufgereinigt. Die geeigneten Produktfraktionen wurden vereinigt und die Lösungsmittel am Rotationsverdampfer entfernt, wobei [3-(2-Ethinyl-7-morpholinyl-chinazolin-4-yl)-4-fluorphenyl]-thiazol-2-yl-methanol (BEISPIEL 208, 221 mg, 0,495 mmol, MS: 447,1 [M+H⁺], 50% Ausbeute) als Feststoff erhalten wurde.

Verbindungen die analog der BEISPIELE 207 un 208 hergestellt wurden, finden sich in der nachfolgenden Tabelle 6.

**Tabelle 6: Verbindungen der Formel (I)**

| Nr. | Strukturformel | Name | IC₅₀ DNA-PK | IC₅₀ pDNA-PK | Kᵢ [Kv1.11 hERG] |
|---|---|---|---|---|---|
| **207** | 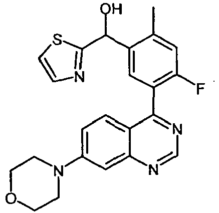 | [4-Fluoro-2-methyl-5-(7-morpholin-4-yl-quinazolin-4-yl)-phenyl]-thiazol-2-yl-methanol | A | B | B |
| | MS: 437.1 (M+H⁺) | 1H NMR (500 MHz, DMSO-d6) ppm = 9.09 (s, 1H), 7.71 (d, J=3.2, 1H), 7.64 (d, J=3.2, 1H), 7.62 (d, J=7.5, 1H), 7.58 - 7.51 (m, 2H), 7.28 (d, J=10.9, 1H), 7.19 (d, J=2.1, 1H), 6.84 (s, 1H), 6.20 (s, 1H), 3.80 - 3.75 (m, 4H), 3.48 - 3.43 (m, 4H), 2.49 (s, 3H) | | | |
| **208** | 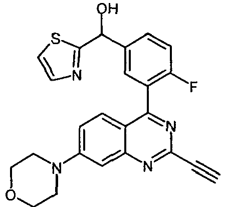 | [3-(2-Ethynyl-7-morpholin-4-yl-quinazolin-4-yl)-4-fluoro-phenyl]-thiazol-2-yl-methanol | B | B | D |
| | MS: 447.0 (M+H⁺) | 1H NMR (500 MHz, DMSO-d6) ppm = 7.81 - 7.47 (m, 6H), 7.46 - 7.36 (m, 1H), 7.18 (d, J=2.4, 1H), 6.99 (d, J=4.6, 1H), 6.08 (d, J=4.3, 1H), 4.30 (s, 1H), 3.80 - 3.74 (m, 4H), 3.49 - 3.41 (m, 4H) | | | |
| **209** | 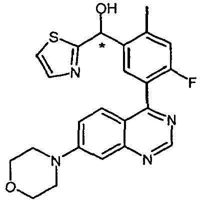 | [4-Fluoro-2-methyl-5-(7-morpholin-4-yl-quinazolin-4-yl)-phenyl]-thiazol-2-yl-methanol (*Ena 1*) | D | D | A |
| | MS: 437.1 (m+H⁺); Rₜ 4.77min (SFC, Chiracel OJ-H, CO₂/ 15 Vol.% Methanol, 0.5 vol.% Diethylamin) | 1H NMR (400 MHz, DMSO-d6) ppm = 9.09 (s, 1H), 7.72 (d, J=3.3, 1H), 7.68 - 7.59 (m, 2H), 7.60 - 7.47 (m, 2H), 7.28 (d, J=10.9, 1H), 7.19 (s, 1H), 6.99 - 6.71 (m, 1H), 6.20 (s, 1H), 3.82 - 3.73 (m, 4H), 3.48 - 3.42 (m, 4H), 2.58 - 2.50 (m, 3H) | | | |
| **210** | 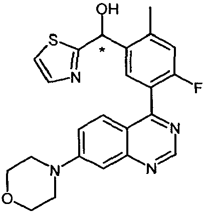 | [4-Fluoro-2-methyl-5-(7-morpholin-4-yl-quinazolin-4-yl)-phenyl]-thiazol-2-yl-methanol (*Ena 2*) | B | B | A |
| | MS: 437.1 (M+H⁺); Rₜ 6.01 min (SFC, Chiracel OJ-H, CO₂/15 vol.% Methanol, 0.5 vol.% Diethylamin) | 1H NMR (400 MHz, DMSO-d6) ppm = 9.09 (s, 1H), 7.72 (d, J=3.3, 1H), 7.68 - 7.59 (m, 2H), 7.60 - 7.47 (m, 2H), 7.28 (d, J=10.9, 1H), 7.19 (s, 1H), 6.99 - 6.71 (m, 1H), 6.20 (s, 1H), 3.82 - 3.73 (m, 4H), 3.48 - 3.42 (m, 4H), 2.58 - 2.50 (m, 3H) | | | |
| **211** | 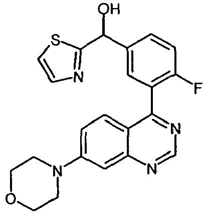 | [4-Fluoro-3-(7-morpholin-4-yl-quinazolin-4-yl)-phenyl]-thiazol-2-yl-methanol | B | B | A |
| | MS: 423.1 (M+H⁺) | 1H NMR (400 MHz, DMSO-d6) ppm = 9.10 (s, 1H), 7.78 - 7.61 (m, 4H), 7.57 - 7.49 (m, 2H), 7.46 - 7.37 (m, 1H), 7.23 - 7.17 (m, 1H), 6.95 (s, 1H), 6.08 (s, 1H), 3.81 - 3.74 (m, 4H), 3.48 - 3.41 (m, 4H) | | | |
| **212** | 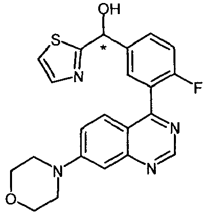 | [4-Fluoro-3-(7-morpholin-4-yl-quinazolin-4-yl)-phenyl]-thiazol-2-yl-methanol (*Ena 1*) | A | D | A |
| | MS: 423.1 (M+H⁺); Rₜ 15.65min (SFC, Chiracel OJ-H, CO₂/10 vol.% Methanol, 0.5 vol.% Diethylamin) | 1H NMR (400 MHz, DMSO-d6) ppm = 9.10 (s, 1H), 7.73 (d, J=3.2, 1H), 7.71 - 7.63 (m, 3H), 7.55 - 7.49 (m, 2H), 7.44 - 7.38 (m, 1H), 7.22 - 7.18 (m, 1H), 6.95 (d, J=4.6, 1H), 6.08 (d, J=4.5, 1H), 3.81 - 3.75 (m, 4H), 3.47 - 3.42 (m, 4H) | | | |
| **213** | 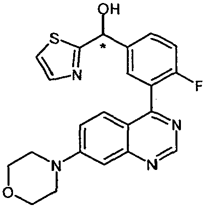 | [4-Fluoro-3-(7-morpholin-4-yl-quinazolin-4-yl)-phenyl]-thiazol-2-yl-methanol (*Ena 2*) | A | C | A |
| | MS: 423.1 (M+H⁺); Rₜ 18.36min (SFC, Chiracel OJ-H, CO₂/ 10 vol.% Methanol, 0.5 vol.% Diethylamin) | 1H NMR (400 MHz, DMSO-d6) ppm = 9.10 (s, 1H), 7.73 (d, J=3.2, 1H), 7.72 - 7.65 (m, 2H), 7.65 - 7.64 (m, 1H), 7.54 - 7.52 (m, 2H), 7.44 - 7.39 (m, 1H), 7.21 - 7.19 (m, 1H), 6.96 (d, J=4.6, 1H), 6.08 (d, J=4.6, 1H), 3.80 - 3.75 (m, 4H), 3.46 - 3.42 (m, 4H) | | | |
| **214** | 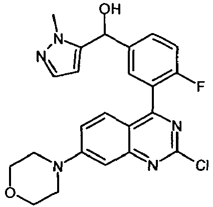 | [3-(2-Chloro-7-morpholin-4-yl-quinazolin-4-yl)-4-fluoro-phenyl]-(2-methyl-2H-pyrazol-3-yl)-methanol | B | D | B |
| | MS: 454.1/456.1 (M+H⁺) (Cl Isotopie, rel. Peakintensitäts- Verhältnis [%] 100:35) | 1H NMR (500 MHz, DMSO-d6) ppm = 7.71 - 7.59 (m, 3H), 7.58 - 7.51 (m, 2H), 7.50 - 7.41 (m, 1H), 7.31 - 7.26 (m, 1H), 7.18 - 7.12 (m, 1H), 5.99 (s, 1H), 5.95 - 5.90 (m, 1H), 3.78 (s, 3H), 3.78 - 3.73 (m, 4H), 3.52 - 3.45 (m, 4H) | | | |
| **215** | 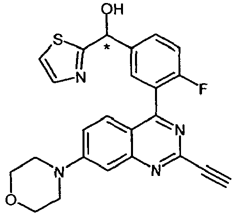 | [3-(2-Ethynyl-7-morpholin-4-yl-quinazolin-4-yl)-4-fluoro-phenyl]-thiazol-2-yl-methanol (*Ena 1*) | A | C | D |
| | MS: 447.1 (M+H⁺); Rₜ 10.67min (HPLC, Chiralpak AD-H, n-Heptan / 40 vol.% 2-Propanol | 1H NMR (500 MHz, DMSO-d6) ppm = 7.73 (d, J=3.2, 1H), 7.72 - 7.65 (m, 2H), 7.65 (d, J=3.2, 1H), 7.53 (qd, J=9.4, 2.7, 2H), 7.46 - 7.38 (m, 1H), 7.18 (d, J=2.3, 1H), 6.97 (d, J=4.6, 1H), 6.09 (d, J=4.6, 1H), 4.30 (s, 1H), 3.80 - 3.74 (m, 4H), 3.49 - 3.43 (m, 4H) | | | |
| **216** | 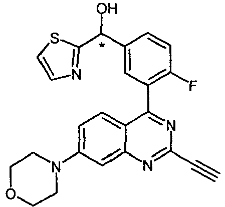 | [3-(2-Ethynyl-7-morpholin-4-yl-quinazolin-4-yl)-4-fluoro-phenyl]-thiazol-2-yl-methanol (*Ena 2*) | A | D | C |
| | MS: 447.1 (M+H⁺); Rₜ 13.14min (HPLC, Chiralpak AD-H, n-Heptan / 40 vol.% 2-Propanol | 1H NMR (500 MHz, DMSO-d6) ppm = 7.73 (d, J=3.2, 1H), 7.72 - 7.65 (m, 2H), 7.65 (d, J=3.2, 1H), 7.53 (qd, J=9.4, 2.7, 2H), 7.46 - 7.38 (m, 1H), 7.18 (d, J=2.3, 1H), 6.97 (d, J=4.6, 1H), 6.09 (d, J=4.6, 1H), 4.30 (s, 1H), 3.80 - 3.74 (m, 4H), 3.49 - 3.43 (m, 4H) | | | |

### BEISPIEL 217

### [2,4-Difluor-5-7-morpholin-4-yl-chinazolin-4-yl)-phenyl]-pyridin-3-yl-methanol (217)

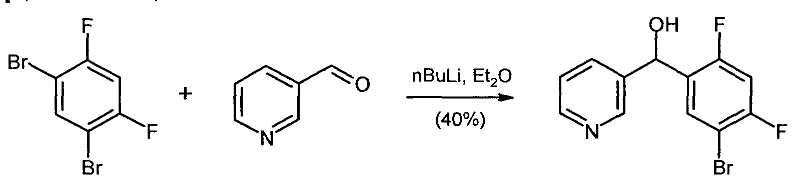

Unter Argon wurde 1,5-Dibrom-2,4-difluor-benzen (500 mg, 1,78 mmol) in trockenem Diethylether (10 ml) gelöst. Die Reaktionslösung wurde mittels Aceton/Trockeneis-Bad auf (-)65°C gekühlt. n-Butyllithium (15%ige Lösung in n-Hexan, 1,23 ml, 1,96 mmol) wurde über 15 min bei einer konstanten Temperatur von (-)65°C zugetropft und die Reaktionslösung weitere 30 min bei (-)65°C gerührt. Anschließend wurde eine vorbereitete Lösung von Nicotinaldehyd (201 µl, 2,14 mmol) in trockenen Diethylether (5 ml) bei (-)65°C über einen Zeitraum von 15 min zugetropft und die Reaktionsmischung weitere 10 min gerührt, um danach über den Zeitraum von einer Stunde langsam auf 0°C zu erwärmen. Nach Beendigung der Reaktion wurden gesättigte Ammoniumchlorid-Lösung (5 ml) und Wasser (30 ml) der Reaktionslösung zugeführt. Die wässrige Phase wurde dreimal mit t-Butylmethylether extrahiert. Die vereinigten organischen Phasen wurden mit gesättigter Natriumchloridlösung gewaschen, über Natriumsulfat getrocknet, filtriert und am Rotationsverdampfer eingeengt. Das Rohprodukt (Öl) wurde mittels präparativer RP-Säulenchromatographie aufgereinigt (Lösemittelgradient Wasser/Acetonitril/0,1vol.% Trifluoressigsäure [5,5 min], CombiFlash Rf 200). Die geeigneten Produktfraktionen wurden vereinigt und im Vakuum eingeengt. Der wässrige Rückstand wurde mit gesättigter Natriumhydrogencarbonat-Lösung neutralisiert und dreimal mit Essigsäureethylester extrahiert. Die vereinigten organischen Phasen wurden mit gesättigter Natriumchloridlösung gewaschen, über Natriumsulfat getrocknet, filtriert und im Vakuum am Rotationsverdampfer zur Trockne eingeengt, wobei (5-Brom-2,4-difluor-phenyl)-(3-pyridyl)methanol (215 mg, 0,717 mmol, MS: 300,0/302,0 [M+H^{+]}, 40% Ausbeute) als farbloses Öl erhalten wurde.

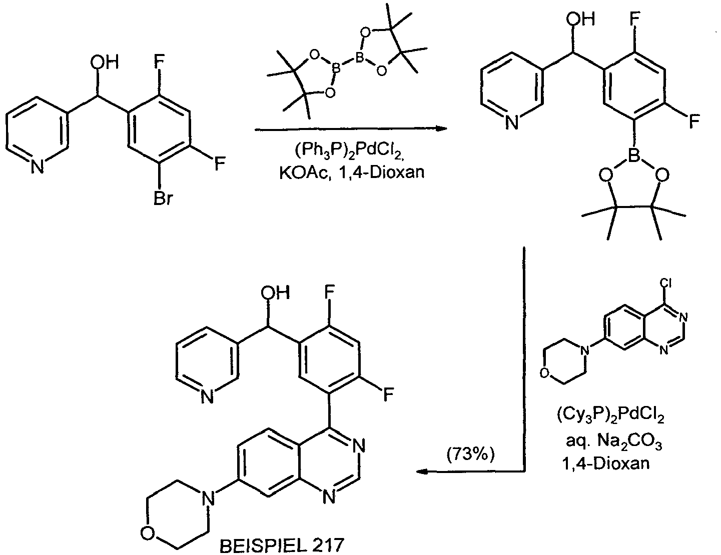

[2,4-Difluor-5-(7-morpholin-4-yl-chinazolin-4-yl)-phenyl]-pyridin-3-yl-methanol (BEISPIEL 217) wurde ausgehend von (5-Brom-2,4-difluor-phenyl)-(3-pyridyl)methanol analog der Syntheseverfahren zur Herstellung von 1-[5-(7-Morpholin-4-yl-chinazolin-4-yl)-pyridin-3-yl]-1-thiazol-2-yl-ethanol (BEISPIEL 138) erhalten.

Verbindungen die analog BEISPIEL 217 hergestellt wurden, finden sich in der nachfolgenden Tabelle 7.

**Tabelle 7: Verbindungen der Formel (I)**

| Nr. | Strukturformel | Name | IC₅₀ DNA-PK | IC₅₀ pDNA-PK | Kᵢ [Kv1.1 1 hERG] |
|---|---|---|---|---|---|
| **217** | 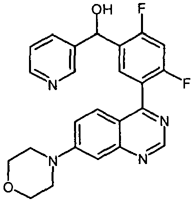 | [2,4-Difluoro-5-(7-morpholin-4-yl-quinazolin-4-yl)-phenyl]-pyridin-3-yl-methanol | B | A | A |
| | MS: 435.2 (M+H⁺) | 1H NMR (500 MHz, DMSO-d6) ppm = 9.11 (s, 1H), 8.62 - 8.59 (m, 1H), 8.47 (dd, J=4.8, 1.6, 1H), 7.84 (t, J=8.2, 1H), 7.77 - 7.72 (m, 1H), 7.55 (qd, J=9.4, 2.8, 2H), 7.46 (t, J=10.1, 1H), 7.39 - 7.34 (m, 1H), 7.21 (d, J=2.4, 1H), 6.36 (d, J=4.5, 1H), 6.06 (d, J=4.5, 1H), 3.81 - 3.74 (m, 4H), 3.48 - 3.42 (m, 4H) | | | |
| **218** | 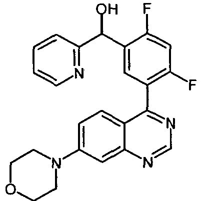 | [2,4-Difluoro-5-(7-morpholin-4-yl-quinazolin-4-yl)-phenyl]-pyridin-2-yl-methanol | B | A | B |
| | MS: 435.2 (M+H⁺) | 1H NMR (500 MHz, DMSO-d6) ppm = 9.09 (s, 1H), 8.50 - 8.46 (m, 1H), 7.82 (td, J=7.7, 1.8, 1H), 7.68 (t, J=8.1, 1H), 7.64 - 7.59 (m, 1H), 7.57 - 7.50 (m, 2H), 7.43 (t, J=10.1, 1H), 7.29 - 7.24 (m, 1H), 7.21 - 7.17 (m, 1H), 6.34 (d, J=5.0, 1H), 6.02 (d, J=5.0, 1H), 3.82 - 3.72 (m, 4H), 3.48 - 3.41 (m, 4H). | | | |
| **219** | 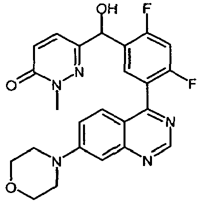 | 6-{[2,4-Difluoro-5-(7-morpholin-4-yl-quinazolin-4-yl)-phenyl]-hydroxymethyl}-2-methyl-2H-pyridazin-3-one | B | B | B |
| | MS: 466.2 (M+H⁺) | 1H NMR (500 MHz, DMSO-d6) ppm = 9.11 (s, 1H), 7.85 (t, J=8.1, 1H), 7.61 - 7.51 (m, 3H), 7.47 (t, J=10.1, 1H), 7.21 (d, J=2.3, 1H), 6.94 (d, J=9.6, 1H), 6.56 (d, J=4.8, 1H), 5.85 (d, J=4.8, 1H), 3.81 - 3.75 (m, 4H), 3.60 (s, 3H), 3.48 - 3.42 (m, 4H) | | | |
| **220** | 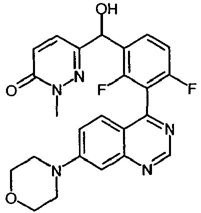 | 6-{[2,4-Difluoro-3-(7-morpholin-4-yl-quinazolin-4-yl)-phenyl]-hydroxymethyl}-2-methyl-2H-pyridazin-3-one | D | C | A |
| | MS: 466.2 (M+H⁺) | 1H NMR (500 MHz, DMSO-d6) ppm = 9.10 (s, 1H), 7.75 (d, J=9.6, 1H), 7.67 - 7.61 (m, 1H), 7.51 (qd, J=9.4, 2.6, 2H), 7.32 (t, J=8.8, 1H), 7.20 (d, J=2.3, 1H), 6.99 (d, J=9.7, 1H), 6.64 (d, J=5.1, 1H), 6.02 (d, J=5.1, 1H), 3.78 (t, J=4.9, 4H), 3.57 (s, 3H), 3.45 (t, J=4.9, 4H) | | | |
| **221** | 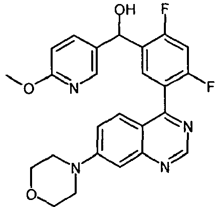 | [2,4-Difluoro-5-(7-morpholin-4-yl-quinazolin-4-yl)-phenyl]-(6-methoxy-pyridin-3-yl)-methanol | B | B | B |
| | MS: 465.2 (M+H⁺) | 1H NMR (500 MHz, DMSO-d6) ppm = 9.11 (s, 1H), 8.17 (d, J=2.4, 1H), 7.84 (t, J=8.2, 1H), 7.63 (dd, J=8.6, 2.5, 1H), 7.60 - 7.52 (m, 2H), 7.44 (t, J=10.2, 1H), 7.21 (d, J=2.3, 1H), 6.79 (d, J=8.6, 1H), 6.22 (d, J=4.0, 1H), 5.99 (d, J=3.7, 1H), 3.83 (s, 3H), 3.80 - 3.76 (m, 4H), 3.47 - 3.43 (m, 4H) | | | |
| **222** | 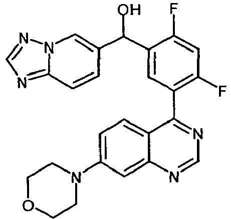 | [2,4-Difluoro-5-(7-morpholin-4-yl-quinazolin-4-yl)-phenyl]-[1,2,4]triazolo[1,5-a]pyridin-6-yl-methanol | B | B | A |
| | MS: 475.2 (M+H⁺) | 1H NMR (500 MHz, DMSO-d6) ppm = 9.10 (s, 1H), 8.98 - 8.95 (m, 1H), 8.49 (s, 1H), 7.88 (t, J=8.1, 1H), 7.83 - 7.80 (m, 1H), 7.62 - 7.56 (m, 2H), 7.53 (dd, J=9.5, 2.5, 1H), 7.48 (t, J=10.1, 1H), 7.20 (d, J=2.4, 1H), 6.52 (s, 1H), 6.17 (s, 1H), 3.82 - 3.74 (m, 4H), 3.49 - 3.41 (m, 4H) | | | |
| **223** | 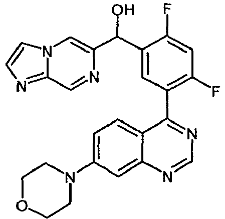 | [2,4-Difluoro-5-(7-morpholin-4-yl-quinazolin-4-yl)-phenyl]-imidazo[1,2-a]pyrazin-6-yl-methanol | C | B | A |
| | MS: 475.2 (M+H⁺) | 1H NMR (500 MHz, DMSO-d6) ppm = 9.13 (s, 1H), 9.06 - 9.02 (m, 1H), 8.83 (t, J=1.3, 1H), 8.25 - 8.22 (m, 1H), 7.86 (d, J=1.0, 1H), 7.76 (t, J=8.1, 1H), 7.63 -7.56 (m, 2H), 7.52 (t, J=10.1, 1H), 7.24 (d, J=2.1, 1H), 6.53 (d, J=4.7, 1H), 6.16 (d, J=4.5, 1H), 3.87 - 3.80 (m, 4H), 3.54 - 3.46 (m, 4H) | | | |
| **224** | 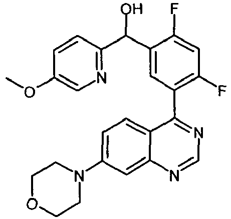 | [2,4-Difluoro-5-(7-morpholin-4-yl-quinazolin-4-yl)-phenyl]-(5-methoxy-pyridin-2-yl)-methanol | B | B | B |
| | MS: 465.2 (M+H⁺) | 1H NMR (500 MHz, DMSO-d6) ppm = 9.09 (s, 1H), 8.20 - 8.17 (m, 1H), 7.68 (t, J=8.1, 1H), 7.55 - 7.51 (m, 3H), 7.43 - 7.37 (m, 2H), 7.21 - 7.18 (m, 1H), 6.23 (d, J=4.9, 1H), 5.98 (d, J=4.6, 1H), 3.80 (s, 3H), 3.80 - 3.74 (m, 4H), 3.48 - 3.41 (m, 4H) | | | |

BEISPIEL 225:

### [2-Chlor-5-(5,6-dideuterio-7-morpholin-chinazolin-4-yl)-4-fluor-phenyl]-(6-methoxypyridazin-3-yl)methanol (225)

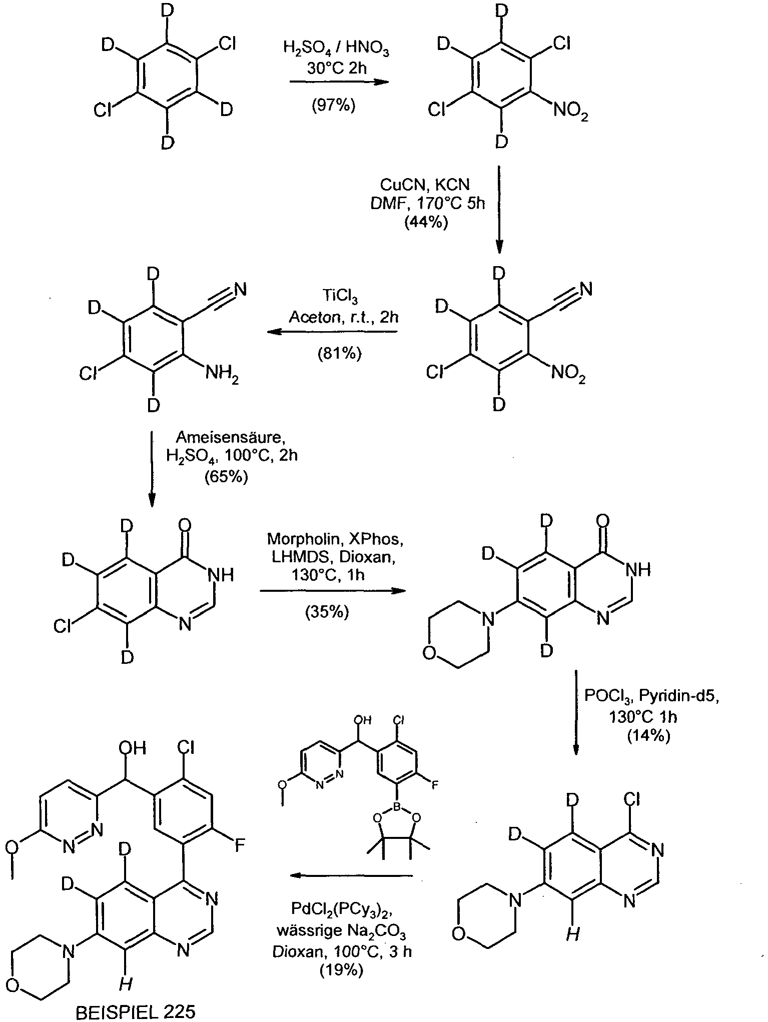

Durch Umsetzung von 5,6,8-Trideuterio-7-morpholin-3H-chinazolin-4-on mit Phosphoroxychlorid wurde 4-Chlor-5,6-dideuterio-7-*N*-morpholinyl-chinazolin erhalten.

### BEISPIEL 237:

### [4-Fluor-3-(7-morpholin-4-yl-pyrido[3,2-d]pyrimidin-4-yl)-phenyl]-(3-methyl-pyrazin-2-yl)-methanol (237)

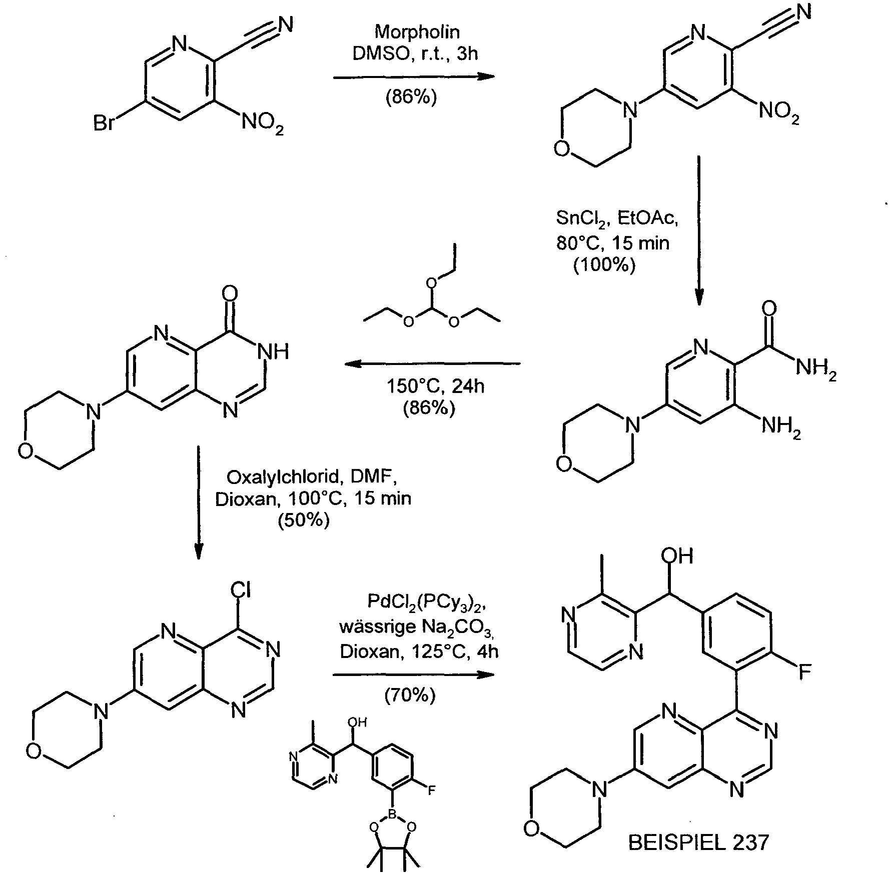

### BEISPIEL 258:

### [2-Chlor-4-fluor-5-[7-(2,2,3,3,5,5,6,6-octadeuterio-morpholin-4-yl)chinazolin-4-yl]phenyl]-(3-methoxypyrazin-2-yl)methanol (258)

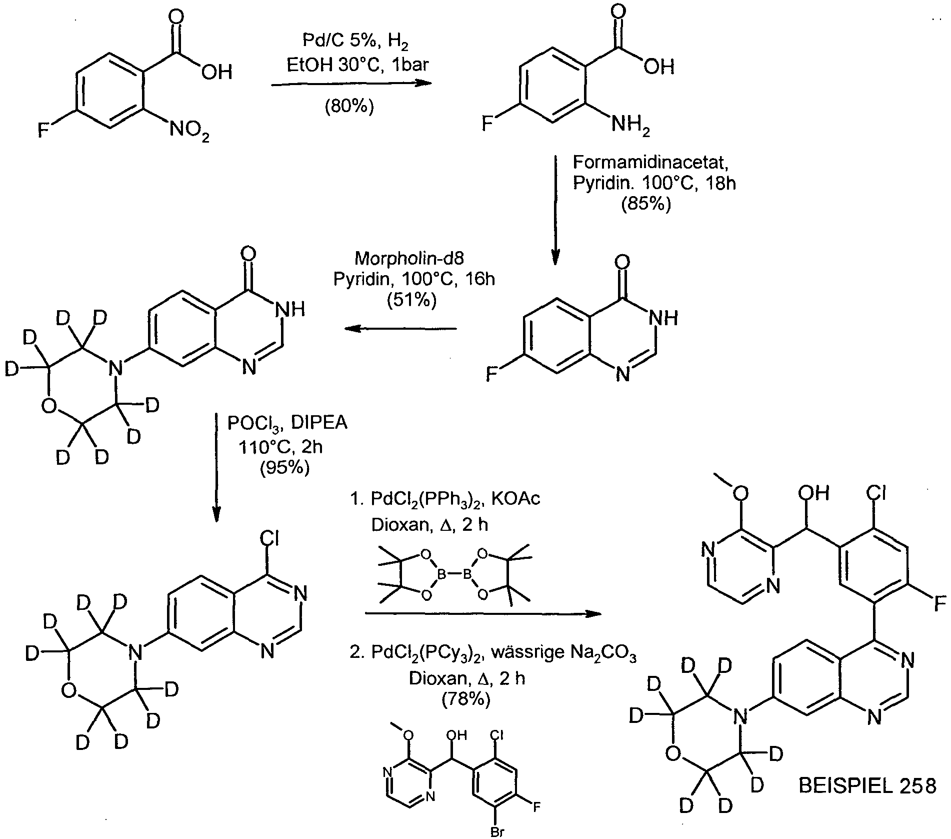

### BEISPIELE 268 und 278:

### [4-Fluoro-3-(5-fluor-7-morpholin-4-yl-chinazolin-4-yl)-phenyl]-(3-methyl-pyrazin-2-yl)-methanol (268), [2-Chlor-4-fluor-5-(5-fluor-7-morpholin-4-yl-chinazolin-4-yl)-phenyl]-(6-methoxy-pyridazin-3-yl)-methanol (278)

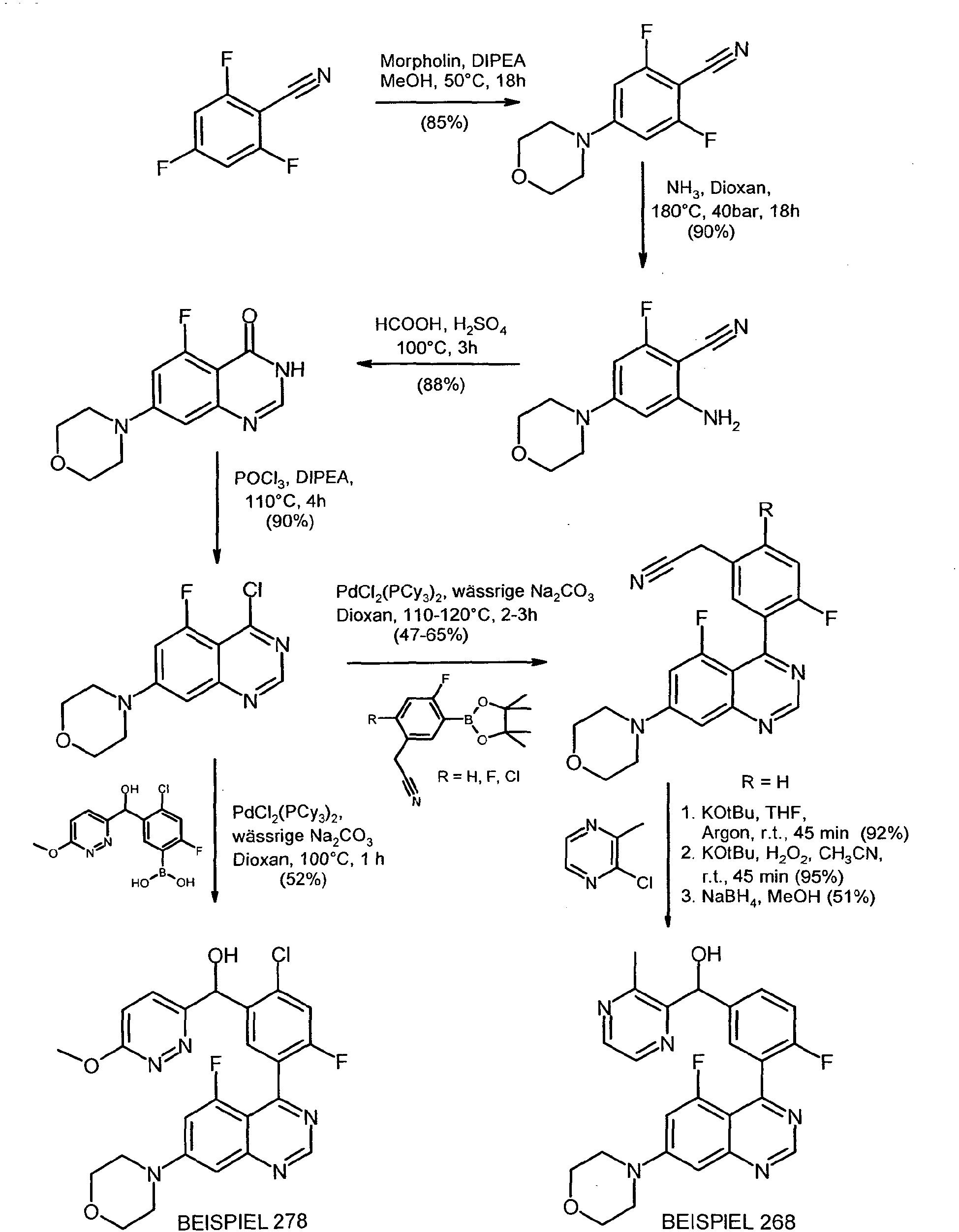

### BEISPIEL 319:

### 2-[2,4-Difluor-5-(7-morpholin-4-yl-chinazolin-4-yl)-phenyl]-2-(3-methoxy-pyrazin-2-yl)-acetamid (319)

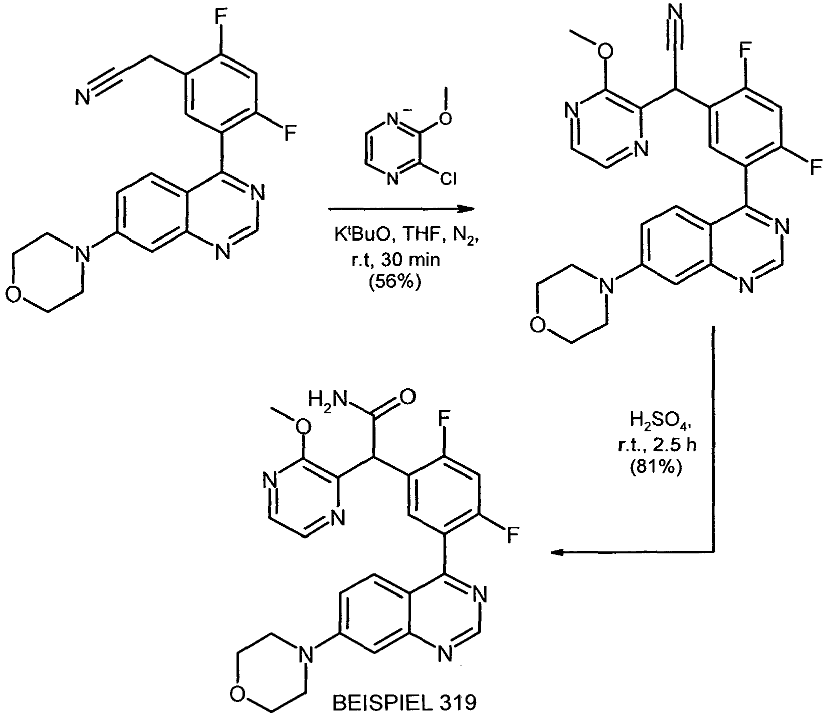

[2,4-Difluoro-5-(7-morpholin-4-yl-chinazolin-4-yl)-phenyl]-acetonitril (300 mg, 0,82 mmol) und 2-Chloro-3-methoxy-pyrazin (297 mg, 1,97 mmol) wurden in Tetrahydrofuran gelöst. Anschließend wurde für die Dauer von 10 min Stickstoff in die Lösung eingeleitet. Danach wurde zur Reaktionslösung Kalium-tert.-butylat (193 mg, 1,72 mmol) zugegeben und für die Dauer von 30 min unter einer Argon-Atmosphäre bei Raumtemperatur gerührt. Nach beendeter Reaktion wurde die Reaktionsmischung mit gesättigter NH₄Cl-Lösung neutralisiert, mit destilliertem Wasser (30 ml) verdünnt und dreimal mit Dichlormethan (je 30 ml) extrahiert. Die organische Phase wurde über NaSO₄ getrocknet, abgesaugt und im Vakuum zur Trockne eingeengt. Der Rückstand wurde mittels Flash-Säulenchromatographie (Gradient: Dichlormethan/0-5 vol.% Ethanol, CombiFlash Rf 200, 40 g Silica-Säule, λ = 220 nm) aufgereinigt. Die geeigneten Produktfraktionen wurden vereinigt und Lösemittel am Rotationsverdampfer entfernt. Man erhielt [2,4-Difluoro-5-(7-morpholin-4-yl-chinazolin-4-yl)-phenyl]-(3-methoxy-pyrazin-2-yl)-acetonitril (218 mg, 0,46 mmol; MS: 475,2 [M+H⁺], 56% Ausbeute) als Feststoff.

[2,4-Difluoro-5-(7-morpholin-4-yl-chinazolin-4-yl)-phenyl]-(3-methoxy-pyrazin-2-yl)-acetonitril (218 mg, 0,46 mmol) wurde im Reaktionskolben vorgelegt und anschließend mit H₂SO₄ (95-98%ig, 3,53 ml, 64 mmol) gelöst. Die Reaktionslösung wurde 2,5 h bei Raumtemperatur gerührt. Nach beendeter Reaktion Eis (80g) wurde zur Reaktionslösung gegeben. Anschließend wurde mit NaOH-Lösung (32%ig, 10,6 ml) vorsichtig neutralisiert. Die erhaltene Suspension wurde mit destilliertem Wasser (50 ml) verdünnt und dreimal mit Dichlormethan (je 100 ml) extrahiert. Die organische Phase wurde über über NaSO₄ getrocknet, abgesaugt und im Vakuum zur Trockne eingeengt. Der Rückstand wurde mittels Flash-Säulenchromatographie (Gradient: Dichlormethan/0-12 vol.% Ethanol, CombiFlash Rf 200, 40 g Silica-Säule, λ = 220 nm) aufgereinigt. Die geeigneten Produktfraktionen wurden vereinigt und Lösemittel am Rotationsverdampfer entfernt. Man erhielt 2-[2,4-Difluor-5-(7-morpholin-4-yl-chinazolin-4-yl)-phenyl]-2-(3-methoxy-pyrazin-2-yl)-acetamid (182 mg, 0,37 mmol, MS: 493,4 [M+H⁺], 81% Ausbeute) als Feststoff.

Verbindungen die entsprechend den BEISPIELEN 225, 237, 258, 268, 278 und 319 sowie analog den Synthesesequenzen der BEISPIELE 1, 2, 37, 137, 121, 217 hergestellt wurden, finden sich in der nachfolgenden Tabelle 8:

| Nr. | Strukturformel | Name | IC₅₀ DNA-PK | IC₅₀ pDNA-PK | Kᵢ [Kv1.11 hERG] |
|---|---|---|---|---|---|
| **225** | 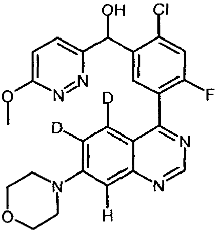 | [2-Chloro-5-(5,6-dideuterio-7-morpholino-quinazolin-4-yl)-4-fluoro-phenyl]-(6-methoxypyridazin-3-yl)methanol | B | A | A |
| | MS: 484.3/486.3 (M+H⁺) (Cl Isotopie, rel. Peakintensitäts-Verhältnis [%] 100:37) | 1H NMR (500 MHz, DMSO-d6) ppm = 9.12 (s, 1H), 7.91 (d, J=7.8, 1H), 7.72 - 7.64 (m, 2H), 7.27 - 7.17 (m, 2H), 6.61 (d, J=5.0, 1H), 6.23 (d, J=5.0, 1H), 4.00 (s, 3H), 3.81 - 3.72 (m, 4H), 3.49 - 3.36 (m, 4H). | | | |
| **226** | 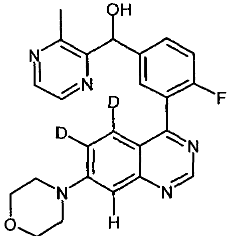 | [3-(5,6-Dideuterio-7-morpholino-quinazolin-4-yl)-4-fluoro-phenyl]-(3-methylpyrazin-2-yl)methanol | B | A | A |
| | MS: 434.4/436.4 (M+H⁺) (Cl Isotopie, rel. Peakintensitäts-Verhältnis [%] 100:40) | 1H NMR (500 MHz, DMSO-d6) ppm = 9.09 (s, 1H), 8.43 (s, 2H), 7.64 - 7.55 (m, 2H), 7.42 - 7.34 (m, 1H), 7.19 (s, 1H), 6.34 - 6.20 (m, 1H), 6.08 (s, 1H), 3.86 - 3.68 (m, 4H), 3.50 - 3.36 (m, 4H), 2.55 (s, 3H). | | | |
| **227** | 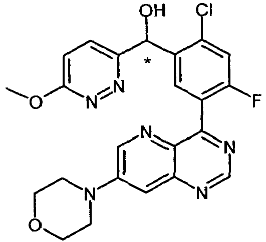 | [2-Chloro-4-fluoro-5-(7-morpholin-4-yl-pyrido[3,2-d]pyrimidin-4-yl)-phenyl]-(6-methoxy-pyridazin-3-yl)-methanol (*Ena 1*) | B | B | A |
| | MS: 483.2/485.1 (M+H⁺) (Cl Isotopie, rel. Peakintensitäts-Verhältnis [%] 100:33); Rₜ 8.37min (HPLC, Chiralpak AD-H, n-Heptan / 90 vol.% 2-Propanol) | s. Racemat | | | |
| **228** | 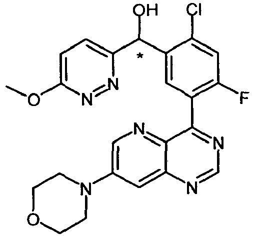 | [2-Chloro-4-fluoro-5-(7-morpholin-4-yl-pyrido[3,2-d]pyrimidin-4-yl)-phenyl]-(6-methoxy-pyridazin-3-yl)-methanol (*Ena 2*) | D | D | A |
| | MS: 483.2/485.1 (M+H⁺) (Cl Isotopie, rel. Peakintensitäts-Verhältnis [%] 100:33); Rₜ 12.45min (HPLC, Chiralpak AD-H, n-Heptan / 90 vol.% 2-Propanol) | s. Racemat | | | |
| **229** | 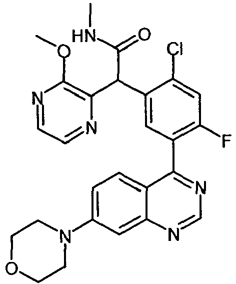 | 2-[2-Chloro-4-fluoro-5-(7-morpholin-4-yl-quinazolin-4-yl)-phenyl]-2-(3-methoxy-pyrazin-2-yl)-N-methyl-acetamide | C | B | A |
| | MS: 523.3/525.2 (M+H⁺) (Cl Isotopie, rel. Peakintensitäts-Verhältnis [%] 100:40) | 1H NMR (500 MHz, DMSO-d6) ppm = 9.09 (s, 1H), 8.26 (q, J=4.5, 1H), 8.13 (d, J=2.8, 1H), 8.11 (d, J=2.8, 1H), 7.73 (d, J=9.6, 1H), 7.59 (dd, J=9.4, 2.6, 1H), 7.56 (dd, J=9.5, 2.3, 1H), 7.47 (d, J=7.7, 1H), 7.20 (d, J=2.2, 1H), 5.66 (s, 1H), 3.94 (s, 3H), 3.80 - 3.71 (m, 4H), 3.48 - 3.42 (m, 4H), 2.61 (d, J=4.6, 3H). | | | |
| **230** | 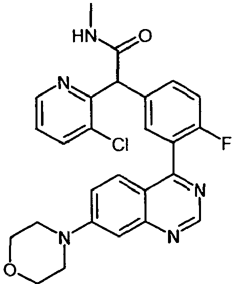 | 2-(3-Chloro-pyridin-2-yl)-2-[4-fluoro-3-(7-morpholin-4-yl-quinazolin-4-yl)-phenyl]-N-methyl-acetamide | C | C | B* |
| | MS: 492.3/494.2 (M+H⁺) (Cl Isotopie, rel. Peakintensitäts-Verhältnis [%] 100:40) | 1H NMR (500 MHz, DMSO-d6) ppm = 9.09 (s, 1H), 8.51 (dd, J=4.7, 1.4, 1H), 8.03 (q, J=4.4, 1H), 7.92 (dd, J=8.1, 1.5, 1H), 7.63 (dd, J=9.4, 2.7, 1H), 7.58 - 7.54 (m, 3H), 7.41 - 7.33 (m, 2H), 7.20 (d, J=2.4, 1H), 5.52 (s, 1H), 3.85 - 3.71 (m, 4H), 3.51 - 3.40 (m, 4H), 2.60 (d, J=4.6, 3H). | | | |
| **231** | 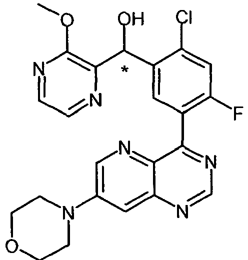 | [2-Chloro-4-fluoro-5-(7-morpholin-4-yl-pyrido[3,2-d]pyrimidin-4-yl)-phenyl]-(3-methoxy-pyrazin-2-yl)-methanol *(Ena 2*) | C | B | A |
| | MS: 483.1/485.1 (M+H⁺) (Cl Isotopie, rel. Peakintensitäts-Verhältnis [%] 100:38); Rₜ 8.72min (SFC, Chiralpak AD-H, CO₂ / 40 vol.% Methanol) | s. Racemat | | | |
| **232** | 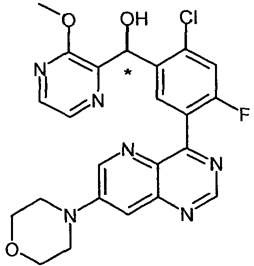 | [2-Chloro-4-fluoro-5-(7-morpholin-4-yl-pyrido[3,2-d]pyrimidin-4-yl)-phenyl]-(3-methoxy-pyrazin-2-yl)-methanol (*Ena 1*) | C | D | A |
| | MS: 483.1/485.1 (M+H⁺) (Cl Isotopie, rel. Peakintensitäts-Verhältnis [%] 100:38); Rₜ 7.27min (SFC, Chiralpak AD-H, CO₂ / 40 vol.% Methanol) | s. Racemat | | | |
| **233** | 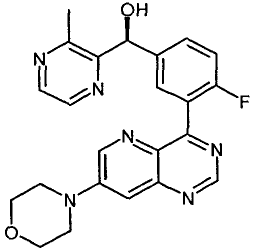 | (S)-[4-Fluoro-3-(7-morpholin-4-yl-pyrido[3,2-d]pyrimidin-4-yl)-phenyl]-(3-methyl-pyrazin-2-yl)-methanol | C | D | A |
| | MS: 433.1 (M+H⁺); Rₜ 4.40min (SFC, Chiralcel OD-H, CO₂ /40 vol.% 2-Propanol, 0.5 vol.% Diethylamin) | s. Racemat | | | |
| **234** | 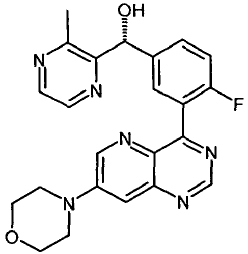 | (R)-[4-Fluoro-3-(7-morpholin-4-yl-pyrido[3,2-d]pyrimidin-4-yl)-phenyl]-(3-methyl-pyrazin-2-yl)-methanol | A | A | A |
| | MS: 433.1 (M+H⁺); Rₜ 3.01min (SFC, Chiralcel OD-H, CO₂/40 vol.% 2-Propanol, 0.5 vol.% Diethylamin) | s. Racemat | | | |
| **235** | 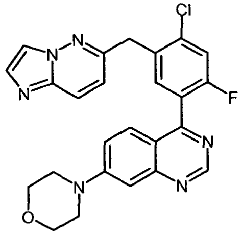 | 4-(4-Chloro-2-fluoro-5-imidazo[1,2-b]pyridazin-6-ylmethyl-phenyl)-7-morpholin-4-yl-quinazoline | A | A | A* |
| | MS: 475.1/477.1 (M+H⁺) (Cl Isotopie, rel. Peakintensitäts-Verhältnis [%] 100:35) | 1H NMR (500 MHz, DMSO-d6) ppm = 9.10 (s, 1H), 8.22 (s, 1H), 8.07 (d, J=9.4, 1H), 7.78 - 7.74 (m, 2H), 7.74 - 7.71 (m, 1H), 7.60 (dd, J=9.4, 3.1, 1H), 7.54 (dd, J=9.4, 2.5, 1H), 7.20 (d, J=2.5, 1H), 7.16 (d, J=9.4, 1H), 4.42 (s, 2H), 3.80 - 3.75 (m, 4H), 3.47 - 3.43 (m, 4H). | | | |
| **236** | 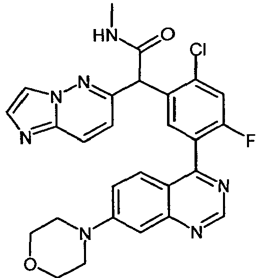 | 2-[2-Chloro-4-fluoro-5-(7-morpholin-4-yl-quinazolin-4-yl)-phenyl]-2-imidazo[1,2-b]pyridazin-6-yl-N-methyl-acetamide | D | C | A* |
| | MS: 532.2/534.2 (M+H⁺) (Cl Isotopie, rel. Peakintensitäts-Verhältnis [%] 100:35) | 1H NMR (500 MHz, DMSO-d6) ppm = 9.11 (s, 1H), 8.56 (q, J=4.5, 1H), 8.26 (s, 1H), 8.10 (d, J=9.5, 1H), 7.81 (d, J=9.5, 1H), 7.77 (d, J=1.2, 1H), 7.74 (d, J=7.6, 1H), 7.60 (dd, J=9.4, 3.0, 1H), 7.55 (dd, J=9.4, 2.5, 1H), 7.21 (d, J=2.4, 1H), 7.12 (d, J=9.5, 1H), 5.62 (s, 1H), 3.81 - 3.75 (m, 4H), 3.48 - 3.40 (m, 4H), 2.65 (d, J=4.6, 3H). | | | |
| **237** | 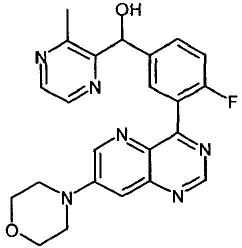 | [4-Fluoro-3-(7-morpholin-4-yl-pyrido[3,2-d]pyrimidin-4-yl)-phenyl]-(3-methyl-pyrazin-2-yl)-methanol | A | A | C |
| | MS: 433.3 (M+H⁺) | 1H NMR (500 MHz, DMSO-d6) ppm = 9.18 (s, 1H), 9.02 (d, J=2.9, 1H), 8.47 - 8.39 (m, 2H), 7.64 (dd, J=6.7, 2.2, 1H), 7.59 - 7.50 (m, 1H), 7.45 (d, J=2.9, 1H), 7.31 (dd, J=9.7, 8.6, 1H), 6.28 (d, J=5.4, 1H), 6.06 (d, J=5.4, 1H), 3.81 - 3.76 (m, 4H), 3.56 - 3.50 (m, 4H), 2.55 (s, 3H). | | | |
| **238** | 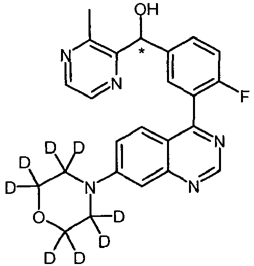 | [4-Fluoro-3-[7-(2,2,3,3,5,5,6,6-octadeuteriomorpholi n-4-yl)quinazolin-4-yl]phenyl]-(3-methylpyrazin-2-yl)methanol (*Ena 2*) | A | A | B |
| | MS: 440.4 (M+H⁺);Rₜ 10.58min (SFC, Chiralpak AD-H, CO₂ / 30 vol.% 2-Propanol, 0.5 vol.% Diethylamin) | S. Racemat | | | |
| **239** | 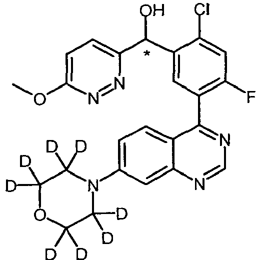 | [2-Chloro-4-fluoro-5-[7-(2,2,3,3,5,5,6,6-octadeuteriomorpholi n-4-yl)quinazolin-4-yl]phenyl]-(6-methoxypyridazin-3-yl)methanol (*Ena 2*) | C | D | B |
| | MS: 490.3/492.2 (M+H⁺) (Cl Isotopie, rel. Peakintensitäts-Verhältnis [%] 100:38); Rₜ 5.20min (SFC, Chiralpak AD-H, CO₂ / 40 vol.% Methanol, 0.5 vol.% Diethylamin) | s. Racemat | | | |
| **240** | 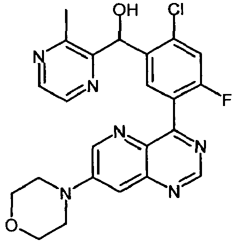 | [2-Chloro-4-fluoro-5-(7-morpholin-4-yl-pyrido[3,2-d]pyrimidin-4-yl)-phenyl]-(3-methyl-pyrazin-2-yl)-methanol | C | B | A |
| | MS: 467.3/469.2 (M+H⁺) (Cl Isotopie, rel. Peakintensitäts-Verhältnis [%] 100:38) | 1H NMR (500 MHz, DMSO-d6) ppm = 9.23 (s, 1H), 9.06 (d, J=2.9, 1H), 8.43 (d, J=2.5, 1H), 8.33 (d, J=2.5, 1H), 8.05 (d, J=7.5, 1H), 7.54 (d, J=9.2, 1H), 7.48 (d, J=2.9, 1H), 6.65 - 6.27 (m, 1H), 6.25 - 6.22 (m, 1H), 3.82 - 3.77 (m, 4H), 3.58 - 3.52 (m, 4H), 2.74 (s, 3H). | | | |
| **241** | 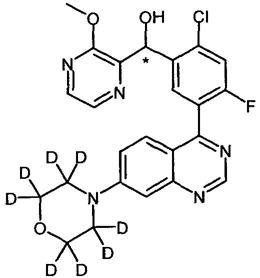 | [2-Chloro-4-fluoro-5-[7-(2,2,3,3,5,5,6,6-octadeuteriomorpholi n-4-yl)quinazolin-4-yl]phenyl]-(3-methoxypyrazin-2-yl)methanol (*Ena 2*) | A | A | C |
| | MS: 490.2/492.2 (M+H⁺) (Cl Isotopie, rel. Peakintensitäts-Verhältnis [%] 100:38); Rₜ 6.47min (SFC, Chiralpak AD-H, CO₂ / 40 vol.% Methanol, 0.5 vol.% Diethylamin) | s. Racemat | | | |
| **242** | 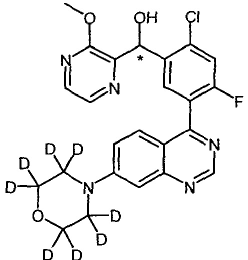 | [2-Chloro-4-fluoro-5-[7-(2,2,3,3,5,5,6,6-octadeuteriomorpholi n-4-yl)quinazolin-4-yl]phenyl]-(3-methoxypyrazin-2-yl)methanol (*Ena 1*) | A | A | B |
| | MS: 490.2/492.2 (M+H⁺) (Cl Isotopie, rel. Peakintensitäts-Verhältnis [%] 100:35); Rₜ 2.91min (SFC, Chiralpak AD-H, CO₂ / 40 vol.% Methanol, 0.5 vol.% *Diethylamin*) | s. Racemat | | | |
| **243** | 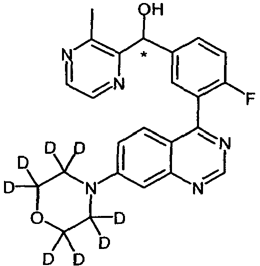 | (S)-[4-fluoro-3-[7-(2,2,3,3,5,5,6,6-octadeuteriomorpholi n-4-yl)quinazolin-4-yl]phenyl]-(3-methylpyrazin-2-yl)methanol (*Ena 1*) | C | B | B |
| | MS: 440.4 (M+H⁺);Rₜ 8.16min (SFC, Chiralpak AD-H, CO₂ / 30 vol.% 2-Propanol, 0.5 vol.% Diethylamin) | s. Racemat | | | |
| **244** | 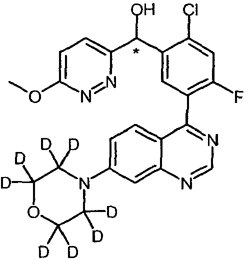 | [2-chloro-4-fluoro-5-[7-(2,2,3,3,5,5,6,6-octadeuteriomorpholi n-4-yl)quinazolin-4-yl]phenyl]-(6-methoxypyridazin-3-yl)methanol (*Ena 1*) | A | A | B |
| | MS: 490.1/492.1 (M+H⁺) (Cl Isotopie, rel. Peakintensitäts-Verhältnis [%] 100:33); Rₜ 3.39min (SFC, Chiralpak AD-H, CO₂ / 40 vol.% Methanol, 0.5 vol.% Diethylamin) | s. Racemat | | | |
| **245** | 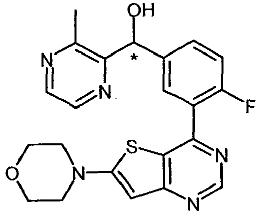 | [4-Fluoro-3-(6-morpholin-4-yl-thieno[3,2-d]pyrimidin-4-yl)-phenyl]-(3-methyl-pyrazin-2-yl)-methanol (*Ena 1*) | A | A | A* |
| | MS: 438.1 (M+H⁺); Rₜ 4.70min (SFC, Chiralcel OD-H, CO₂ / 30 vol.% 2-Propanol, 0.5 vol.% Diethylamin) | s. Racemat | | | |
| **246** | 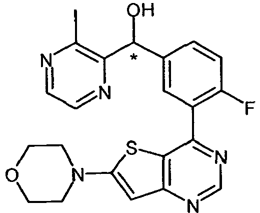 | [4-Fluoro-3-(6-morpholin-4-yl-thieno[3,2-d]pyrimidin-4-yl)-phenyl]-(3-methyl-pyrazin-2-yl)-methanol (*Ena 2*) | B | B | A* |
| | MS: 438.1 (M+H⁺); Rₜ 7.04min (SFC, Chiralcel OD-H, CO₂ / 30 vol.% 2-Propanol, 0.5 vol.% Diethylamin) | s. Racemat | | | |
| **247** | 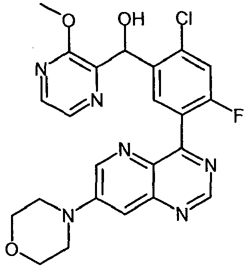 | [2-Chloro-4-fluoro-5-(7-morpholin-4-yl-pyrido[3,2-d]pyrimidin-4-yl)-phenyl]-(3-methoxy-pyrazin-2-yl)-methanol | C | C | B |
| | MS: 483.1/485.1 (M+H⁺) (Cl Isotopie, rel. Peakintensitäts-Verhältnis [%] 100:35) | 1H NMR (500 MHz, DMSO-d6) ppm = 9.22 (s, 1H), 9.04 (d, J=2.9, 1H), 8.16 (d, J=2.7, 1H), 8.08 (d, J=2.7, 1H), 8.02 (d, J=7.6, 1H), 7.51 (d, J=9.3, 1H), 7.47 (d, J=2.8, 1H), 6.32 - 6.28 (m, 2H), 4.00 (s, 3H), 3.82 - 3.77 (m, 4H), 3.57 - 3.52 (m, 4H). | | | |
| **248** | 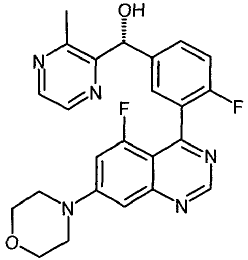 | (R)-[4-Fluoro-3-(5-fluoro-7-morpholin-4-yl-quinazolin-4-yl)-phenyl]-(3-methyl-pyrazin-2-yl)-methanol | A | A | A |
| | MS: 450.1 (M+H⁺); Rₜ 7.18min (SFC, Chiralcel OD-H, CO₂ / 25 vol.% 2-Propanol, 0.5 vol.% Diethylamin) | s. Racemat | | | |
| **249** | 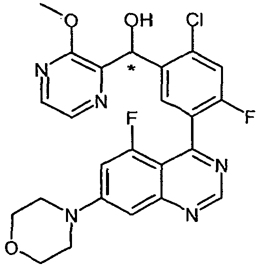 | [2-Chloro-4-fluoro-5-(5-fluoro-7-morpholin-4-yl-quinazolin-4-yl)-phenyl]-(3-methoxy-pyrazin-2-yl)-methanol (*Ena 2*) | A | A | A* |
| | MS: 500.2/502.1 (M+H⁺) (Cl Isotopie, rel. Peakintensitäts-Verhältnis [%] 100:35); Rₜ 7.94min (SFC, Chiralpak AD-H, CO₂ / 40 vol.% Methanol) | s. Racemat | | | |
| **250** | 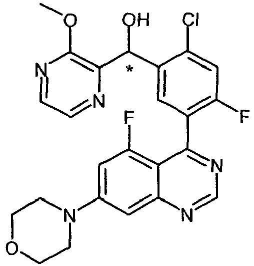 | [2-Chloro-4-fluoro-5-(5-fluoro-7-morpholin-4-yl-quinazolin-4-yl)-phenyl]-(3-methoxy-pyrazin-2-yl)-methanol (*Ena 1*) | A | A | A* |
| | MS: 500.2/502.1 (M+H⁺) (Cl Isotopie, rel. Peakintensitäts-Verhältnis [%] 100:35); Rₜ 3.46min (SFC, Chiralpak AD-H, CO₂ / 40 vol.% Methanol) | s. Racemat | | | |
| **251** | 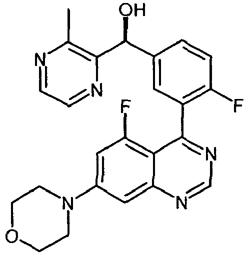 | (S)-[4-Fluoro-3-(5-fluoro-7-morpholin-4-yl-quinazolin-4-yl)-phenyl]-(3-methyl-pyrazin-2-yl)-methanol | B | B | A* |
| | MS: 450.1 (M+H⁺); Rₜ 8.84min (SFC, Chiralcel OD-H, CO₂ / 25 vol.% 2-Propanol, 0.5 vol.% Diethylamin) | s. Racemat | | | |
| **252** | 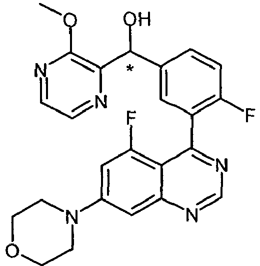 | [4-Fluoro-3-(5-fluoro-7-morpholin-4-yl-quinazolin-4-yl)-phenyl]-(3-methoxy-pyrazin-2-yl)-methanol (*Ena 2*) | A | A | A* |
| | MS: 466.2 (M+H⁺); Rₜ 10.46min (SFC, Chiralcel OD-H, CO₂ / 25 vol.% 2-Propanol, 0.5 vol.% Diethylamin) | s. Racemat | | | |
| **253** | 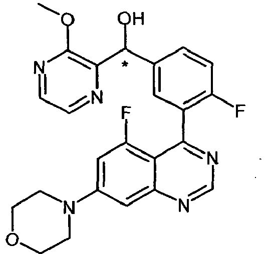 | [4-Fluoro-3-(5-fluoro-7-morpholin-4-yl-quinazolin-4-yl)-phenyl]-(3-methoxy-pyrazin-2-yl)-methanol (*Ena 1*) | C | C | A* |
| | MS: 466.2 (M+H⁺); Rₜ 7.37min (SFC, Chiralcel OD-H, CO₂ / 25 vol.% 2-Propanol, 0.5 vol.% Diethylamin) | s. Racemat | | | |
| **254** | 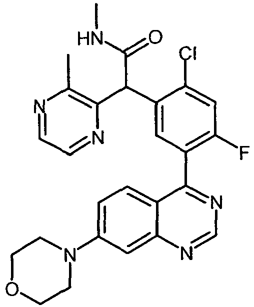 | 2-[2-Chloro-4-fluoro-5-(7-morpholin-4-yl-quinazolin-4-yl)-phenyl]-N-methyl-2-(3-methyl-pyrazin-2-yl)-acetamide | C | D | A* |
| | MS: 507.2/509.2 (M+H⁺) (Cl Isotopie, rel. Peakintensitäts-Verhältnis [%] 100:35) | 1H NMR (500 MHz, DMSO-d6) ppm = 9.08 (s, 1H), 8.41 (d, J=2.6, 1H), 8.39 (d, J=2.5, 1H), 8.30 (q, J=4.5, 1H), 7.74 (d, J=9.5, 1H), 7.59 (dd, J=9.4, 2.6, 1H), 7.55 (dd, J=9.5, 2.4, 1H), 7.47 (d, J=7.7, 1H), 7.20 (d, J=2.3, 1H), 5.66 (s, 1H), 3.78 (t, J=5.8, 3.9, 4H), 3.46 (t, J=4.9, 4H), 2.63 (d, J=4.5, 3H), 2.52 (s, 3H). | | | |
| **255** | 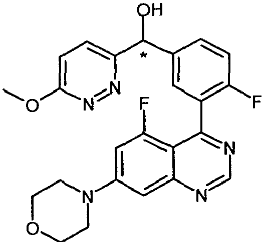 | [4-Fluoro-3-(5-fluoro-7-morpholin-4-yl-quinazolin-4-yl)-phenyl]-(6-methoxy-pyridazin-3-yl)-methanol (*Ena 2*) | C | A | A* |
| | MS: 466.2 (M+H⁺); Rₜ 10.43min (SFC, Chiralpak AS-H, CO₂ / 15 vol.% Methanol, 0.5 vol.% Diethylamin) | s. Racemat | | | |
| **256** | 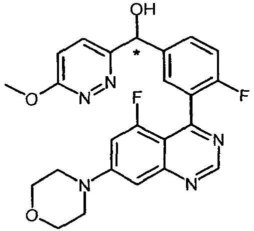 | [4-Fluoro-3-(5-fluoro-7-morpholin-4-yl-quinazolin-4-yl)-phenyl]-(6-methoxy-pyridazin-3-yl)-methanol (*Ena 1*) | A | A | A* |
| | MS: 466.2 (M+H⁺); Rₜ 7.26min (SFC, Chiralpak AS-H, CO₂ / 15 vol.% Methanol, 0.5 vol.% Diethylamin) | s. Racemat | | | |
| **257** | 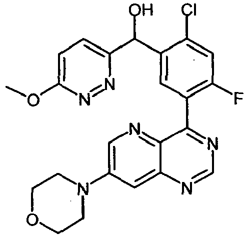 | [2-Chloro-4-fluoro-5-(7-morpholin-4-yl-pyrido[3,2-d]pyrimidin-4-yl)-phenyl]-(6-methoxy-pyridazin-3-yl)-methanol | B | B | A |
| | MS: 483.2/485.1 (M+H⁺) (Cl Isotopie, rel. Peakintensitäts-Verhältnis [%] 100:33) | 1H NMR (500 MHz, DMSO-d6) ppm = 9.21 (s, 1H), 9.04 (d, J=2.9, 1H), 7.96 (d, J=7.5, 1H), 7.68 (d, J=9.2, 1H), 7.58 (d, J=9.3, 1H), 7.46 (d, J=2.9, 1H), 7.22 (d, J=9.1, 1H), 6.60 (d, J=4.9, 1H), 6.23 (d, J=4.9, 1H), 4.00 (s, 3H), 3.84 - 3.76 (m, 4H), 3.57 - 3.51 (m, 4H). | | | |
| **258** | 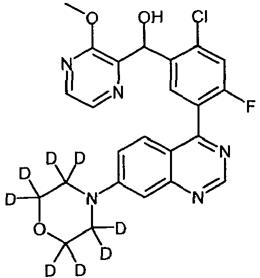 | [2-Chloro-4-fluoro-5-[7-(2,2,3,3,5,5,6,6-octadeuteriomorpholin-4-yl)quinazolin-4-yl]phenyl]-(3-methoxypyrazin-2-yl)methanol | B | A | A* |
| | MS: 490.1/492.3 (M+H⁺) (Cl Isotopie, rel. Peakintensitäts-Verhältnis [%] 100:36) | 1H NMR (500 MHz, DMSO-d6) ppm = 9.13 (s, 1H), 8.17 (d, J=2.7, 1H), 8.11 (d, J=2.7, 1H), 7.97 (d, J=7.8, 1H), 7.67 - 7.58 (m, 2H), 7.56 (dd, J=9.4, 2.6, 1H), 7.21 (d, J=2.5, 1H), 6.34 (d, J=5.9, 1H), 6.31 (d, J=5.9, 1H), 4.00 (s, 3H). | | | |
| **259** | 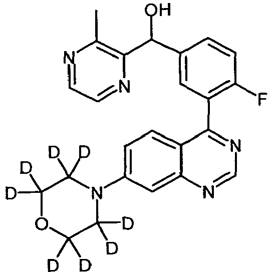 | [4-Fluoro-3-[7-(2,2,3,3,5,5,6,6-octadeuteriomorpholi n-4-yl)quinazolin-4-yl]phenyl]-(3-methylpyrazin-2-yl)methanol | A | A | A* |
| | MS: 440.4 (M+H⁺) | 1H NMR (500 MHz, DMSO-d6) ppm = 9.09 (s, 1H), 8.44 (s, 2H), 7.63 - 7.55 (m, 2H), 7.53 (s, 2H), 7.42 - 7.34 (m, 1H), 7.19 (s, 1H), 6.30 (d, J=5.3, 1H), 6.08 (d, J=4.6, 1H), 2.55 (s, 3H). | | | |
| **260** | 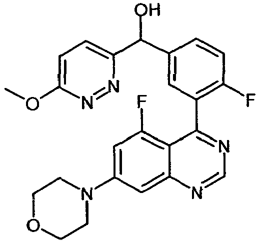 | [4-Fluoro-3-(5-fluoro-7-morpholin-4-yl-quinazolin-4-yl)-phenyl]-(6-methoxy-pyridazin-3-yl)-methanol | A | A | A |
| | MS: 466.2 (M+H⁺) | 1H NMR (500 MHz, DMSO-d6) ppm = 9.09 (d, J=6.1, 1H), 7.69 (d, J=9.2, 1H), 7.66 - 7.48 (m, 2H), 7.37 (dt, J=15.3, 2.7, 1H), 7.29 (ddd, J=9.8, 8.5, 3.1, 1H), 7.21 (t, J=9.2, 1H), 7.08 (d, J=2.4, 1H), 6.57 - 6.43 (m, 1H), 6.00 (d, J=4.4, 1H), 4.00 (d, J=2.9, 3H), 3.81 - 3.69 (m, 4H), 3.53 - 3.40 (m, 4H). | | | |
| **261** | 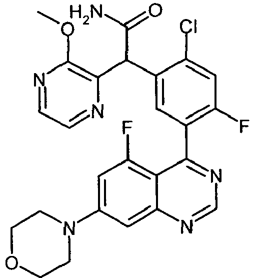 | 2-[2-Chloro-4-fluoro-5-(5-fluoro-7-morpholin-4-yl-quinazolin-4-yl)-phenyl]-2-(3-methoxy-pyrazin-2-yl)-acetamide | C | B | B |
| | MS: 527.2/529.2 (M+H⁺) (Cl Isotopie, rel. Peakintensitäts-Verhältnis [%] 100:35) | 1H NMR (400 MHz, DMSO-d6) ppm = 9.07 (s, 1H), 8.10 (d, J=2.8, 1H), 8.07 (d, J=2.8, 1H), 7.56 (d, J=7.7, 1H), 7.51 (d, J=9.7, 1H), 7.41 - 6.85 (m, 5H), 3.96 (s, 3H), 3.82 - 3.74 (m, 4H), 3.52 - 3.45 (m, 4H). | | | |
| **262** | 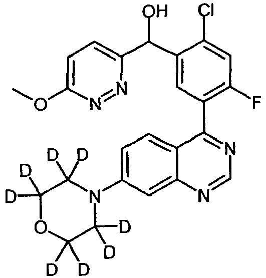 | [2-Chloro-4-fluoro-5-[7-(2,2,3,3,5,5,6,6-octadeuteriomorpholi n-4-yl)quinazolin-4-yl]phenyl]-(6-methoxypyridazin-3-yl)methanol | A | A | A* |
| | MS: 490.2/492.2 (M+H⁺) (Cl Isotopie, rel. Peakintensitäts-Verhältnis [%] 100:36) | 1H NMR (500 MHz, DMSO-d6) ppm = 9.12 (s, 1H), 7.91 (d, J=7.8, 1H), 7.71 - 7.64 (m, 2H), 7.59 (dd, J=9.4, 3.4, 1H), 7.54 (dd, J=9.4, 2.6, 1H), 7.23 - 7.19 (m, 2H), 6.63 (d, J=5.0, 1H), 6.23 (d, J=5.0, 1H), 4.00 (s, 3H). | | | |
| **263** | 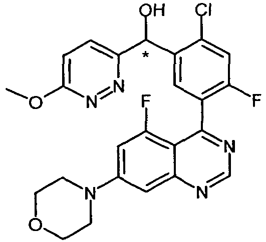 | [2-Chloro-4-fluoro-5-(5-fluoro-7-morpholin-4-yl-quinazolin-4-yl)-phenyl]-(6-methoxy-pyridazin-3-yl)-methanol (*Ena 2*) | C | D | A* |
| | MS: 500.1/502.1 (M+H⁺) (Cl Isotopie, rel. Peakintensitäts-Verhältnis [%] 100:30); Rₜ 6.17min (SFC, Chiralpak AD-H, CO₂ / 40 vol.% Methanol, 0.5 vol.% Diethylamin) | s. Racemat | | | |
| **264** | 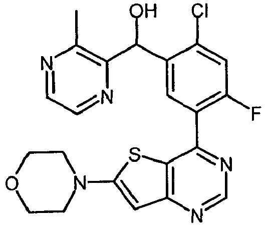 | [2-Chloro-4-fluoro-5-(6-morpholin-4-yl-thieno[3,2-d]pyrimidin-4-yl)-phenyl]-(3-methyl-pyrazin-2-yl)-methanol | A | A | A* |
| | MS: 472.1/474.0 (M+H⁺) (Cl Isotopie, rel. Peakintensitäts-Verhältnis [%] 100:40) | 1H NMR (500 MHz, DMSO-d6) ppm = 8.93 (s, 1H), 8.43 (d, J=2.5, 1H), 8.33 (d, J=2.4, 1H), 8.11 (d, J=7.9, 1H), 7.63 (d, J=9.9, 1H), 6.56 (s, 1H), 6.47 (d, J=6.0, 1H), 6.23 (d, J=4.6, 1H), 3.79 - 3.71 (m, 4H), 3.48 - 3.39 (m, 4H), 2.73 (s, 3H). | | | |
| **265** | 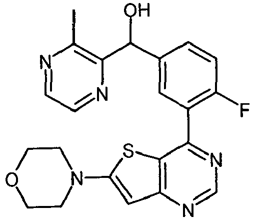 | [4-Fluoro-3-(6-morpholin-4-yl-thieno[3,2-d]pyrimidin-4-yl)-phenyl]-(3-methyl-pyrazin-2-yl)-methanol | A | A | A* |
| | MS: 438.1 (M+H⁺) | 1H NMR (500 MHz, DMSO-d6) ppm = 8.88 (s, 1H), 8.46 - 8.42 (m, 2H), 7.67 (dd, J=7.0, 2.3, 1H), | | | |
| | | 7.60 - 7.55 (m, 1H), 7.38 (dd, J=10.3, 8.6, 1H), 6.52 (s, 1H), 6.32 (d, J=5.4, 1H), 6.08 - 6.05 (m, 1H), 3.79 - 3.70 (m, 4H), 3.44 - 3.37 (m, 4H), 2.54 (s, 3H). | | | |
| **266** | 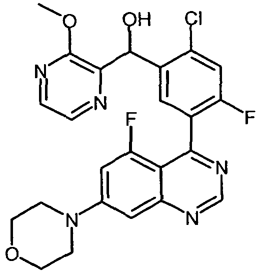 | [2-Chloro-4-fluoro-5-(5-fluoro-7-morpholin-4-yl-quinazolin-4-yl)-phenyl]-(3-methoxy-pyrazin-2-yl)-methanol | A | A | A* |
| | MS: 500.2/502.1 (M+H⁺) (Cl Isotopie, rel. Peakintensitäts-Verhältnis [%] 100:35) | 1H NMR (400 MHz, 90°C, DMSO-d6) ppm = 9.08 (s, 1H), 8.11 (d, J=2.7, 1H), 8.07 (d, J=2.7, 1H), 7.90 (d, J=8.0, 1H), 7.39 (d, J=9.7, 1H), 7.29 (dd, J=15.2, 2.3, 1H), 7.07 (d, J=2.3, 1H), 6.30 (s, 1H), 3.96 (s, 3H), 3.80 - 3.72 (m, 4H), 3.51 - 3.44 (m, 4H). | | | |
| **267** | 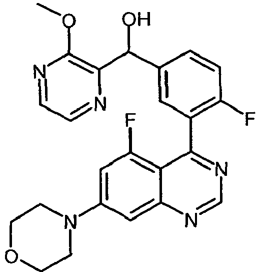 | [4-Fluoro-3-(5-fluoro-7-morpholin-4-yl-quinazolin-4-yl)-phenyl]-(3-methoxy-pyrazin-2-yl)-methanol | A | A | A* |
| | MS: 466.2 (M+H⁺) | 1H NMR (400 MHz, 90°C, DMSO-d6) ppm = 9.05 (s, 1H), 8.15 (d, J=2.5, 1H), 8.11 (d, J=2.5, 1H), 7.62 - 7.44 (m, 2H), 7.30 - 7.12 (m, 2H), 7.05 (d, J=2.2, 1H), 6.09 - 5.95 (m, 2H), 3.91 (s, 3H), 3.81 - 3.70 (m, 4H), 3.52 - 3.41 (m, 4H). | | | |
| **268** | 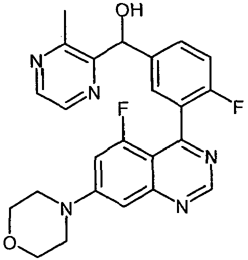 | [4-Fluoro-3-(5-fluoro-7-morpholin-4-yl-quinazolin-4-yl)-phenyl]-(3-methyl-pyrazin-2-yl)-methanol | A | A | A* |
| | MS: 450.1 (M+H⁺) | 1H NMR (500 MHz, DMSO-d6) ppm = 9.08 (s, 1H), 8.45 (s, 2H), 7.63 - 7.56 (m, 1H), 7.56 - 7.46 (m, 1H), 7.46 - 7.34 (m, 1H), 7.34 - 7.22 (m, 1H), 7.08 (s, 1H), 6.36 - 6.27 (m, 1H), 6.06 (d, J=5.4, 1H), 3.81 - 3.73 (m, 4H), 3.53 - 3.43 (m, 4H), 2.53 (s, 3H). | | | |
| **269** | 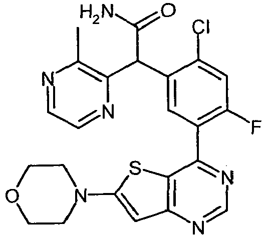 | 2-[2-Chloro-4-fluoro-5-(6-morpholin-4-yl-thieno[3,2-d]pyrimidin-4-yl)-phenyl]-2-(3-methyl-pyrazin-2-yl)-acetamide | B | B | A* |
| | MS: 499.1/501.1 (M+H⁺) (Cl Isotopie, rel. Peakintensitäts-Verhältnis [%] 100:39) | 1H NMR (500 MHz, DMSO-d6) ppm = 8.87 (s, 1H), 8.43 (d, J=2.6, 1H), 8.40 (d, J=2.7, 1H), 7.82 - 7.78 (m, 1H), 7.74 (d, J=10.0, 1H), 7.55 (d, J=7.8, 1H), 7.41 - 7.36 (m, 1H), 6.53 (s, 1H), 5.63 (s, 1H), 3.79 - 3.73 (m, 4H), 3.43 - 3.38 (m, 4H), 2.51 (s, 3H). | | | |
| **270** | 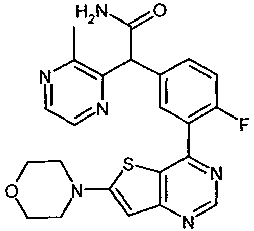 | 2-[4-Fluoro-3-(6-morpholin-4-yl-thieno[3,2-d]pyrimidin-4-yl)-phenyl]-2-(3-methyl-pyrazin-2-yl)-acetamide | B | C | A* |
| | MS: 465.1 (M+H⁺) | 1H NMR (500 MHz, DMSO-d6) ppm = 8.88 (s, 1H), 8.42 - 8.37 (m, 2H), 7.67 (dd, J=7.1, 2.3, 1H), 7.61 - 7.55 (m, 2H), 7.41 - 7.34 (m, 1H), 7.25 - 7.19 (m, 1H), 6.53 (s, 1H), 5.40 (s, 1H), 3.79 - 3.71 (m, 4H), 3.44 - 3.39 (m, 4H), 2.53 (s 3H). | | | |
| **271** | 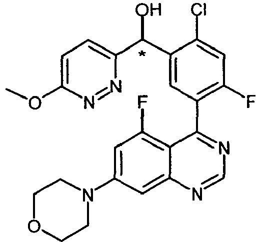 | [2-Chloro-4-fluoro-5-(5-fluoro-7-morpholin-4-yl-quinazolin-4-yl)-phenyl]-(6-methoxy-pyridazin-3-yl)-methanol (*Ena 1*) | A | A | A |
| | MS: 500.1/502.1 (M+H⁺) (Cl Isotopie, rel. Peakintensitäts-Verhältnis [%] 100:32); Rₜ 4.25min (SFC, Chiralpak AD-H, CO₂ / 40 vol.% Methanol, 0.5 vol.% Diethylamin) | 1H NMR (400 MHz, DMSO-d6/DMSO, 120 °C) ppm = 9.05 (s, 1H), 7.84 (d, J = 7.7 Hz, 1H), 7.60 (d, J = 9.0 Hz, 1H), 7.40 (d, J = 9.5 Hz, 1H), 7.23 (d, J = 14.5 Hz, 1H), 7.11 (d, J = 9.1 Hz, 1H), 7.06 (s, 1H), 6.24 (d, J = 4.7 Hz, 1H), 6.16 (d, J = 4.7 Hz, 1H), 4.02 (s, 3H), 3.80 - 3.72 (m, 4H), 3.51 - 3.43 (m, 4H). | | | |
| **272** | 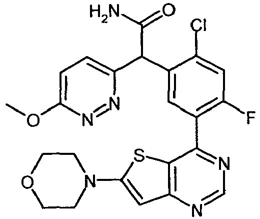 | 2-[2-Chloro-4-fluoro-5-(6-morpholin-4-yl-thieno[3,2-d]pyrimidin-4-yl)-phenyl]-2-(6-methoxy-pyridazin-3-yl)-acetamide | B | B | A* |
| | MS: 515.2/517.2 (M+H⁺) (Cl Isotopie, rel. Peakintensitäts-Verhältnis [%] 100:41) | 1H NMR (400 MHz, DMSO-d6) ppm = 8.89 (s, 1H), 7.94 (s, 1H), 7.81 (d, J=7.7, 1H), 7.74 (d, J=10.0, 1H), 7.47 (d, J=9.2, 1H), 7.40 (s, 1H), 7.19 (d, J=9.2, 1H), 6.53 (s, 1H), 5.65 (s, 1H), 4.00 (s, 3H), 3.79 - 3.71 (m, 4H), 3.45 - 3.39 (m, 4H). | | | |
| **278** | 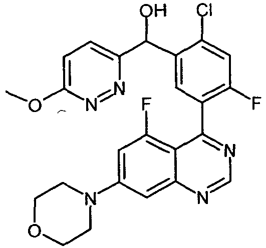 | [2-Chloro-4-fluoro-5-(5-fluoro-7-morpholin-4-yl-quinazolin-4-yl)-phenyl]-(6-methoxy-pyridazin-3-yl)-methanol | A | A | A* |
| | MS: 500.2/502.2 (M+H⁺) (Cl Isotopie, rel. Peakintensitäts-Verhältnis [%] 100:35) | 1H NMR (400 MHz, 120°C, DMSO-d6) ppm = 9.05 (s, 1H), 7.84 (d, J=7.8, 1H), 7.60 (d, J=9.1, 1H), 7.40 (d, J=9.6, 1H), 7.23 (dd, J=15.2, 2.5, 1H), 7.11 (d, J=9.1, 1H), 7.06 (d, J=2.5, 1H), 6.25 (d, J=5.1, 1H), 6.16 (d, J=5.1, 1H), 4.03 (s, 3H), 3.80 - 3.73 (m, 4H), 3.49 - 3.43 (m, 4H). | | | |
| **279** | 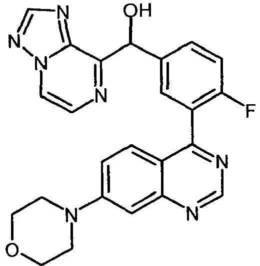 | [4-Fluoro-3-(7-morpholin-4-yl-quinazolin-4-yl)-phenyl]-[1,2,4]triazolo[1,5-a]pyrazin-8-yl-methanol | B | C | A* |
| | MS: 458.2 (M+H⁺) | 1H NMR (400 MHz, DMSO-d6) ppm = 9.10 (s, 1H), 9.03 (d, J=4.5, 1H), 8.76 (s, 1H), 8.25 (d, J=4.5, 1H), 7.84 - 7.76 (m, 2H), 7.59 - 7.51 (m, 2H), 7.39 (dd, J=9.9, 8.5, 1H), 7.20 (d, J=2.1, 1H), 6.48 (s, 1H), 3.82 - 3.75 (m, 4H), 3.51 - 3.43 (m, 4H). | | | |
| **280** | 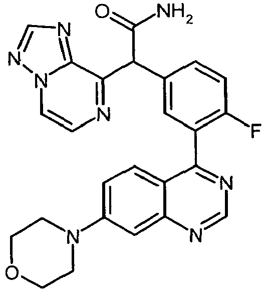 | 2-[4-Fluoro-3-(7-morpholin-4-yl-quinazolin-4-yl)-phenyl]-2-[1,2,4]triazolo[1,5-a]pyrazin-8-yl-acetamide | C | D | A* |
| | MS: 485.2 (M+H⁺) | 1H NMR (500 MHz, DMSO-d6) ppm = 9.10 (s, 1H), 9.02 (d, J=4.5, 1H), 8.74 (s, 1H), 8.21 (d, J=4.5, 1H), 7.79 - 7.75 (m, 1H), 7.74 - 7.69 (m, 2H), 7.64 (dd, J=9.4, 2.9, 1H), 7.56 (dd, J=9.5, 2.5, 1H), 7.45 - 7.40 (m, 1H), 7.31 - 7.27 (m, 1H), 7.20 (d, J=2.4, 1H), 5.89 (s, 1H), 3.81 - 3.74 (m, 4H), 3.49 - 3.41 (m, 4H). | | | |
| **282** | 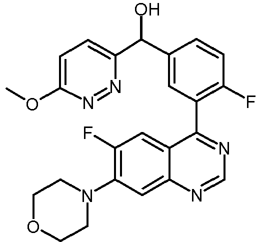 | [4-Fluoro-3-(6-fluoro-7-morpholin-4-yl-quinazolin-4-yl)-phenyl]-(6-methoxy-pyridazin-3-yl)-methanol | D | D | A* |
| | MS: 466.2 (M+H⁺) | 1H NMR (500 MHz, DMSO-d6) ppm = 9.24 (s, 1H), 7.74 - 7.63 (m, 3H), 7.48 - 7.39 (m, 2H), 7.34 (dd, J=13.6, 3.1, 1H), 7.21 (d, J=9.2, 1H), 6.53 (d, J=4.3, 1H), 6.03 (d, J=4.4, 1H), 4.00 (s, 3H), 3.83 - 3.77 (m, 4H), 3.36 - 3.27 (m, 4H). | | | |
| **283** | 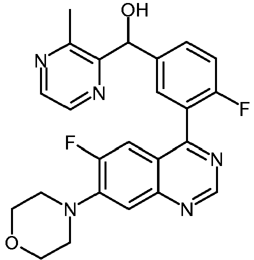 | [4-Fluoro-3-(6-fluoro-7-morpholin-4-yl-quinazolin-4-yl)-phenyl]-(3-methyl-pyrazin-2-yl)-methanol | C | D | A |
| | MS: 450.2 (M+H⁺) | 1H NMR (400 MHz, DMSO-d6) ppm = 9.23 (s, 1H), 8.46 - 8.41 (m, 2H), 7.66 - 7.58 (m, 2H), 7.49 - 7.31 (m, 3H), 6.32 (d, J=5.5, 1H), 6.10 (d, J=5.5, 1H), 3.84 - 3.78 (m, 4H), 3.36 - 3.27 (m, 4H), 2.57 (s, 3H). | | | |
| **284** | 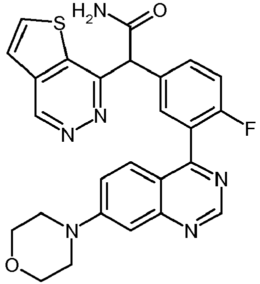 | 2-[4-Fluoro-3-(7-morpholin-4-yl-quinazolin-4-yl)-phenyl]-2-thieno[2,3-d]pyridazin-7-yl-acetamide | B | D | A* |
| | MS: 501.1 (M+H⁺) | 1H NMR (500 MHz, DMSO-d6) ppm = 9.59 (s, 1H), 9.09 (s, 1H), 8.24 (d, J=5.3, 1H), 7.92 (s, 1H), 7.73 - 7.65 (m, 3H), 7.57 (dd, J=9.4, 3.1, 1H), 7.50 (dd, J=9.5, 2.5, 1H), 7.47 - 7.36 (m, 2H), 7.19 (d, J=2.5, 1H), 5.72 (s, 1H), 3.84 - 3.73 (m, 4H), 3.46 - 3.41 (m, 4H), 9.09 - 9.09 (m, 0H). | | | |
| **285** | 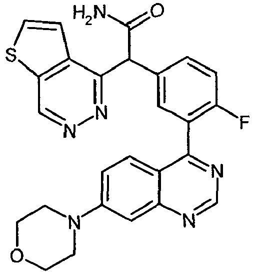 | 2-[4-Fluoro-3-(7-morpholin-4-yl-quinazolin-4-yl)-phenyl]-2-thieno[2,3-d]pyridazin-4-yl-acetamide | B | D | B |
| | MS: 501.1 (M+H⁺) | 1H NMR (500 MHz, DMSO-d6) ppm = 9.83 - 9.79 (m, 1H), 9.08 (s, 1H), 8.30 (d, J=5.4, 1H), 7.81 - 7.75 (m, 2H), 7.75 - 7.68 (m, 2H), 7.56 (dd, J=9.4, 3.1, 1H), 7.49 (dd, J=9.4, 2.6, 1H), 7.40 (dd, J=9.8, 8.4, 1H), 7.32 (s, 1H), 7.19 (d, J=2.5, 1H), 5.90 (s, 1H), 3.81 - 3.74 (m, 4H), 3.47 - 3.40 (m, 4H). | | | |
| **286** | 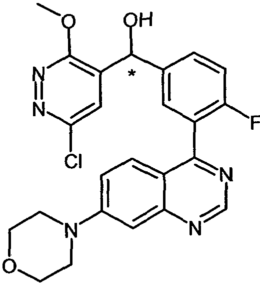 | (6-Chloro-3-methoxy-pyridazin-4-yl)-[4-fluoro-3-(7-morpholin-4-yl-quinazolin-4-yl)-phenyl]-methanol (*Ena 1)* | C | C | A* |
| | MS: 482.1/484.2 (M+H⁺) (Cl Isotopie, rel. Peakintensitäts-Verhältnis [%] 100:36); Rₜ 5.83min (SFC, Chiralpak AS-H, CO₂ / 20 vol.% Methanol, 0.5 vol.% Diethylamin) | s. Racemat | | | |
| **288** | 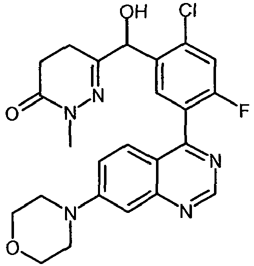 | 6-{[2-Chloro-4-fluoro-5-(7-morpholin-4-yl-quinazolin-4-yl)-phenyl]-hydroxymethyl}-2-methyl-4,5-dihydro-2H-pyridazin-3-one | C | C | A* |
| | MS: 484.2/486.2 (M+H⁺) (Cl Isotopie, rel. Peakintensitäts-Verhältnis [%] 100:35) | 1H NMR (500 MHz, DMSO-d6) ppm = 9.12 (s, 1H), 7.84 (d, J=7.8, 1H), 7.68 (d, J=9.5, 1H), 7.57 (dd, J=9.4, 2.8, 1H), 7.54 (dd, J=9.5, 2.4, 1H), 7.21 (d, J=2.3, 1H), 6.42 (s, 1H), 5.57 (s, 1H), 3.81 - 3.75 (m, 4H), 3.47 - 3.43 (m, 4H), 3.16 (s, 3H), 2.61 - 2.51 (m, 1H), 2.43 - 2.28 (m, 3H). | | | |
| **289** | 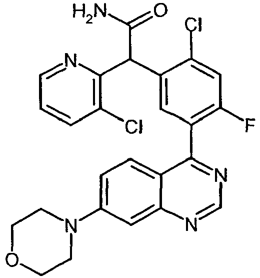 | 2-[2-Chloro-4-fluoro-5-(7-morpholin-4-yl-quinazolin-4-yl)-phenyl]-2-(3-chloro-pyridin-2-yl)-acetamide | A | B | B |
| | MS: 512.2/514.2/516.2 (M+H⁺) (Cl₂ Isotopie, rel. Peakintensitäts-Verhältnis [%] 100:71:21) | 1H NMR (500 MHz, DMSO-d6) ppm = 9.08 (s, 1H), 8.44 (dd, J=4.7, 1.5, 1H), 7.95 (dd, J=8.1, 1.5, 1H), 7.76 (s, 1H), 7.72 (d, J=9.6, 1H), 7.58 (dd, J=9.4, 2.9, 1H), 7.54 (dd, J=9.5, 2.5, 1H), 7.41 (d, J=7.6, 1H), 7.35 (dd, J=8.1, 4.7, 1H), 7.28 (s, 1H), 7.19 (d, J=2.4, 1H), 5.81 (s, 1H), 3.80 - 3.75 (m, 4H), 3.48 - 3.42 (m, 4H). | | | |
| **290** | 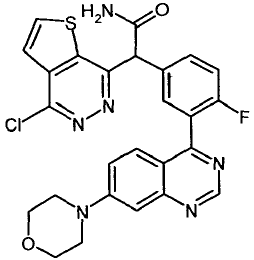 | 2-(4-Chloro-thieno[2,3-d]pyridazin-7-yl)-2-[4-fluoro-3-(7-morpholin-4-yl-quinazolin-4-yl)-phenyl]-acetamide | B | C | D |
| | MS: 535.3/537.2 (M+H⁺) (Cl Isotopie, rel. Peakintensitäts-Verhältnis [%] 100:41) | 1H NMR (500 MHz, DMSO-d6) ppm = 9.09 (s, 1H), 8.41 (d, J=5.4, 1H), 7.94 (s, 1H), 7.71 - 7.65 (m, 3H), 7.56 (dd, J=9.4, 3.1, 1H), 7.51 (dd, J=9.4, 2.5, 1H), 7.48 - 7.40 (m, 2H), 7.20 (d, J=2.4, 1H), 5.75 (s, 1H), 3.82-3.73 (m, 4H), 3.48 - 3.40 (m, 4H). | | | |
| **291** | 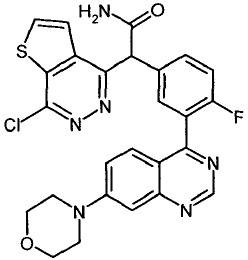 | 2-(7-Chloro-thieno[2,3-d]pyridazin-4-yl)-2-[4-fluoro-3-(7-morpholin-4-yl-quinazolin-4-yl)-phenyl]-acetamide | B | C | D |
| | MS: 535.2/537.2 (M+H⁺) (Cl Isotopie, rel. Peakintensitäts-Verhältnis [%] 100:41) | 1H NMR (500 MHz, DMSO-d6) ppm = 9.09 (s, 1H), 8.43 (d, J=5.4, 1H), 7.86 (d, J=5.4, 1H), 7.77 (s, 1H), 7.74 - 7.67 (m, 2H), 7.56 (dd, J=9.4, 3.1, 1H), 7.50 (dd, J=9.5, 2.5, 1H), 7.44 - 7.35 (m, 2H), 7.19 (d, J=2.4, 1H), 5.92 (s, 1H), 3.81 - 3.74 (m, 4H), 3.47 - 3.40 (m, 4H). | | | |
| **292** | 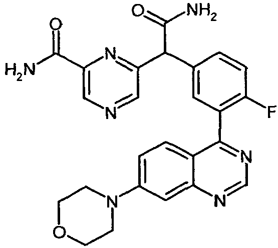 | 6-{Carbamoyl-[4-fluoro-3-(7-morpholin-4-yl-quinazolin-4-yl)-phenyl]-methyl}-pyrazine-2-carboxylic acid amide | C | D | A |
| | MS: 488.2 (M+H⁺) | 1H NMR (500 MHz, DMSO-d6) ppm = 9.12 (s, 1H), 9.06 (s, 1H), 8.90 (s, 1H), 8.08 - 8.01 (m, 1H), 7.99 - 7.93 (m, 1H), 7.85 - 7.77 (m, 2H), 7.77 - 7.71 (m, 1H), 7.59 (dd, J=9.4, 3.6, 1H), 7.52 (dd, J=9.5, 2.5, 1H), 7.46 - 7.39 (m, 1H), 7.38 - 7.32 (m, 1H), 7.20 (d, J=2.5, 1H), 5.39 (s, 1H), 3.80 - 3.75 (m, 4H), 3.47 - 3.42 (m, 4H). | | | |
| **293** | 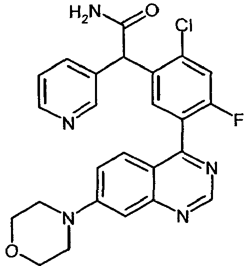 | 2-[2-Chloro-4-fluoro-5-(7-morpholin-4-yl-quinazolin-4-yl)-phenyl]-2-pyridin-3-yl-acetamide | B | C | A |
| | MS: 478.2/480.2 (M+H⁺) (Cl Isotopie, rel. Peakintensitäts-Verhältnis [%] 100:30) | 1H NMR (500 MHz, DMSO-d6) ppm = 9.09 (s, 1H), 8.49 (s, 1H), 8.46 (d, J=3.9, 1H), 7.94 (s, 1H), 7.74 (d, J=9.5, 1H), 7.67 (d, J=7.7, 1H), 7.66 - 7.63 (m, 1H), 7.56 (dd, J=9.4, 3.2, 1H), 7.53 (dd, J=9.5, 2.4, 1H), 7.37 (dd, J=7.9, 4.8, 1H), 7.33 (s, 1H), 7.20 (d, J=2.3, 1H), 5.41 (s, 1H), 3.81 - 3.74 (m, 4H), 3.49 - 3.42 (m, 4H). | | | |
| **294** | 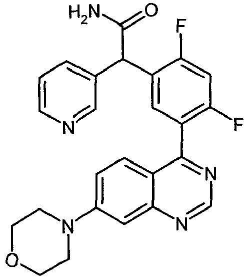 | 2-[2,4-Difluoro-5-(7-morpholin-4-yl-quinazolin-4-yl)-phenyl]-2-pyridin-3-yl-acetamide | B | D | A |
| | MS: 462.3 (M+H⁺) | 1H NMR (500 MHz, DMSO-d6) ppm = 9.08 (s, 1H), 8.53 (d, J=2.3, 1H), 8.48 (dd, J=4.8, 1.6, 1H), 7.89 (s, 1H), 7.72 (dt, J=8.0, 2.0, 1H), 7.61 (t, J=8.2, 1H), 7.59 - 7.48 (m, 3H), 7.38 (dd, J=7.9, 4.7, 1H), 7.32 (s, 1H), 7.19 (d, J=2.3, 1H), 5.31 (s, 1H), 3.82 - 3.73 (m, 4H), 3.47 - 3.42 (m, 4H). | | | |
| **295** | 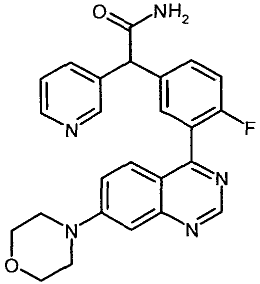 | 2-[4-Fluoro-3-(7-morpholin-4-yl-quinazolin-4-yl)-phenyl]-2-pyridin-3-yl-acetamide | C | D | A |
| | MS: 444.2 (M+H⁺) | 1H NMR (500 MHz, DMSO-d6) ppm = 9.09 (s, 1H), 8.55 (s, 1H), 8.47 (s, 1H), 7.82 (s, 1H), 7.79 (d, J=8.0, 1H), 7.62 - 7.56 (m, 2H), 7.56 - 7.49 (m, 2H), 7.45 - 7.35 (m, 2H), 7.25 (s, 1H), 7.20 (d, J=2.0, 1H), 5.12 (s, 1H), 3.80 - 3.74 (m, 4H), 3.47 - 3.41 (m, 4H). | | | |
| **296** | 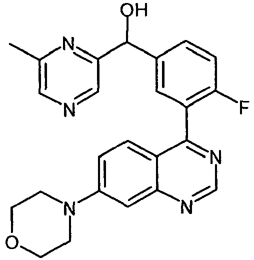 | [4-Fluoro-3-(7-morpholin-4-yl-quinazolin-4-yl)-phenyl]-(6-methyl-pyrazin-2-yl)-methanol | C | C | A |
| | MS: 432.2 (M+H⁺) | 1H NMR (500 MHz, DMSO-d6) ppm = 9.10 (s, 1H), 8.66 (s, 1H), 8.43 (s, 1H), 7.68 - 7.63 (m, 2H), 7.54 - 7.49 (m, 2H), 7.42 - 7.35 (m, 1H), 7.21 - 7.18 (m, 1H), 6.40 (d, J=4.3, 1H), 5.87 (d, J=4.3, 1H), 3.81 - 3.74 (m, 4H), 3.47 - 3.41 (m, 4H), 2.46 (s, 3H). | | | |
| **297** | 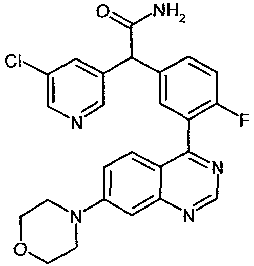 | 2-(5-Chloro-pyridin-3-yl)-2-[4-fluoro-3-(7-morpholin-4-yl-quinazolin-4-yl)-phenyl]-acetamide | C | D | C |
| | MS: 478.3/480.2 (M+H⁺) (Cl Isotopie, rel. Peakintensitäts-Verhältnis [%] 100:38) | 1H NMR (500 MHz, DMSO-d6) ppm = 9.10 (s, 1H), 8.54 (d, J=2.4, 1H), 8.52 (d, J=1.9, 1H), 7.90 - 7.87 (m, 1H), 7.85 (s, 1H), 7.61 (tt, J=6.9, 2.3, 2H), 7.53 (qd, J=9.4, 2.8, 2H), 7.47 - 7.39 (m, 1H), 7.33 (s, 1H), 7.20 (d, J=2.3, 1H), 5.16 (s, 1H), 3.82 - 3.73 (m, 4H), 3.48 - 3.40 (m, 4H). | | | |
| **298** | 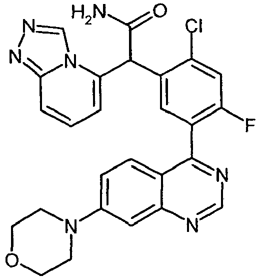 | 2-[2-Chloro-4-fluoro-5-(7-morpholin-4-yl-quinazolin-4-yl)-phenyl]-2-[1,2,4]triazolo[4,3-a]pyridin-5-yl-acetamide | C | D | A |
| | MS: 518.2/520.2 (M+H⁺) (Cl Isotopie, rel. Peakintensitäts-Verhältnis [%] 100:35) | 1H NMR (500 MHz, DMSO-d6) ppm = 9.21 (d, J=0.8, 1H), 9.08 (s, 1H), 8.13 (s, 1H), 7.88 (d, J=9.5, 1H), 7.79 (d, J=9.2, 1H), 7.73 (s, 1H), 7.57 - 7.48 (m, 3H), 7.38 (dd, J=9.2, 6.9, 1H), 7.19 (d, J=2.3, 1H), 6.57 (d, J=6.9, 1H), 5.84 (s, 1H), 3.80 - 3.75 (m, 4H), 3.48 - 3.42 (m, 4H). | | | |
| **299** | 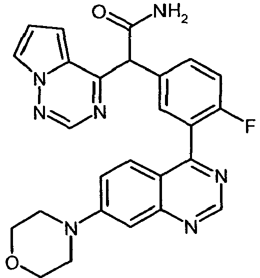 | 2-[4-Fluoro-3-(7-morpholin-4-yl-quinazolin-4-yl)-phenyl]-2-pyrrolo[2,1-f][1,2,4]triazin-4-yl-acetamide | C | D | D |
| | MS: 484.2 (M+H⁺) | 1H NMR (500 MHz, DMSO-d6) ppm = 13.68 (d, J=4.0, 1H), 9.09 (s, 1H), 7.84 - 7.74 (m, 2H), 7.57 - 7.46 (m, 3H), 7.46 - 7.39 (m, 1H), 7.31 - 7.25 (m, 1H), 7.24 - 7.16 (m, 1H), 7.02 (s, 1H), 6.21 (dd, J=4.4, 2.7, 1H), 6.08 (s, 1H), 4.81 (dd, J=4.3, 1.7, 1H), 3.90 - 3.70 (m, 4H), 3.53 - 3.38 (m, 4H). | | | |
| **300** | 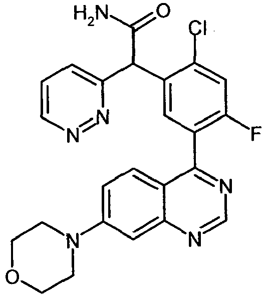 | 2-[2-Chloro-4-fluoro-5-(7-morpholin-4-yl-quinazolin-4-yl)-phenyl]-2-pyridazin-3-yl-acetamide | C | D | A |
| | MS: 479.2/481.2 (M+H⁺) (Cl Isotopie, rel. Peakintensitäts-Verhältnis [%] 100:33) | 1H NMR (500 MHz, DMSO-d6) ppm = 9.14 (dd, J=4.9, 1.6, 1H), 9.10 (s, 1H), 8.03 (s, 1H), 7.76 (d, J=9.6, 1H), 7.71 (d, J=7.6, 1H), 7.65 (dd, J=8.5, 4.9, 1H), 7.60 (dd, J=9.4, 3.4, 1H), 7.53 (dd, J=5.6, 2.1, 1H), 7.52 (dd, J=4.7, 2.0, 1H), 7.43 (s, 1H), 7.20 (d, J=2.5, 1H), 5.78 (s, 1H), 3.78 (dd, J=5.8, 4.0, 4H), 3.46 (dd, J=6.0, 3.9, 4H). | | | |
| **301** | 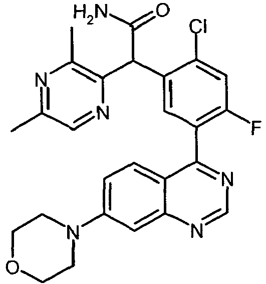 | 2-[2-Chloro-4-fluoro-5-(7-morpholin-4-yl-quinazolin-4-yl)-phenyl]-2-(3,5-dimethyl-pyrazin-2-yl)-acetamide | C | B | A |
| | MS: 507.2/509.2 (M+H⁺) (Cl Isotopie, rel. Peakintensitäts-Verhältnis [%] 100:35) | 1H NMR (500 MHz, DMSO-d6) ppm = 9.08 (s, 1H), 8.25 (s, 1H), 7.75 - 7.69 (m, 2H), 7.58 (dd, J=9.4, 3.0, 1H), 7.54 (dd, J=9.5, 2.5, 1H), 7.47 (d, J=7.7, 1H), 7.28 (s, 1H), 7.19 (d, J=2.4, 1H), 5.60 (s, 1H), 3.81 - 3.74 (m, 4H), 3.49 - 3.42 (m, 4H), 2.49 (s, 3H), 2.41 (s, 3H). | | | |
| **302** | 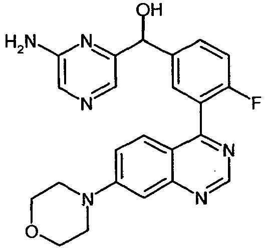 | (6-Amino-pyrazin-2-yl)-[4-fluoro-3-(7-morpholin-4-yl-quinazolin-4-yl)-phenyl]-methanol | B | C | A |
| | MS: 433.1 (M+H⁺) | 1H NMR (500 MHz, DMSO-d6) ppm = 9.09 (s, 1H), 7.91 (s, 1H), 7.74 (s, 1H), 7.63 - 7.56 (m, 2H), 7.54 - 7.51 (m, 2H), 7.39 - 7.34 (m, 1H), 7.21 - 7.19 (m, 1H), 6.36 (s, 2H), 6.10 (d, J=4.3, 1H), 5.60 (d, J=4.3, 1H), 3.81 - 3.74 (m, 4H), 3.47 - 3.41 (m, 4H). | | | |
| **303** | 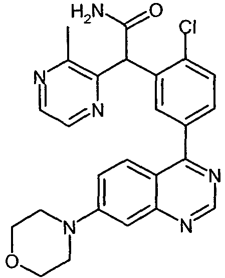 | 2-[2-Chloro-5-(7-morpholin-4-yl-quinazolin-4-yl)-phenyl]-2-(3-methyl-pyrazin-2-yl)-acetamide | C | C | B |
| | MS: 475.2/477.2 (M+H⁺) (Cl Isotopie, rel. Peakintensitäts-Verhältnis [%] 100:38) | 1H NMR (500 MHz, DMSO-d6) ppm = 9.05 (s, 1H), 8.43 - 8.37 (m, 2H), 7.87 (d, J=9.4, 1H), 7.80 (s, 1H), 7.75 - 7.65 (m, 3H), 7.51 (dd, J=9.5, 2.6, 1H), 7.33 (s, 1H), 7.19 (d, J=2.5, 1H), 5.71 (s, 1H), 3.85 - 3.74 (m, 4H), 3.52 - 3.39 (m, 4H), 2.55 (s, 3H). | | | |
| **304** | 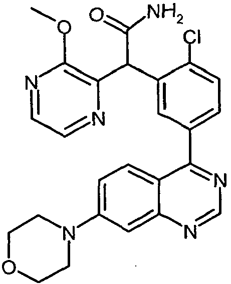 | 2-[2-Chloro-5-(7-morpholin-4-yl-quinazolin-4-yl)-phenyl]-2-(3-methoxy-pyrazin-2-yl)-acetamide | C | C | A |
| | MS: 491.3/493.2 (M+H⁺) (Cl Isotopie, rel. Peakintensitäts-Verhältnis [%] 100:38) | 1H NMR (500 MHz, DMSO-d6) ppm = 9.06 (s, 1H), 8.12 (d, J=2.8, 1H), 8.10 (d, J=2.8, 1H), 7.89 (d, J=9.4, 1H), 7.79 - 7.75 (m, 1H), 7.73 - 7.69 (m, 2H), 7.67 (d, J=8.1, 1H), 7.52 (dd, J=9.5, 2.6, 1H), 7.27 - 7.23 (m, 1H), 7.20 (d, J=2.6, 1H), 5.71 (s, 1H), 3.95 (s, 3H), 3.81 - 3.75 (m, 4H), 3.49 - 3.41 (m, 4H). | | | |
| **305** | 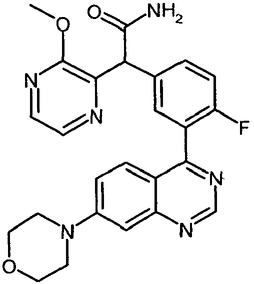 | 2-[4-Fluoro-3-(7-morpholin-4-yl-quinazolin-4-yl)-phenyl]-2-(3-methoxy-pyrazin-2-yl)-acetamide | C | C | A |
| | MS: 475.3 (M+H⁺) | 1H NMR (500 MHz, DMSO-d6) ppm = 9.09 (s, 1H), 8.13 (d, J=2.8, 1H), 8.10 (d, J=2.8, 1H), 7.63 (dd, J=9.4, 2.8, 1H), 7.61 - 7.52 (m, 4H), 7.41 - 7.35 (m, 1H), 7.20 (d, J=2.4, 1H), 7.11 (s, 1H), 5.34 (s, 1H), 3.93 (s, 3H), 3.82 - 3.74 (m, 4H), 3.48 - 3.41 (m, 4H). | | | |
| **306** | 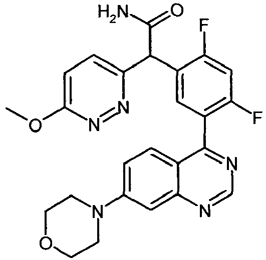 | 2-[2,4-Difluoro-5-(7-morpholin-4-yl-quinazolin-4-yl)-phenyl]-2-(6-methoxy-pyridazin-3-yl)-acetamide | C | D | A |
| | MS: 493.3 (M+H⁺) | 1H NMR (500 MHz, DMSO-d6) ppm = 9.10 (s, 1H), 7.90 (s, 1H), 7.71 (t, J=8.2, 1H), 7.61 - 7.49 (m, 4H), 7.37 (s, 1H), 7.20 (d, J=2.4, 1H), 7.18 (d, J=9.2, 1H), 5.57 (s, 1H), 4.00 (s, 3H), 3.81 - 3.74 (m, 4H), 3.49 - 3.41 (m, 4H). | | | |
| **307** | 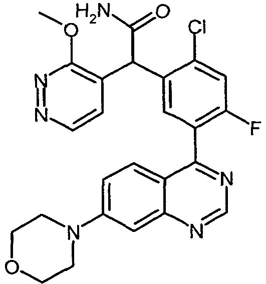 | 2-[2-Chloro-4-fluoro-5-(7-morpholin-4-yl-quinazolin-4-yl)-phenyl]-2-(3-methoxy-pyridazin-4-yl)-acetamide | B | C | A |
| | MS: 509.2/511.1 (M+H⁺) (Cl Isotopie, rel. Peakintensitäts-Verhältnis [%] 100:39) | 1H NMR (500 MHz, DMSO-d6) ppm = 9.09 (s, 1H), 8.80 (d, J=4.7, 1H), 8.02 (s, 1H), 7.80 (d, J=9.5, 1H), 7.59 (dd, J=9.4, 3.3, 1H), 7.53 (dd, J=9.5, 2.5, 1H), 7.46 (d, J=7.5, 1H), 7.42 (s, 1H), 7.20 (d, J=2.4, 1H), 7.08 (dd, J=4.7, 0.5, 1H), 5.46 (s, 1H), 4.07 (s, 3H), 3.80 - 3.76 (m, 4H), 3.49 - 3.43 (m, 4H). | | | |
| **308** | 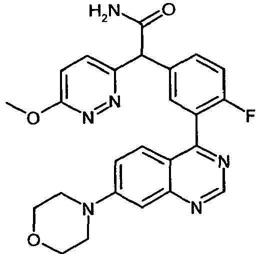 | 2-[4-Fluoro-3-(7-morpholin-4-yl-quinazolin-4-yl)-phenyl]-2-(6-methoxy-pyridazin-3-yl)-acetamide | C | D | A |
| | MS: 475.3 (M+H⁺) | 1H NMR (500 MHz, DMSO-d6) ppm = 9.10 (s, 1H), 7.87 (s, 1H), 7.67 (d, J=9.2, 1H), 7.64 - 7.57 (m, 2H), 7.57 - 7.49 (m, 2H), 7.46 - 7.38 (m, 1H), 7.31 (s, 1H), 7.20 (d, J=2.2, 1H), 7.18 (d, J=9.2, 1H), 5.40 (s, 1H), 4.00 (s, 3H), 3.78 (dd, J=5.8, 3.9, 4H), 3.44 (dd, J=5.8, 4.1, 4H). | | | |
| **309** | 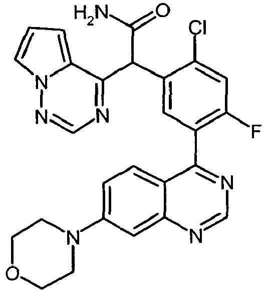 | 2-[2-Chloro-4-fluoro-5-(7-morpholin-4-yl-quinazolin-4-yl)-phenyl]-2-[3H-pyrrolo[2,1-f][1,2,4]triazin-(4E)-ylidene]-acetamid | D | D | D |
| | MS: 518.2/520.2 (M+H⁺) (Cl Isotopie, rel. Peakintensitäts-Verhältnis [%] 100:37) | 1H NMR (500 MHz, DMSO-d6) ppm = 13.76 (d, J=3.9, 1H), 9.07 (s, 1H), 7.86 (d, J=9.7, 1H), 7.84 - 7.76 (m, 2H), 7.56 (d, J=7.8, 1H), 7.51 (dd, J=9.5, 2.5, 1H), 7.32 (dd, J=2.7, 1.7, 1H), 7.18 (d, J=2.4, 1H), 6.99 (s, 1H), 6.37 (s, 1H), 6.26 (dd, J=4.4, 2.7, 1H), 4.87 (dd, J=4.4, 1.7, 1H), 3.81 - 3.74 (m, 4H), 3.49 - 3.42 (m, 4H). | | | |
| **310** | 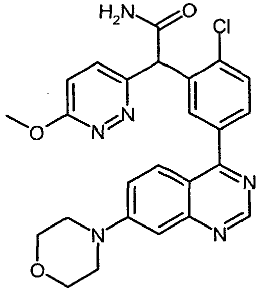 | 2-[2-Chloro-5-(7-morpholin-4-yl-quinazolin-4-yl)-phenyl]-2-(6-methoxy-pyridazin-3-yl)-acetamide | D | D | B |
| | MS: 491.2/493.2 (M+H⁺) (Cl Isotopie, rel. Peakintensitäts-Verhältnis [%] 100:35) | 1H NMR (500 MHz, DMSO-d6) ppm = 9.07 (s, 1H), 7.97 (s, 1H), 7.89 - 7.85 (m, 2H), 7.73 (dd, J=8.2, 2.1, 1H), 7.70 (d, J=8.2, 1H), 7.52 (dd, J=9.5, 2.6, 1H), 7.46 (d, J=9.2, 1H), 7.39 (s, 1H), 7.21 (d, J=2.5, 1H), 7.18 (d, J=9.2, 1H), 5.72 (s, 1H), 4.01 (s, 3H), 3.82 - 3.75 (m, 4H), 3.48 - 3.42 (m, 4H). | | | |
| **311** | 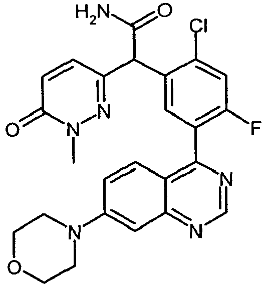 | 2-[2-Chloro-4-fluoro-5-(7-morpholin-4-yl-quinazolin-4-yl)-phenyl]-2-(1-methyl-6-oxo-1,6-dihydro-pyridazin-3-yl)-acetamide | D | D | A |
| | MS: 509.3/511.3 (M+H⁺) (Cl Isotopie, rel. Peakintensitäts-Verhältnis [%] 100:38) | 1H NMR (500 MHz, DMSO-d6) ppm = 9.11 (s, 1H), 7.92 (s, 1H), 7.76 (d, J=9.5, 1H), 7.68 (d, J=7.5, 1H), 7.58 (dd, J=9.4, 3.1, 1H), 7.54 (dd, J=9.5, 2.5, 1H), 7.41 (s, 1H), 7.33 (d, J=9.6, 1H), 7.21 (d, J=2.4, 1H), 6.90 (d, J=9.6, 1H), 5.33 (s, 1H), 3.81 - 3.75 (m, 4H), 3.59 (s, 3H), 3.49 - 3.42 (m, 4H). | | | |
| **312** | 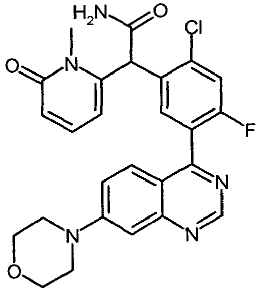 | 2-[2-Chloro-4-fluoro-5-(7-morpholin-4-yl-quinazolin-4-yl)-phenyl]-2-(1-methyl-6-oxo-1,6-dihydro-pyridin-2-yl)-acetamide | D | D | A |
| | MS: 508.3/510.2 (M+H⁺) (Cl Isotopie, rel. Peakintensitäts-Verhältnis [%] 100:38) | 1H NMR (500 MHz, DMSO-d6) ppm = 9.09 (s, 1H), 8.04 (s, 1H), 7.83 (d, J=9.4, 1H), 7.62 - 7.44 (m, 3H), 7.42 - 7.27 (m, 2H), 7.19 (d, J=2.4, 1H), 6.36 (dd, J=9.1, 1.3, 1H), 5.90 (dd, J=7.1, 1.3, 1H), 5.48 (s, 1H), 3.77 (dd, J=5.8, 4.0, 4H), 3.52 - 3.42 (m, 4H), 3.40 (s, 3H). | | | |
| **313** | 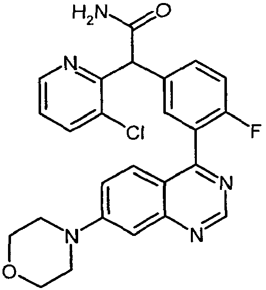 | 2-(3-Chloro-pyridin-2-yl)-2-[4-fluoro-3-(7-morpholin-4-yl-quinazolin-4-yl)-phenyl]-acetamide | C | C | B |
| | MS: 478.2/480.2 (M+H⁺) (Cl Isotopie, rel. Peakintensitäts-Verhältnis [%] 100:36) | 1H NMR (500 MHz, DMSO-d6) ppm = 9.10 (s, 1H), 8.51 (dd, J=4.7, 1.5, 1H), 7.93 (dd, J=8.1, 1.5, 1H), 7.64 (dd, J=9.4, 2.9, 1H), 7.61 - 7.53 (m, 4H), 7.39 (dd, J=9.9, 8.7, 1H), 7.36 (dd, J=8.1, 4.7, 1H), 7.21 (d, J=2.5, 1H), 7.17 (s, 1H), 5.53 (s, 1H), 3.84 - 3.73 4H), 3.50 - 3.41 (m, 4H). | | | |
| **314** | 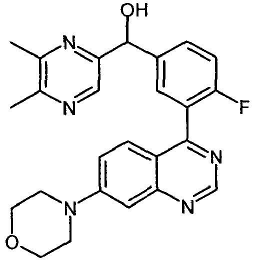 | (5,6-Dimethyl-pyrazin-2-yl)-[4-fluoro-3-(7-morpholin-4-yl-quinazolin-4-yl)-phenyl]-methanol | C | B | B |
| | MS: 446.2 (M+H⁺) | 1H NMR (500 MHz, DMSO-d6) ppm = 9.09 (s, 1H), 8.51 (s, 1H), 7.66 - 7.61 (m, 2H), 7.54 - 7.49 (m, 2H), 7.40 - 7.35 (m, 1H), 7.21 - 7.19 (m, 1H), 6.32 (d, J=4.2, 1H), 5.84 (d, J=4.0, 1H), 3.80 - 3.75 (m, 4H), 3.46 - 3.42 (m, 4H), 2.45 (s, 3H), 2.44 (s, 3H). | | | |
| **315** | 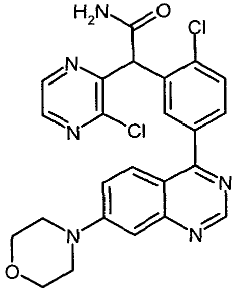 | 2-[2-Chloro-5-(7-morpholin-4-yl-quinazolin-4-yl)-phenyl]-2-(3-chloro-pyrazin-2-yl)-acetamide | D | D | C |
| | MS: 495.0/497.1/499.1 (M+H⁺) (Cl₂ Isotopie, rel. Peakintensitäts-Verhältnis [%] 100:75:20) | 1H NMR (500 MHz, DMSO-d6) ppm = 9.07 (s, 1H), 8.60 (d, J=2.5, 1H), 8.45 (d, J=2.5, 1H), 7.97 - 7.91 (m, 1H), 7.88 (d, J=9.5, 1H), 7.75 (dd, J=8.3, 2.1,1H), 7.71 (d, J=8.2, 1H), 7.66 (d, J=2.1, 1H), 7.53 (dd, J=9.5, 2.6, 1H), 7.47 - 7.42 (m, 1H), 7.22 - 7.19 (m, 1H), 5.87 (s, 1H), 3.81 - 3.76 (m, 4H), 3.47 - 3.42 (m, 4H). | | | |
| **316** | 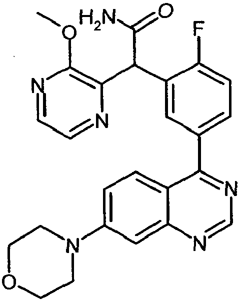 | 2-[2-Fluoro-5-(7-morpholin-4-yl-quinazolin-4-yl)-phenyl]-2-(3-methoxy-pyrazin-2-yl)-acetamide | D | C | B |
| | MS: 475.4 (M+H⁺) | 1H NMR (500 MHz, DMSO-d6) ppm = 9.05 (s, 1H), 8.13 (d, J=2.9, 2H), 7.94 (d, J=9.4, 1H), 7.78 - 7.70 (m, 3H), 7.53 (dd, J=9.5, 2.6, 1H), 7.41 (dd, J=9.6, 8.6, 1H), 7.23 (s, 1H), 7.19 (d, J=2.5, 1H), 5.59 (s, 1H), 3.95 (s, 3H), 3.82 - 3.74 (m, 4H), 3.48 - 3.40 (m, 4H). | | | |
| **317** | 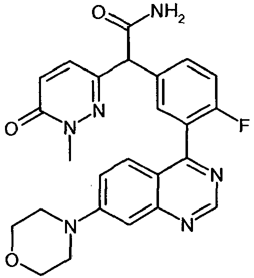 | 2-[4-Fluoro-3-(7-morpholin-4-yl-quinazolin-4-yl)-phenyl]-2-(1-methyl-6-oxo-1,6-dihydro-pyridazin-3-yl)-acetamide | D | D | B |
| | MS: 475.3 (M+H⁺) | 1H NMR (500 MHz, DMSO-d6) ppm = 9.10 (s, 1H), 7.86 (s, 1H), 7.64 - 7.50 (m, 4H), 7.47 (d, J=9.6, 1H), 7.44 (dd, J=9.8, 8.5, 1H), 7.34 (s, 1H), 7.20 (d, J=2.2, 1H), 6.90 (d, J=9.6, 1H), 5.05 (s, 1H), 3.83 - 3.73 (m, 4H), 3.61 (s, 3H), 3.44 (t, J=4.9, 4H). | | | |
| **318** | 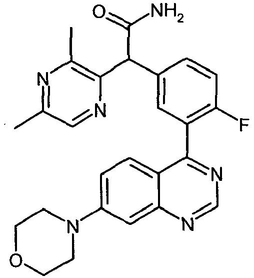 | 2-(3,5-Dimethyl-pyrazin-2-yl)-2-[4-fluoro-3-(7-morpholin-4-yl-quinazolin-4-yl)-phenyl]-acetamide | C | D | A |
| | MS: 473.4 (M+H⁺) | 1H NMR (500 MHz, DMSO-d6) ppm = 9.09 (s, 1H), 8.29 (s, 1H), 7.60 (dd, J=9.5, 2.9, 1H), 7.59 - 7.55 (m, 2H), 7.54 (dd, J=9.5, 2.5, 1H), 7.50 (s, 1H), 7.42 - 7.33 (m, 1H), 7.20 (d, J=2.5, 1H), 7.15 (s, 1H), 5.35 (s, 1H), 3.78 (dd, J=5.8, 4.0, 4H), 3.44 (dd, J=6.0, 3.9, 4H), 2.49 (s, 3H), 2.41 (s, 3H). | | | |
| **319** | 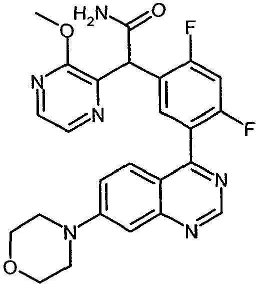 | 2-[2,4-Difluoro-5-(7-morpholin-4-yl-quinazolin-4-yl)-phenyl]-2-(3-methoxy-pyrazin-2-yl)-acetamide | C | C | A |
| | MS: 493.4 (M+H⁺) | 1H NMR (500 MHz, DMSO-d6) ppm = 9.08 (s, 1H), 8.13 (d, J=2.8, 1H), 8.11 (d, J=2.8, 1H), 7.73 (s, 1H), 7.61 (dd, J=9.4, 3.2, 1H), 7.58 - 7.46 (m, 3H), 7.24 (s, 1H), 7.20 (d, J=2.4, 1H), 5.53 (s, 1H), 3.95 (s, 3H), 3.78 (dd, J=5.8, 4.0, 4H), 3.45 (dd, J=6.0, 3.9, 4H). | | | |
| **320** | 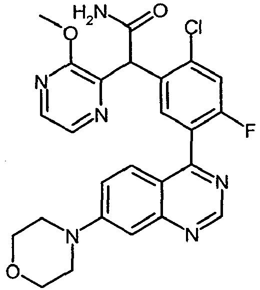 | 2-[2-Chloro-4-fluoro-5-(7-morpholin-4-yl-quinazolin-4-yl)-phenyl]-2-(3-methoxy-pyrazin-2-yl)-acetamide | C | B | A |
| | MS: 509.3/511.3 (M+H⁺) (Cl Isotopie, rel. Peakintensitäts-Verhältnis [%] 100:38) | 1H NMR (500 MHz, DMSO-d6) ppm = 9.09 (s, 1H), 8.12 (d, J=2.8, 1H), 8.09 (d, J=2.8, 1H), 7.78 (s, 1H), 7.72 (d, J=9.6, 1H), 7.60 (dd, J=9.4, 3.1, 1H), 7.55 (dd, J=9.5, 2.5, 1H), 7.51 (d, J=7.6, 1H), 7.26 (s, 1H), 7.20 (d, J=2.4, 1H), 5.65 (s, 1H), 3.95 (s, 3H), 3.86 - 3.66 (m, 4H), 3.56 - 3.37 (m, 4H). | | | |
| **321** | 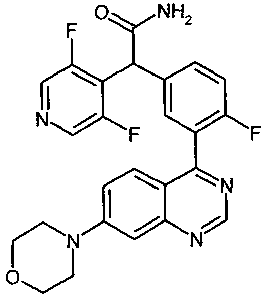 | 2-(3,5-Difluoro-pyridin-4-yl)-2-[4-fluoro-3-(7-morpholin-4-yl-quinazolin-4-yl)-phenyl]-acetamide | C | B | B |
| | MS: 480.2 (M+H⁺) | 1H NMR (500 MHz, DMSO-d6) ppm = 9.09 (s, 1H), 8.50 (s, 2H), 7.59 (s, 1H), 7.58 - 7.49 (m, 4H), 7.46 - 7.37 (m, 2H), 7.20 (d, J=2.2, 1H), 5.42 (s, 1H), 3.87 - 3.64 (m, 4H), 3.59 - 3.40 (m, 4H). | | | |
| **322** | 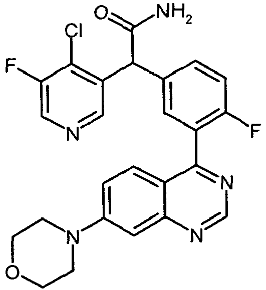 | 2-(4-Chloro-5-fluoro-pyridin-3-yl)-2-[4-fluoro-3-(7-morpholin-4-yl-quinazolin-4-yl)-phenyl]-acetamide | B | B | C |
| | MS: 496.2/498.1 (M+H⁺) (Cl Isotopie, rel. Peakintensitäts-Verhältnis [%] 100:37) | 1H NMR (500 MHz, DMSO-d6) ppm = 9.09 (d, J=2.3, 1H), 8.65 (s, 1H), 8.33 (s, 1H), 7.94 (s, 1H), 7.61 - 7.50 (m, 4H), 7.44 (dt, J=20.5, 9.0, 2H), 7.20 (d, J=2.1, 1H), 5.44 (s, 1H), 3.80 - 3.75 (m, 4H), 3.47 - 3.42 (m, 4H). | | | |
| **323** | 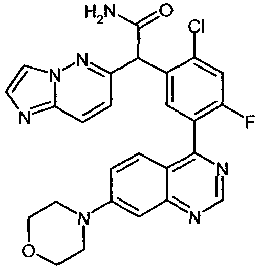 | 2-[2-Chloro-4-fluoro-5-(7-morpholin-4-yl-quinazolin-4-yl)-phenyl]-2-imidazo[1,2-b]pyridazin-6-yl-acetamide | B | B | B |
| | MS: 518.2/520.2 (M+H⁺) (Cl Isotopie, rel. Peakintensitäts-Verhältnis [%] 100:35) | 1H NMR (500 MHz, DMSO-d6) ppm = 9.10 (d, J=10.0, 1H), 8.24 (s, 1H), 8.08 (d, J=9.5, 1H), 8.02 (s, 1H), 7.79 (d, J=9.5, 1H), 7.76 (d, J=1.2, 1H), 7.72 (d, J=7.6, 1H), 7.60 (dd, J=9.5, 3.2, 1H), 7.53 (dd, J=9.5, 2.5, 1H), 7.47 (s, 1H), 7.22 (d, J=2.5, 1H), 7.11 (d, J=9.5, 1H), 5.61 (s, 1H), 3.81 - 3.74 (m, 4H), 3.49 - 3.42 (m, 4H). | | | |
| **324** | 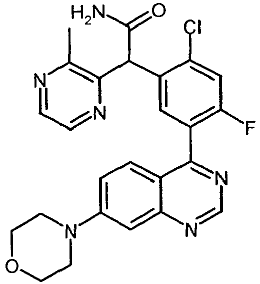 | 2-[2-Chloro-4-fluoro-5-(7-morpholin-4-yl-quinazolin-4-yl)-phenyl]-2-(3-methyl-pyrazin-2-yl)-acetamide | B | B | A |
| | MS: 493.3/495.3 (M+H⁺) (Cl Isotopie, rel. Peakintensitäts-Verhältnis [%] 100:38) | 1H NMR (500 MHz, DMSO-d6) ppm = 9.08 (s, 1H), 8.40 (d, J=2.5, 1H), 8.38 (d, J=2.5, 1H), 7.80 (s, 1H), 7.73 (d, J=9.5, 1H), 7.59 (dd, J=9.4, 3.0, 1H), 7.54 (dd, J=9.5, 2.5, 1H), 7.48 (d, J=7.6, 1H), 7.34 (s, 1H), 7.19 (d, J=2.4, 1H), 5.66 (s, 1H), 3.78 (dd, J=5.8, 4.0, 4H), 3.45 (dd, J=5.9, 3.9, 4H), 2.55 (s, 3H). | | | |
| **325** | 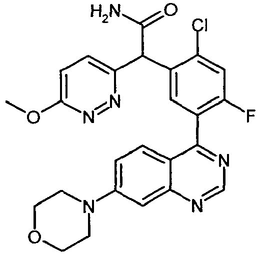 | 2-[2-Chloro-4-fluoro-5-(7-morpholin-4-yl-quinazolin-4-yl)-phenyl]-2-(6-methoxy-pyridazin-3-yl)-acetamide | D | D | B |
| | MS: 509.2/511.1 (M+H⁺) (Cl Isotopie, rel. Peakintensitäts-Verhältnis [%] 100:37) | 1H NMR (500 MHz, DMSO-d6) ppm = 9.11 (s, 1H), 7.96 (s, 1H), 7.75 (d, J=9.5, 1H), 7.71 (d, J=7.6, 1H), 7.63 - 7.51 (m, 2H), 7.47 (d, J=9.2, 1H), 7.39 (s, 1H), 7.21 (d, J=2.4, 1H), 7.17 (d, J=9.2, 1H), 5.67 (s, 1H), 4.00 (s, 3H), 3.82 - 3.74 (m, 4H), 3.50 - 3.42 (m, 4H). | | | |
| **326** | 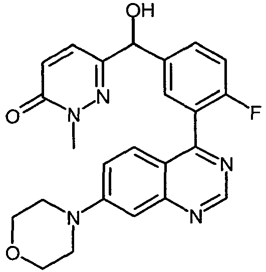 | 6-{[4-Fluoro-3-(7-morpholin-4-yl-quinazolin-4-yl)-phenyl]-hydroxymethyl}-2-methyl-2H-pyridazin-3-one | C | B | A |
| | MS: 448.2 (M+H⁺) | 1H NMR (500 MHz, DMSO-d6) ppm = 9.10 (s, 1H), 7.66 - 7.60 (m, 2H), 7.55 - 7.49 (m, 2H), 7.47 (d, J=9.6, 1H), 7.44 - 7.39 (m, 1H), 7.21 - 7.19 (m, 1H), 6.93 (d, J=9.6, 1H), 6.46 (d, J=4.3, 1H), 5.66 (d, J=4.3, 1H), 3.80 - 3.75 (m, 4H), 3.62 (s, 3H), 3.46 - 3.42 (m, 4H). | | | |
| **327** | 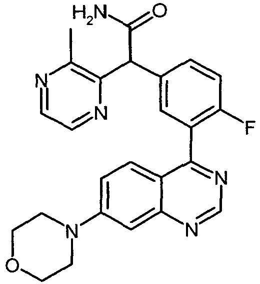 | 2-[4-Fluoro-3-(7-morpholin-4-yl-quinazolin-4-yl)-phenyl]-2-(3-methyl-pyrazin-2-yl)-acetamide | C | D | A |
| | MS: 459.3 (M+H⁺) | 1H NMR (500 MHz, DMSO-d6) ppm = 9.09 (s, 1H), 8.41 (d, J=2.4, 1H), 8.38 (d, J=2.6, 1H), 7.64 - 7.51 (m, 5H), 7.38 (dd, J=10.6, 8.5, 1H), 7.21 - 7.16 (m, 2H), 5.41 (s, 1H), 3.83 - 3.74 (m, 4H), 3.46 - 3.40 (m, 4H), 2.54 (s, 3H). | | | |
| **328** | 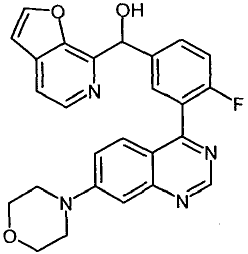 | [4-Fluoro-3-(7-morpholin-4-yl-quinazolin-4-yl)-phenyl]-furo[2,3-c]pyridin-7-yl-methanol | A | A | D |
| | MS: 457.2 (M+H⁺) | 1H NMR (500 MHz, DMSO-d6) ppm = 9.08 (s, 1H), 8.32 (d, J=5.2, 1H), 8.21 (d, J=2.2, 1H), 7.74 - 7.67 (m, 2H), 7.64 (d, J=5.2, 1H), 7.52 - 7.49 (m, 2H), 7.36 (dd, J=9.9, 8.4, 1H), 7.22 - 7.16 (m, 1H), 7.06 (d, J=2.2, 1H), 6.32 (d, J=5.0, 1H), 6.25 (d, J=5.0, 1H), 3.80 - 3.75 (m, 4H), 3.46 - 3.41 (m, 4H). | | | |
| **329** | 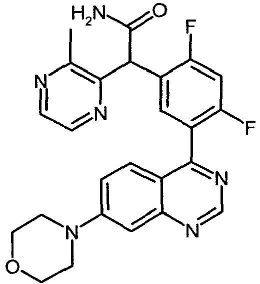 | 2-[2,4-Difluoro-5-(7-morpholin-4-yl-quinazolin-4-yl)-phenyl]-2-(3-methyl-pyrazin-2-yl)-acetamide | C | C | A |
| | MS: 477.2 (M+H⁺) | 1H NMR (500 MHz, DMSO-d6) ppm = 9.08 (s, 1H), 8.41 (d, J=2.6, 1H), 8.39 (d, J=2.7, 1H), 7.77 - 7.68 (m, 1H), 7.61 (dd, J=9.4, 3.1, 1H), 7.59 - 7.47 (m, 3H), 7.32 (s, 1H), 7.19 (d, J=2.5, 1H), 5.57 (s, 1H), 3.84 - 3.74 (m, 4H), 3.48 - 3.41 (m, 4H), 2.55 (s, 3H). | | | |
| **330** | 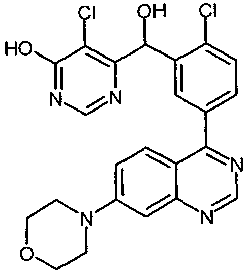 | 5-Chloro-6-{[2-chloro-5-(7-morpholin-4-yl-quinazolin-4-yl)-phenyl]-hydroxymethyl}-pyrimidin-4-ol | C | D | A |
| | MS: 484.1/486.1/488.1 (M+H⁺) (Cl₂ Isotopie. rel. Peakintensitäts-Verhältnis [%] 100:70:18) | 1H NMR (500 MHz, DMSO-d6) ppm = 13.06 (s, 1H), 9.09 (s, 1H), 8.22 - 8.14 (m, 2H), 7.97 (d, J=9.4, 1H), 7.71 (dd, J=8.2, 2.2, 1H), 7.62 - 7.56 (m, 2H), 7.22 (d, J=2.6, 1H), 6.46 (d, J=5.5, 1H), 6.31 (d, J=5.5, 1H), 3.83 - 3.77 (m, 4H), 3.48 - 3.43 (m, 4H). | | | |
| **331** | 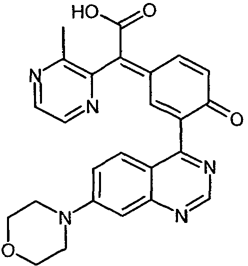 | (3-Methyl-pyrazin-2-yl)-[3-(7-morpholin-4-yl-quinazolin-4-yl)-4-oxo-cyclohexa-2,5-dien-(E)-ylidene]-acetic acid | C | C | A |
| | MS: 456.2 (M+H⁺) | 1H NMR (500 MHz, DMSO-d6) ppm = 9.06 (s, 1H), 8.70 (d, J=2.5, 1H), 8.56 (d, J=2.6, 1H), 8.01 (dd, J=8.8, 2.3, 1H), 7.84 (d, J=2.3, 1H), 7.48 (d, J=1.4, 2H), 7.38 (d, J=8.8, 1H), 7.17 - 7.14 (m, 1H), 3.81 - 3.74 (m, 4H), 3.45 - 3.39 (m, 4H), 2.55 (s, 3H). | | | |
| **332** | 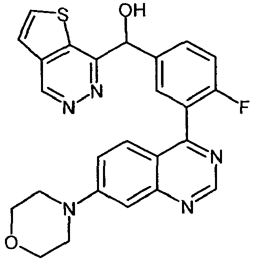 | [4-Fluoro-3-(7-morpholin-4-yl-quinazolin-4-yl)-phenyl]-thieno[2,3-d]pyridazin-7-yl-methanol | A | A | B |
| | MS: 474.1 (M+H⁺) | 1H NMR (500 MHz, DMSO-d6) ppm = 9.56 (s, 1H), 9.08 (s, 1H), 8.26 (d, J=5.4, 1H), 7.79 - 7.72 (m, 2H), 7.70 (d, J=5.4, 1H), 7.51 - 7.44 (m, 2H), 7.43 - 7.35 (m, 1H), 7.21 - 7.16 (m, 1H), 7.10 (d, J=3.8, 1H), 6.38 (d, J=3.9, 1H), 3.82 - 3.74 (m, 4H), 3.47 - 3.40 (m, 4H). | | | |
| **333** | 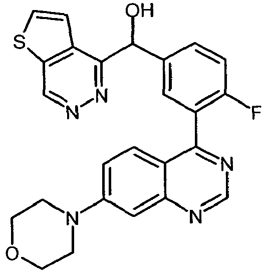 | [4-Fluoro-3-(7-morpholin-4-yl-quinazolin-4-yl)-phenyl]-thieno[2,3-d]pyridazin-4-yl-methanol | A | A | B |
| | MS: 474.2 (M+H⁺) | 1H NMR (500 MHz, DMSO-d6) ppm = 9.84 (d, J=0.6, 1H), 9.08 (s, 1H), 8.28 (d, J=5.3, 1H), 7.89 (dd, J=5.4, 0.7, 1H), 7.75 - 7.67 (m, 2H), 7.49 (dd, J=9.4, 2.4, 1H), 7.45 (dd, J=9.4, 2.9, 1H), 7.42 - 7.34 (m, 1H), 7.19 (d, J=2.3, 1H), 6.82 (s, 1H), 6.42 (s, 1H), 3.81 - 3.75 (m, 4H), 3.47 - 3.41 (m, 4H). | | | |
| **334** | 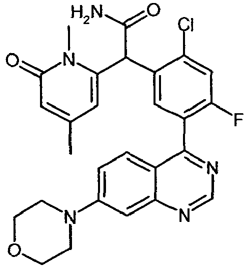 | 2-[2-Chloro-4-fluoro-5-(7-morpholin-4-yl-quinazolin-4-yl)-phenyl]-2-(1,4-dimethyl-6-oxo-1,6-dihydro-pyridin-2-yl)-acetamide | D | D | A |
| | MS: 522.1/524.1 (M+H⁺) (Cl Isotopie, rel. Peakintensitäts-Verhältnis [%] 100:35) | 1H NMR (500 MHz, DMSO-d6) ppm = 9.10 (s, 1H), 8.10 - 7.99 (m, 1H), 7.85 (d, J=9.4, 1H), 7.58 - 7.49 (m, 3H), 7.34 (d, J=7.4, 1H), 7.24 - 7.17 (m, 1H), 6.22 - 6.15 (m, 1H), 5.79 - 5.77 (m, 1H), 5.47 (s, 1H), 3.82 - 3.75 (m, 4H), 3.46 (t, J=5.0, 4H), 3.36 (s, 3H), 2.05 (s, 3H). | | | |
| **335** | 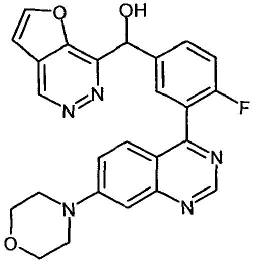 | [4-Fluoro-3-(7-morpholin-4-yl-quinazolin-4-yl)-phenyl]-furo[2,3-d]pyridazin-7-yl-methanol | A | A | A |
| | MS: 458.2 (M+H⁺) | 1H NMR (500 MHz, DMSO-d6) ppm = 9.59 (s, 1H), 9.09 (s, 1H), 8.37 (d, J=2.1, 1H), 7.82 - 7.70 (m, 2H), 7.54 - 7.47 (m, 2H), 7.40 (dd, J=9.8, 8.6, 1H), 7.23 - 7.16 (m, 2H), 6.69 (d, J=4.6, 1H), 6.40 (d, J=4.6, 1H), 3.81 - 3.75 (m, 4H), 3.47 - 3.41 (m, 4H). | | | |
| **336** | 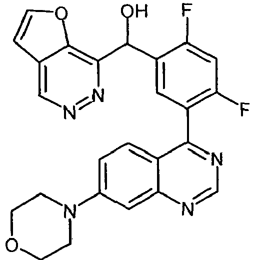 | [2,4-Difluoro-5-(7-morpholin-4-yl-quinazolin-4-yl)-phenyl]-furo[2,3-d]pyridazin-7-yl-methanol | A | A | A |
| | MS: 475.7 (M+H⁺) | 1H NMR (500 MHz, DMSO-d6) ppm = 9.63 (s, 1H), 9.14 (s, 1H), 8.41 (d, J=2.1, 1H), 8.10 (t, J=8.1, 1H), 7.63 (dd, J=9.4, 3.0, 1H), 7.58 (dd, J=9.4, 2.5, 1H), 7.45 (t, J=10.1, 1H), 7.27 - 7.19 (m, 2H), 6.82 (d, J=5.3, 1H), 6.63 (d, J=5.3, 1H), 3.83 - 3.77 (m, 4H), 3.50 - 3.45 (m, 4H). | | | |
| **337** | 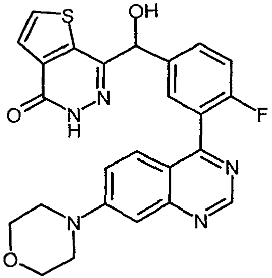 | 7-[[4-Fluoro-3-(7-morpholinoquinazolin -4-yl)phenyl]-hydroxy-methyl]-5H-thieno[2,3-d]pyridazin-4-one | A | B | B |
| | MS: 490.1 (M+H⁺) | 1H NMR (500 MHz, DMSO-d6) ppm = 12.73 (s, 1H), 9.10 (s, 1H), 8.04 (d, J=5.3, 1H), 7.74 - 7.63 (m, 2H), 7.59 (d, J=5.2, 1H), 7.53 - 7.45 (m, 2H), 7.44 - 7.37 (m, 1H), 7.23 - 7.13 (m, 1H), 6.88 (s, 1H), 5.94 (s, 1H), 3.82 - 3.71(m, 4H), 3.46 - 3.41 (m, 4H). | | | |
| **338** | 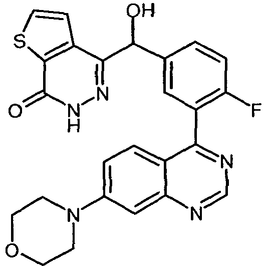 | 4-[[4-Fluoro-3-(7-morpholinoquinazolin -4-yl)phenyl]-hydroxy-methyl]-6H-thieno[2,3-d]pyridazin-7-one | A | B | A |
| | MS: 490.1 (M+H⁺) | 1H NMR (500 MHz, DMSO-d6) ppm = 12.82 (s, 1H), 9.09 (s, 1H), 8.18 (d, J=5.2, 1H), 7.70 - 7.64 (m, 2H), 7.62 (d, J=5.2, 1H), 7.50 (dd, J=9.4, 2.4, 1H), 7.47 (dd, J=9.4, 2.8, 1H), 7.44 - 7.36 (m, 1H), 7.19 (d, J=2.3, 1H), 6.62 (s, 1H), 6.01 (s, 1H), 3.81 - 3.73 (m, 4H), 3.48 - 3.40 (m, 4H). | | | |
| **339** | 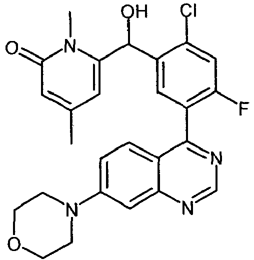 | 6-{[2-Chloro-4-fluoro-5-(7-morpholin-4-yl-quinazolin-4-yl)-phenyl]-hydroxymethyl}-1,4-dimethyl-1H-pyridin-2-one | C | B | A |
| | MS: 494.7/496.7 (M+H⁺) (Cl Isotopie, rel. Peakintensitäts-Verhältnis [%] 100:37) | 1H NMR (500 MHz, DMSO-d6) ppm = 9.13 (s, 1H), 7.79 (d, J=9.5, 1H), 7.71 (d, J=7.6, 1H), 7.60 (dd, J=9.4, 3.2, 1H), 7.54 (dd, J=9.4, 2.5, 1H), 7.22 (d, J=2.4, 1H), 6.60 (d, J=6.2, 1H), 6.27 - 6.15 (m, 1H), 6.05 (d, J=6.2, 1H), 5.84 - 5.68 (m, 1H), 3.81 - 3.75 (m, 4H), 3.52 - 3.43 (m, 7H), 2.05 (s, 3H). | | | |
| **340** | 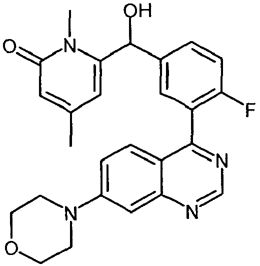 | 6-{[4-Fluoro-3-(7-morpholin-4-yl-quinazolin-4-yl)-phenyl]-hydroxymethyl}-1,4-dimethyl-1H-pyridin-2-one | A | B | A |
| | MS: 461.2 (M+H⁺) | 1H NMR (500 MHz, DMSO-d6) ppm = 9.10 (s, 1H), 7.62 - 7.54 (m, 2H), 7.54 - 7.49 (m, 2H), 7.49 - 7.41 (m, 1H), 7.22 - 7.18 (m, 1H), 6.50 (d, J=5.1, 1H), 6.21 - 6.15 (m, 2H), 5.90 (d, J=5.1, 1H), 3.81 - 3.74 (m, 4H), 3.48 - 3.41 (m, 4H), 3.30 (s, 3H), 2.12 (s, 3H). | | | |
| **341** | 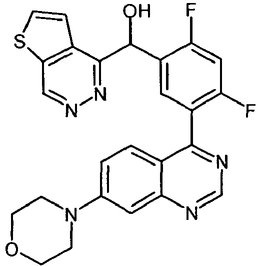 | [2,4-Difluoro-5-(7-morpholin-4-yl-quinazolin-4-yl)-phenyl]-thieno[2,3-d]pyridazin-4-yl-methanol | A | A | B |
| | MS: 492.2 (M+H⁺) | 1H NMR (500 MHz, DMSO-d6) ppm = 9.85 (d, J=0.8, 1H), 9.12 (s, 1H), 8.33 (d, J=5.3, 1H), 8.01 (t, J=8.1, 1H), 7.91 (dd, J=5.4, 0.8, 1H), 7.66 - 7.50 (m, 2H), 7.43 (t, J=10.1, 1H), 7.21 (d, J=2.4, 1H), 6.84 (s, 1H), 6.63 (s, 1H), 3.86 - 3.70 (m, 4H), 3.54 - 3.40 (m, 4H). | | | |
| **342** | 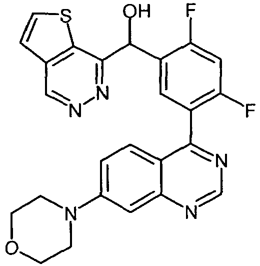 | [2,4-Difluoro-5-(7-morpholin-4-yl-quinazolin-4-yl)-phenyl]-thieno[2,3-d]pyridazin-7-yl-methanol | A | A | A |
| | MS: 492.2 (M+H⁺) | 1H NMR (500 MHz, DMSO-d6) ppm = 9.57 (s, 1H), 9.09 (s, 1H), 8.27 (d, J=5.4, 1H), 7.86 (t, J=8.0, 1H), 7.71 (d, J=5.4, 1H), 7.61 - 7.42 (m, 3H), 7.19 (d, J=2.4, 1H), 7.16 (d, J=4.8, 1H), 6.54 (d, J=4.8, 1H), 3.85 - 3.69 (m, 4H), 3.50 - 3.38 (m, 4H). | | | |
| **343** | 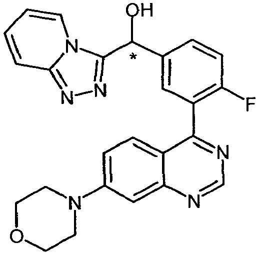 | [4-Fluoro-3-(7-morpholin-4-yl-quinazolin-4-yl)-phenyl]-[1,2,4]triazolo[4,3-a]pyridin-3-yl-methanol (*Ena 2*) | A | B | B |
| | MS: 457.2 (M+H⁺); Rₜ 11.36min (SFC, Chiralpak AS-H, CO₂ / 20 vol.% Methanol, 0.5 vol.% Diethylamin) | s. Racemat | | | |
| **344** | 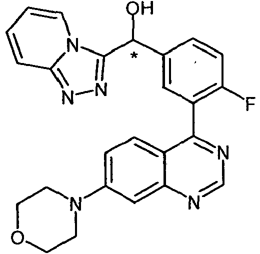 | [4-Fluoro-3-(7-morpholin-4-yl-quinazolin-4-yl)-phenyl]-[1,2,4]triazolo[4,3-a]pyridin-3-yl-methanol (*Ena 1*) | A | C | A |
| | MS: 457.2 (M+H⁺); Rₜ 8.65min (SFC, Chiralpak AS-H, CO₂ / 20 vol.% Methanol, 0.5 vol.% Diethylamin) | s. Racemat | | | |
| **345** | 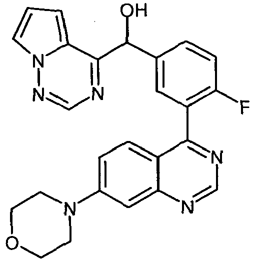 | [4-Fluoro-3-(7-morpholin-4-yl-quinazolin-4-yl)-phenyl]-pyrrolo[2,1-f][1,2,4]triazin-4-yl-methanol | A | A | D |
| | MS: 457.2 (M+H⁺) | 1H NMR (500 MHz, DMSO-d6) ppm = 9.09 (s, 1H), 8.49 (s, 1H), 8.08 (dd, J=2.6, 1.4, 1H), 7.84 - 7.76 (m, 2H), 7.53 - 7.45 (m, 2H), 7.42 - 7.36 (m, 1H), 7.29 (dd, J=4.6, 1.4, 1H), 7.19 (d, J=2.0, 1H), 7.02 (dd, J=4.6, 2.6, 1H), 6.62 (s, 1H), 6.07 (s, 1H), 3.80 - 3.74 (m, 4H), 3.46 - 3.41 (m, 4H). | | | |
| **346** | 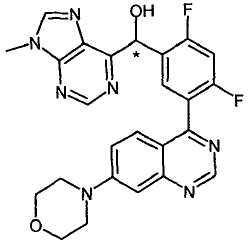 | [2,4-Difluoro-5-(7-morpholin-4-yl-quinazolin-4-yl)-phenyl]-(9-methyl-9H-purin-6-yl)-methanol (*Ena 2*) | C | D | B |
| | MS: 490.2 (M+H⁺); Rₜ 7.65min (SFC, Chiralpak AD-H, CO₂ / 40 vol.% Methanol, 0.5 vol.% Diethylamin) | s. Racemat | | | |
| **347** | 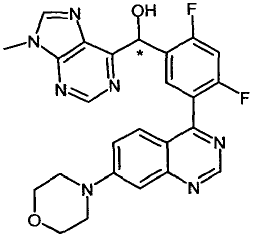 | [2,4-Difluoro-5-(7-morpholin-4-yl-quinazolin-4-yl)-phenyl]-(9-methyl-9H-purin-6-yl)-methanol (*Ena 1*) | B | B | A |
| | MS: 490.2 (M+H⁺); Rₜ 4.59min (SFC, Chiralpak AD-H, CO₂ / 40 vol.% Methanol, 0.5 vol.% Diethylamin) | s. Racemat | | | |
| **348** | 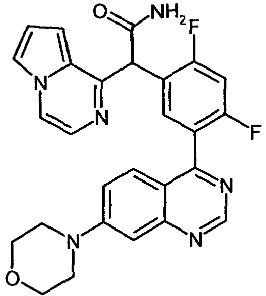 | 2-[2,4-Difluoro-5-(7-morpholin-4-yl-quinazolin-4-yl)-phenyl]-2-pyrrolo[1,2-a]pyrazin-1-yl-acetamide | A | B | C |
| | MS: 501.3 (M+H⁺) | 1H NMR (500 MHz, DMSO-d6) ppm = 9.10 (s, 1H), 8.23 - 8.19 (m, 1H), 7.93 (s, 1H), 7.80 (t, J=8.2, 1H), 7.77 (dd, J=2.4, 1.4, 1H), 7.63 (dd, J=9.4, 3.1, 1H), 7.55 (dd, J=12.3, 2.8, 1H), 7.50 (d, J=10.0, 1H), 7.44 (d, J=4.8, 1H), 7.33 (s, 1H), 7.21 (d, J=2.5, 1H), 6.94 - 6.89 (m, 2H), 5.75 (s, 1H), 3.82 - 3.77 (m, 4H), 3.49 - 3.45 (m, 4H). | | | |
| **349** | 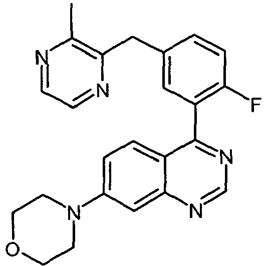 | 4-[2-Fluoro-5-(3-methyl-pyrazin-2-ylmethyl)-phenyl]-7-morpholin-4-yl-quinazoline | B | A | A |
| | MS: 416.3 (M+H⁺) | 1H NMR (500 MHz, DMSO-d6) ppm = 9.09 (s, 1H), 8.43 - 8.36 (m, 2H), 7.56 - 7.49 (m, 2H), 7.46 (dd, J=7.8, 5.5, 2H), 7.39 - 7.33 (m, 1H), 7.22 - 7.16 (m, 1H), 4.28 (s, 2H), 3.81 - 3.75 (m, 4H), 3.46 - 3.41 (m, 4H), 2.53 (s, 3H). | | | |
| **350** | 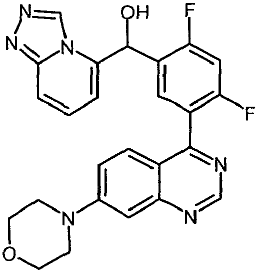 | [2,4-Difluoro-5-(7-morpholin-4-yl-quinazolin-4-yl)-phenyl]-[1,2,4]triazolo[4,3-a]pyridin-5-yl-methanol | B | C | A |
| | MS: 475.2 (M+H⁺) | 1H NMR (500 MHz, DMSO-d6) ppm = 9.33 (s, 1H), 9.13 (s, 1H), 7.94 (t, J=8.1, 1H), 7.80 (d, J=9.2, 1H), 7.61 - 7.50 (m, 3H), 7.42 (dd, J=9.2, 6.8, 1H), 7.22 (d, J=2.4, 1H), 6.93 (d, J=5.7, 1H), 6.87 (d, J=6.8, 1H), 6.49 (d, J=5.6, 1H), 3.83 - 3.75 (m, 4H), 3.49 - 3.43 (m, 4H). | | | |
| **351** | 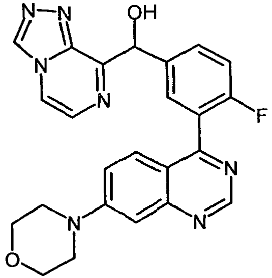 | [4-Fluoro-3-(7-morpholin-4-yl-quinazolin-4-yl)-phenyl]-[1,2,4]triazolo[4,3-a]pyrazin-8-yl-methanol | C | D | A |
| | MS: 458.1 (M+H⁺) | 1H NMR (500 MHz, DMSO-d6) ppm = 9.44 (s, 1H), 9.09 (s, 1H), 8.52 (d, J=4.7, 1H), 7.91 (d, J=4.7, 1H), 7.85 - 7.78 (m, 2H), 7.53 (dd, J=9.4, 2.9, 1H), 7.50 (dd, J=9.4, 2.4, 1H), 7.41 - 7.35 (m, 1H), 7.20 (d, J=2.3, 1H), 6.51 (s, 1H), 6.43 (s, 1H), 3.82 - 3.74 (m, 4H), 3.47 - 3.41 (m, 4H). | | | |
| **352** | 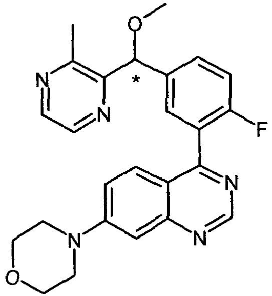 | 4-{2-Fluoro-5-[methoxy-(3-methyl-pyrazin-2-yl)-methyl]-phenyl}-7-morpholin-4-yl-quinazoline (Ena 1) | D | | A* |
| | MS: 446.2 (M+H⁺); Rₜ 3.08min (SFC, Chiralcel OJ-H, CO₂ / 20 vol.% 2-Propanol, 0.5 vol.% Diethylamin) | 1H NMR (500 MHz, DMSO-d6) ppm = 9.11 (s, 1H), 8.53 - 8.42 (m, 2H), 7.69 - 7.58 (m, 2H), 7.58 - 7.48 (m, 2H), 7.48 - 7.38 (m, 1H), 7.21 (d, J=1.9, 1H), 5.80 (s, 1H), 3.81 - 3.70 (m, 4H), 3.48 - 3.39 (m, 4H), 3.36 (s, 3H), 2.59 (s, 3H). | | | |
| **353** | 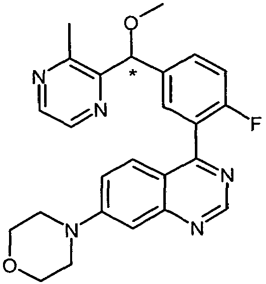 | 4-{2-Fluoro-5-[methoxy-(3-methyl-pyrazin-2-yl)-methyl]-phenyl}-7-morpholin-4-yl-quinazoline (*Ena* 2) | C | D | A |
| | MS: 446.2 (M+H⁺); Rₜ 3.78min (SFC, Chiralcel OJ-H, CO₂ / 20 vol.% 2-Propanol, 0.5 vol.% Diethylamin) | 1H NMR (500 MHz, DMSO-d6) ppm = 9.11 (s, 1H), 8.52 - 8.43 (m, 2H), 7.69 - 7.58 (m, 2H), 7.58 - 7.51 (m, 2H), 7.49 - 7.39 (m, 1H), 7.28 - 7.15 (m, 1H), 5.81 (s, 1H), 3.84 - 3.74 (m, 4H), 3.49 - 3.42 (m, 4H), 3.36 (s, 3H), 2.59 (s, 3H). | | | |
| **354** | 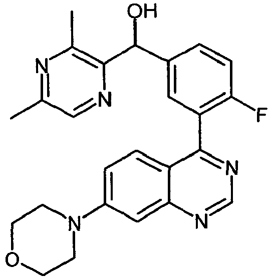 | (3,5-Dimethyl-pyrazin-2-yl)-[4-fluoro-3-(7-morpholin-4-yl-quinazolin-4-yl)-phenyl]-methanol | A | A | A |
| | MS: 446.2 (M+H⁺) | 1H NMR (500 MHz, DMSO-d6) ppm = 9.09 (s, 1H), 8.30 (s, 1H), 7.60 - 7.50 (m, 4H), 7.41 - 7.33 (m, 1H), 7.23 - 7.17 (m, 1H), 6.20 (d, J=5.4, 1H), 6.04 (d, J=5.2, 1H), 3.81 - 3.76 (m, 4H), 3.47 - 3.42 (m, 4H), 2.50 (s, 3H), 2.43 (s, 3H). | | | |
| **355** | 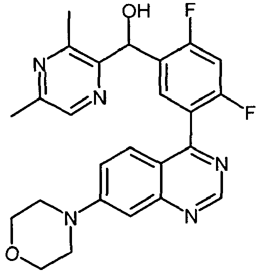 | [2,4-Difluoro-5-(7-morpholin-4-yl-quinazolin-4-yl)-phenyl]-(3,5-dimethyl-pyrazin-2-yl)-methanol | B | B | B |
| | MS: 464.3 (M+H⁺) | 1H NMR (500 MHz, DMSO-d6) ppm = 9.12 (s, 1H), 8.26 (s, 1H), 7.89 (t, *J*=8.2, 1H), 7.61 (dd, J=9.4, 3.2, 1H), 7.56 (dd, J=9.4, 2.5, 1H), 7.39 (t, J=10.1, 1H), 7.21 (d, J=2.4, 1H), 6.28 (d, J=6.0, 1H), 6.23 (d, J=6.0, 1H), 3.82 - 3.75 (m, 4H), 3.49 - 3.41 (m, 4H), 2.62 (s, 3H), 2.43 (s, 3H). | | | |
| **356** | 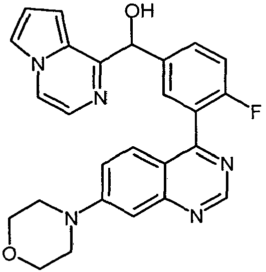 | [4-Fluoro-3-(7-morpholin-4-yl-quinazolin-4-yl)-phenyl]-pyrrolo[1,2-a]pyrazin-1-yl-methanol | A | A | D |
| | MS: 456.2 (M+H⁺) | 1H NMR (500 MHz, DMSO-d6) ppm = 9.10 (s, 1H), 8.20 (dd, J=4.8, 1.0, 1H), 7.79 - 7.72 (m, 3H), 7.53 - 7.45 (m, 3H), 7.40 - 7.34 (m, 1H), 7.20 (d, J=2.1, 1H), 7.02 (dt, J=4.1, 1.2, 1H), 6.87 (dd, J=4.1, 2.5, 1H), 6.33 (d, J=4.9, 1H), 6.06 (d, J=4.8, 1H), 3.82 - 3.76 (m, 4H), 3.48 - 3.42 (m, 4H). | | | |
| **357** | 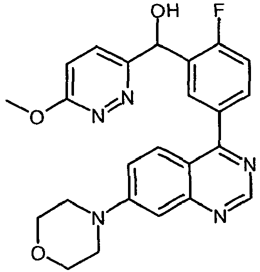 | [2-Fluoro-5-(7-morpholin-4-yl-quinazolin-4-yl)-phenyl]-(6-methoxy-pyridazin-3-yl)-methanol | B | B | A |
| | MS: 448.2 (M+H⁺) | 1H NMR (500 MHz, DMSO-d6) ppm = 9.08 (s, 1H), 8.00 (dd, J=7.1, 2.3, 1H), 7.89 (d, J=9.4, 1H), 7.80 - 7.71 (m, 2H), 7.55 (dd, J=9.5, 2.6, 1H), 7.38 (dd, J=10.0, 8.4, 1H), 7.27 - 7.18 (m, 2H), 6.57 (d, J=4.8, 1H), 6.24 (d, J=4.8, 1H), 4.01 (s, 3H), 3.85 - 3.71 (m, 4H), 3.51 - 3.40 (m, 4H). | | | |
| **358** | 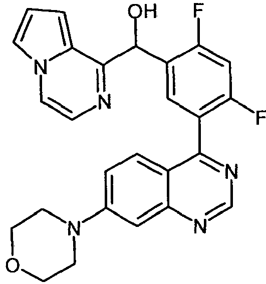 | [2,4-Difluoro-5-(7-morpholin-4-yl-quinazolin-4-yl)-phenyl]-pyrrolo[1,2-a]pyrazin-1-yl-methanol | A | A | C |
| | MS: 474.2 (M+H⁺) | 1H NMR (500 MHz, DMSO-d6) ppm = 9.12 (s, 1H), 8.25 - 8.19 (m, 1H), 7.94 (t, J=8.2, 1H), 7.78 (dd, J=2.5, 1.3, 1H), 7.58 (dd, J=9.3, 3.0, 1H), 7.55 (dd, J=9.4, 2.4, 1H), 7.47 - 7.39 (m, 2H), 7.22 (d, J=2.3, 1H), 6.97 - 6.93 (m, 1H), 6.91 (dd, J=4.1, 2.5, 1H), 6.43 (d, J=5.9, 1H), 6.30 (d, J=5.9, 1H), 3.82 - 3.76 (m, 4H), 3.49 - 3.44 (m, 4H). | | | |
| **359** | 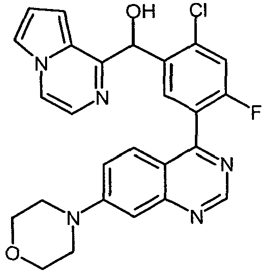 | [2-Chloro-4-fluoro-5-(7-morpholin-4-yl-quinazolin-4-yl)-phenyl]-pyrrolo[1,2-a]pyrazin-1-yl-methanol | A | A | D |
| | MS: 490.2/492.2 (M+H⁺) (Cl Isotopie, rel. Peakintensitäts-Verhältnis [%] 100:31) | 1H NMR (500 MHz, DMSO-d6) ppm = 9.12 (s, 1H), 8.20 (dd, J=4.7, 1.0, 1H), 7.97 (d, J=7.8, 1H), 7.76 (dd, J=2.5, 1.3, 1H), 7.63 (d, J=9.5, 1H), 7.60 (dd, J=9.4, 3.2, 1H), 7.55 (dd, J=9.4, 2.6, 1H), 7.40 (d, J=4.8, 1H), 7.21 (d, J=2.5, 1H), 7.00 - 6.95 (m, 1H), 6.91 (dd, J=4.1, 2.5, 1H), 6.48 (d, J=6.2, 1H), 6.36 (d, J=6.1, 1H), 3.81 - 3.75 (m, 4H), 3.48 - 3.43 (m, 4H). | | | |
| **360** | 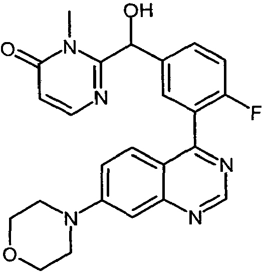 | 2-{[4-Fluoro-3-(7-morpholin-4-yl-quinazolin-4-yl)-phenyl]-hydroxymethyl}-3-methyl-3H-pyrimidin-4-one | B | B | A |
| | MS: 448.3 (M+H⁺) | 1H NMR (400 MHz, DMSO-d6) ppm = 9.10 (s, 1H), 7.91 (d, J=6.5, 1H), 7.67 - 7.60 (m, 2H), 7.60 - 7.50 (m, 2H), 7.44 (t, J=9.5, 1H), 7.20 (d, J=1.7, 1H), 6.72 (s, 1H), 6.38 (d, J=6.5, 1H), 6.00 (s, 1H), 3.82 - 3.73 (m, 4H), 3.48 (s, 3H), 3.47 - 3.42 (m, 4H). | | | |
| **361** | 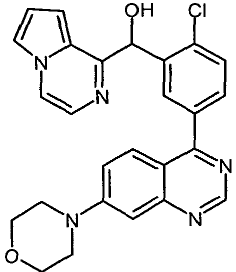 | [2-Chloro-5-(7-morpholin-4-yl-quinazolin-4-yl)-phenyl]-pyrrolo[1,2-a]pyrazin-1-yl-methanol | B | B | D |
| | MS: 472.2/474.2 (M+H⁺) (Cl Isotopie, rel. Peakintensitäts-Verhältnis [%] 100:33) | 1H NMR (500 MHz, DMSO-d6) ppm = 9.08 (s, 1H), 8.22 - 8.19 (m, 1H), 8.14 (d, J=2.2, 1H), 7.89 (d, J=9.4, 1H), 7.76 (dd, J=2.5, 1.3, 1H), 7.70 (dd, J=8.2, 2.2, 1H), 7.61 (d, J=8.2, 1H), 7.53 (dd, J=9.5, 2.6, 1H), 7.43 (d, J=4.8, 1H), 7.21 (d, J=2.6, 1H), 7.00 - 6.97 (m, 1H), 6.93 - 6.90 (m, 1H), 6.45 - 6.40 (m, 2H), 3.81 - 3.76 (m, 4H), 3.48 - 3.42 (m, 4H). | | | |
| **362** | 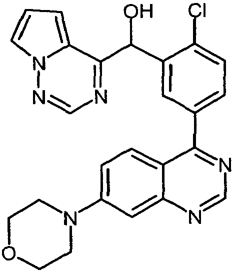 | [2-Chloro-5-(7-morpholin-4-yl-quinazolin-4-yl)-phenyl]-pyrrolo[2,1-f][1,2,4]triazin-4-yl-methanol | B | B | D |
| | MS: 473.2/475.2 (M+H⁺) (Cl Isotopie, rel. Peakintensitäts-Verhältnis [%] 100:38) | 1H NMR (500 MHz, DMSO-d6) ppm = 9.08 (s, 1H), 8.49 (s, 1H), 8.16 (d, J=2.2, 1H), 8.12 (dd, J=2.6, 1.4, 1H), 7.88 (d, J=9.4, 1H), 7.74 (dd, J=8.2, 2.2, 1H), 7.64 (d, J=8.2, 1H), 7.54 (dd, J=9.5, 2.6, 1H), 7.23 - 7.19 (m, 2H), 7.06 (dd, J=4.6, 2.6, 1H), 6.80 (d, J=5.7, 1H), 6.44 (d, J=5.7, 1H), 3.81 - 3.76 (m, 4H), 3.48 - 3.43 (m, 4H). | | | |
| **363** | 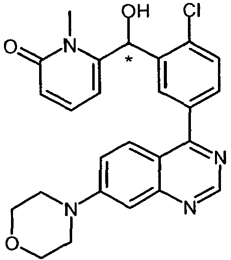 | 6-{[2-Chloro-5-(7-morpholin-4-yl-quinazolin-4-yl)-phenyl]-hydroxymethyl}-1-methyl-1 H-pyridin-2-one (*Ena 2*) | C | C | B |
| | MS: 463.2/465.1 (M+H⁺) (Cl Isotopie, rel. Peakintensitäts-Verhältnis [%] 100:42); Rₜ 4.63min (SFC, Chiralcel OJ-H, CO₂ / 20 vol.% Methanol, 0.5 vol.% Diethylamin) | s. Racemat | | | |
| **364** | 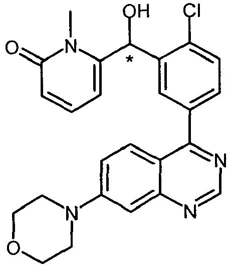 | 6-{[2-Chloro-5-(7-morpholin-4-yl-quinazolin-4-yl)-phenyl]-hydroxymethyl}-1-methyl-1H-pyridin-2-one (*Ena 1*) | A | A | A |
| | MS: 463.2/465.2 (M+H⁺) (Cl Isotopie, rel. Peakintensitäts-Verhältnis [%] 100:41); Rₜ 2.74min (SFC, Chiralcel OJ-H, CO₂ / 20 vol.% Methanol, 0.5 vol.% Diethylamin) | s. Racemat | | | |
| **365** | 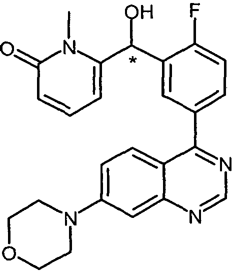 | 6-{[2-Fluoro-5-(7-morpholin-4-yl-quinazolin-4-yl)-phenyl]-hydroxymethyl}-1-methyl-1H-pyridin-2-one (*Ena 2*) | B | B | A |
| | MS: 447.2 (M+H⁺); Rₜ 7.64min (SFC, Chiralpak AS-H, CO₂ / 25 vol.% Methanol) | s. Racemat | | | |
| **366** | 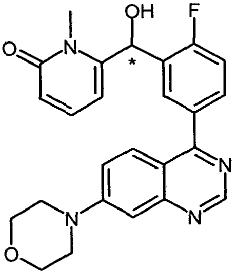 | 6-{[2-Fluoro-5-(7-morpholin-4-yl-quinazolin-4-yl)-phenyl]-hydroxymethyl}-1-methyl-1H-pyridin-2-one (*Ena 1*) | C | C | A |
| | MS: 447.2 (M+H⁺); Rₜ 4.61min (SFC, Chiralpak AS-H, CO₂ / 25 vol.% Methanol) | s. Racemat | | | |
| **367** | 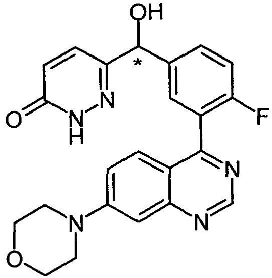 | 6-{[4-Fluoro-3-(7-morpholin-4-yl-quinazolin-4-yl)-phenyl]-hydroxymethyl}-2H-pyridazin-3-one (*Ena 1*) | A | B | A |
| | MS: 434.1 (M+H⁺); Rₜ 13.79 min, (SFC, Chiralcel OJ-H, CO₂ / 15 vol.% Methanol, 0.5 vol.% Diethylamin) | s. Enantiomer | | | |
| | *Ena 2 zu dieser Verbindung : Beispiel 76* | | | | |
| **368** | 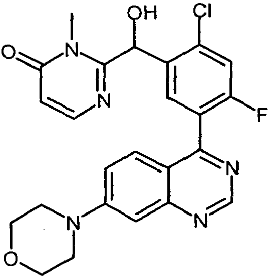 | 2-{[2-Chloro-4-fluoro-5-(7-morpholin-4-yl-quinazolin-4-yl)-phenyl]-hydroxymethyl}-3-methyl-3H-pyrimidin-4-one | C | C | A |
| | MS: 482.2/484.2 (M+H⁺) (Cl Isotopie, rel. Peakintensitäts-Verhältnis [%] 100:41) | 1H NMR (500 MHz, DMSO-d6) ppm = 9.14 (s, 1H), 7.88 (d, J=7.7, 1H), 7.83 (d, J=6.5, 1H), 7.70 (d, J=9.4, 1H), 7.63 (dd, J=9.4, 3.2, 1H), 7.57 (dd, J=9.5, 2.5, 1H), 7.23 (d, J=2.4, 1H), 6.91 (d, J=7.0, 1H), 6.39 (d, J=6.5, 1H), 6.15 (d, J=7.0, 1H), 3.83 - 3.76 (m, 4H), 3.72 (s, 3H), 3.50 - 3.44 (m, 4H). | | | |
| **369** | 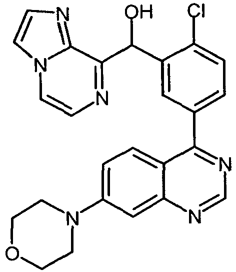 | [2-Chloro-5-(7-morpholin-4-yl-quinazolin-4-yl)-phenyl]-imidazo[1,2-a]pyrazin-8-yl-methanol | C | C | B |
| | MS: 473.1/475.2 (M+H⁺) (Cl Isotopie, rel. Peakintensitäts-Verhältnis [%] 100:25) | 1H NMR (500 MHz, DMSO-d6) ppm = 9.11 (s, 1H), 8.57 (d, J=4.5, 1H), 8.27 (d, J=2.2, 1H), 8.18 (d, J=1.1, 1H), 8.04 (d, J=9.4, 1H), 7.88 (d, J=1.1, 1H), 7.83 (d, J=4.5, 1H), 7.72 (dd, J=8.2, 2.3, 1H), 7.62 - 7.55 (m, 2H), 7.23 (d, J=2.6, 1H), 6.87 (d, J=5.8, 1H), 6.50 (d, J=5.9, 1H), 3.83 - 3.78 (m, 4H), 3.50 - 3.45 (m, 4H). | | | |
| **370** | 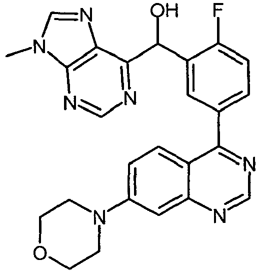 | [2-Fluoro-5-(7-morpholin-4-yl-quinazolin-4-yl)-phenyl]-(9-methyl-9H-purin-6-yl)-methanol | C | B | A |
| | MS: 472.2 (M+H⁺) | 1H NMR (500 MHz, DMSO-d6) ppm = 9.07 (s, 1H), 8.91 (s, 1H), 8.58 (s, 1H), 8.28 (dd, J=7.0, 2.3, 1H), 8.05 (d, J=9.4, 1H), 7.72 (ddd, J=8.3, 5.0, 2.4, 1H), 7.58 (dd, J=9.5, 2.6, 1H), 7.30 (dd, J=10.0, 8.5, 1H), 7.21 (d, J=2.5, 1H), 6.66 (d, J=5.5, 1H), 6.44 (d, J=5.6, 1H), 3.85 (s, 3H), 3.82 - 3.78 (m, 4H), 3.49 - 3.45 (m, 4H). | | | |
| **371** | 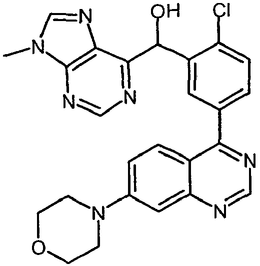 | [2-Chloro-5-(7-morpholin-4-yl-quinazolin-4-yl)-phenyl]-(9-methyl-9H-purin-6-yl)-methanol | C | B | C |
| | MS: 488.2/490.2 (M+H⁺) (Cl Isotopie, rel. Peakintensitäts-Verhältnis [%] 100:33) | 1H NMR (500 MHz, DMSO-d6) ppm = 9.10 (s, 1H), 8.90 (s, 1H), 8.59 (s, 1H), 8.38 (d, J=2.2, 1H), 8.12 (d, J=9.4, 1H), 7.70 (dd, J=8.2, 2.3, 1H), 7.60 (dd, J=9.5, 2.6, 1H), 7.56 (d, J=8.2, 1H), 7.23 (d, J=2.6, 1H), 6.71 (d, J=5.4, 1H), 6.53 (d, J=5.4, 1H), 3.86 (s, 3H), 3.82 - 3.77 (m, 4H), 3.49 - 3.45 (m, 4H). | | | |
| **372** | 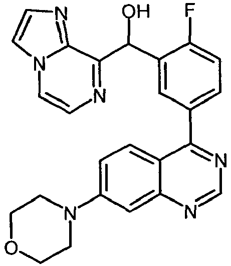 | [2-Fluoro-5-(7-morpholin-4-yl-quinazolin-4-yl)-phenyl]-imidazo[1,2-a]pyrazin-8-yl-methanol | C | B | A |
| | MS: 457.2 (M+H⁺) | 1H NMR (500 MHz, DMSO-d6) ppm = 9.08 (s, 1H), 8.58 (d, J=4.5, 1H), 8.24 - 8.16 (m, 2H), 8.00 (d, J=9.4, 1H), 7.91 - 7.83 (m, 2H), 7.74 (ddd, J=8.1, 5.0, 2.4, 1H), 7.57 (dd, J=9.5, 2.6, 1H), 7.33 (dd, J=10.0, 8.5, 1H), 7.22 (d, J=2.5, 1H), 6.80 (d, J=5.9, 1H), 6.39 (d, J=6.0, 1H), 3.86 - 3.75 (m, 4H), 3.52 - 3.43 (m, 4H). | | | |
| **373** | 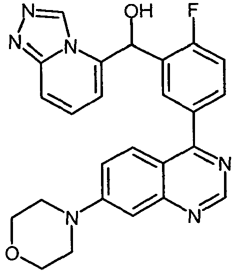 | [2-Fluoro-5-(7-morpholin-4-yl-quinazolin-4-yl)-phenyl]-[1,2,4]triazolo[4,3-a]pyridin-5-yl-methanol | B | B | B |
| | MS: 457.2 (M+H⁺) | 1H NMR (500 MHz, DMSO-d6) ppm = 9.36 - 9.30 (m, 1H), 9.07 (s, 1H), 8.03 (dd, J=7.2, 2.3, 1H), 7.86 - 7.76 (m, 3H), 7.52 - 7.38 (m, 3H), 7.21 (d, J=2.6, 1H), 6.91 (d, J=5.6, 1H), 6.87 - 6.80 (m, 1H), 6.53 (d, J=5.6, 1H), 3.83 - 3.74 (m, 4H), 3.48 - 3.40 (m, 4H). | | | |
| **374** | 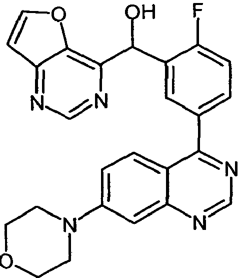 | [2-Fluoro-5-(7-morpholin-4-yl-quinazolin-4-yl)-phenyl]-furo[3,2-d]pyrimidin-4-yl-methanol | A | B | B |
| | MS: 458.2 (M+H⁺) | 1H NMR (500 MHz, DMSO-d6) ppm = 9.08 (s, 1H), 9.02 (s, 1H), 8.59 (d, J=2.2, 1H), 8.20 (dd, J=7.0, 2.3, 1H), 7.92 (d, J=9.4, 1H), 7.77 (ddd, J=8.4, 5.0, 2.4, 1H), 7.57 (dd, J=9.5, 2.6, 1H), 7.37 (dd, J=10.0, 8.5, 1H), 7.27 (d, J=2.3, 1H), 7.22 (d, J=2.5, 1H), 6.73 (d, J=5.2, 1H), 6.48 (d, J=5.1, 1H), 3.82 - 3.76 (m, 4H), 3.49 - 3.44 (m, 4H). | | | |
| **375** | 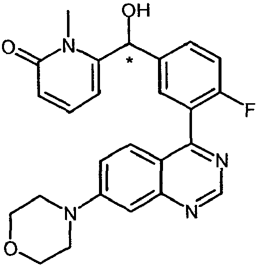 | 6-{[4-Fluoro-3-(7-morpholin-4-yl-quinazolin-4-yl)-phenyl]-hydroxymethyl}-1-methyl-1H-pyridin-2-one (*Ena 2*) | A | B | A |
| | MS: 447.3 (M+H⁺); Rₜ 4.15min (SFC, Chiralcel OJ-H, CO₂ / 20 vol.% Methanol, 0.5 vol.% Diethylamin) | s. Racemat | | | |
| **376** | 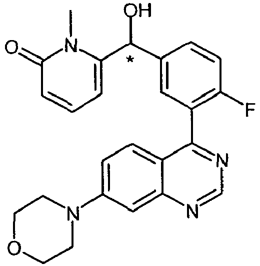 | 6-{[4-Fluoro-3-(7-morpholin-4-yl-quinazolin-4-yl)-phenyl]-hydroxymethyl}-1-methyl-1H-pyridin-2-one (*Ena 1*) | A | A | A |
| | MS: 447.3 (M+H⁺); Rₜ 3.23min (SFC, Chiralcel OJ-H, CO₂ / 20 vol.% Methanol, 0.5 vol.% Diethylamin) | s. Racemat | | | |
| **377** | 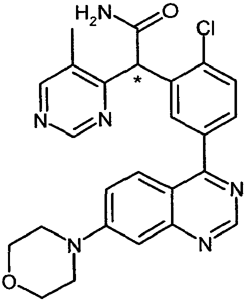 | 2-[2-Chloro-5-(7-morpholin-4-yl-quinazolin-4-yl)-phenyl]-2-(5-methyl-pyrimidin-4-yl)-acetamide (*Ena 2*) | B | B | B |
| | MS: 475.1/477.1 (M+H⁺) (Cl Isotopie, rel. Peakintensitäts-Verhältnis [%] 100:34); Rₜ 6.14min (SFC, Chiralpak AD-H, CO₂ / 40 vol.% Methanol, 0.5 vol.% Diethylamin) | s. Racemat | | | |
| **378** | 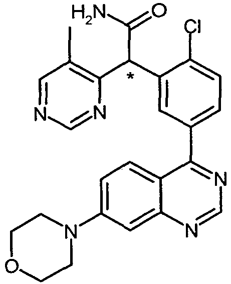 | 2-[2-Chloro-5-(7-morpholin-4-yl-quinazolin-4-yl)-phenyl]-2-(5-methyl-pyrimidin-4-yl)-acetamide (*Ena 1*) | B | B | A |
| | MS: 475.1/477.1 (M+H⁺) (Cl Isotopie, rel. Peakintensitäts-Verhältnis [%] 100:34); Rₜ 3.81min (SFC, Chiralpak AD-H, CO₂ / 40 vol.% Methanol, 0.5 vol.% Diethylamin) | s. Racemat | | | |
| **379** | 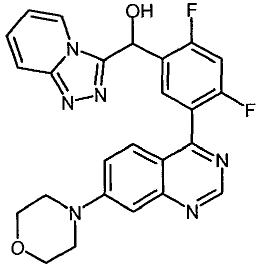 | [2,4-Difluoro-5-(7-morpholin-4-yl-quinazolin-4-yl)-phenyl]-[1,2,4]triazolo[4,3-a]pyridin-3-yl-methanol | B | B | A |
| | MS: 475.2 (M+H⁺) | 1H NMR (500 MHz, DMSO-d6) ppm = 9.14 (s, 1H), 8.63 (dt, J=7.1, 1.2, 1H), 8.02 (t, J=8.1, 1H), 7.81 (dt, J=9.3, 1.1, 1H), 7.64 (dd, J=9.4, 3.3, 1H), 7.60 - 7.50 (m, 2H), 7.44 (ddd, J=9.3, 6.6, 1.1, 1H), 7.23 (d, J=2.5, 1H), 7.06 (td, J=6.8, 1.0, 1H), 6.87 (d, J=6.0, 1H), 6.69 (d, J=6.0, 1H), 3.82 - 3.77 (m, 4H), 3.49 - 3.45 (m, 4H). | | | |
| **380** | 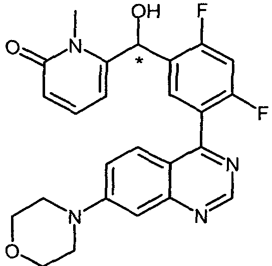 | 6-{[2,4-Difluoro-5-(7-morpholin-4-yl-quinazolin-4-yl)-phenyl]-hydroxymethyl}-1-methyl-1H-pyridin-2-one (*Ena 2*) | B | B | A |
| | MS: 465.2 (M+H⁺); Rₜ 10.95min (SFC, Chiralpak AD-H, CO₂ / 20 vol.% Methanol, 0.5 vol.% Diethylamin) | s. Racemat | | | |
| **381** | 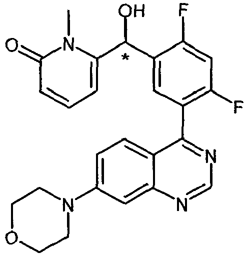 MS: 465.2 (M+H⁺); Rₜ 7.49min (SFC, Chiralpak AD-H, CO₂ / 20 vol.% Methanol, 0.5 vol.% Diethylamin) | 6-{[2,4-Difluoro-5-(7-morpholin-4-yl-quinazolin-4-yl)-phenyl]-hydroxymethyl}-1-methyl-1H-pyridin-2-one (*Ena 1*) | A | B | A |
| | | s. Racemat | | | |
| **382** | 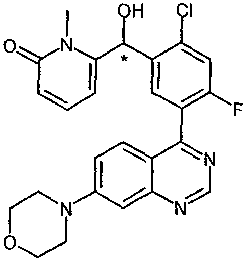 | 6-{[2-Chloro-4-fluoro-5-(7-morpholin-4-yl-quinazolin-4-yl)-phenyl]-hydroxymethyl}-1-methyl-1H-pyridin-2-one (*Ena 2*) | C | B | B |
| | MS: 481.1/483.1 (M+H⁺) (Cl Isotopie, rel. Peakintensitäts-Verhältnis [%] 100:35); Rₜ 4.55min (SFC, Chiralpak AD-H, CO₂ / 40 vol.% Methanol, 0.5 vol.% Diethylamin) | s. Racemat | | | |
| **383** | 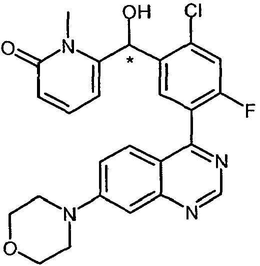 | 6-{[2-Chloro-4-fluoro-5-(7-morpholin-4-yl-quinazolin-4-yl)-phenyl]-hydroxymethyl}-1-methyl-1H-pyridin-2-one (*Ena 1*) | A | A | A |
| | MS: 481.1/483.1 (M+H⁺) (Cl Isotopie, rel. Peakintensitäts-Verhältnis [%] 100:35); Rₜ 2.24min (SFC, Chiralpak AD-H, CO₂ / 40 vol.% Methanol, 0.5 vol.% Diethylamin) | s. Racemat | | | |
| **384** | 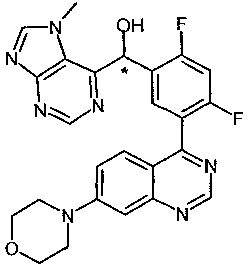 | (R)-[2,4-Difluoro-5-(7-morpholin-4-yl-quinazolin-4-yl)-phenyl]-(7-methyl-7H-purin-6-yl)-methanol (*Ena 2*) | C | D | A |
| | MS: 490.2 (M+H⁺); Rₜ 5.77min (SFC, Chiralpak AD-H, CO₂ / 40 vol.% Methanol, 0.5 vol.% Diethylamin) | s. Racemat | | | |
| **385** | 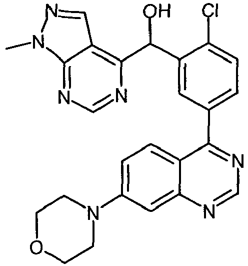 | [2-Chloro-5-(7-morpholin-4-yl-quinazolin-4-yl)-phenyl]-(1-methyl-1H-pyrazolo[3,4-d]pyrimidin-4-yl)-methanol | C | B | A |
| | MS: 488.2/490.2 (M+H⁺) (Cl Isotopie, rel. Peakintensitäts-Verhältnis [%] 100:31) | 1H NMR (500 MHz, DMSO-d6) ppm = 9.05 (s, 1H), 8.92 (s, 1H), 8.44 (s, 1H), 7.95 (d, J=2.2, 1H), 7.80 (d, J=9.4, 1H), 7.72 (dd, J=8.2, 2.2, 1H), 7.66 (d, J=8.2, 1H), 7.50 (dd, J=9.5, 2.6, 1H), 7.19 (d, J=2.5, 1H), 6.90 (d, J=4.9, 1H), 6.49 (d, J=4.9, 1H), 4.05 (s, 3H), 3.82 - 3.76 (m, 4H), 3.47 - 3.41 (m, 4H). | | | |
| **386** | 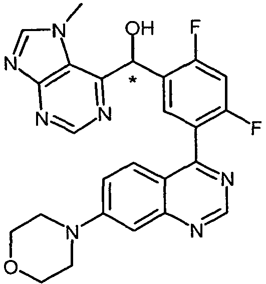 | [2,4-Difluoro-5-(7-morpholin-4-yl-quinazolin-4-yl)-phenyl]-(7-methyl-7H-purin-6-yl)-methanol (*Ena 1*) | B | C | A |
| | MS: 490.2 (M+H⁺); Rₜ 3.50min (SFC, Chiralpak AD-H, CO₂ / 40 vol.% Methanol, 0.5 vol.% Diethylamin) | s. Racemat | | | |
| **387** | 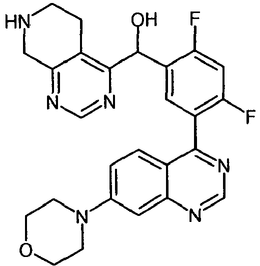 | [2,4-Difluoro-5-(7-morpholin-4-yl-quinazolin-4-yl)-phenyl]-(5,6,7,8-tetrahydro-pyrido[3,4-d]pyrimidin-4-yl)-methanol | C | B | B |
| | MS: 491.2 (M+H⁺) | 1H NMR (500 MHz, DMSO-d6) ppm = 9.11 (s, 1H), 8.83 (s, 1H), 7.87 (t, J=8.2, 1H), 7.60 (dd, J=9.4, 3.0, 1H), 7.56 (dd, J=9.4, 2.5, 1H), 7.42 (t, J=10.1, 1H), 7.21 (d, J=2.4, 1H), 6.33 (d, J=6.3, 1H), 6.16 (d, J=6.3, 1H), 3.86 (s, 2H), 3.82 - 3.75 (m, 4H), 3.49 - 3.42 (m, 4H), 3.09 - 2.94 (m, 2H), 2.94 - 2.72 (m, 2H). | | | |
| **388** | 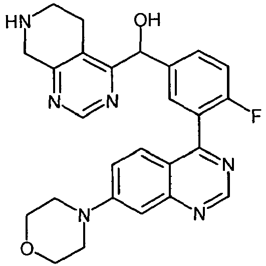 | [4-Fluoro-3-(7-morpholin-4-yl-quinazolin-4-yl)-phenyl]-(5,6,7,8-tetrahydro-pyrido[3,4-d]pyrimidin-4-yl)-methanol | C | B | C |
| | MS: 473.2 (M+H⁺) | 1H NMR (500 MHz, DMSO-d6) ppm = 9.09 (s, 1H), 8.88 (s, 1H), 7.64 - 7.56 (m, 2H), 7.55 - 7.50 (m, 2H), 7.38 (dd, J=10.5, 8.1, 1H), 7.23 - 7.16 (m, 1H), 6.20 (d, J=5.8, 1H), 5.96 (d, J=5.8, 1H), 3.83 (s, 2H), 3.81 - 3.72 (m, 4H), 3.49 - 3.39 (m, 4H), 3.05 - 2.84 (m, 2H), 2.77 - 2.72 (m, 2H). | | | |
| **389** | 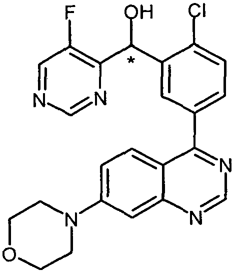 | [2-Chloro-5-(7-morpholin-4-yl-quinazolin-4-yl)-phenyl]-(5-fluoro-pyrimidin-4-yl)-methanol (*Ena 2*) | C | C | C |
| | MS: 452.1/454.1 (M+H⁺) (Cl Isotopie, rel. Peakintensitäts-Verhältnis [%] 100:34); Rₜ 3.98min (SFC, Chiralpak AD-H, CO₂ / 40 vol.% 2-Propanol, 0.5 vol.% Diethylamin) | s. Racemat | | | |
| **390** | 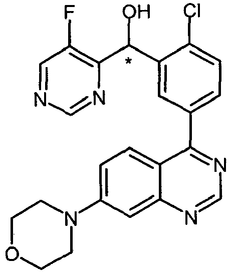 | [2-Chloro-5-(7-morpholin-4-yl-quinazolin-4-yl)-phenyl]-(5-fluoro-pyrimidin-4-yl)-methanol (*Ena 1*) | B | B | B |
| | MS: 452.1/454.1 (M+H⁺) (Cl Isotopie, rel. Peakintensitäts-Verhältnis [%] 100:34); Rₜ 2.20min (SFC, Chiralpak AD-H, CO₂ / 40 vol.% 2-Propanol, 0.5 vol.% Diethylamin) | s. Racemat | | | |
| **391** | 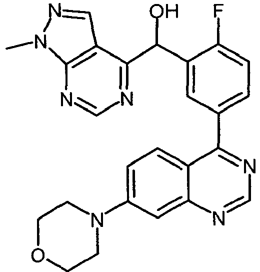 | [2-Fluoro-5-(7-morpholin-4-yl-quinazolin-4-yl)-phenyl]-(1-methyl-1H-pyrazolo[3,4-d]pyrimidin-4-yl)-methanol | C | B | B |
| | MS: 472.2 (M+H⁺) | 1H NMR (500 MHz, DMSO-d6) ppm = 9.05 (s, 1H), 8.90 (s, 1H), 8.49 (s, 1H), 7.93 (dd, J=6.9, 2.3, 1H), 7.81 (d, J=9.4, 1H), 7.74 (ddd, J=8.4, 4.9, 2.3, 1H), 7.52 (dd, J=9.5, 2.6, 1H), 7.40 (dd, J=9.9, 8.5, 1H), 7.19 (d, J=2.6, 1H), 6.88 (s, 1H), 6.37 (s, 1H), 4.05 (s, 3H), 3.81 - 3.75 (m, 4H), 3.51 - 3.42 (m, 4H). | | | |
| **392** | 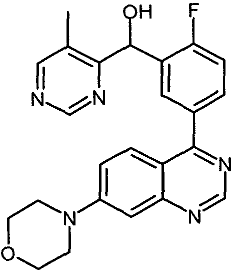 | [2-Fluoro-5-(7-morpholin-4-yl-quinazolin-4-yl)-phenyl]-(5-methyl-pyrimidin-4-yl)-methanol | B | B | B |
| | MS: 432.2 (M+H⁺) | 1H NMR (500 MHz, DMSO-d6) ppm = 9.08 (s, 1H), 8.98 (s, 1H), 8.64 (s, 1H), 8.07 (dd, J=7.1, 2.3, 1H), 7.93 (d, J=9.4, 1H), 7.74 (ddd, J=8.3, 5.0, 2.3, 1H), 7.57 (dd, J=9.5, 2.6, 1H), 7.35 (dd, J=10.1, 8.4, 1H), 7.21 (d, J=2.6, 1H), 6.38 (d, J=6.0, 1H), 6.25 (d, J=6.0, 1H), 3.82 - 3.76 (m, 4H), 3.51 - 3.43 (m, 4H), 2.41 (s, 3H). | | | |
| **393** | 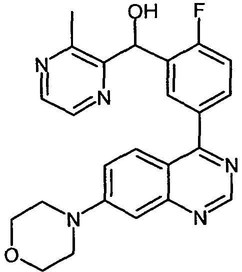 | [2-Fluoro-5-(7-morpholin-4-yl-quinazolin-4-yl)-phenyl]-(3-methyl-pyrazin-2-yl)-methanol | B | C | B |
| | MS: 432.2 (M+H⁺) | 1H NMR (500 MHz, DMSO-d6) ppm = 9.08 (s, 1H), 8.45 (d, J=2.5, 1H), 8.41 (d, J=2.5, 1H), 8.09 (dd, J=7.1, 2.3, 1H), 7.94 (d, J=9.4, 1H), 7.74 (ddd, J=7.9, 5.0, 2.3, 1H), 7.56 (dd, J=9.5, 2.6, 1H), 7.33 (dd, J=10.1, 8.4, 1H), 7.21 (d, J=2.5, 1H), 6.35 (d, J=5.8, 1H), 6.32 (d, J=5.9, 1H), 3.83 - 3.76 (m, 4H), 3.45 (t, J=4.9, 4H), 2.68 (s, 3H). | | | |
| **394** | 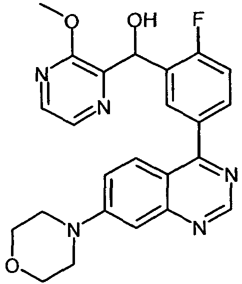 | [2-Fluoro-5-(7-morpholin-4-yl-quinazolin-4-yl)-phenyl]-(3-methoxy-pyrazin-2-yl)-methanol | B | B | D |
| | MS: 448.2 (M+H⁺) | 1H NMR (500 MHz, DMSO-d6) ppm = 9.08 (s, 1H), 8.19 (d, J=2.7, 1H), 8.17 (d, J=2.7, 1H), 8.07 (dd, J=7.1, 2.3, 1H), 7.93 (d, J=9.4, 1H), 7.73 (ddd, J=8.3, 5.0, 2.4, 1H), 7.57 (dd, J=9.5, 2.6, 1H), 7.33 (dd, J=10.1, 8.4, 1H), 7.22 (d, J=2.5, 1H), 6.35 (d, J=6.0, 1H), 6.21 (d, J=6.0, 1H), 3.98 (s,3H), 3.82 - 3.76 (m, 4H), 3.46 (dd, J=5.9, 3.9, 4H). | | | |
| **395** | 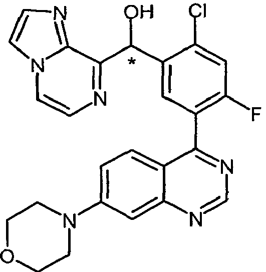 | [2-Chloro-4-fluoro-5-(7-morpholin-4-yl-quinazolin-4-yl)-phenyl]-imidazo[1,2-a]pyrazin-8-yl-methanol (*Ena 2*) | C | C | B |
| | MS: 491.2/493.2 (M+H⁺) (Cl Isotopie, rel. Peakintensitäts- Verhältnis [%] 100:37); Rₜ 14.57min (SFC, Chiralpak AD-H, CO₂ / 40 vol.% Methanol, 0.5 vol.% Diethylamin) | s. Racemat | | | |
| **396** | 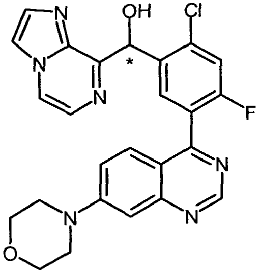 | (S)-[2-Chloro-4-fluoro-5-(7-morpholin-4-yl-quinazolin-4-yl)-phenyl]-imidazo[1,2-a]pyrazin-8-yl-methanol (*Ena 1*) | B | A | A |
| | MS: 491.2/493.1 (M+H⁺) (Cl Isotopie, rel. Peakintensitäts- Verhältnis [%] 100:35); Rₜ 4.27min (SFC, Chiralpak AD-H, CO₂ / 40 vol.% Methanol, 0.5 vol.% Diethylamin) | s. Racemat | | | |
| **397** | 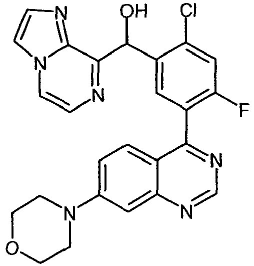 | [2-Chloro-4-fluoro-5-(7-morpholin-4-yl-quinazolin-4-yl)-phenyl]-imidazo[1,2-a]pyrazin-8-yl-methanol | B | A | A |
| | MS: 491.2/493.1 (M+H⁺) (Cl Isotopie, rel. Peakintensitäts-Verhältnis [%] 100:35) | 1H NMR (500 MHz, DMSO-d6) ppm = 9.13 (s, 1H), 8.55 (d, J=4.5, 1H), 8.17 (d, J=1.1, 1H), 8.06 (d, J=7.8, 1H), 7.85 (d, J=1.1, 1H), 7.80 (d, J=4.5, 1H), 7.67 (dd, J=9.4, 3.1, 1H), 7.61 (d, J=9.5, 1H), 7.57 (dd, J=9.5, 2.6, 1H), 7.22 (d, J=2.5, 1H), 6.81 (d, J=5.3, 1H), 6.52 (d, J=6.1, 1H), 3.81 - 3.77 (m, 4H), 3.49 - 3.45 (m, 4H). | | | |
| **398** | 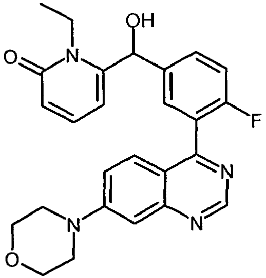 | 1-Ethyl-6-{[4-fluoro-3-(7-morpholin-4-yl-quinazolin-4-yl)-phenyl]-hydroxy-methyl}-1H-pyridin-2-one | B | B | A |
| | MS: 461.2 (M+H⁺) | 1H NMR (500 MHz, DMSO-d6) ppm = 9.10 (s, 1H), 7.63 - 7.56 (m, 2H), 7.54 (dd, J=9.4, 2.5, 1H), 7.52 - 7.43 (m, 2H), 7.41 (dd, J=9.1, 6.9, 1H), 7.20 (d, J=2.4, 1H), 6.58 (d, J=5.0, 1H), 6.35 (dd, J=9.1, 1.4, 1H), 6.31 (dd, J=7.0, 1.4, 1H), 5.91 (d, J=5.0, 1H), 4.05 - 3.88 (m, 2H), 3.81 - 3.74 (m, 4H), 3.48 - 3.39 (m, 4H), 0.95 (t J=6.9, 3H). | | | |
| **399** | 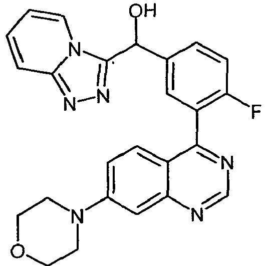 | [4-Fluoro-3-(7-morpholin-4-yl-quinazolin-4-yl)-phenyl]-[1,2,4]triazolo[4,3-a]pyridin-3-yl-methanol | A | B | A |
| | MS: 457.2 (M+H⁺) | 1H NMR (500 MHz, DMSO-d6) ppm = 9.10 (s, 1H), 8.51 - 8.39 (m, 1H), 7.83 - 7.74 (m, 1H), 7.74 - 7.64 (m, 2H), 7.55 (dd, J=9.4, 3.0, 1H), 7.51 (dd, J=9.4, 2.4, 1H), 7.48 - 7.42 (m, 1H), 7.42 - 7.35 (m, 1H), 7.20 (d, J=2.3, 1H), 7.04 - 6.94 (m, 1H), 6.83 (d, J=5.2, 1H), 6.52 (d, J=4.8, 1H), 3.88 - 3.72 (m, 4H), 3.49 - 3.37 (m, 4H). | | | |
| **400** | 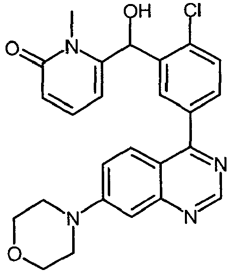 | 6-{[2-Chloro-5-(7-morpholin-4-yl-quinazolin-4-yl)-phenyl]-hydroxy-methyl}-1-methyl-1H-pyridin-2-one | A | A | B |
| | MS: 463.1/465.1 (M+H⁺) (Cl Isotopie, rel. Peakintensitäts-Verhältnis [%] 100:37) | 1H NMR (500 MHz, DMSO-d6) ppm = 9.08 (s, 1H), 7.90 (d, J=2.1, 1H), 7.84 (d, J=9.4, 1H), 7.79 (dd, J=8.2, 2.2, 1H), 7.72 (d, J=8.2, 1H), 7.51 (dd, J=9.5, 2.6, 1H), 7.33 (dd, J=9.1, 7.0, 1H), 7.21 (d, J=2.5, 1H), 6.60 (d, J=6.2, 1H), 6.38 (dd, J=9.1, 1.3, 1H), 6.10 (d, J=6.1, 1H), 5.88 (dd, J=7.0, 1.4, 1H), 3.84 - 3.74 (m, 4H), 3.56 (s, 3H), 3.48 - 3.40 (m, 4H). | | | |
| **401** | 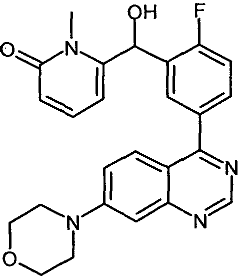 | 6-{[2-Fluoro-5-(7-morpholin-4-yl-quinazolin-4-yl)-phenyl]-hydroxy-methyl}-1-methyl-1H-pyridin-2-one | B | B | C |
| | MS: 447.2 (M+H⁺) | 1H NMR (500 MHz, DMSO-d6) ppm = 9.06 (s, 1H), 7.85 - 7.75 (m, 3H), 7.51 (dd, J=9.5, 2.6, 1H), 7.46 (dd, J=10.1, 8.4, 1H), 7.38 (dd, J=9.1, 7.0, 1H), 7.20 (d, J=2.5, 1H), 6.59 (d, J=5.9, 1H), 6.38 (dd, J=9.1, 1.1, 1H), 6.21 -6.15 (m, 1H), 6.14 (d, J=5.8, 1H), 3.81 - 3.74 (m, 4H), 3.46 (s, 3H), 3.45 - 3.41 (m, 4H). | | | |
| **402** | 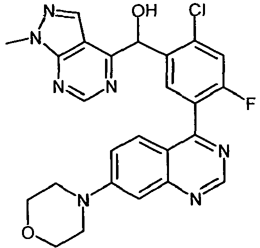 | [2-Chloro-4-fluoro-5-(7-morpholin-4-yl-quinazolin-4-yl)-phenyl]-(1-methyl-1H-pyrazolo[3,4-d]pyrimidin-4-yl)-methanol | B | B | B |
| | MS: 506.2/508.1 (M+H⁺) (Cl Isotopie, rel. Peakintensitäts-Verhältnis [%] 100:38) | 1H NMR (500 MHz, DMSO-d6) ppm = 9.08 (s, 1H), 8.90 (s, 1H), 8.44 (s, 1H), 7.81 (d, J=7.6, 1H), 7.69 (d, J=9.5, 1H), 7.56 - 7.48 (m, 2H), 7.22 - 7.16 (m, 1H), 6.92 (d, J=5.0, 1H), 6.43 (d, J=5.0, 1H), 4.04 (s, 3H), 3.81 - 3.74 (m, 4H), 3.48 - 3.40 (m, 4H). | | | |
| **403** | 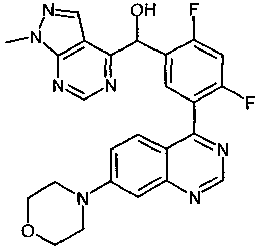 | [2,4-Difluoro-5-(7-morpholin-4-yl-quinazolin-4-yl)-phenyl]-(1-methyl-1H-pyrazolo[3,4-d]pyrimidin-4-yl)-methanol | B | B | B |
| | MS: 490.2 (M+H⁺) | 1H NMR (500 MHz, DMSO-d6) ppm = 9.07 (s, 1H), 8.89 (s, 1H), 8.49 (s, 1H), 7.79 (t, J=8.0, 1H), 7.56 - 7.50 (m, 2H), 7.47 (t, J=10.0, 1H), 7.19 (s, 1H), 6.88 (s, 1H), 6.32 (s, 1H), 4.04 (s, 3H), 3.81 - 3.74 (m, 4H), 3.48 - 3.40 (m, 4H). | | | |
| **404** | 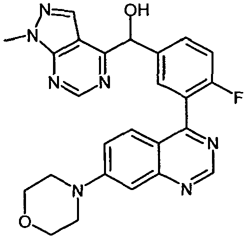 | [4-Fluoro-3-(7-morpholin-4-yl-quinazolin-4-yl)-phenyl]-(1-methyl-1H-pyrazolo[3,4-d]pyrimidin-4-yl)-methanol | B | A | A |
| | MS: 472.2 (M+H⁺) | 1H NMR (500 MHz, DMSO-d6) ppm = 9.09 (s, 1H), 8.91 (s, 1H), 8.53 (s, 1H), 7.79 - 7.72 (m, 2H), 7.54 - 7.44 (m, 2H), 7.38 (dd, J=9.8, 8.4, 1H), 7.19 (d, J=2.1, 1H), 6.79 (d, J=4.0, 1H), 6.10 (d, J=3.3, 1H), 4.03 (s, 3H), 3.80 - 3.75 (m, 4H), 3.46 - 3.41 (m, 4H). | | | |
| **405** | 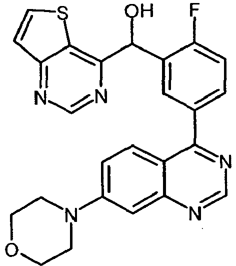 | [2-Fluoro-5-(7-morpholin-4-yl-quinazolin-4-yl)-phenyl]-thieno[3,2-d]pyrimidin-4-yl-methanol | A | A | D |
| | MS: 474.2 (M+H⁺) | 1H NMR (500 MHz, DMSO-d6) ppm = 9.07 (s, 1H), 9.04 (s, 1H), 8.49 (d, J=5.5, 1H), 7.91 (dd, J=6.9, 2.3, 1H), 7.80 (d, J=9.4, 1H), 7.79 - 7.74 (m, 1H), 7.63 (d, J=5.5, 1H), 7.49 (dd, J=9.5, 2.6, 1H), 7.43 (dd, J=9.9, 8.5, 1H), 7.21 - 7.15 (m, 2H), 6.38 (d, J=2.1, 1H), 3.83 - 3.76 (m, 4H), 3.47 - 3.42 (m, 4H). | | | |
| **406** | 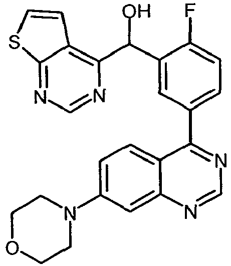 | [2-Fluoro-5-(7-morpholin-4-yl-quinazolin-4-yl)-phenyl]-thieno[2,3-d]pyrimidin-4-yl-methanol | A | A | C |
| | MS: 474.1 (M+H⁺) | 1H NMR (500 MHz, DMSO-d6) ppm = 9.06 (s, 1H), 9.03 (s, 1H), 8.48 (d, J=5.6, 1H), 7.90 (dd, J=6.9, 2.3, 1H), 7.79 (d, J=9.4, 1H), 7.78 - 7.73 (m, 1H), 7.62 (d, J=5.6, 1H), 7.48 (dd, J=9.5, 2.6, 1H), 7.41 (dd, J=9.9, 8.5, 1H), 7.18 (d, J=2.6, 1H), 7.16 (d, J=4.7, 1H), 6.36 (d, J=4.5, 1H), 3.82 - 3.74 (m, 4H), 3.48 - 3.40 (m, 4H). | | | |
| **407** | 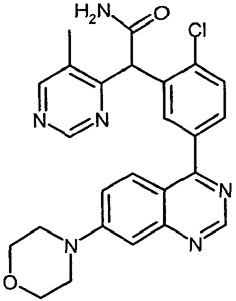 | 2-[2-Chloro-5-(7-morpholin-4-yl-quinazolin-4-yl)-phenyl]-2-(5-methyl-pyrimidin-4-yl)-acetamide | A | B | B |
| | MS: 475.2/477.2 (M+H⁺) (Cl Isotopie, rel. Peakintensitäts-Verhältnis [%] 100:36) | 1H NMR (400 MHz, DMSO-d6) ppm = 9.06 (s, 1H), 8.95 (s, 1H), 8.64 - 8.60 (m, 1H), 7.89 (d, J=9.5, 1H), 7.85 - 7.79 (m, 1H), 7.76 - 7.67 (m, 3H), 7.54 (dd, J=9.4, 2.6, 1H), 7.42 - 7.34 (m, 1H), 7.20 (d, J=2.5, 1H), 5.67 (s, 1H), 3.84 - 3.74 (m, 4H), 3.50 - 3.42 (m, 4H), 2.31 (s, 3H). | | | |
| **408** | 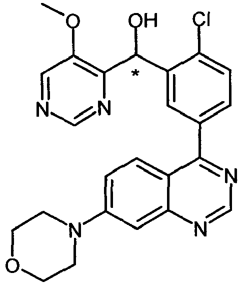 | [2-Chloro-5-(7-morpholin-4-yl-quinazolin-4-yl)-phenyl]-(5-methoxy-pyrimidin-4-yl)-methanol (*Ena 2*) | B | B | C |
| | MS: 464.2/466.2 (M+H⁺) (Cl Isotopie, rel. Peakintensitäts-Verhältnis [%] 100:36); Rₜ 5.41min (SFC, Chiralpak AD-H, CO₂ / 40 vol.% 2-Propanol, 0.5 vol.% Diethylamin) | s. Racemat | | | |
| **409** | 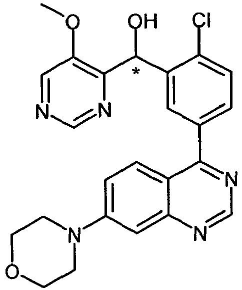 | [2-Chloro-5-(7-morpholin-4-yl-quinazolin-4-yl)-phenyl]-(5-methoxy-pyrimidin-4-yl)-methanol (*Ena 1*) | B | B | A |
| | MS: 464.1/466.1 (M+H⁺) (Cl Isotopie, rel. Peakintensitäts-Verhältnis [%] 100:35); Rₜ 3.05min (SFC, Chiralpak AD-H, CO₂ / 40 vol.% 2-Propanol, 0.5 vol.% Diethylamin) | s. Racemat | | | |
| **410** | 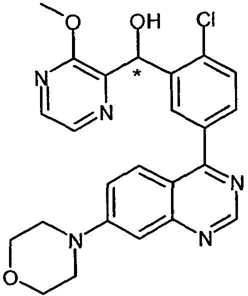 | [2-Chloro-5-(7-morpholin-4-yl-quinazolin-4-yl)-phenyl]-(3-methoxy-pyrazin-2-yl)-methanol (*Ena 2*) | A | A | D |
| | MS: 464.2/466.2 (M+H⁺) (Cl Isotopie, rel. Peakintensitäts-Verhältnis [%] 100:35); Rₜ 5.47min (SFC, Chiralpak AD-H, CO₂ / 40 vol.% 2-Propanol, 0.5 vol.% Diethylamin) | s. Racemat | | | |
| **411** | 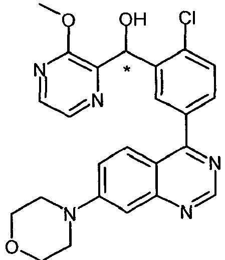 | (S)-[2-Chloro-5-(7-morpholin-4-yl-quinazolin-4-yl)-phenyl]-(3-methoxy-pyrazin-2-yl)-methanol (*Ena 1*) | B | B | C |
| | MS: 464.1/466.1 (M+H⁺) (Cl Isotopie, rel. Peakintensitäts-Verhältnis [%] 100:35); Rₜ 2.84min (SFC, Chiralpak AD-H, CO₂ / 40 vol.% 2-Propanol, 0.5 vol.% Diethylamin) | s. Racemat | | | |
| **412** | 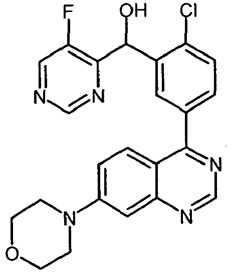 | [2-Chloro-5-(7-morpholin-4-yl-quinazolin-4-yl)-phenyl]-(5-fluoro-pyrimidin-4-yl)-methanol | C | C | B |
| | MS: 452.1/454.1 (M+H⁺) (Cl Isotopie, rel. Peakintensitäts-Verhältnis [%] 100:35) | 1H NMR (500 MHz, DMSO-d6) ppm = 9.10 (s, 1H), 9.03 (d, J=2.9, 1H), 8.93 (d, J=2.1, 1H), 8.24 (d, J=2.2, 1H), 7.96 (d, J=9.4, 1H), 7.74 (dd, J=8.2, 2.3, 1H), 7.62 (d, J=8.2, 1H), 7.59 (dd, J=9.5, 2.6, 1H), 7.23 (d, J=2.5, 1H), 6.77 (d, J=5.5, 1H), 6.38 (d, J=5.5, 1H), 3.82 - 3.76 (m, 4H), 3.49 - 3.44 (m, 4H). | | | |
| **413** | 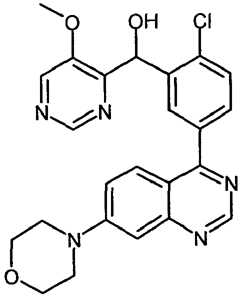 | [2-Chloro-5-(7-morpholin-4-yl-quinazolin-4-yl)-phenyl]-(5-methoxy-pyrimidin-4-yl)-methanol | B | B | B |
| | MS: 464.2/466.2 (M+H⁺) (Cl Isotopie, rel. Peakintensitäts-Verhältnis [%] 100:36) | 1H NMR (500 MHz, DMSO-d6) ppm = 9.09 (s, 1H), 8.74 (s, 1H), 8.64 (s, 1H), 8.15 (d, J=2.2, 1H), 7.95 (d, J=9.4, 1H), 7.69 (dd, J=8.2, 2.3, 1H), 7.57 (dd, J=8.9, 2.8, 2H), 7.22 (d, J=2.5, 1H), 6.43 (d, J=6.0, 1H), 6.31 (d, J=6.0, 1H), 4.01 (s, 3H), 3.83 - 3.74 (m, 4H), 3.50 - 3.41 (m, 4H). | | | |
| **414** | 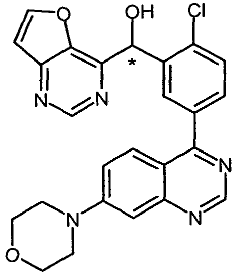 | [2-Chloro-5-(7-morpholin-4-yl-quinazolin-4-yl)-phenyl]-furo[3,2-d]pyrimidin-4-yl-methanol (*Ena 2*) | C | A | C |
| | MS: 474.1/476.1 (M+H⁺) (Cl Isotopie, rel. Peakintensitäts-Verhältnis [%] 100:34); Rₜ 6.16min (SFC, Chiralpak AD-H, CO₂ / 40 vol.% 2-Propanol, 0.5 vol.% Diethylamin) | s. Racemat | | | |
| **415** | 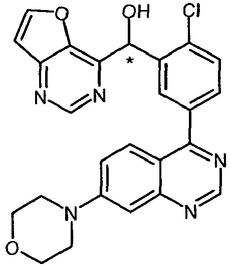 | [2-Chloro-5-(7-morpholin-4-yl-quinazolin-4-yl)-phenyl]-furo[3,2-d]pyrimidin-4-yl-methanol (*Ena 1*) | B | A | C |
| | MS: 474.1/476.1 (M+H⁺) (Cl Isotopie, rel. Peakintensitäts-Verhältnis [%] 100:35); Rₜ 3.19min (SFC, Chiralpak AD-H, CO₂ / 40 vol.% 2-Propanol, 0.5 vol.% Diethylamin) | s. Racemat | | | |
| **416** | 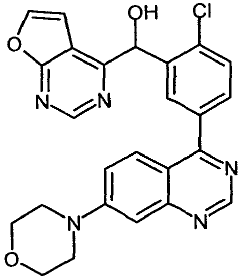 | [2-Chloro-5-(7-morpholin-4-yl-quinazolin-4-yl)-phenyl]-furo[2,3-d]pyrimidin-4-yl-methanol | B | B | C |
| | MS: 474.1/476.1 (M+H⁺) (Cl Isotopie, rel. Peakintensitäts-Verhältnis [%] 100:34) | 1H NMR (500 MHz, DMSO-d6) ppm = 9.07 (s, 1H), 8.88 (s, 1H), 8.20 (d, J=2.5, 1H), 8.03 (d, J=2.2, 1H), 7.84 (d, J=9.4, 1H), 7.72 (dd, J=8.2, 2.2, 1H), 7.65 (d, J=8.3, 1H), 7.52 (dd, J=9.4, 2.6, 1H), 7.26 (d, J=2.5, 1H), 7.20 (d, J=2.5, 1H), 6.88 - 6.72 (m, 1H), 6.46 (s, 1H), 3.82 - 3.76 (m, 4H), 3.48 - 3.42 (m, 4H). | | | |
| **417** | 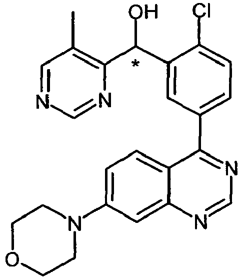 | [2-Chloro-5-(7-morpholin-4-yl-quinazolin-4-yl)-phenyl]-(5-methyl-pyrimidin-4-yl)-methanol (*Ena 2*) | C | B | B |
| | MS: 448.1/450.1 (M+H⁺) (Cl Isotopie, rel. Peakintensitäts-Verhältnis [%] 100:36); Rₜ 6.39min (SFC, Chiralpak AD-H, CO₂ / 40 vol.% Methanol, 0.5 vol.% Diethylamin) | s. Racemat | | | |
| **418** | 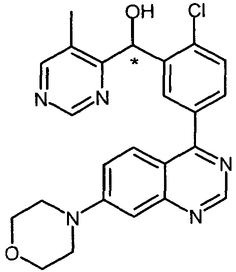 | [2-Chloro-5-(7-morpholin-4-yl-quinazolin-4-yl)-phenyl]-(5-methyl-pyrimidin-4-yl)-methanol (*Ena 1*) | C | B | A |
| | MS: 448.1/450.1 (M+H⁺) (Cl Isotopie, rel. Peakintensitäts-Verhältnis [%] 100:36); Rₜ 4.95min (SFC, Chiralpak AD-H, CO₂ / 40 vol.% Methanol, 0.5 vol.% Diethylamin) | s. Racemat | | | |
| **419** | 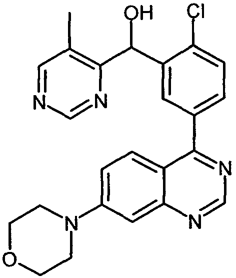 | [2-Chloro-5-(7-morpholin-4-yl-quinazolin-4-yl)-phenyl]-(5-methyl-pyrimidin-4-yl)-methanol | C | B | B |
| | MS: 448.1/450.1 (M+H⁺) (Cl Isotopie, rel. Peakintensitäts-Verhältnis [%] 100:37 | 1H NMR (400 MHz, DMSO-d6) ppm = 9.10 (s, 1H), 8.94 (s, 1H), 8.67 (s, 1H), 8.16 (d, J=2.2, 1H), 7.98 (d, J=9.4, 1H), 7.72 (dd, J=8.2, 2.2, 1H), 7.64 - 7.53 (m, 2H), 7.22 (d, J=2.5, 1H), 6.47 (d, J=6.0, 1H), 6.25 (d, J=6.0, 1H), 3.84 - 3.73 (m, 4H), 3.51 - 3.42 (m, 4H), 2.49 (s, 3H). | | | |
| **420** | 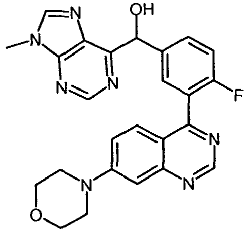 | [4-Fluoro-3-(7-morpholin-4-yl-quinazolin-4-yl)-phenyl]-(9-methyl-9H-purin-6-yl)-methanol | C | C | A |
| | MS: 472.3 (M+H⁺) | 1H NMR (500 MHz, DMSO-d6) ppm = 9.08 (s, 1H), 8.91 (s, 1H), 8.55 (s, 1H), 7.82 - 7.73 (m, 2H), 7.56 - 7.47 (m, 2H), 7.40 - 7.31 (m, 1H), 7.19 (d, J=1.3, 1H), 6.43 (d, J=3.0, 1H), 6.30 (d, J=5.2, 1H), 3.83 (s, 3H), 3.81 - 3.75 (m, 4H), 3.47 - 3.40 (m, 4H). | | | |
| **421** | 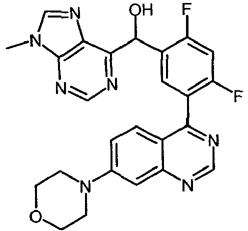 | [2,4-Difluoro-5-(7-morpholin-4-yl-quinazolin-4-yl)-phenyl]-(9-methyl-9H-purin-6-yl)-methanol | C | B | A |
| | MS: 490.3 (M+H⁺) | 1H NMR (500 MHz, DMSO-d6) ppm = 9.11 (s, 1H), 8.90 (s, 1H), 8.56 (s, 1H), 8.07 (t, J=8.2, 1H), 7.66 (dd, J=9.4, 2.9, 1H), 7.56 (dd, J=9.4, 2.5, 1H), 7.38 (t, J=10.1, 1H), 7.21 (d, J=2.4, 1H), 6.61 (d, J=4.8, 1H), 6.48 (d, J=5.6, 1H), 3.85 (s, 3H), 3.82 - 3.75 (m, 4H), 3.52 - 3.43 (m, 4H). | | | |
| **422** | 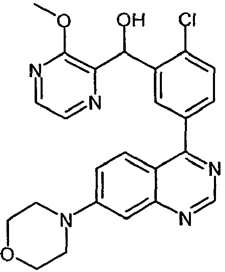 | [2 -Chloro-5-(7-morpholin-4-yl-quinazolin-4-yl)-phenyl]-(3-methoxy-pyrazin-2-yl)-methanol | B | B | C |
| | MS: 464.1/466.1 (M+H⁺) (Cl Isotopie, rel. Peakintensitäts- Verhältnis [%] 100:37) | 1H NMR (500 MHz, DMSO-d6) ppm = 9.09 (s, 1H), 8.19 - 8.14 (m, 2H), 8.11 (d, J=2.7, 1H), 7.95 (d, J=9.4, 1H), 7.69 (dd, J=8.2, 2.3, 1H), 7.60 - 7.53 (m, 2H), 7.22 (d, J=2.6, 1H), 6.38 (d, J=5.8, 1H), 6.27 (d, J=5.8, 1H), 4.00 (s, 3H), 3.82 - 3.75 (m, 4H), 3.49 - 3.42 (m, 4H). | | | |
| **423** | 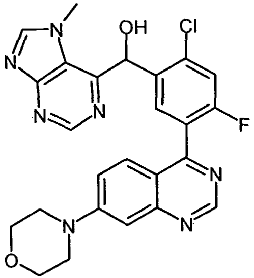 | [2-Chloro-4-fluoro-5-(7-morpholin-4-yl-quinazolin-4-yl)-phenyl]-(7-methyl-7H-purin-6-yl)-methanol | C | C | A |
| | MS: 506.2/4508.3 (M+H⁺) (Cl Isotopie, rel. Peakintensitäts-Verhältnis [%] 100:40) | 1H NMR (500 MHz, DMSO-d6) ppm = 9.14 (s, 1H), 8.83 (s, 1H), 8.69 (s, 1H), 8.01 (d, J=7.8, 1H), 7.71 - 7.63 (m, 2H), 7.59 (dd, J=9.4, 2.5, 1H), 7.22 (d, J=2.4, 1H), 6.87 (d, J=6.3, 1H), 6.66 (d, J=6.3, 1H), 4.25 (s, 3H), 3.84 - 3.73 (m, 4H), 3.52 - 3.42 (m, 4H). | | | |
| **424** | 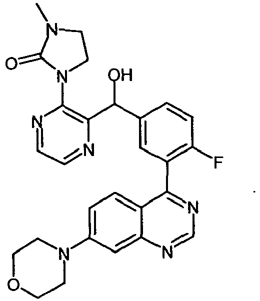 | 1-(3-{[4-Fluoro-3-(7-morpholin-4-yl-quinazolin-4-yl)-phenyl]-hydroxy-methyl}-pyrazin-2-yl)-3-methyl-imidazolidin-2-one | D | D | A |
| | MS: 516.4 (M+H⁺) | 1H NMR (500 MHz, DMSO-d6) ppm = 9.09 (s, 1H), 8.50 (d, J=2.4, 1H), 8.43 (d, J=2.4, 1H), 7.69 - 7.59 (m, 2H), 7.59 - 7.49 (m, 2H), 7.35 (t, J=9.2, 1H), 7.19 (d, J=1.9, 1H), 6.23 (d, J=4.4, 1H), 5.95 (d, J=5.3, 1H), 3.85 (t, J=7.8, 2H), 3.82 - 3.73 (m, 4H), 3.52 - 3.40 (m, 6H), 2.78 (s, 3H). | | | |
| **425** | 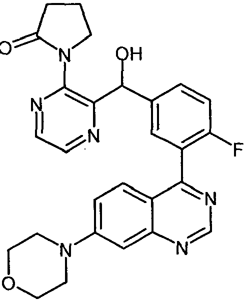 | 1-(3-{[4-Fluoro-3-(7-morpholin-4-yl-quinazolin-4-yl)-phenyl]-hydroxymethyl}-pyrazin-2-yl)-pyrrolidin-2-one | D | D | A |
| | MS: 501.4 (M+H⁺) | 1H NMR (500 MHz, DMSO-d6) ppm = 9.09 (s, 1H), 8.61 (d, J=2.4, 1H), 8.52 (d, J=2.4, 1H), 7.65 - 7.56 (m, 2H), 7.56 - 7.49 (m, 2H), 7.36 (t, J=9.5, 1H), 7.19 (s, 1H), 6.02 (s, 2H), 3.89 - 3.82 (m, 1H), 3.81 - 3.74 (m, 4H), 3.74 - 3.64 (m, 1H), 3.50 - 3.39 (m, 4H), 2.57 - 2.41 (m, 2H), 2.17 - 2.08 (m, 1H), 2.07 - 1.98 (m, 1H). | | | |
| **426** | 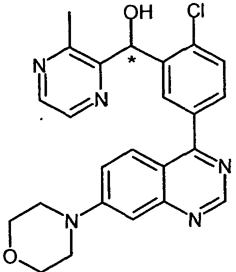 | [2-Chloro-5-(7-morpholin-4-yl-quinazolin-4-yl)-phenyl]-(3-methyl-pyrazin-2-yl)-methanol (*Ena 2*) | C | B | A |
| | MS: 448.1/450.1 (M+H⁺) (Cl Isotopie, rel. Peakintensitäts-Verhältnis [%] 100:36); Rₜ 5.95min (SFC, Chiralpak AD-H, CO₂ / 40 vol.% Methanol, 0.5 vol.% Diethylamin) | s. Racemat | | | |
| **427** | 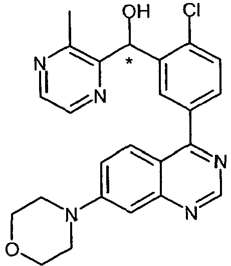 | [2-Chloro-5-(7-morpholin-4-yl-quinazolin-4-yl)-phenyl]-(3-methyl-pyrazin-2-yl)-methanol (*Ena 1*) | C | B | B |
| | MS: 448.1/450.2 (M+H⁺) (Cl Isotopie, rel. Peakintensitäts-Verhältnis [%] 100:31); Rₜ 3.91min (SFC, Chiralpak AD-H, CO₂ / 40 vol.% Methanol, 0.5 vol.% Diethylamin) | s. Racemat | | | |
| **428** | 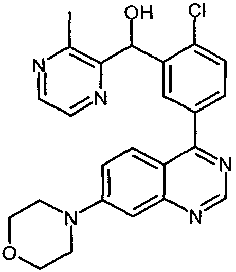 | [2-Chloro-5-(7-morpholin-4-yl-quinazolin-4-yl)-phenyl]-(3-methyl-pyrazin-2-yl)-methanol | C | A | C |
| | MS: 448.2/450.2 (M+H⁺) (Cl Isotopie, rel. Peakintensitäts-Verhältnis [%] 100:36) | 1H NMR (500 MHz, DMSO-d6) ppm = 9.10 (s, 1H), 8.43 (d, J=2.5, 1H), 8.36 (d, J=2.5, 1H), 8.18 (d, J=2.2, 1H), 7.97 (d, J=9.4, 1H), 7.71 (dd, J=8.2, 2.3, 1H), 7.61 - 7.54 (m, 2H), 7.22 (d, J=2.6, 1H), 6.41 (d, J=5.8, 1H), 6.30 (d, J=5.8, 1H), 3.81 - 3.75 (m, 4H), 3.49 - 3.42 (m, 4H), 2.76 (s, 3H). | | | |
| **429** | 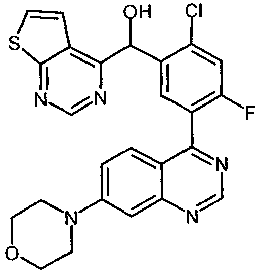 | [2-Chloro-4-fluoro-5-(7-morpholin-4-yl-quinazolin-4-yl)-phenyl]-thieno[2,3-d]pyrimidin-4-yl-methanol | A | A | D |
| | MS: 508.1/510.1 (M+H⁺) (Cl Isotopie, rel. Peakintensitäts-Verhältnis [%] 100:40) | 1H NMR (500 MHz, DMSO-d6) ppm = 9.07 (s, 1H), 9.05 (s, 1H), 8.46 (d, J=5.6, 1H), 7.75 (d, J=7.6, 1H), 7.72 (d, J=9.6, 1H), 7.61 (d, J=5.6, 1H), 7.55 - 7.49 (m, 2H), 7.20 (d, J=5.0, 1H), 7.19 - 7.17 (m, 1H), 6.42 (d, J=5.1, 1H), 3.80 - 3.75 (m, 4H), 3.46 - 3.42 (m, 4H). | | | |
| **430** | 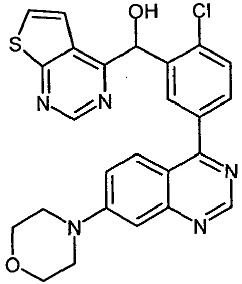 | [2-Chloro-5-(7-morpholin-4-yl-quinazolin-4-yl)-phenyl]-thieno[2,3-d]pyrimidin-4-yl-methanol | B | A | A |
| | MS: 490.2/492.1 (M+H⁺) (Cl Isotopie, rel. Peakintensitäts-Verhältnis [%] 100:40) | 1H NMR (500 MHz, DMSO-d6) ppm = 9.06 (s, 1H), 9.04 (s, 1H), 8.47 (d, J=5.6, 1H), 7.89 (d, J=2.1, 1H), 7.76 (d, J=9.4, 1H), 7.73 (dd, J=8.2, 2.2, 1H), 7.69 (d, J=8.3, 1H), 7.62 (d, J=5.5, 1H), 7.47 (dd, J=9.5, 2.6, 1H), 7.20 - 7.16 (m, 2H), 6.47 (d, J=4.9, 1H), 3.80 - 3.75 (m, 4H), 3.46 - 3.41 (m, 4H). | | | |
| **431** | 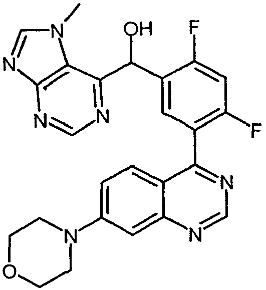 | [2,4-Difluoro-5-(7-morpholin-4-yl-quinazolin-4-yl)-phenyl]-(7-methyl-7H-purin-6-yl)-methanol | C | B | A |
| | MS: 490.2 (M+H⁺) | 1H NMR (500 MHz, DMSO-d6) ppm = 9.12 (s, 1H), 8.85 (s, 1H), 8.67 (s, 1H), 7.91 (t, J=8.1, 1H), 7.64 (dd, J=9.4, 3.2, 1H), 7.58 (dd, J=9.4, 2.5, 1H), 7.46 (t, J=10.1, 1H), 7.21 (d, J=2.5, 1H), 6.82 (d, J=6.5, 1H), 6.65 (d, J=6.5, 1H), 4.19 (s, 3H), 3.81 - 3.76 (m, 4H), 3.49 - 3.44 (m, 4H). | | | |
| **432** | 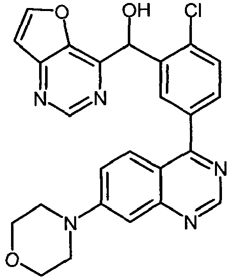 | [2-Chloro-5-(7-morpholin-4-yl-quinazolin-4-yl)-phenyl]-furo[3,2-d]pyrimidin-4-yl-methanol | C | B | C |
| | MS: 474.1/476.1 (M+H⁺) (Cl Isotopie, rel. Peakintensitäts-Verhältnis [%] 100:34) | 1H NMR (500 MHz, DMSO-d6) ppm = 9.10 (s, 1H), 9.02 (s, 1H), 8.59 (d, J=2.3, 1H), 8.30 (d, J=2.2, 1H), 7.96 (d, J=9.4, 1H), 7.74 (dd, J=8.2, 2.3, 1H), 7.62 (d, J=8.2, 1H), 7.59 (dd, J=9.5, 2.6, 1H), 7.28 (d, J=2.3, 1H), 7.23 (d, J=2.6, 1H), 6.79 (d, J=5.2, 1H), 6.54 (d, J=5.2, 1H), 3.83 - 3.75 (m, 4H), 3.50 - 3.42 (m, 4H). | | | |
| **433** | 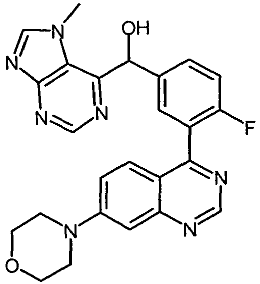 | [4-Fluoro-3-(7-morpholin-4-yl-quinazolin-4-yl)-phenyl]-(7-methyl-7H-purin-6-yl)-methanol | C | B | A |
| | MS: 472.2 (M+H⁺) | 1H NMR (500 MHz, DMSO-d6) ppm = 9.08 (s, 1H), 8.90 (s, 1H), 8.60 (s, 1H), 7.68 - 7.60 (m, 2H), 7.56 (dd, J=9.4, 2.8, 1H), 7.52 (dd, J=9.4, 2.4, 1H), 7.44 - 7.38 (m, 1H), 7.19 (d, J=2.3, 1H), 6.84 (d, J=5.5, 1H), 6.37 (d, J=5.4, 1H), 4.04 (s, 3H), 3.81 - 3.75 (m, 4H), 3.47 - 3.41 (m, 4H). | | | |
| **434** | 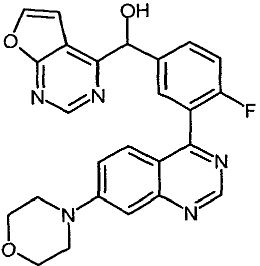 | [4-Fluoro-3-(7-morpholin-4-yl-quinazolin-4-yl)-phenyl]-furo[2,3-d]pyrimidin-4-yl-methanol | A | A | A |
| | MS: 458.2 (M+H⁺) | 1H NMR (500 MHz, DMSO-d6) ppm = 9.10(s, 1H), 8.89 (s, 1H), 8.18 (d, J=2.5, 1H), 7.77 - 7.72 (m, 2H), 7.54 - 7.47 (m, 2H), 7.40 (t, J=9.5, 1H), 7.36 (d, J=2.5, 1H), 7.20 (d, J=2.2, 1H), 6.71 (d, J=4.2, 1H), 6.13 (d, J=4.2, 1H), 3.81-3.76 (m, 4H), 3.47 - 3.42 (m, 4H). | | | |
| **435** | 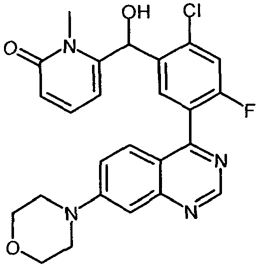 | 6-{[2-Chloro-4-fluoro-5-(7-morpholin-4-yl-quinazolin-4-yl)-phenyl]-hydroxy-methyl}-1-methyl-1H-pyridin-2-one | B | A | A |
| | MS: 481.2/483.2 (M+H⁺) (Cl Isotopie, rel. Peakintensitäts-Verhältnis [%] 100:38) | 1H NMR (500 MHz, DMSO-d6) ppm = 9.11 (s, 1H), 7.77 (d, J=9.5, 1H), 7.74 (d, J=7.6, 1H), 7.60 (dd, J=9.4, 3.3, 1H), 7.53 (dd, J=9.4, 2.5, 1H), 7.33 (dd, J=9.1, 7.0, 1H), 7.21 (d, J=2.5, 1H), 6.62 (d, J=6.3, 1H), 6.37 (dd, J=9.1, 1.3, 1H), 6.06 (d, J=6.2, 1H), 5.89 - 5.85 (m, 1H), 3.81 - 3.75 (m, 4H), 3.55 (s, 3H), 3.49 - 3.43 (m, 4H). | | | |
| **436** | 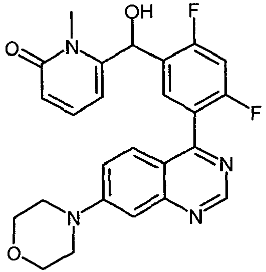 | 6-{[2,4-Difluoro-5-(7-morpholin-4-yl-quinazolin-4-yl)-phenyl]-hydroxymethyl}-1-methyl-1H-pyridin-2-one | B | B | A |
| | MS: 465.3 (M+H⁺) | 1H NMR (500 MHz, DMSO-d6) ppm = 9.09 (s, 1H), 7.63 (t, J=8.1, 1H), 7.59 - 7.51 (m, 3H), 7.38 (dd, J=9.1, 7.0, 1H), 7.20 (d, J=2.3, 1H), 6.60 (d, J=5.9, 1H), 6.39 - 6.35 (m, 1H), 6.20 - 6.16 (m, 1H), 6.09 (d, J=5.9, 1H), 3.81 - 3.75 (m, 4H), 3.49 - 3.40 (m, 7H). | | | |
| **437** | 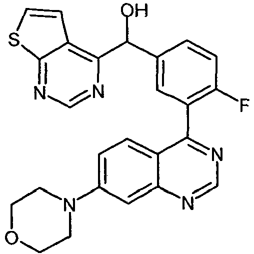 | [4-Fluoro-3-(7-morpholin-4-yl-quinazolin-4-yl)-phenyl]-thieno[2,3-d]pyrimidin-4-yl-methanol | A | A | C |
| | MS: 474.1 (M+H⁺) | 1H NMR (400 MHz, DMSO-d6) ppm = 9.09 (s, 1H), 9.08 (s, 1H), 8.47 (d, J=5.6, 1H), 7.78 - 7.73 (m, 2H), 7.61 (d, J=5.6, 1H), 7.54 - 7.46 (m, 2H), 7.43 - 7.36 (m, 1H), 7.21 - 7.18 (m, 1H), 7.13 (d, J=3.9, 1H), 6.12 (d, J=3.9, 1H), 3.82 - 3.73 (m, 4H), 3.48 - 3.40 (m, 4H). | | | |
| **438** | 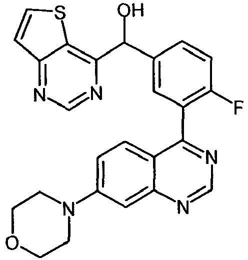 | [4-Fluoro-3-(7-morpholin-4-yl-quinazolin-4-yl)-phenyl]-thieno[3,2-d]pyrimidin-4-yl-methanol | A | A | C |
| | MS: 474.2 (M+H⁺) | 1H NMR (500 MHz, DMSO-d6) ppm = 9.09 (s, 1H), 9.07 (s, 1H), 8.46 (s, 1H), 8.45 (s, 1H), 7.78 - 7.73 (m, 2H), 7.60 (d, J=5.5, 1H), 7.52 - 7.46 (m, 2H), 7.41 - 7.36 (m, 1H), 7.20 - 7.18 (m, 1H), 7.11 (d, J=3.9, 1H), 6.12 (d, J=3.9, 1H), 3.80 - 3.75 (m, 4H), 3.46 - 3.41 (m, 4H). | | | |
| **439** | 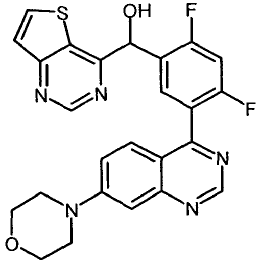 | [2,4-Difluoro-5-(7-morpholin-4-yl-quinazolin-4-yl)-phenyl]-thieno[3,2-d]pyrimidin-4-yl-methanol | A | A | C |
| | MS: 492.2 (M+H⁺) | 1H NMR (500 MHz, DMSO-d6) ppm = 9.07 (s, 1H), 9.05 (s, 1H), 8.47 (d, J=5.6, 1H), 7.76 (t, J=8.0, 1H), 7.61 (d, J=5.6, 1H), 7.54 - 7.46 (m, 3H), 7.21 - 7.16 (m, 2H), 6.32 (d, J=4.8, 1H), 3.80 - 3.74 (m, 4H), 3.46 - 3.41 (m, 4H). | | | |
| **440** | 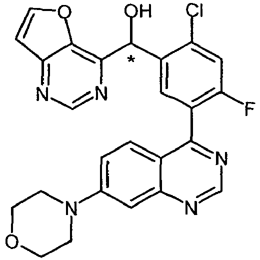 | [2-Chloro-4-fluoro-5-(7-morpholin-4-yl-quinazolin-4-yl)-phenyl]-furo[3,2-d]pyrimidin-4-yl-methanol (*Ena 2*) | A | A | B |
| | MS: 492.1/494.1 (M+H⁺) (Cl Isotopie, rel. Peakintensitäts-Verhältnis [%] 100:38); Rₜ 8.33min (SFC, Chiralpak AD-H, CO₂ /40 vol.% Methanol, 0.5 vol.% Diethylamin) | s. Racemat | | | |
| **441** | 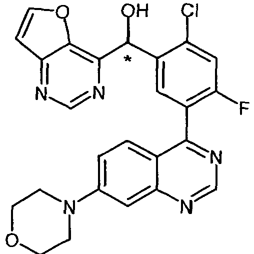 | [2-Ch loro-4-fluoro-5-(7-morpholin-4-yl-quinazolin-4-yl)-phenyl]-furo[3,2-d]pyrimidin-4-yl-methanol (*Ena 1*) | A | A | C |
| | MS: 492.1/494.1 (M+H⁺) (Cl Isotopie, rel. Peakintensitäts-Verhältnis [%] 100:38); Rₜ 3.83min (SFC, Chiralpak AD-H, CO₂ / 40 vol.% Methanol, 0.5 vol.% Diethvlamin) | s. Racemat | | | |
| **442** | 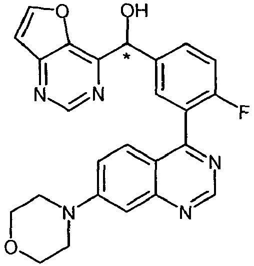 | [4-Fluoro-3-(7-morpholin-4-yl-quinazolin-4-yl)-phenyl]-furo[3,2-d]pyrimidin-4-yl-methanol (*Ena 2*) | A | A | A |
| | MS: 458.2 (M+H⁺); Rₜ 7.52min (SFC, Chiralpak AD-H, CO₂/40 vol.% 2-Propanol, 0.5 vol.% Diethylamin) | s. Racemat | | | |
| **443** | 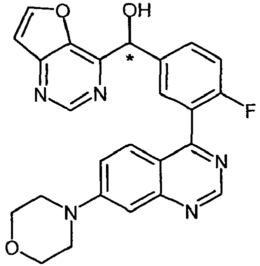 | [4-Fluoro-3-(7-morpholin-4-yl-quinazolin-4-yl)-phenyl]-furo[3,2-d]pyrimidin-4-yl-methanol (*Ena 1*) | B | B | A* |
| | MS: 458.1 (M+H⁺); Rₜ 3.43min (SFC, Chiralpak AD-H, CO₂/40 vol.% 2-Propanol, 0.5 vol.% Diethylamin) | s. Racemat | | | |
| **444** | 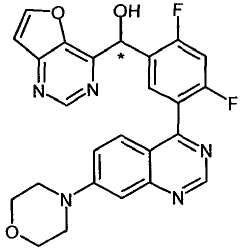 | [2,4-Difluoro-5-(7-morpholin-4-yl-quinazolin-4-yl)-phenyl]-furo[3,2-d]pyrimidin-4-yl-methanol (*Ena 2*) | A | B | A |
| | MS: 476.2 (M+H⁺); Rₜ 9.29min (SFC, Chiralpak AD-H, CO₂/30 vol.% Methanol, 0.5 vol.% Diethylamin) | s. Racemat | | | |
| **445** | 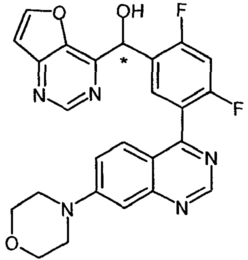 | [2,4-Difluoro-5-(7-morpholin-4-yl-quinazolin-4-yl)-phenyl]-furo[3,2-d]pyrimidin-4-yl-methanol (*Ena 1*) | B | A | A |
| | MS: 476.1 (M+H⁺); Rₜ 5.74min (SFC, Chiralpak AD-H, CO₂/30 vol.% Methanol, 0.5 vol.% Diethylamin) | s. Racemat | | | |
| **446** | 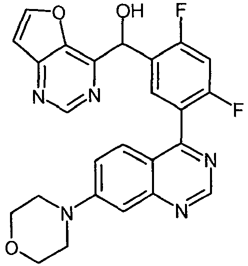 | [2,4-Difluoro-5-(7-morpholin-4-yl-quinazolin-4-yl)-phenyl]-furo[3,2-d]pyrimidin-4-yl-methanol | A | B | A |
| | MS: 476.2 (M+H⁺) | 1H NMR (500 MHz, DMSO-d6) ppm = 9.12 (s, 1H), 9.00 (s, 1H), 8.57 (d, J=2.3, 1H), 8.00 (t, J=8.1, 1H), 7.59 (dd, J=9.4, 2.8, 1H), 7.56 (dd, J=9.4, 2.4, 1H), 7.45 (t, J=10.1, 1H), 7.27 (d, J=2.3, 1H), 7.21 (d, J=2.3, 1H), 6.75 (d, J=5.4, 1H), 6.43 (d, J=5.3, 1H), 3.81 - 3.76 (m, 4H), 3.49 - 3.42 (m, 4H). | | | |
| **447** | 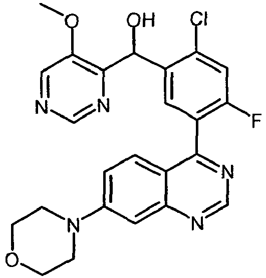 | [2-Chloro-4-fluoro-5-(7-morpholin-4-yl-quinazolin-4-yl)-phenyl]-(5-methoxy-pyrimidin-4-yl)-methanol | A | B | C |
| | MS: 482.2/484.1 (M+H⁺) (Cl Isotopie, rel. Peakintensitäts-Verhältnis [%] 100:40) | 1H NMR (400 MHz, DMSO-d6) ppm = 9.14 (s, 1H), 8.74 (s, 1H), 8.65 (s, 1H), 7.97 (d, J=7.9, 1H), 7.69 - 7.52 (m, 3H),7.23 (d, J=2.4, 1H), 6.43 - 6.32 (m, 2H), 4.01 (s, 3H), 3.80 (dd, J=5.9, 3.9, 4H), 3.47 (t, J=4.9, 4H). | | | |
| **448** | 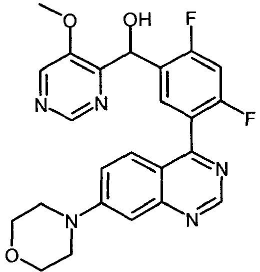 | [2,4-Difluoro-5-(7-morpholin-4-yl-quinazolin-4-yl)-phenyl]-(5-methoxy-pyrimidin-4-yl)-methanol | A | B | C |
| | MS: 466.2 (M+H⁺) | 1H NMR (400 MHz, DMSO-d6) ppm = 9.11 (s, 1H), 8.77 (s, 1H), 8.62 (s, 1H), 7.86 (t, J=8.2, 1H), 7.66 - 7.50 (m, 2H), 7.41 (t, J=10.1, 1H), 7.21 (d, J=2.1, 1H), 6.32 (d, J=6.2, 1H), 6.25 (d, J=6.3, 1H), 3.97 (s, 3H), 3.78 (t, J=4.9, 4H), 3.45 (t, J=4.9, 4H). | | | |
| **449** | 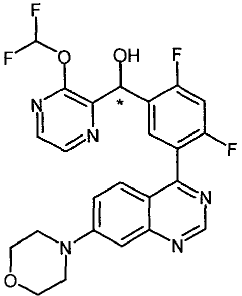 | (3-Difluoromethoxy-pyrazin-2-yl)-[2,4-difluoro-5-(7-morpholin-4-yl-quinazolin-4-yl)-phenyl]-methanol (*Ena 2*) | A | B | A |
| | MS: 502.2 (M+H⁺); Rₜ 3.40min (SFC, Chiralpak AD-H, CO₂/40 vol.% Methanol, 0.5 vol.% Diethylamin) | s. Racemat | | | |
| **450** | 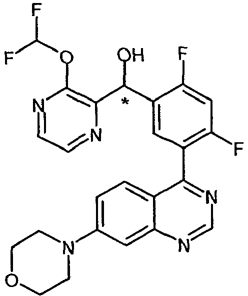 | (3-Difluoromethoxy-pyrazin-2-yl)-[2,4-difluoro-5-(7-morpholin-4-yl-quinazolin-4-yl)-phenyl]-methanol (*Ena 1*) | C | C | B |
| | MS: 502.2 (M+H⁺); Rₜ 2.00min (SFC, Chiralpak AD-H, CO₂/40 vol.% Methanol, 0.5 vol.% Diethylamin) | s. Racemat | | | |
| **451** | 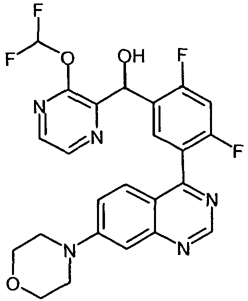 | (3-Difluoromethoxy-pyrazin-2-yl)-[2,4-difluoro-5-(7-morpholin-4-yl-quinazolin-4-yl)-phenyl]-methanol | B | B | B |
| | MS: 502.2 (M+H⁺) | 1H NMR (500 MHz, DMSO-d6) ppm = 9.12 (s, 1H), 8.49 (d, J=2.6, 1H), 8.32 (d, J=2.6, 1H), 7.91 (t, J=8.2, 1H), 7.77 (t, J=71.7, 1H), 7.61 - 7.51 (m, 2H), 7.43 (t, J=10.1, 1H), 7.21 (d, J=2.4, 1H), 6.48 (d, J=5.9, 1H), 6.29 (d, J=4.7, 1H), 3.81 - 3.75 (m, 4H), 3.48 - 3.42 (m, 4H). | | | |
| **452** | 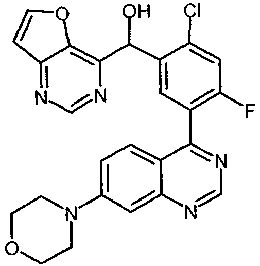 | [2-Chloro-4-fluoro-5-(7-morpholin-4-yl-quinazolin-4-yl)-phenyl]-furo[3,2-d]pyrimidin-4-yl-methanol | A | A | C |
| | MS: 492.1/494.1 (M+H⁺) (Cl Isotopie, rel. Peakintensitäts-Verhältnis [%] 100:41) | 1H NMR (500 MHz, DMSO-d6) ppm = 9.15 (s, 1H), 9.01 (s, 1H), 8.59 (d, J=2.3, 1H), 8.11 (d, J=7.7, 1H), 7.67 (d, J=9.4, 1H), 7.64 (dd, J=9.4, 2.9, 1H), 7.59 (dd, J=9.4, 2.5, 1H), 7.29 (d, J=2.3, 1H), 7.24 (d, J=2.4, 1H), 6.85 (d, J=5.4, 1H), 6.50 (d, J=5.3, 1H), 3.83 - 3.77 (m, 4H), 3.51 - 3.44 (m, 4H). | | | |
| **453** | 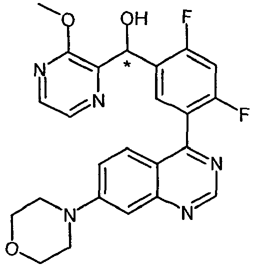 | [2,4-Difluoro-5-(7-morpholin-4-yl-quinazolin-4-yl)-phenyl]-(3-methoxy-pyrazin-2-yl)-methanol (*Ena 1*) | B | A | C |
| | MS: 466.1 (M+H⁺); Rₜ 2.76min (SFC, Chiralpak AD-H, CO₂ / 40 vol.% Methanol, 0.5 vol.% Diethylamin) | s. Racemat | | | |
| **454** | 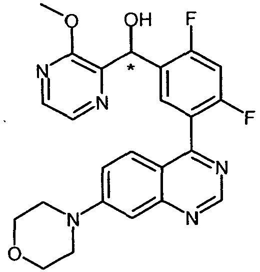 | [2,4-Difluoro-5-(7-morpholin-4-yl-quinazolin-4-yl)-phenyl]-(3-methoxy-pyrazin-2-yl)-methanol (*Ena 2*) | A | A | A |
| | MS: 466.2 (M+H⁺); Rₜ 4.60min (SFC, Chiralpak AD-H, CO₂/40 vol.% Methanol, 0.5 vol.% Diethylamin) | s. Racemat | | | |
| **455** | 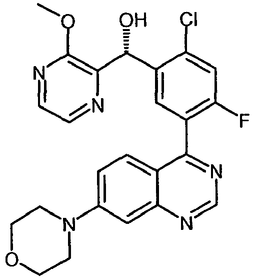 | (R)-[2-Chloro-4-fluoro-5-(7-morpholin-4-yl-quinazolin-4-yl)-phenyl]-(3-methoxy-pyrazin-2-yl)-methanol | A | A | B |
| | MS: 482.1/484.1 (M+H⁺) (Cl Isotopie, rel. Peakintensitäts-Verhältnis [%] 100:36); Rₜ 5.48min (SFC, Chiralpak AD-H, CO₂ / 40 vol.% Methanol, 0.5 vol.% Diethylamin) | s. Racemat | | | |
| **456** | 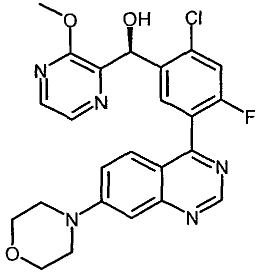 | (S)-[2-Chloro-4-fluoro-5-(7-morpholin-4-yl-quinazolin-4-yl)-phenyl]-(3-methoxy-pyrazin-2-yl)-methanol | A | A | C |
| | MS: 482.1/484.1 (M+H⁺) (Cl Isotopie, rel. Peakintensitäts-Verhältnis [%] 100:35); Rₜ 2.58min (SFC, Chiralpak AD-H, CO₂ / 40 vol.% Methanol, 0.5 vol.% Diethvlamin) | s. Racemat | | | |
| **457** | 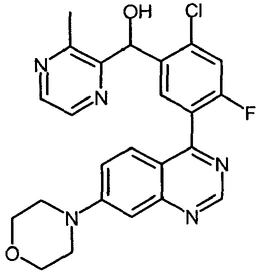 | [2-Chloro-4-fluoro-5-(7-morpholin-4-yl-quinazolin-4-yl)-phenyl]-(3-methyl-pyrazin-2-yl)-methanol | B | A | A |
| | MS: 466.1/468.1 (M+H⁺) (Cl Isotopie, rel. Peakintensitäts-Verhältnis [%] 100:36) | 1H NMR (500 MHz, DMSO-d6) ppm = 9.13 (s, 1H), 8.43 (d, J=2.5, 1H), 8.36 (d, J=2.5, 1H), 7.99 (d, J=7.8, 1H), 7.67 - 7.60 (m, 2H), 7.57 (dd, J=9.4, 2.5, 1H), 7.22 (d, J=2.4, 1H), 6.44 (d, J=5.9, 1H), 6.25 (d, J=5.6, 1H), 3.86 - 3.72 (m, 4H), 3.52 - 3.42 (m, 4H), 2.74 (s, 3H). | | | |
| **458** | 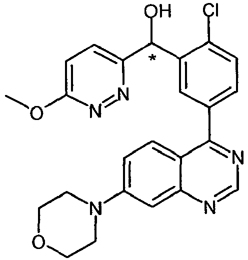 | [2-Chloro-5-(7-morpholin-4-yl-quinazolin-4-yl)-phenyl]-(6-methoxy-pyridazin-3-yl)-methanol (*Ena 1*) | A | A | C |
| | MS: 464.1/466.1 (M+H⁺) (Cl Isotopie, rel. Peakintensitäts-Verhältnis [%] 100:36); Rₜ 11.22min (SFC, Chiralcel OJ-H, CO₂ / 15 vol.% 2-Propanol, 0.5 vol.% Diethylamin) | 1H NMR (400 MHz, DMSO-d6) ppm = 9.08 (s, 1H), 8.07 (d, J=2.2, 1H), 7.89 (d, J=9.5, 1H), 7.73 J=8.2, 1H), 7.54 (dd, - 7.67 (m, 2H), 7.63 (d, J=8.2, 1H), 7.54 (dd, J=9.5, 2.6, 1H), 7.24 - 7.19 (m, 2H), 6.59 (d, J=4.8, 1H), 6.28 (d, J=4.8, 1H), 4.00 (s, 3H), 3.82 - 3.75 (m, 4H), 3.48 - 3.41 (m, 4H). | | | |
| **459** | 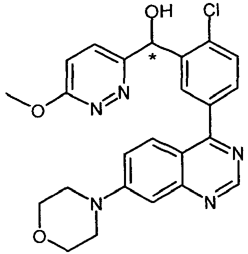 | [2-Chloro-5-(7-morpholin-4-yl-quinazolin-4-yl)-phenyl]-(6-methoxy-pyridazin-3-yl)-methanol (*Ena 2*) | B | B | A* |
| | MS: 464.1/466.1 (M+H⁺) (Cl Isotopie, rel. Peakintensitäts-Verhältnis [%] 100:36); Rₜ 14.88min (SFC, Chiralcel OJ-H, CO₂ / 15 vol.% 2-Propanol, 0.5 vol.% Diethylamin) | s. Racemat | | | |
| **460** | 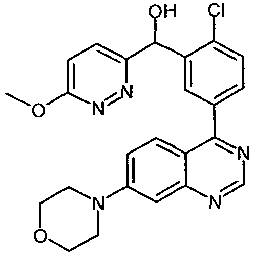 | [2-Chloro-5-(7-morpholin-4-yl-quinazolin-4-yl)-phenyl]-(6-methoxy-pyridazin-3-yl)-methanol | B | A | A* |
| | MS: 464.1/466.1 (M+H⁺) (Cl Isotopie, rel. Peakintensitäts-Verhältnis [%] 100:36) | 1H NMR (500 MHz, DMSO-d6) ppm = 9.09 (s, 1H), 8.08 (d, J=2.2, 1H), 7.89 (d, J=9.5, 1H), 7.73 - 7.67 (m, 2H), 7.63 (d, J=8.2, 1H), 7.54 (dd, J=9.4, 2.7, 1H), 7.23 - 7.19 (m, 2H), 6.59 (d, J=4.9, 1H), 6.28 (d, J=4.8, 1H), 4.00 (s, 3H), 3.81 - 3.75 (m, 4H), 3.48 - 3.42 (m, 4H). | | | |
| **461** | 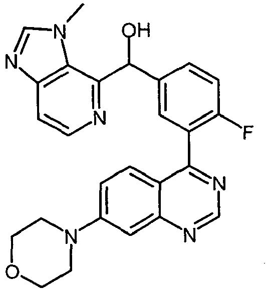 | [4-Fluoro-3-(7-morpholin-4-yl-quinazolin-4-yl)-phenyl]-(3-methyl-3H-imidazo[4,5-c]pyridin-4-yl)-methanol | A | A | A* |
| | MS: 471.2 (M+H⁺) | 1H NMR (500 MHz, DMSO-d6) ppm= 9.08 (s, 1H), 8.33 (s, 1H), 8.25 (d, J=5.5, 1H), 7.62 (d, J=5.5, 1H), 7.61 - 7.50 (m, 4H), 7.42 - 7.37 (m, 1H), 7.19 (d, J=2.3, 1H), 6.61 (d, J=5.5, 1H), 6.43 (d, J=5.4, 1H), 4.01 (s, 3H), 3.80 - 3.74 (m, 4H), 3.46 - 3.41 (m, 4H). | | | |
| **462** | 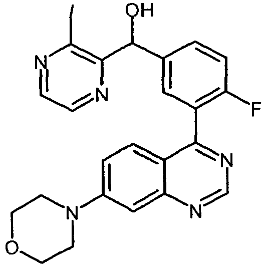 | [4-Fluoro-3-(7-morpholin-4-yl-quinazolin-4-yl)-phenyl]-(3-iodo-pyrazin-2-yl)-methanol | A | A | B |
| | MS: 544.1 (M+H⁺) | 1H NMR (500 MHz, DMSO-d6) ppm = 9.09 (s, 1H), 8.62 (d, J=2.4, 1H), 8.37 (d, J=2.4, 1H), 7.69 - 7.61 (m, 2H), 7.58 - 7.49 (m, 2H), 7.44 - 7.36 (m, 1H), 7.22 - 7.17 (m, 1H), 6.33 (d, J=5.7, 1H), 6.17 (d, J=5.7, 1H), 3.81 - 3.75 (m, 4H), 3.47 - 3.41 (m, 4H). | | | |
| **463** | 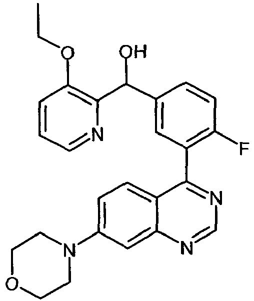 | (3-Ethoxy-pyridin-2-yl)-[4-fluoro-3-(7-morpholin-4-yl-quinazolin-4-yl)-phenyl]-methanol | B | A | B |
| | MS: 461.2 (M+H⁺) | 1H NMR (500 MHz, DMSO-d6) ppm = 9.09 (s, 1H), 8.14 (dd, J=4.7, 1.2, 1H), 7.63 - 7.55 (m, 2H), 7.55 - 7.47 (m, 2H), 7.41 (dd, J=8.3, 1.3, 1H), 7.38 - 7.31 (m, 1H), 7.29 (dd, J=8.3, 4.7, 1H), 7.21 - 7.17 (m, 1H), 6.07 (d, J=6.3, 1H), 5.79 (d, J=6.6, 1H), 4.06 (q, J=7.0, 2H), 3.81 - 3.75 (m, 4H), 3.47 - 3.41 (m, 4H), 1.28 (t, J=6.9, 3H). | | | |
| **464** | 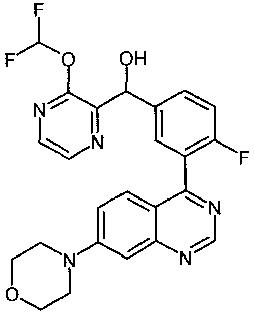 | (3-Difluoromethoxy-pyrazin-2-yl)-[4-fluoro-3-(7-morpholin-4-yl-quinazolin-4-yl)-phenyl]-methanol | B | B | B |
| | MS: 484.2 (M+H⁺) | 1H NMR (500 MHz, DMSO-d6) ppm = 9.09 (s, 1H), 8.51 (d, J=2.6, 1H), 8.28 (d, J=2.6, 1H), 7.86 - 7.55 (m, 3H), 7.54 - 7.49 (m, 2H), 7.43 - 7.36 (m, 1H), 7.22 - 7.17 (m, 1H), 6.30 (d, J=5.6, 1H), 6.10 (d, J=4.9, 1H), 3.83 - 3.73 (m, 4H), 3.48 - 3.40 (m, 4H). | | | |
| **465** | 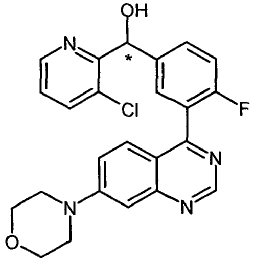 | (3-Chloro-pyridin-2-yl)-[4-fluoro-3-(7-morpholin-4-yl-quinazolin-4-yl)-phenyl]-methanol (*Ena 2*) | A | A | B |
| | MS: 451.1/453.1 (M+H⁺) (Cl Isotopie, rel. Peakintensitäts-Verhältnis [%] 100:35); Rₜ 6.63min (SFC, Chiralpak AD-H, CO₂ /40 vol.% 2-Propanol, 0.5 vol.% Diethvlamin) | s. Racemat | | | |
| **466** | 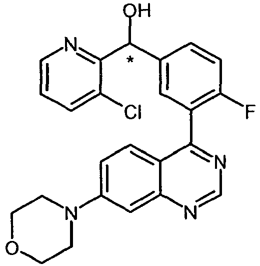 | (3-Chloro-pyridin-2-yl)-[4-fluoro-3-(7-morpholin-4-yl-quinazolin-4-yl)-phenyl]-methanol (*Ena 1*) | C | B | B |
| | MS: 451.1/453.1 (M+H⁺) (Cl Isotopie, rel. Peakintensitäts-Verhältnis [%] 100:35); Rₜ 3.98min (SFC, Chiralpak AD-H, CO₂ /40 vol.% 2-Propanol, 0.5 vol.% Diethylamin) | s. Racemat | | | |
| **467** | 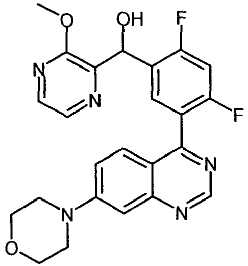 | [2,4-Difluoro-5-(7-morpholin-4-yl-quinazolin-4-yl)-phenyl]-(3-methoxy-pyrazin-2-yl)-methanol | B | A | A |
| | MS: 466.2 (M+H⁺) | 1H NMR (500 MHz, DMSO-d6) ppm = 9.11 (s, 1H), 8.16 (dd, J=16.4, 2.7, 2H), 7.87 (t, J=8.2, 1H), 7.59 (dd, J=9.4, 3.0, 1H), 7.56 (dd, J=9.4, 2.4, 1H), 7.40 (t, J=10.1, 1H), 7.21 (d, J=2.5, 1H), 6.28 (s, 1H), 6.23 (s, 1H), 3.96 (s, 3H), 3.81 - 3.75 (m, 4H), 3.49 - 3.43 (m, 4H). | | | |
| **468** | 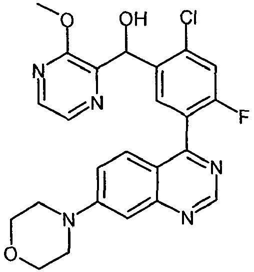 | [2-Chloro-4-fluoro-5-(7-morpholin-4-yl-quinazolin-4-yl)-phenyl]-(3-methoxy-pyrazin-2-yl)-methanol | A | A | B |
| | MS: 482.1/484.1 (M+H⁺) (Cl Isotopie, rel. Peakintensitäts-Verhältnis [%] 100:37) | 1H NMR (500 MHz, DMSO-d6) ppm = 9.13 (s, 1H), 8.17 (d, J=2.7, 1H), 8.11 (d, J=2.7, 1H), 7.97 (d, J=7.8, 1H), 7.65 - 7.58 (m, 2H), 7.56 (dd, J=9.4, 2.5, 1H), 7.22 (d, J=2.4, 1H), 6.31 (s, 2H), 4.00 (s, 3H), 3.81 - 3.76 (m, 4H), 3.48 - 3.43 (m, 4H). | | | |
| **469** | 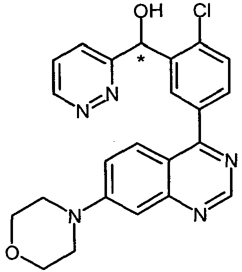 | [2-Chloro-5-(7-morpholin-4-yl-quinazolin-4-yl)-phenyl]-pyridazin-3-yl-methanol (*Ena 2*) | D | D | C |
| | MS: 434.1/436.1 (M+H⁺) (Cl Isotopie, rel. Peakintensitäts-Verhältnis [%] 100:38); Rₜ 4.70min (SFC, Chiralcel OJ-H, CO₂ / 20 vol.% Methanol, 0.5 vol.% Diethylamin) | s. Racemat | | | |
| **470** | 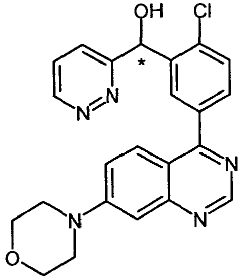 | [2-Chloro-5-(7-morpholin-4-yl-quinazolin-4-yl)-phenyl]-pyridazin-3-yl-methanol (*Ena 1*) | B | A | A |
| | MS: 434.1/436.1 (M+H⁺) (Cl Isotopie, rel. Peakintensitäts-Verhältnis [%] 100:38); Rₜ 2.52min (SFC, Chiralcel OJ-H, CO₂ / 20 vol.% Methanol, 0.5 vol.% Diethylamin) | s. Racemat | | | |
| **471** | 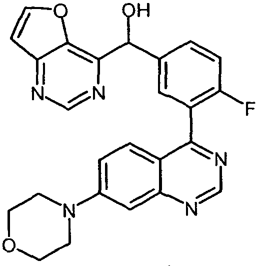 | [4-Fluoro-3-(7-morpholin-4-yl-quinazolin-4-yl)-phenyl]-furo[3,2-d]pyrimidin-4-yl-methanol | A | A | A* |
| | MS: 458.1 (M+H⁺) | 1H NMR (500 MHz, DMSO-d6) ppm = 9.10 (s, 1H), 9.01 (s, 1H), 8.55 (d, J=2.3, 1H), 7.79 - 7.73 (m, 2H), 7.54 - 7.49 (m, 2H), 7.44 - 7.38 (m, 1H), 7.24 (d, J=2.2, 1H), 7.22 - 7.18 (m, 1H), 6.62 (d, J=4.6, 1H), 6.20 (d, J=4.6, 1H), 3.80 - 3.75 (m, 4H), 3.47 - 3.42 (m, 4H). | | | |
| **472** | 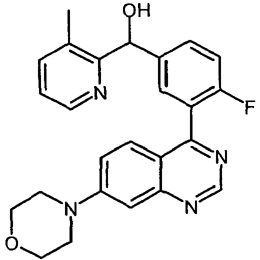 | [4-Fluoro-3-(7-morpholin-4-yl-quinazolin-4-yl)-phenyl]-(3-methyl-pyridin-2-yl)-methanol | B | A | A |
| | MS: 431.2 (M+H⁺) | 1H NMR (400 MHz, DMSO-d6) ppm = 9.08 (s, 1H), 8.43 - 8.39 (m, 1H), 7.63 - 7.57 (m, 1H), 7.56 - 7.49 (m, 4H), 7.39 - 7.32 (m, 1H), 7.28 - 7.23 (m, 1H), 7.21 - 7.17 (m, 1H), 6.09 (s, 1H), 6.01 (s, 1H), 3.82 - 3.73 (m, 4H), 3.48 - 3.40 (m, 4H), 2.28 (s, 3H). | | | |
| **473** | 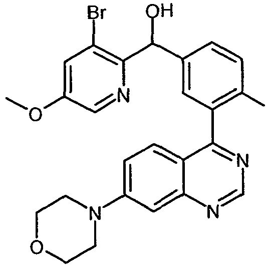 | (3-Bromo-5-methoxy-pyridin-2-yl)-[4-fluoro-3-(7-morpholin-4-yl-quinazolin-4-yl)-phenyl]-methanol | B | A | B |
| | MS: 525.1/527.1 (M+H⁺) (Br Isotopie, rel. Peakintensitäts-Verhältnis [%] 100:97) | 1H NMR (400 MHz, DMSO-d6) ppm = 9.09 (s, 1H), 8.31 (d, J=2.6, 1H), 7.70 (d, J=2.6, 1H), 7.62 - 7.56 (m, 2H), 7.54 - 7.50 (m, 2H), 7.39 - 7.33 (m, 1H), 7.21 - 7.18 (m, 1H), 6.16 (d, J=6.1, 1H), 6.01 (d, J=6.1, 1H), 3.84 (s, 3H), 3.80 - 3.75 (m, 4H), 3.46 - 3.41 (m, 4H). | | | |
| **474** | 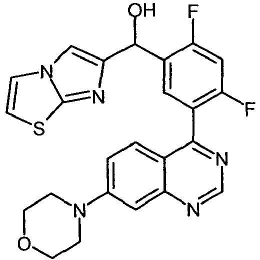 | [2,4-Difluoro-5-(7-morpholin-4-yl-quinazolin-4-yl)-phenyl]-imidazo[2,1-b]thiazol-6-yl-methanol | D | D | C |
| | MS: 480.1 (M+H⁺) | 1H NMR (400 MHz, DMSO-d6) ppm = 9.09 (s, 1H), 7.85 (d, J=4.5, 1H), 7.71 - 7.68 (m, 1H), 7.62 - 7.55 (m, 1H), 7.54 - 7.51 (m, 2H), 7.26 (t, J=9.1, 1H), 7.21 - 7.17 (m, 2H), 6.18 - 6.13 (m, 2H), 3.80 - 3.75 (m, 4H), 3.47 - 3.41 (m, 4H). | | | |
| **475** | 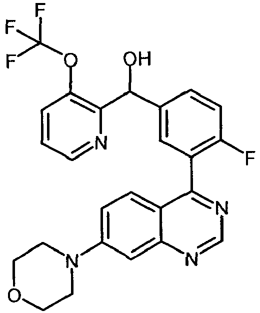 | [4-Fluoro-3-(7-morpholin-4-yl-quinazolin-4-yl)-phenyl]-(3-trifluoromethoxy-pyridin-2-yl)-methanol | A | A | B |
| | MS: 501.2 (M+H⁺) | 1H NMR (400 MHz, DMSO-d6) ppm = 9.11 (s, 1H), 8.61 (dd, J=4.6, 1.3, 1H), 7.87 (dp, J=8.4, 1.6, 1H), 7.69 - 7.57 (m, 2H), 7.57 - 7.46 (m, 3H), 7.40 (dd, J=9.9, 8.4, 1H), 7.21 (d, J=1.9, 1H), 6.26 (d, J=5.9, 1H), 6.13 (d, J=5.9, 1H), 3.89 - 3.65 (m, 4H), 3.54 - 3.38 (m, 4H). | | | |
| **476** | 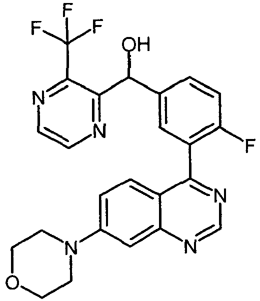 | [4-Fluoro-3-(7-morpholin-4-yl-quinazolin-4-yl)-phenyl]-(3-trifluoromethyl-pyrazin-2-yl)-methanol | B | A | A |
| | MS: 486.2 (M+H⁺) | 1H NMR (500 MHz, DMSO-d6) ppm = 9.10 (s, 1H), 9.00 (d, J=2.3, 1H), 8.77 (d, J=2.3, 1H), 7.66 (dd, J=6.9, 2.3, 1H), 7.63 - 7.59 (m, 1H), 7.56 - 7.50 (m, 2H), 7.43 - 7.37 (m, 1H), 7.21 - 7.18 (m, 1H), 6.54 (d, J=5.7, 1H), 6.21 (d, J=4.3, 1H), 3.81 - 3.74 (m, 4H), 3.48 - 3.40 (m, 4H). | | | |
| **478** | 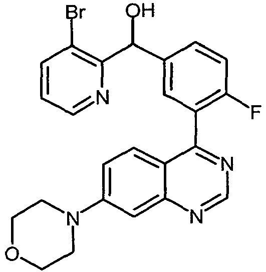 | (3-Bromo-pyridin-2-yl)-[4-fluoro-3-(7-morpholin-4-yl-quinazolin-4-yl)-phenyl]-methanol | A | A | D |
| | MS: 495.1/497.1 (M+H⁺) (Br Isotopie, rel. Peakintensitäts-Verhältnis [%] 100:96) | 1H NMR (400 MHz, DMSO-d6) ppm = 9.09 (s, 1H), 8.60 (dd, J=4.6, 1.4, 1H), 8.08 (dd, J=8.1, 1.5, 1H), 7.65 - 7.59 (m, 2H), 7.54 - 7.50 (m, 2H), 7.41 - 7.34 (m, 1H), 7.29 (dd, J=8.1, 4.6, 1H), 7.21 - 7.18 (m, 1H), 6.20 (d, J=6.3, 1H), 6.12 (d, J=6.3, 1H), 3.82 - 3.74 (m, 4H), 3.49 - 3.40 (m, 4H). | | | |
| **479** | 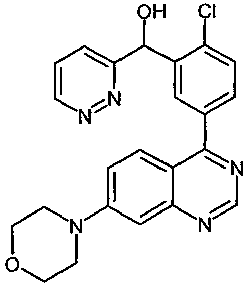 | [2-Chloro-5-(7-morpholin-4-yl-quinazolin-4-yl)-phenyl]-pyridazin-3-yl-methanol | B | A | B |
| | MS: 434.1/436.1 (M+H⁺) (Cl Isotopie, rel. Peakintensitäts-Verhältnis [%] 100:37) | 1H NMR (500 MHz, DMSO-d6) ppm = 9.15 (dd, J=4.9, 1.7, 1H), 9.08 (s, 1H), 8.04 (d, J=2.2, 1H), 7.87 (d, J=9.4, 1H), 7.80 (dd, J=8.5, 1.7, 1H), 7.74 - 7.69 (m, 2H), 7.64 (d, J=8.2, 1H), 7.52 (dd, J=9.5, 2.6, 1H), 7.21 (d, J=2.5, 1H), 6.69 (d, J=4.9, 1H), 6.38 (d, J=4.9, 1H), 3.83 - 3.72 (m, 4H), 3.49 - 3.40 (m, 4H). | | | |

| | | | | | |
|---|---|---|---|---|---|
| (*) In der zweiten Spalte: Chromatographisch isoliertes Enantiomer, das entweder die reine R- oder S-Konfiguration des Moleküls repräsentiert (*) In der letzten Spalte: Kaliumkanalaktivität gemessen mit hERG binding assay anstelle von hERG patch clamp assay | | | | | |

Beispielnummern 273-277, 281-283, 287 und 477 wurden absichtlich herausgelassen.

## Patentansprüche

1. Verbindung der Formel (I) 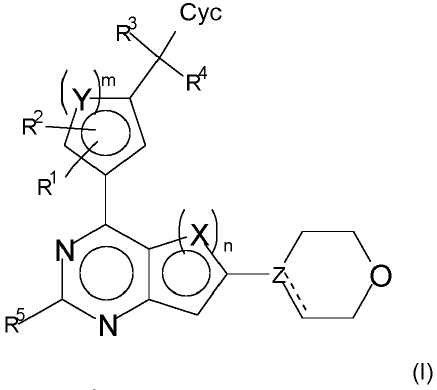 worin
X CH, CF, S oder N ist,
Y CH, S oder N ist,
Z C oder N ist,
---- bildet, wenn Z = C ist, zusammen mit der Einfachbindung eine Doppelbindung,
ist abwesend, wenn Z = N ist,
n 1 oder 2 ist, wobei
wenn n = 1 ist, ist X = S,
und wenn n = 2 ist, sind beide X = CH, oder das mit dem Pyrimidinring verknüpfte X ist CF und das nicht mit dem Pyrimidinring verknüpfte X ist CH, oder ein X ist CH und das andere X ist N;
m 1 oder 2 ist, wobei
wenn m = 1 ist, ist Y = S,
und wenn m = 2 ist, sind beide Y = CH, oder ein Y ist CH und das andere Y ist N;
R¹, R², R³, R⁴ unabhängig voneinander H, Hal, CN, OH, CONH₂, CONH(LA) oder LA sind;
R⁵ H, Hal, CN oder C≡CH ist;
Cyc Phenyl ist, das unsubstituiert oder ein- oder zweifach unabhängig voneinander durch R⁶ substituiert sein kann, oder Het¹ ist;
Het¹ ein ein- oder zweikerniger, 5-10 gliedriger Heterozyklus ist, mit 1-3 N-, O- und/oder S-Atomen, oder 1-4 N-Atomen, der unsubstituiert oder ein-, zwei oder dreifach unabhängig voneinander durch R⁶ substituiert sein kann, oder einfach durch Het² substituiert sein kann;
R⁶ Hal, LA, Oxo, CN, oder NH₂ ist;
LA unverzweigtes oder verzweigtes Alkyl mit 1-5 C-Atomen ist, das gesättigt oder partiell ungesättigt sein kann, worin 1-3 H-Atome durch Hal, und/oder ein H-Atom durch CN oder Het², und/oder ein oder zwei CH₂-Gruppen durch O, NH, NH₂, N(CH₃) oder CO ersetzt sein können;
Het² ein 3-5 gliedriger aliphatischer Homo- oder Heterozyklus mit 0, 1, 2 oder 3 N-, O- und/oder S-Atomen ist, der unsubstituiert ist;
Hal F, Cl, Br oder I ist;
wobei H, C, N, O, P, S, F und Cl auch die schwereren Isotope dieser Atome umfassen,
und/oder physiologisch unbedenkliches Salz, Tautomer und/oder Stereoisomer davon, einschließlich deren Mischungen in allen Verhältnissen.

2. Verbindung nach Anspruch 1, die der Formel (Ib) entspricht, 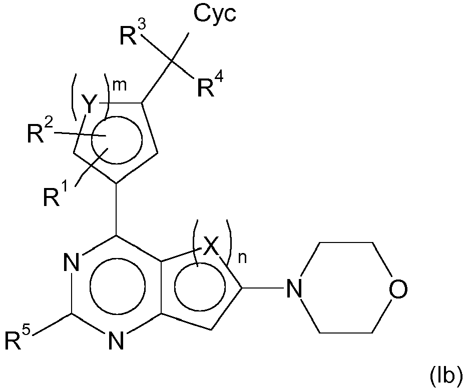 worin alle Substituenten die für die Formel (I) angegebene Bedeutung haben, und/oder physiologisch unbedenkliches Salz, Tautomer und/oder Stereoisomer davon, einschließlich deren Mischungen in allen Verhältnissen.

3. Verbindung nach Anspruch 1 oder 2, die der Formel (II) entspricht 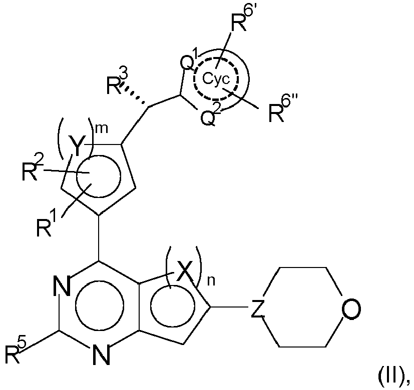 worin
R³ Hal, CN, OH, CONH₂, CONH(LA) oder LA ist;
R^{6'}, R^{6"} unabhängig voneinander H, Hal, LA, Oxo, CN, NH₂ oder Het² sind;
Q¹,Q² unabhängig voneinander CH, N oder NH und in jedem Fall unsubstituiert sind;
---- die An- oder Abwesenheit von Doppelbindungen in Cyc bedeutet;
und die übrigen Substituenten die für die Formel (I) angegebene Bedeutung haben, und/oder physiologisch unbedenkliches Salz, Tautomer und/oder Stereoisomer davon, einschließlich deren Mischungen in allen Verhältnissen.

4. Verbindung nach Anspruch 1 oder 2, die der Formel (III) entspricht 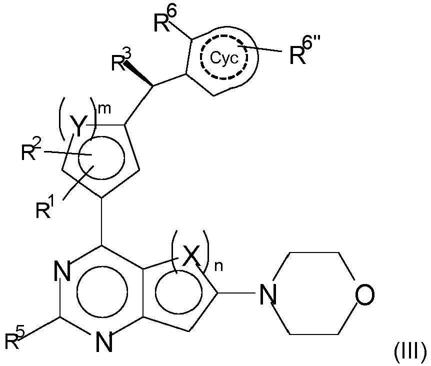 worin
R³ Hal, CN, OH, CONH₂, CONH(LA) oder LA ist;
R⁶ Hal, LA, Oxo, CN, NH₂ oder Het² ist;
R^{6"} H, Hal, LA, Oxo, CN, NH₂ oder Het² ist;
---- die An- oder Abwesenheit von Doppelbindungen in Cyc bedeutet;
und die übrigen Substituenten die für die Formel (I) angegebene Bedeutung haben, und/oder physiologisch unbedenkliches Salz, Tautomer und/oder Stereoisomer davon, einschließlich deren Mischungen in allen Verhältnissen.

5. Verbindung nach Anspruch 3, die der Formel (IIa) entspricht 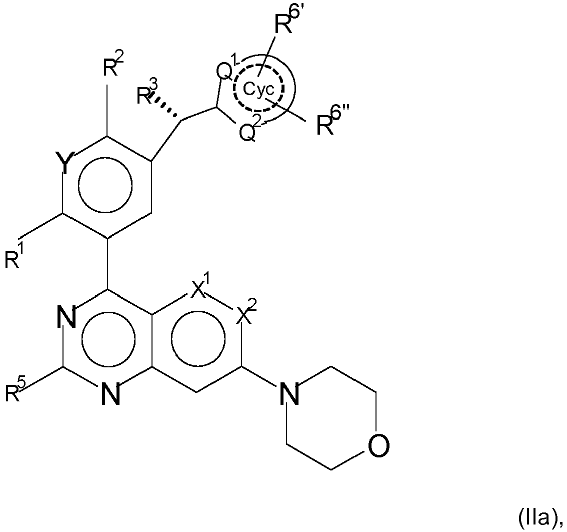 worin
R², R³ unabhängig voneinander Hal, CN, OH, CONH₂, CON(LA) oder LA sind;
R^{6'}, R^{6"} unabhängig voneinander H, Hal, LA, Oxo, CN, NH₂ oder Het² sind;
Q¹,Q² unabhängig voneinander CH, N oder NH und in jedem Fall unsubstituiert sind;
X¹ CH, CF oder N ist;
X² CH oder N ist, wobei X¹, X² nicht gleichzeitig N sind;
Y CH oder N ist;
---- die An- oder Abwesenheit von Doppelbindungen in Cyc bedeutet;
und die übrigen Substituenten die für die Formel (I) angegebene Bedeutung haben, und/oder physiologisch unbedenkliches Salz, Tautomer und/oder Stereoisomer davon, einschließlich deren Mischungen in allen Verhältnissen.

6. Verbindung nach Anspruch 3, die der Formel (IIb) entspricht 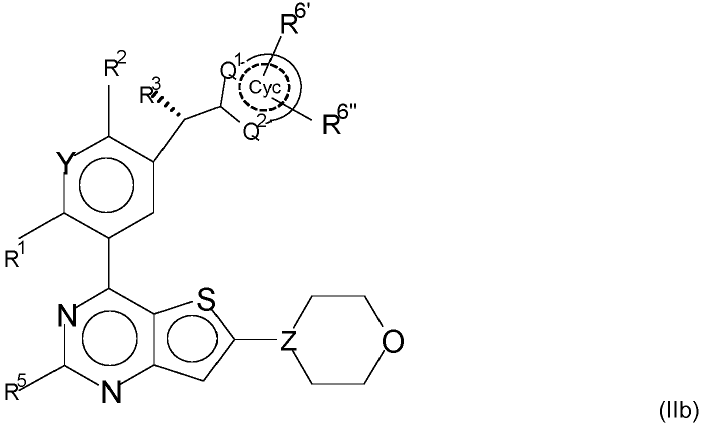 worin
R², R³ unabhängig voneinander Hal, CN, OH, CONH₂, CON(LA) oder LA sind;
R^{6'}, R^{6"} unabhängig voneinander H, Hal, LA, Oxo, CN, NH₂ oder Het² sind;
Q¹,Q² unabhängig voneinander CH, N oder NH und in jedem Fall unsubstituiert sind;
Y CH oder N ist,
---- die An- oder Abwesenheit von Doppelbindungen in Cyc bedeutet;
und alle übrigen Substituenten die für die Formel (I) angegebene Bedeutung haben, und/oder physiologisch unbedenkliches Salz, Tautomer und/oder Stereoisomer davon, einschließlich deren Mischungen in allen Verhältnissen.

7. Verbindung nach Anspruch 4, die der Formel (IIIa) entspricht 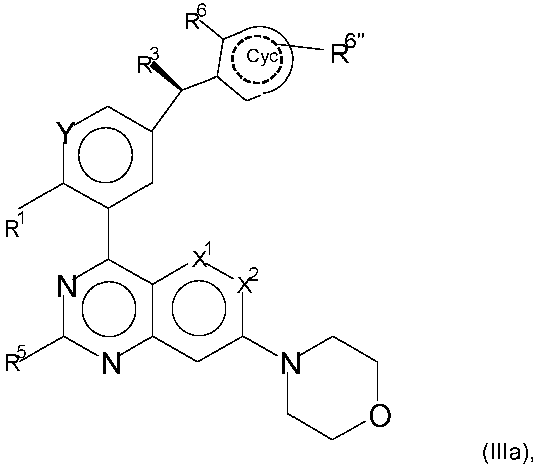 worin
R³ Hal, CN, OH, CONH₂, CON(LA) oder LA ist;
R⁶ Hal, LA, Oxo, CN, NH₂ oder Het² ist;
R^{6"} H, Hal, LA, Oxo, CN, NH₂ oder Het² ist;
X¹ CH, CF oder N ist;
X² CH oder N ist, wobei X¹, X² nicht gleichzeitig N sind;
Y CH oder N ist;
---- die An- oder Abwesenheit von Doppelbindungen in Cyc bedeutet;
und die übrigen Substituenten die für die Formel (I) angegebene Bedeutung haben, und/oder physiologisch unbedenkliches Salz, Tautomer und/oder Stereoisomer davon, einschließlich deren Mischungen in allen Verhältnissen.

8. Verbindung nach Anspruch 4, die der Formel (IIIb) entspricht 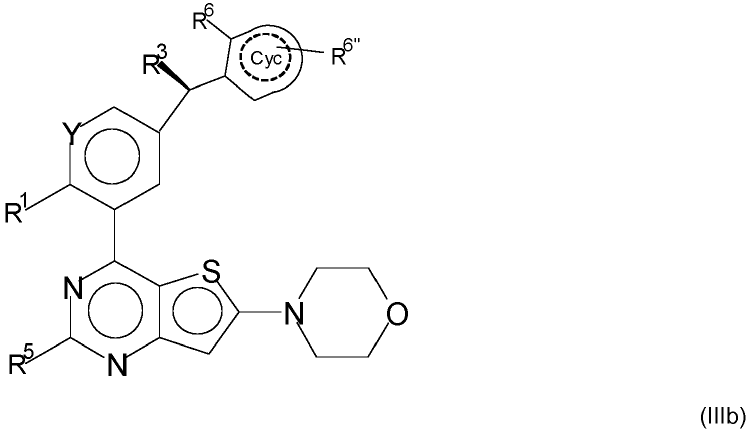 worin
R³ Hal, CN, OH, CONH₂, CON(LA) oder LA ist;
R⁶ Hal, LA, Oxo, CN, NH₂ oder Het² ist;
R^{6"} H, Hal, LA, Oxo, CN, NH₂ oder Het² ist;
Y CH oder N ist,
---- die An- oder Abwesenheit von Doppelbindungen in Cyc bedeutet;
und alle übrigen Substituenten die für die Formel (I) angegebene Bedeutung haben, und/oder physiologisch unbedenkliches Salz, Tautomer und/oder Stereoisomer davon, einschließlich deren Mischungen in allen Verhältnissen.

9. Verbindung nach Anspruch 5, worin die nicht näher bezeichneten Reste die für die Formel (IIa) angegebene Bedeutung haben, worin jedoch
bei der Teilformel (IIa-A)
X¹ CH,
R¹ F oder Cl,
R² F oder Cl,
bei der Teilformel (IIa-B)
R¹ F,
R² F oder Cl,
bei der Teilformel (IIa-C)
X¹, X² CH,
bei der Teilformel (IIa-D)
X¹ CH,
R⁵ H,
bei der Teilformel (IIa-E)
R³ H, OH,
bei der Teilformel (IIa-F)
X¹ CH,
R³ OH,
bei der Teilformel (IIa-G)
X¹ CH,
Y CH,
bei der Teilformel (IIa-H)
X¹ CH,
Cyc Pyridin, Pyrazin oder Pyridazin, oder Pyrazolo[1,5-a]pyrimidinyl oder Imidazo[1,2-b]pyridazinyl,
bei der Teilformel (IIa-J)
Cyc Pyridin, Pyrazin, Pyridazin, Pyrazolo[1,5-a]pyrimidinyl, Imidazo[1,2-b]pyridazinyl, Furo[2,3-c]pyridinyl, Furo[2,3-d]pyridazinyl, Thieno[2,3-d]pyridazinyl, Thieno[2,3-d]pyrimidinyl oder Imidazo[4,5-c]pyridinyl, die jeweils unsubstituiert sein können, oder ein- oder zweifach mit Methoxy, Methyl, Oxo, Cl oder CHF₂O substituiert sein können,
bei der Teilformel (IIa-K)
R¹ F oder Cl,
R² F oder Cl,
R³ OH,
R⁵ H,
X¹, X² CH,
bei der Teilformel (IIa-L)
R¹ F,
R² F oder Cl,
R³ H oder OH,
R⁵ H,
bei der Teilformel (IIa-M)
R¹ F oder Cl,
R² F oder Cl,
R³ OH,
R⁵ H,
X¹, X² CH,
Cyc Pyridin, Pyrazin oder Pyridazin, oder Pyrazolo[1,5-a]pyrimidinyl oder Imidazo[1,2-b]pyridazinyl,
bei der Teilformel (IIa-N)
R¹ F,
R² F oder Cl,
R³ H oder OH,
R⁵ H,
Cyc Pyridin, Pyrazin, Pyridazin, Pyrazolo[1,5-a]pyrimidinyl, Imidazo[1,2-b]pyridazinyl, Furo[2,3-c]pyridinyl, Furo[2,3-d]pyridazinyl, Thieno[2,3-d]pyridazinyl, Thieno[2,3-d]pyrimidinyl oder Imidazo[4,5-c]pyridinyl, die jeweils unsubstituiert sein können, oder ein- oder zweifach mit Methoxy, Methyl, Oxo, Cl oder CHF₂O substituiert sein können,
bei der Teilformel (IIa-O)
R¹ F,
R² F oder Cl,
R³ H oder OH,
R⁵ H,
Cyc 5-Methoxy-pyridazin-3-yl, Imidazo[1,2-b]pyridazin-6-yl, 3-Chloro-6-methoxy-pyrazin-2-yl, 3-Chloro-pyrazin-2-yl, Pyridazin-4-yl, 3-Methoxy-pyrazin-2-yl, 6-Methoxy-pyridazin-3-yl, 3-Difluoromethoxy-pyridin-2-yl, 3-Methyl-pyrazin-2-yl, Thieno[2,3-d]pyrimidin-4-yl, 1-Methyl-1H-pyridin-2-one-6-yl, 1H-Pyridazin-6-one-3-yl, Furo[2,3-d]pyridazin-7-yl, Thieno[2,3-d]pyridazin-7-yl, 3,5-Dimethyl-pyrazin-2-yl, Furo[2,3-d]pyrimidin-4-yl, 3-Methyl-3H-imidazo[4,5-c]pyridin-4-yl,
ist und/oder physiologisch unbedenkliches Salz, Tautomer und/oder Stereoisomer davon, einschließlich deren Mischungen in allen Verhältnissen.

10. Verbindung nach Anspruch 7, worin die nicht näher bezeichneten Reste die für die Formel (IIIa) angegebene Bedeutung haben, worin jedoch
bei der Teilformel (IIIa-B)
R¹ F,
bei der Teilformel (IIIa-C)
X¹, X² CH,
bei der Teilformel (IIIa-D)
X¹ CH,
R⁵ H,
bei der Teilformel IIIa-(E)
R³ H, OH,
bei der Teilformel (IIIa-F)
X¹ CH,
R³ OH,
bei der Teilformel (IIIa-G)
X¹ CH,
Y CH,
bei der Teilformel (IIIa-H)
X¹ CH,
Cyc Pyridin, Pyrazin oder Pyridazin, oder Pyrazolo[1,5-a]pyrimidinyl oder Imidazo[1,2-b]pyridazinyl,
bei der Teilformel (IIIa-J)
Cyc Pyridin, Pyrazin, Pyridazin, Pyrazolo[1,5-a]pyrimidinyl, Imidazo[1,2-b]pyridazinyl, Furo[2,3-c]pyridinyl, Furo[2,3-d]pyridazinyl, Thieno[2,3-d]pyridazinyl, Thieno[2,3-d]pyrimidinyl oder Imidazo[4,5-c]pyridinyl, die jeweils unsubstituiert sein können, oder ein- oder zweifach mit Methoxy, Methyl, Oxo, Cl oder CHF₂O substituiert sein können,
bei der Teilformel (IIIa-K)
R¹ F oder Cl,
R³ OH,
R⁵ H,
X¹, X² CH,
bei der Teilformel (IIIa-L)
R¹ F,
R³ H oder OH,
R⁵ H,
bei der Teilformel (IIIa-M)
R¹ F oder Cl,
R³ OH,
R⁵ H,
X¹, X² CH,
Cyc Pyridin, Pyrazin oder Pyridazin, oder Pyrazolo[1,5-a]pyrimidinyl oder Imidazo[1,2-b]pyridazinyl,
bei der Teilformel (IIIa-N)
R¹ F,
R³ H oder OH,
R⁵ H,
Cyc Pyridin, Pyrazin, Pyridazin, Pyrazolo[1,5-a]pyrimidinyl, Imidazo[1,2-b]pyridazinyl, Furo[2,3-c]pyridinyl, Furo[2,3-d]pyridazinyl, Thieno[2,3-d]pyridazinyl, Thieno[2,3-d]pyrimidinyl oder Imidazo[4,5-c]pyridinyl, die jeweils unsubstituiert sein können, oder ein- oder zweifach mit Methoxy, Methyl, Oxo, Cl oder CHF₂O substituiert sein können,
bei der Teilformel (IIIa-O)
R¹ F,
R³ H oder OH,
R⁵ H,
Cyc 5-Methoxy-pyridazin-3-yl, Imidazo[1,2-b]pyridazin-6-yl, 3-Chloro-6-methoxy-pyrazin-2-yl, 3-Chloro-pyrazin-2-yl, Pyridazin-4-yl, 3-Methoxy-pyrazin-2-yl, 6-Methoxy-pyridazin-3-yl, 3-Difluoromethoxy-pyridin-2-yl, 3-Methyl-pyrazin-2-yl, Thieno[2,3-d]pyrimidin-4-yl, 1-Methyl-1H-pyridin-2-one-6-yl, 1H-Pyridazin-6-one-3-yl, Furo[2,3-d]pyridazin-7-yl, Thieno[2,3-d]pyridazin-7-yl, 3,5-Dimethyl-pyrazin-2-yl, Furo[2,3-d]pyrimidin-4-yl, 3-Methyl-3H-imidazo[4,5-c]pyridin-4-yl,
ist und/oder physiologisch unbedenkliches Salz, Tautomer und/oder Stereoisomer davon, einschließlich deren Mischungen in allen Verhältnissen.

11. Verbindung nach Anspruch 6, worin die nicht näher bezeichneten Reste die für die Formeln (IIb) angegebene Bedeutung haben, worin jedoch
bei der Teilformel (IIb-Q)
R¹ F oder Cl,
R² F oder Cl,
R³ OH,
R⁵ H,
Y CH,
bei der Teilformel (IIb-R)
R¹ F,
R² F oder Cl,
R³ OH,
R⁵ H,
Y CH,
bei der Teilformel (IIb-S)
Cyc Pyridin, Pyrazin oder Pyridazin,
bei der Teilformel (IIb-T)
R¹ F oder Cl,
R² F oder Cl,
R³ OH,
R⁵ H,
Cyc Pyridin, Pyrazin oder Pyridazin,
bei der Teilformel (IIb-U)
R¹ F,
R² F oder Cl,
R³ OH,
R⁵ H,
Cyc Pyridin, Pyrazin, Pyridazin oder 3-Methyl-pyrazin-2-yl,
ist und/oder physiologisch unbedenkliches Salz, Tautomer und/oder Stereoisomer, einschließlich deren Mischungen in allen Verhältnissen.

12. Verbindung nach Anspruch 8, worin die nicht näher bezeichneten Reste die für die Formeln (IIIb) angegebene Bedeutung haben, worin jedoch
bei der Teilformel (IIIb-Q)
R¹ F oder Cl,
R³ OH,
R⁵ H,
Y CH,
bei der Teilformel (IIIb-R)
R¹ F,
R³ OH,
R⁵ H,
Y CH,
bei der Teilformel (IIIb-S)
Cyc Pyridin, Pyrazin oder Pyridazin,
bei der Teilformel (IIIb-T)
R¹ F oder Cl,
R³ OH,
R⁵ H,
Cyc Pyridin, Pyrazin oder Pyridazin,
bei der Teilformel (IIIb-U)
R¹ F,
R³ OH,
R⁵ H,
Cyc Pyridin, Pyrazin, Pyridazin oder 3-Methyl-pyrazin-2-yl,
ist und/oder physiologisch unbedenkliches Salz, Tautomer und/oder Stereoisomer davon, einschließlich deren Mischungen in allen Verhältnissen.

13. Verbindung nach einem der Ansprüche 1 bis 12, ausgewählt aus der Gruppe:
[2-Chloro-4-fluoro-5-(7-morpholin-4-yl-quinazolin-4-yl)-phenyl]-(5-methoxy-pyridazin-3-yl)-methanol,
[4-Fluoro-3-(7-morpholin-4-yl-quinazolin-4-yl)-phenyl]-(5-methoxy-pyridazin-3-yl)-methanol, [2,4-Difluoro-5-(7-morpholin-4-yl-quinazolin-4-yl)-phenyl]-imidazo[1,2-b]pyridazin-6-yl-methanol,
(3-Chloro-6-methoxy-pyrazin-2-yl)-[4-fluoro-3-(7-morpholin-4-yl-quinazolin-4-yl)-phenyl]-methanol,
(R)-(3-Chloro-pyrazin-2-yl)-[4-fluoro-3-(7-morpholin-4-yl-quinazolin-4-yl)-phenyl]-methanol,
[2-Chloro-4-fluoro-5-(7-morpholin-4-yl-quinazolin-4-yl)-phenyl]-pyridazin-4-yl-methanol,
[4-Fluoro-3-(7-morpholin-4-yl-quinazolin-4-yl)-phenyl]-(3-methoxy-pyrazin-2-yl)-methanol,
[4-Fluoro-3-(7-morpholin-4-yl-quinazolin-4-yl)-phenyl]-(6-methoxy-pyridazin-3-yl)-methanol,
(3-Difluoromethoxy-pyridin-2-yl)-[4-fluoro-3-(7-morpholin-4-yl-quinazolin-4-yl)-phenyl]-methanol,
(R)-[4-Fluoro-3-(7-morpholin-4-yl-quinazolin-4-yl)-phenyl]-(3-methyl-pyrazin-2-yl)-methanol,
[2,4-Difluoro-5-(7-morpholin-4-yl-quinazolin-4-yl)-phenyl]-(3-methyl-pyrazin-2-yl)-methanol,
[2,4-Difluoro-5-(7-morpholin-4-yl-quinazolin-4-yl)-phenyl]-thieno[2,3-d]pyrimidin-4-yl-methanol,
6-{[4-Fluoro-3-(7-morpholin-4-yl-quinazolin-4-yl)-phenyl]-hydroxy-methyl}-1-methyl-1H-pyridin-2-one,
3-[[2-Chloro-4-fluoro-5-(7-morpholino-quinazolin-4-yl)phenyl]-hydroxy-methyl]-1H-pyridazin-6-one,
(S)-[2-Chloro-4-fluoro-5-(7-morpholin-4-yl-quinazolin-4-yl)-phenyl]-(6-methoxy-pyridazin-3-yl)-methanol,
(R)-[4-Fluoro-3-(7-morpholin-4-yl-pyrido[3,2-d]pyrimidin-4-yl)-phenyl]-(3-methyl-pyrazin-2-yl)-methanol,
4-(4-Chloro-2-fluoro-5-imidazo[1,2-b]pyridazin-6-ylmethyl-phenyl)-7-morpholin-4-yl-quinazoline,
[4-Fluoro-3-(6-morpholin-4-yl-thieno[3,2-d]pyrimidin-4-yl)-phenyl]-(3-methyl-pyrazin-2-yl)-methanol,
(R)-[4-Fluoro-3-(5-fluoro-7-morpholin-4-yl-quinazolin-4-yl)-phenyl]-(3-methyl-pyrazin-2-yl)-methanol,
[2-Chloro-4-fluoro-5-(5-fluoro-7-morpholin-4-yl-quinazolin-4-yl)-phenyl]-(3-methoxy-pyrazin-2-yl)-methanol,
[4-Fluoro-3-(5-fluoro-7-morpholin-4-yl-quinazolin-4-yl)-phenyl]-(3-methoxy-pyrazin-2-yl)-methanol,
[4-Fluoro-3-(5-fluoro-7-morpholin-4-yl-quinazolin-4-yl)-phenyl]-(6-methoxy-pyridazin-3-yl)-methanol,
[4-Fluoro-3-[7-(2,2,3,3,5,5,6,6-octadeuteriomorpholin-4-yl)quinazolin-4-yl]phenyl]-(3-methylpyrazin-2-yl)methanol,
[4-Fluoro-3-(5-fluoro-7-morpholin-4-yl-quinazolin-4-yl)-phenyl]-(6-methoxy-pyridazin-3-yl)-methanol,
[2-Chloro-4-fluoro-5-[7-(2,2,3,3,5,5,6,6-octadeuteriomorpholin-4-yl)quinazolin-4-yl]phenyl]-(6-methoxypyridazin-3-yl)methanol,
[2-Chloro-4-fluoro-5-(6-morpholin-4-yl-thieno[3,2-d]pyrimidin-4-yl)-phenyl]-(3-methyl-pyrazin-2-yl)-methanol,
[4-Fluoro-3-(6-morpholin-4-yl-thieno[3,2-d]pyrimidin-4-yl)-phenyl]-(3-methyl-pyrazin-2-yl)-methanol,
[2-Chloro-4-fluoro-5-(5-fluoro-7-morpholin-4-yl-quinazolin-4-yl)-phenyl]-(3-methoxy-pyrazin-2-yl)-methanol,
[4-Fluoro-3-(5-fluoro-7-morpholin-4-yl-quinazolin-4-yl)-phenyl]-(3-methoxy-pyrazin-2-yl)-methanol,
[4-Fluoro-3-(5-fluoro-7-morpholin-4-yl-quinazolin-4-yl)-phenyl]-(3-methyl-pyrazin-2-yl)-methanol,
[2-Chloro-4-fluoro-5-(5-fluoro-7-morpholin-4-yl-quinazolin-4-yl)-phenyl]-(6-methoxy-pyridazin-3-yl)-methanol,
[4-Fluoro-3-(7-morpholin-4-yl-quinazolin-4-yl)-phenyl]-furo[2,3-d]pyridazin-7-yl-methanol,
[2,4-Difluoro-5-(7-morpholin-4-yl-quinazolin-4-yl)-phenyl]-furo[2,3-d]pyridazin-7-yl-methanol,
[2,4-Difluoro-5-(7-morpholin-4-yl-quinazolin-4-yl)-phenyl]-thieno[2,3-d]pyridazin-7-yl-methanol,
(3,5-Dimethyl-pyrazin-2-yl)-[4-fluoro-3-(7-morpholin-4-yl-quinazolin-4-yl)-phenyl]-methanol,
6-{[2-Chloro-5-(7-morpholin-4-yl-quinazolin-4-yl)-phenyl]-hydroxy-methyl}-1-methyl-1H-pyridin-2-one,
6-{[4-Fluoro-3-(7-morpholin-4-yl-quinazolin-4-yl)-phenyl]-hydroxy-methyl}-2H-pyridazin-3-one,
6-{[4-Fluoro-3-(7-morpholin-4-yl-quinazolin-4-yl)-phenyl]-hydroxy-methyl}-1-methyl-1H-pyridin-2-one,
6-{[2-Chloro-4-fluoro-5-(7-morpholin-4-yl-quinazolin-4-yl)-phenyl]-hydroxy-methyl}-1-methyl-1H-pyridin-2-one,
[4-Fluoro-3-(7-morpholin-4-yl-quinazolin-4-yl)-phenyl]-furo[2,3-d]pyrimidin-4-yl-methanol,
[4-Fluoro-3-(7-morpholin-4-yl-quinazolin-4-yl)-phenyl]-furo[3,2-d]pyrimidin-4-yl-methanol,
[2,4-Difluoro-5-(7-morpholin-4-yl-quinazolin-4-yl)-phenyl]-(3-methoxy-pyrazin-2-yl)-methanol,
4-Fluoro-3-(7-morpholin-4-yl-quinazolin-4-yl)-phenyl]-(3-methyl-3H-imidazo[4,5-c]pyridin-4-yl)-methanol,
[4-Fluoro-3-(7-morpholin-4-yl-quinazolin-4-yl)-phenyl]-furo[3,2-d]pyrimidin-4-yl-methanol,
und/oder physiologisch unbedenkliches Salz, Tautomer und/oder Stereoisomer davon, einschließlich deren Mischungen in allen Verhältnissen.

14. Verfahren zum Herstellen einer Verbindung der Formel (I) nach Anspruch 1 und/oder physiologisch unbedenklichem Salz, Tautomer und/oder Stereoisomer davon mit den folgenden Schritten:
(a) Umsetzen einer Verbindung der Formel (V) 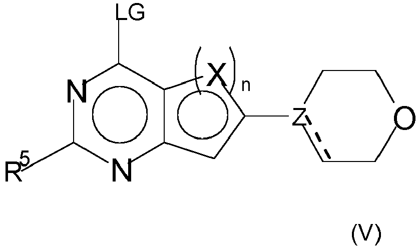 worin LG eine übliche Abgangsgruppe wie Hal ist, mit einer Verbindung der Formel (IV) 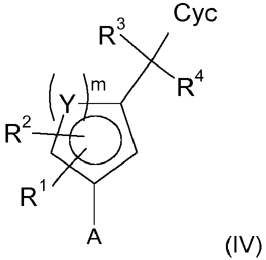 worin A Boronsäure oder ein Boronsäureester ist,
unter Erhalt der Verbindung der Formel (I), und gegebenenfalls
(b) Umwandeln einer Base oder Säure der Verbindung der Formel (I) in eines ihrer Salze.

15. Verbindung nach einem der Ansprüche 1 bis 13 und/oder physiologisch unbedenkliches Salz, Tautomer und/oder Stereoisomer davon, einschließlich deren Mischungen in allen Verhältnissen, zur Verwendung als Medikament.

16. Verbindung nach einem der Ansprüche 1 bis 13 und/oder physiologisch unbedenkliches Salz, Tautomer und/oder Stereoisomer davon, einschließlich deren Mischungen in allen Verhältnissen, zur Verwendung als Medikament zur Sensibilisierung von Krebszellen gegenüber Antikrebsmitteln und/oder ionisierender Strahlung.

17. Verbindung nach einem der Ansprüche 1 bis 13 und/oder physiologisch unbedenkliches Salz, Tautomer und/oder Stereoisomer davon, einschließlich deren Mischungen in allen Verhältnissen, zur Verwendung bei der Prophylaxe und/oder Therapie von Krebs, Tumoren bzw. Metastasen in Kombination mit Radiotherapie und/oder mit mindestens einem Antikrebsmittel.

18. Arzneimittel umfassend mindestens eine Verbindung nach einem der Ansprüche 1 bis 13 und/oder physiologisch unbedenkliches Salz, Tautomer und/oder Stereoisomer davon, einschließlich deren Mischungen in allen Verhältnissen.

19. Pharmazeutische Zusammensetzung umfassend als Wirkstoff eine wirksame Menge von mindestens einer Verbindung nach einem der Ansprüche 1 bis 13 und/oder physiologisch unbedenklichem Salz, Tautomer und/oder Stereoisomer davon, einschließlich deren Mischungen in allen Verhältnissen, zusammen mit pharmazeutisch verträglichen Hilfsstoffen.

20. Pharmazeutische Zusammensetzung umfassend als Wirkstoff eine wirksame Menge von mindestens einer Verbindung nach einem der Ansprüche 1 bis 13 und/oder physiologisch unbedenklichem Salz, Tautomer und/oder Stereoisomer davon, einschließlich deren Mischungen in allen Verhältnissen, zusammen mit pharmazeutisch verträglichen Hilfsstoffen in Kombination mit einer wirksamen Menge mindestens eines Antikrebsmittels.

21. Kit, bestehend aus getrennten Packungen von (a) einer wirksamen Menge von mindestens einer Verbindung nach einem der Ansprüche 1 bis 13 und/oder physiologisch unbedenklichem Salz, Tautomer und/oder Stereoisomer davon, einschließlich deren Mischungen in allen Verhältnissen, und (b) einer wirksamen Menge mindestens eines Antikrebsmittels.

22. Verbindung nach einem der Ansprüche 1 bis 13 und/oder physiologisch unbedenkliches Salz, Tautomer und/oder Stereoisomer davon, einschließlich deren Mischungen in allen Verhältnissen, zur Verwendung nach Anspruch 16 oder 17, wobei das Antikrebsmittel ausgewählt ist aus: Alkylierungsagentien, umfassend Altretamin, Bendamustin, Busulfan, Carmustin, Chlorambucil, Chlormethine, Cyclophosphamid, Dacarbazin, Ifosfamid, Improsulfantosylat, Lomustin, Melphalan, Mitobronitol, Mitolactol, Nimustin, Ranimustin, Temozolomid, Thiotepa, Treosulfan, Mechloretamin, Carboquon, Apaziquon, Fotemustin, Glufosfamid, Palifosfamid, Pipobroman, Trofosfamid, Uramustin; Platinverbindungen, umfassend Carboplatin, Cisplatin, Eptaplatin, Miriplatinhydrat, Oxaliplatin, Lobaplatin, Nedaplatin, Picoplatin, Satraplatin; Topoisomerase-Inhibitoren, umfassend Etoposid, Irinotecan, Razoxan, Sobuzoxan, DNA-verändernden Agentien, wie Amrubicin, Bisantren, Decitabin, Mitoxantron, Procarbazin, Trabectedin, Clofarabin, Amsacrin, Brostallicin, Pixantron, Laromustine; Antikrebs-Antibiotica, umfassend Bleomycin, Dactinomycin, Doxorubicin, Epirubicin, Idarubicin, Levamisol, Miltefosin, Mitomycin C,Romidepsin, Streptozocin, Valrubicin, Zinostatin, Zorubicin, Daunurobicin, Plicamycin, Aclarubicin, Peplomycin, Pirarubicin; Alpha-Emittern, umfassned Alpharadin (²²³Ra Dichlorid, Xofgio), ²¹¹At, ²¹³Bi, ²²⁵Ac, ²²⁷Th.

23. Pharmazeutische Zusammensetzung nach Anspruch 20, wobei das mindestens eine Antikrebsmittel ausgewählt ist aus: Alkylierungsagentien, umfassend Altretamin, Bendamustin, Busulfan, Carmustin, Chlorambucil, Chlormethine, Cyclophosphamid, Dacarbazin, Ifosfamid, Improsulfantosylat, Lomustin, Melphalan, Mitobronitol, Mitolactol, Nimustin, Ranimustin, Temozolomid, Thiotepa, Treosulfan, Mechloretamin, Carboquon, Apaziquon, Fotemustin, Glufosfamid, Palifosfamid, Pipobroman, Trofosfamid, Uramustin; Platinverbindungen, umfassend Carboplatin, Cisplatin, Eptaplatin, Miriplatinhydrat, Oxaliplatin, Lobaplatin, Nedaplatin, Picoplatin, Satraplatin; Topoisomerase-Inhibitoren, umfassend Etoposid, Irinotecan, Razoxan, Sobuzoxan, DNA-verändernden Agentien, wie Amrubicin, Bisantren, Decitabin, Mitoxantron, Procarbazin, Trabectedin, Clofarabin, Amsacrin, Brostallicin, Pixantron, Laromustine; Antikrebs-Antibiotica, umfassend Bleomycin, Dactinomycin, Doxorubicin, Epirubicin, Idarubicin, Levamisol, Miltefosin, Mitomycin C,Romidepsin, Streptozocin, Valrubicin, Zinostatin, Zorubicin, Daunurobicin, Plicamycin, Aclarubicin, Peplomycin, Pirarubicin; Alpha-Emittern, umfassned Alpharadin (²²³Ra Dichlorid, Xofgio), ²¹¹At, ²¹³Bi, ²²⁵Ac, ²²⁷Th.

24. Kit nach Anspruch 21, wobei das mindestens eine Antikrebsmittel ausgewählt ist aus: Alkylierungsagentien, umfassend Altretamin, Bendamustin, Busulfan, Carmustin, Chlorambucil, Chlormethine, Cyclophosphamid, Dacarbazin, Ifosfamid, Improsulfantosylat, Lomustin, Melphalan, Mitobronitol, Mitolactol, Nimustin, Ranimustin, Temozolomid, Thiotepa, Treosulfan, Mechloretamin, Carboquon, Apaziquon, Fotemustin, Glufosfamid, Palifosfamid, Pipobroman, Trofosfamid, Uramustin; Platinverbindungen, umfassend Carboplatin, Cisplatin, Eptaplatin, Miriplatinhydrat, Oxaliplatin, Lobaplatin, Nedaplatin, Picoplatin, Satraplatin; Topoisomerase-Inhibitoren, umfassend Etoposid, Irinotecan, Razoxan, Sobuzoxan, DNA-verändernden Agentien, wie Amrubicin, Bisantren, Decitabin, Mitoxantron, Procarbazin, Trabectedin, Clofarabin, Amsacrin, Brostallicin, Pixantron, Laromustine; Antikrebs-Antibiotica, umfassend Bleomycin, Dactinomycin, Doxorubicin, Epirubicin, Idarubicin, Levamisol, Miltefosin, Mitomycin C,Romidepsin, Streptozocin, Valrubicin, Zinostatin, Zorubicin, Daunurobicin, Plicamycin, Aclarubicin, Peplomycin, Pirarubicin; Alpha-Emittern, umfassned Alpharadin (²²³Ra Dichlorid, Xofgio), ²¹¹At, ²¹³Bi, ²²⁵Ac, ²²⁷Th.

## Claims

1. Compound of the formula (I) 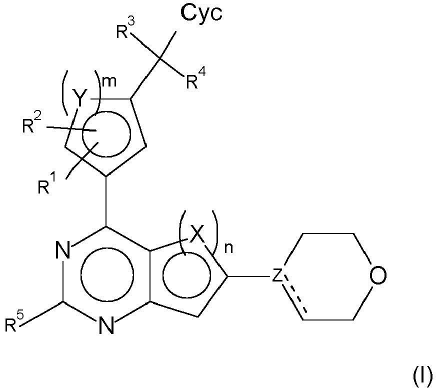 in which
X is CH, CF, S or N,
Y is CH, S or N,
Z is C or N,
---- forms, if Z = C, a double bond together with the single bond, is absent if Z = N,
n is 1 or 2, where
if n = 1, X = S,
and if n = 2, both X = CH, or the X linked to the pyrimidine ring is CF and the X not linked to the pyrimidine ring is CH, or one X is CH and the other X is N;
m is 1 or 2, where
if m = 1, Y = S,
and if m = 2, both Y = CH, or one Y is CH and the other Y is N;
R¹, R², R³, R⁴, independently of one another, are H, Hal, CN, OH, CONH₂, CONH(LA)
or LA;
R⁵ is H, Hal, CN or C≡CH;
Cyc is phenyl, which may be unsubstituted or mono- or disubstituted, independently of one another, by R⁶, or is Het¹;
Het¹ is a mono- or bicyclic, 5-10-membered heterocycle, having 1-3 N, O and/or S atoms, or 1-4 N atoms, which may be unsubstituted or mono-, di- or trisubstituted, independently of one another, by R⁶, or may be monosubstituted by Het²;
R⁶ is Hal, LA, oxo, CN, or NH₂;
LA is unbranched or branched alkyl having 1-5 C atoms, which may be saturated or partially unsaturated, in which 1-3 H atoms may be replaced by Hal, and/or one H atom may be replaced by CN or Het², and/or one or two CH₂ groups may be replaced by O, NH, NH₂, N(CH₃) or CO;
Het² is a 3-5-membered aliphatic homo- or heterocycle having 0, 1, 2 or 3 N, O and/or S atoms, which is unsubstituted;
Hal is F, Cl, Br or I;
where H, C, N, O, P, S, F and Cl also include the heavier isotopes of these atoms,
and/or physiologically acceptable salt, tautomer and/or stereoisomer thereof,
including mixtures thereof in all ratios.

2. Compound according to Claim 1 which conforms to the formula (Ib), 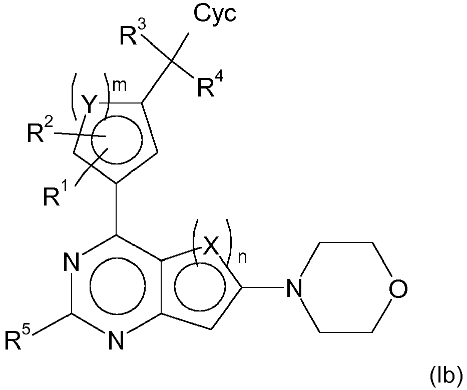 in which all substituents have the meaning indicated for the formula (I),
and/or physiologically acceptable salt, tautomer and/or stereoisomer thereof, including mixtures thereof in all ratios.

3. Compound according to Claim 1 or 2 which conforms to the formula (II) 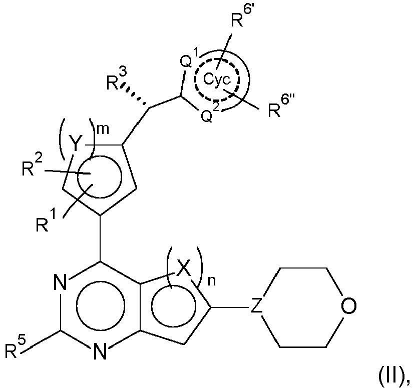 in which
R³ is Hal, CN, OH, CONH₂, CONH(LA) or LA;
R^{6'}, R^{6"}, independently of one another, are H, Hal, LA, oxo, CN, NH₂ or Het²;
Q¹, Q², independently of one another, are CH, N or NH and are in each case unsubstituted;
---- denotes the presence or absence of double bonds in Cyc;
and the other substituents have the meaning indicated for the formula (I),
and/or physiologically acceptable salt, tautomer and/or stereoisomer thereof, including mixtures thereof in all ratios.

4. Compound according to Claim 1 or 2 which conforms to the formula (III) 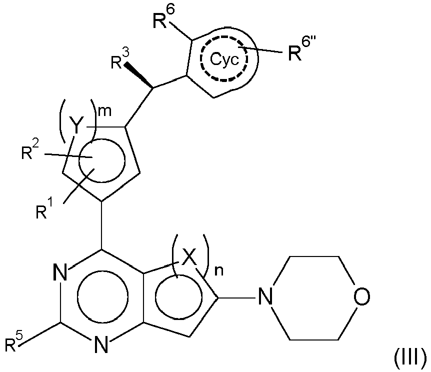 in which
R³ is Hal, CN, OH, CONH₂, CONH(LA) or LA;
R⁶ is Hal, LA, oxo, CN, NH₂ or Het²;
R^{6"} is H, Hal, LA, oxo, CN, NH₂ or Het²;
---- denotes the presence or absence of double bonds in Cyc;
and the other substituents have the meaning indicated for the formula (I), and/or physiologically acceptable salt, tautomer and/or stereoisomer thereof, including mixtures thereof in all ratios.

5. Compound according to Claim 3 which conforms to the formula (IIa) 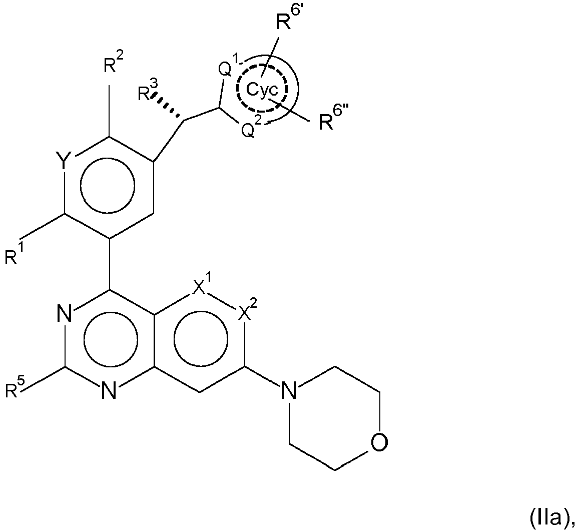 in which
R², R³, independently of one another, are Hal, CN, OH, CONH₂, CON(LA) or LA;
R^{6'}, R^{6"}, independently of one another, are H, Hal, LA, oxo, CN, NH₂ or Het²;
Q¹, Q², independently of one another, are CH, N or NH and are in each case unsubstituted;
X¹ is CH, CF or N;
X² is CH or N,
where X¹, X² are not simultaneously N;
Y is CH or N;
---- denotes the presence or absence of double bonds in Cyc;
and the other substituents have the meaning indicated for the formula (I),
and/or physiologically acceptable salt, tautomer and/or stereoisomer thereof, including mixtures thereof in all ratios.

6. Compound according to Claim 3 which conforms to the formula (IIb) 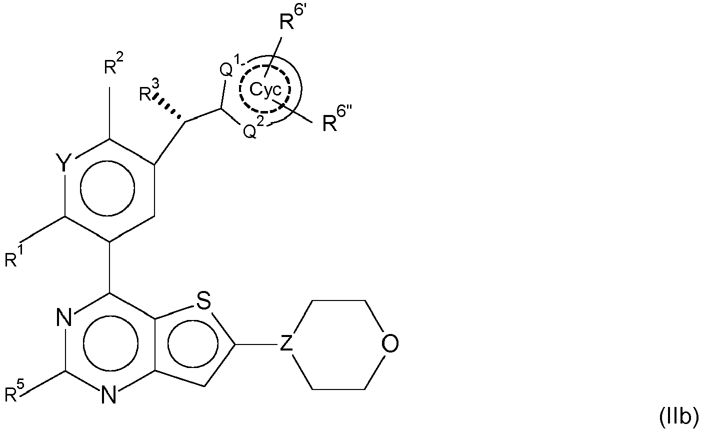 in which
R², R³, independently of one another, are Hal, CN, OH, CONH₂, CON(LA) or LA;
R^{6'}, R^{6"}, independently of one another, are H, Hal, LA, oxo, CN, NH₂ or Het²;
Q¹, Q², independently of one another, are CH, N or NH and are in each case unsubstituted;
Y is CH or N,
---- denotes the presence or absence of double bonds in Cyc;
and all other substituents have the meaning indicated for the formula (I),
and/or physiologically acceptable salt, tautomer and/or stereoisomer thereof, including mixtures thereof in all ratios.

7. Compound according to Claim 4 which conforms to the formula (IIIa) 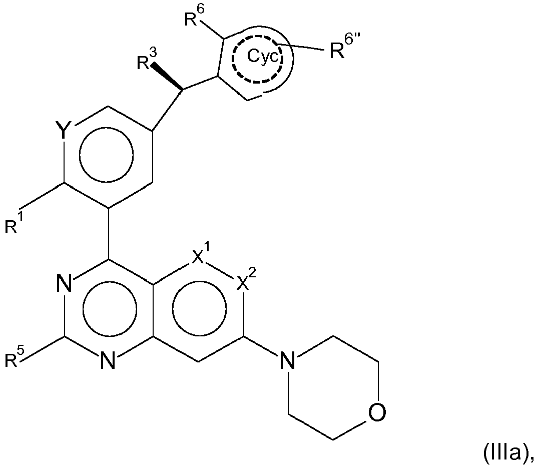 in which
R³ is Hal, CN, OH, CONH₂, CON(LA) or LA;
R⁶ is Hal, LA, oxo, CN, NH₂ or Het²;
R^{6"} is H, Hal, LA, oxo, CN, NH₂ or Het²;
X¹ is CH, CF or N;
X² is CH or N,
where X¹, X² are not simultaneously N;
Y is CH or N;
---- denotes the presence or absence of double bonds in Cyc;
and the other substituents have the meaning indicated for the formula (I),
and/or physiologically acceptable salt, tautomer and/or stereoisomer thereof, including mixtures thereof in all ratios.

8. Compound according to Claim 4 which conforms to the formula (IIIb) 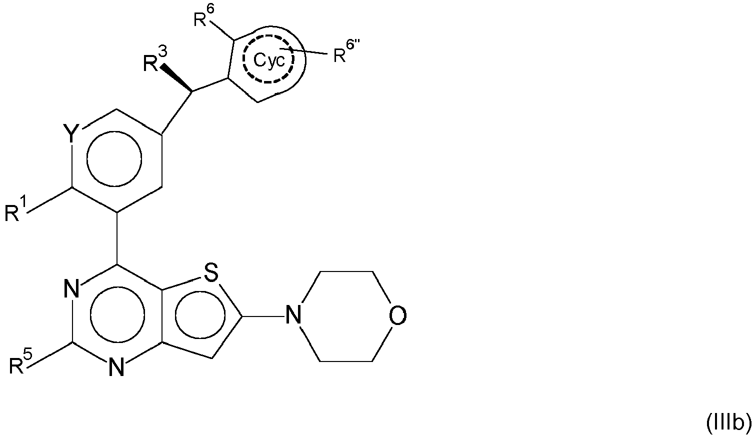 in which
R³ is Hal, CN, OH, CONH₂, CON(LA) or LA;
R⁶ is Hal, LA, oxo, CN, NH₂ or Het²;
R^{6"} is H, Hal, LA, oxo, CN, NH₂ or Het²;
Y is CH or N,
---- denotes the presence or absence of double bonds in Cyc;
and all other substituents have the meaning indicated for the formula (I),
and/or physiologically acceptable salt, tautomer and/or stereoisomer thereof, including mixtures thereof in all ratios.

9. Compound according to Claim 5, in which the radicals not designated in greater detail have the meaning indicated for the formula (IIa), but in which
in the case of the sub-formula (IIa-A)
X¹ is CH,
R¹ is F or Cl,
R² is F or Cl,
in the case of the sub-formula (IIa-B)
R¹ is F,
R² is F or Cl,
in the case of the sub-formula (IIa-C)
X¹, X² are CH,
in the case of the sub-formula (IIa-D)
X¹ is CH,
R⁵ is H,
in the case of the sub-formula (IIa-E)
R³ is H, OH,
in the case of the sub-formula (IIa-F)
X¹ is CH,
R³ is OH,
in the case of the sub-formula (IIa-G)
X¹ is CH,
Y is CH,
in the case of the sub-formula (IIa-H)
X¹ is CH,
Cyc is pyridine, pyrazine or pyridazine, or pyrazolo[1,5-a]pyrimidinyl or imidazo[1,2-b]-pyridazinyl,
in the case of the sub-formula (IIa-J)
Cyc is pyridine, pyrazine, pyridazine, pyrazolo[1,5-a]pyrimidinyl, imidazo[1,2-b]pyridazinyl, furo[2,3-c]pyridinyl, furo[2,3-d]pyridazinyl, thieno[2,3-d]pyridazinyl, thieno[2,3-d]-pyrimidinyl or imidazo[4,5-c]pyridinyl, each of which may be unsubstituted, or may be mono- or disubstituted by methoxy, methyl, oxo, Cl or CHF₂O,
in the case of the sub-formula (IIa-K)
R¹ is F or Cl,
R² is F or Cl,
R³ is OH,
R⁵ is H,
X¹, X² are CH,
in the case of the sub-formula (IIa-L)
R¹ is F,
R² is F or Cl,
R³ is H or OH,
R⁵ is H,
in the case of the sub-formula (IIa-M)
R¹ is F or Cl,
R² is F or Cl,
R³ is OH,
R⁵ is H,
X¹, X² are CH,
Cyc is pyridine, pyrazine or pyridazine, or pyrazolo[1,5-a]pyrimidinyl or imidazo[1,2-b]-pyridazinyl,
in the case of the sub-formula (IIa-N)
R¹ is F,
R² is F or Cl,
R³ is H or OH,
R⁵ is H,
Cyc is pyridine, pyrazine, pyridazine, pyrazolo[1,5-a]pyrimidinyl, imidazo[1,2-b]pyridazinyl, furo[2,3-c]pyridinyl, furo[2,3-d]pyridazinyl, thieno[2,3-d]pyridazinyl, thieno[2,3-d]-pyrimidinyl or imidazo[4,5-c]pyridinyl, each of which may be unsubstituted, or may be mono- or disubstituted by methoxy, methyl, oxo, Cl or CHF₂O,
in the case of the sub-formula (IIa-O)
R¹ is F,
R² is F or Cl,
R³ is H or OH,
R⁵ is H,
Cyc is 5-methoxypyridazin-3-yl, imidazo[1,2-b]pyridazin-6-yl, 3-chloro-6-methoxypyrazin-2-yl, 3-chloropyrazin-2-yl, pyridazin-4-yl, 3-methoxypyrazin-2-yl, 6-methoxypyridazin-3-yl, 3-difluoromethoxypyridin-2-yl, 3-methylpyrazin-2-yl, thieno[2,3-d]pyrimidin-4-yl, 1-methyl-1H-pyridin-2-on-6-yl, 1H-pyridazin-6-on-3-yl, furo[2,3-d]pyridazin-7-yl, thieno[2,3-d]pyridazin-7-yl, 3,5-dimethylpyrazin-2-yl, furo[2,3-d]pyrimidin-4-yl, 3-methyl-3H-imidazo[4,5-c]pyridin-4-yl,
and/or physiologically acceptable salt, tautomer and/or stereoisomer thereof, including mixtures thereof in all ratios.

10. Compound according to Claim 7, in which the radicals not designated in greater detail have the meaning indicated for the formula (IIIa), but in which
in the case of the sub-formula (IIIa-B)
R¹ is F,
in the case of the sub-formula (IIIa-C)
X¹, X² are CH,
in the case of the sub-formula (IIIa-D)
X¹ is CH,
R⁵ is H,
in the case of the sub-formula (IIIa-E)
R³ is H, OH,
in the case of the sub-formula (IIIa-F)
X¹ is CH,
R³ is OH,
in the case of the sub-formula (IIIa-G)
X¹ is CH,
Y is CH,
in the case of the sub-formula (IIIa-H)
X¹ is CH,
Cyc is pyridine, pyrazine or pyridazine, or pyrazolo[1,5-a]pyrimidinyl or imidazo[1,2-b]-pyridazinyl,
in the case of the sub-formula (IIIa-J)
Cyc is pyridine, pyrazine, pyridazine, pyrazolo[1,5-a]pyrimidinyl, imidazo[1,2-b]pyridazinyl, furo[2,3-c]pyridinyl, furo[2,3-d]pyridazinyl, thieno[2,3-d]pyridazinyl, thieno[2,3-d]-pyrimidinyl or imidazo[4,5-c]pyridinyl, each of which may be unsubstituted, or may be mono- or disubstituted by methoxy, methyl, oxo, Cl or CHF₂O,
in the case of the sub-formula (IIIa-K)
R¹ is F or Cl,
R³ is OH,
R⁵ is H,
X¹, X² are CH,
in the case of the sub-formula (IIIa-L)
R¹ is F,
R³ is H or OH,
R⁵ is H,
in the case of the sub-formula (IIIa-M)
R¹ is F or Cl,
R³ is OH,
R⁵ is H,
X¹, X² are CH,
Cyc is pyridine, pyrazine or pyridazine, or pyrazolo[1,5-a]pyrimidinyl or imidazo[1,2-b]-pyridazinyl,
in the case of the sub-formula (IIIa-N)
R¹ is F,
R³ is H or OH,
R⁵ is H,
Cyc is pyridine, pyrazine, pyridazine, pyrazolo[1,5-a]pyrimidinyl, imidazo[1,2-b]pyridazinyl, furo[2,3-c]pyridinyl, furo[2,3-d]pyridazinyl, thieno[2,3-d]pyridazinyl, thieno[2,3-d]-pyrimidinyl or imidazo[4,5-c]pyridinyl, each of which may be unsubstituted, or may be mono- or disubstituted by methoxy, methyl, oxo, Cl or CHF₂O,
in the case of the sub-formula (IIIa-O)
R¹ is F,
R³ is H or OH,
R⁵ is H,
Cyc is 5-methoxypyridazin-3-yl, imidazo[1,2-b]pyridazin-6-yl, 3-chloro-6-methoxypyrazin-2-yl, 3-chloropyrazin-2-yl, pyridazin-4-yl, 3-methoxypyrazin-2-yl, 6-methoxypyridazin-3-yl, 3-difluoromethoxypyridin-2-yl, 3-methylpyrazin-2-yl, thieno[2,3-d]pyrimidin-4-yl, 1-methyl-1H-pyridin-2-one-6-yl, 1H-pyridazin-6-one-3-yl, furo[2,3-d]pyridazin-7-yl, thieno[2,3-d]pyridazin-7-yl, 3,5-dimethylpyrazin-2-yl, furo[2,3-d]pyrimidin-4-yl, 3-methyl-3H-imidazo[4,5-c]pyridin-4-yl,
and/or physiologically acceptable salt, tautomer and/or stereoisomer thereof, including mixtures thereof in all ratios.

11. Compound according to Claim 6, in which the radicals not designated in greater detail have the meaning indicated for the formulae (IIb), but in which
in the case of the sub-formula (IIb-Q)
R¹ is F or Cl,
R² is F or Cl,
R³ is OH,
R⁵ is H,
Y is CH,
in the case of the sub-formula (IIb-R)
R¹ is F,
R² is F or Cl,
R³ is OH,
R⁵ is H,
Y is CH,
in the case of the sub-formula (IIb-S)
Cyc is pyridine, pyrazine or pyridazine,
in the case of the sub-formula (IIb-T)
R¹ is F or Cl,
R² is F or Cl,
R³ is OH,
R⁵ is H,
Cyc is pyridine, pyrazine or pyridazine,
in the case of the sub-formula (IIb-U)
R¹ is F,
R² is F or Cl,
R³ is OH,
R⁵ is H,
Cyc is pyridine, pyrazine, pyridazine or 3-methylpyrazin-2-yl,
and/or physiologically acceptable salt, tautomer and/or stereoisomer thereof, including mixtures thereof in all ratios.

12. Compound according to Claim 8, in which the radicals not designated in greater detail have the meaning indicated for the formulae (IIIb), but in which
in the case of the sub-formula (IIIb-Q)
R¹ is F or Cl,
R³ is OH,
R⁵ is H,
Y is CH,
in the case of the sub-formula (IIIb-R)
R¹ is F,
R³ is OH,
R⁵ is H,
Y is CH,
in the case of the sub-formula (IIIb-S)
Cyc is pyridine, pyrazine or pyridazine,
in the case of the sub-formula (IIIb-T)
R¹ is F or Cl,
R³ is OH,
R⁵ is H,
Cyc is pyridine, pyrazine or pyridazine,
in the case of the sub-formula (IIIb-U)
R¹ is F,
R³ is OH,
R⁵ is H,
Cyc is pyridine, pyrazine, pyridazine or 3-methylpyrazin-2-yl,
and/or physiologically acceptable salt, tautomer and/or stereoisomer thereof, including mixtures thereof in all ratios.

13. Compound according to one of Claims 1 to 12, selected from the group:
[2-chloro-4-fluoro-5-(7-morpholin-4-ylquinazolin-4-yl)phenyl]-(5-methoxypyridazin-3-yl)-methanol,
[4-fluoro-3-(7-morpholin-4-ylquinazolin-4-yl)phenyl]-(5-methoxypyridazin-3-yl)methanol, [2,4-difluoro-5-(7-morpholin-4-ylquinazolin-4-yl)phenyl]imidazo[1,2-b]pyridazin-6-ylmethanol,
(3-chloro-6-methoxypyrazin-2-yl)-[4-fluoro-3-(7-morpholin-4-ylquinazolin-4-yl)phenyl]-methanol,
(R)-(3-chloropyrazin-2-yl)-[4-fluoro-3-(7-morpholin-4-ylquinazolin-4-yl)phenyl]methanol,
[2-chloro-4-fluoro-5-(7-morpholin-4-ylquinazolin-4-yl)phenyl]pyridazin-4-ylmethanol,
[4-fluoro-3-(7-morpholin-4-ylquinazolin-4-yl)phenyl]-(3-methoxypyrazin-2-yl)methanol,
[4-fluoro-3-(7-morpholin-4-ylquinazolin-4-yl)phenyl]-(6-methoxypyridazin-3-yl)methanol,
(3-difluoromethoxypyridin-2-yl)-[4-fluoro-3-(7-morpholin-4-ylquinazolin-4-yl)phenyl]methanol,
(R)-[4-fluoro-3-(7-morpholin-4-ylquinazolin-4-yl)phenyl]-(3-methylpyrazin-2-yl)methanol,
[2,4-difluoro-5-(7-morpholin-4-ylquinazolin-4-yl)phenyl]-(3-methylpyrazin-2-yl)methanol,
[2,4-difluoro-5-(7-morpholin-4-ylquinazolin-4-yl)phenyl]thieno[2,3-d]pyrimidin-4-ylmethanol,
6-{[4-fluoro-3-(7-morpholin-4-ylquinazolin-4-yl)phenyl]hydroxymethyl}-1-methyl-1H-pyridin-2-one,
3-[[2-chloro-4-fluoro-5-(7-morpholinoquinazolin-4-yl)phenyl]hydroxymethyl]-1H-pyridazin-6-one,
(S)-[2-chloro-4-fluoro-5-(7-morpholin-4-ylquinazolin-4-yl)phenyl]-(6-methoxypyridazin-3-yl)-methanol,
(R)-[4-fluoro-3-(7-morpholin-4-ylpyrido[3,2-d]pyrimidin-4-yl)phenyl]-(3-methylpyrazin-2-yl)-methanol,
4-(4-chloro-2-fluoro-5-imidazo[1,2-b]pyridazin-6-ylmethylphenyl)-7-morpholin-4-ylquinazoline, [4-fluoro-3-(6-morpholin-4-ylthieno[3,2-d]pyrimidin-4-yl)phenyl]-(3-methylpyrazin-2-yl)-methanol,
(R)-[4-fluoro-3-(5-fluoro-7-morpholin-4-ylquinazolin-4-yl)phenyl]-(3-methylpyrazin-2-yl)-methanol,
[2-chloro-4-fluoro-5-(5-fluoro-7-morpholin-4-ylquinazolin-4-yl)phenyl]-(3-methoxypyrazin-2-yl)methanol,
[4-fluoro-3-(5-fluoro-7-morpholin-4-ylquinazolin-4-yl)phenyl]-(3-methoxypyrazin-2-yl)-methanol,
[4-fluoro-3-(5-fluoro-7-morpholin-4-ylquinazolin-4-yl)phenyl]-(6-methoxypyridazin-3-yl)-methanol,
[4-fluoro-3-[7-(2,2,3,3,5,5,6,6-octadeuteriomorpholin-4-yl)quinazolin-4-yl]phenyl]-(3-methyl-pyrazin-2-yl)methanol,
[4-fluoro-3-(5-fluoro-7-morpholin-4-ylquinazolin-4-yl)phenyl]-(6-methoxypyridazin-3-yl)-methanol,
[2-chloro-4-fluoro-5-[7-(2,2,3,3,5,5,6,6-octadeuteriomorpholin-4-yl)quinazolin-4-yl]phenyl]-(6-methoxypyridazin-3-yl)methanol,
[2-chloro-4-fluoro-5-(6-morpholin-4-ylthieno[3,2-d]pyrimidin-4-yl)phenyl]-(3-methylpyrazin-2-yl)methanol,
[4-fluoro-3-(6-morpholin-4-ylthieno[3,2-d]pyrimidin-4-yl)phenyl]-(3-methylpyrazin-2-yl)-methanol,
[2-chloro-4-fluoro-5-(5-fluoro-7-morpholin-4-ylquinazolin-4-yl)phenyl]-(3-methoxypyrazin-2-yl)methanol,
[4-fluoro-3-(5-fluoro-7-morpholin-4-ylquinazolin-4-yl)phenyl]-(3-methoxypyrazin-2-yl)-methanol,
[4-fluoro-3-(5-fluoro-7-morpholin-4-ylquinazolin-4-yl)phenyl]-(3-methylpyrazin-2-yl)methanol,
[2-chloro-4-fluoro-5-(5-fluoro-7-morpholin-4-ylquinazolin-4-yl)phenyl]-(6-methoxypyridazin-3-yl)methanol,
[4- fluoro-3-(7-morpholin-4-ylquinazolin-4-yl)phenyl]furo[2,3-d]pyridazin-7-ylmethanol,
[2,4-difluoro-5-(7-morpholin-4-ylquinazolin-4-yl)phenyl]furo[2,3-d]pyridazin-7-ylmethanol,
[2,4-difluoro-5-(7-morpholin-4-ylquinazolin-4-yl)phenyl]thieno[2,3-d]pyridazin-7-ylmethanol,
(3,5-dimethylpyrazin-2-yl)-[4-fluoro-3-(7-morpholin-4-ylquinazolin-4-yl)phenyl]methanol,
6-{[2-chloro-5-(7-morpholin-4-ylquinazolin-4-yl)phenyl]hydroxymethyl}-1-methyl-1H-pyridin-2-one,
6-{[4-fluoro-3-(7-morpholin-4-ylquinazolin-4-yl)phenyl]hydroxymethyl}-2H-pyridazin-3-one,
6-{[4-fluoro-3-(7-morpholin-4-ylquinazolin-4-yl)phenyl]hydroxymethyl}-1-methyl-1H-pyridin-2-one,
6-{[2-chloro-4-fluoro-5-(7-morpholin-4-ylquinazolin-4-yl)phenyl]hydroxymethyl}-1-methyl-1H-pyridin-2-one,
[4-fluoro-3-(7-morpholin-4-ylquinazolin-4-yl)phenyl]furo[2,3-d]pyrimidin-4-ylmethanol,
[4-fluoro-3-(7-morpholin-4-ylquinazolin-4-yl)phenyl]furo[3,2-d]pyrimidin-4-ylmethanol,
[2,4-difluoro-5-(7-morpholin-4-ylquinazolin-4-yl)phenyl]-(3-methoxypyrazin-2-yl)methanol,
4-fluoro-3-(7-morpholin-4-ylquinazolin-4-yl)phenyl]-(3-methyl-3H-imidazo[4,5-c]pyridin-4-yl)-methanol,
[4-fluoro-3-(7-morpholin-4-ylquinazolin-4-yl)phenyl]furo[3,2-d]pyrimidin-4-ylmethanol,
and/or physiologically acceptable salt, tautomer and/or stereoisomer thereof, including mixtures thereof in all ratios.

14. Process for the preparation of a compound of the formula (I) according to Claim 1 and/or physiologically acceptable salt, tautomer and/or stereoisomer thereof having the following steps:
(a) reaction of a compound of the formula (V) 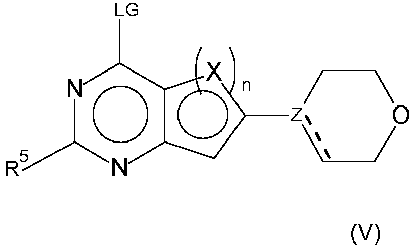 in which LG is a conventional leaving group, such as Hal,
with a compound of the formula (IV) 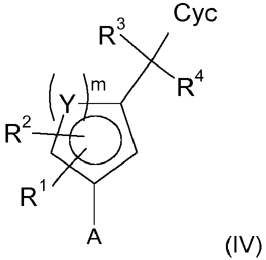 in which A is boronic acid or a boronic acid ester,
giving the compound of the formula (I), and optionally
(b) conversion of a base or acid of the compound of the formula (I) into one of its salts.

15. Compound according to one of Claims 1 to 13 and/or physiologically acceptable salt, tautomer and/or stereoisomer thereof, including mixtures thereof in all ratios, for use as medicament.

16. Compound according to one of Claims 1 to 13 and/or physiologically acceptable salt, tautomer and/or stereoisomer thereof, including mixtures thereof in all ratios, for use as medicament for the sensitisation of cancer cells to anticancer agents and/or ionising radiation.

17. Compound according to one of Claims 1 to 13 and/or physiologically acceptable salt, tautomer and/or stereoisomer thereof, including mixtures thereof in all ratios, for use in the prophylaxis and/or therapy of cancer, tumours or metastases in combination with radiotherapy and/or with at least one anticancer agent.

18. Medicament comprising at least one compound according to one of Claims 1 to 13 and/or physiologically acceptable salt, tautomer and/or stereoisomer thereof, including mixtures thereof in all ratios.

19. Pharmaceutical composition comprising, as active compound, an effective amount of at least one compound according to one of Claims 1 to 13 and/or physiologically acceptable salt, tautomer and/or stereoisomer thereof, including mixtures thereof in all ratios, together with pharmaceutically tolerated adjuvants.

20. Pharmaceutical composition comprising, as active compound, an effective amount of at least one compound according to one of Claims 1 to 13 and/or physiologically acceptable salt, tautomer and/or stereoisomer thereof, including mixtures thereof in all ratios, together with pharmaceutically tolerated adjuvants in combination with an effective amount of at least one anticancer agent.

21. Kit, consisting of separate packs of (a) an effective amount of at least one compound according to one of Claims 1 to 13 and/or physiologically acceptable salt, tautomer and/or stereoisomer thereof, including mixtures thereof in all ratios, and (b) an effective amount of at least one anticancer agent.

22. Compound according to one of Claims 1 to 13 and/or physiologically acceptable salt, tautomer and/or stereoisomer thereof, including mixtures thereof in all ratios, for use according to Claim 16 or 17, where the anticancer agent is selected from: alkylating agents, including altretamine, bendamustine, busulfan, carmustine, chlorambucil, chlormethine, cyclophosphamide, dacarbazine, ifosfamide, improsulfan tosylate, lomustine, melphalan, mitobronitol, mitolactol, nimustine, ranimustine, temozolomide, thiotepa, treosulfan, mechlorethamine, carboquone, apaziquone, fotemustine, glufosfamide, palifosfamide, pipobroman, trofosfamide, uramustine; platinum compounds, including carboplatin, cisplatin, eptaplatin, miriplatin hydrate, oxaliplatin, lobaplatin, nedaplatin, picoplatin, satraplatin; topoisomerase inhibitors, including etoposide, irinotecan, razoxane, sobuzoxane; DNA-modifying agents, including amrubicin, bisantrene, decitabine, mitoxantrone, procarbazine, trabectedine, clofarabine, amsacrine, brostallicin, pixantrone, laromustine; anticancer antibiotics, including bleomycin, dactinomycin, doxorubicin, epirubicin, idarubicin, levamisol, miltefosine, mitomycin C, romidepsin, streptozocin, valrubicin, zinostatin, zorubicin, daunurobicin, plicamycin, aclarubicin, peplomycin, pirarubicin; alpha emitters, including alpharadin (²²³Ra dichloride, Xofigo), ²¹¹At, ²¹³Bi, ²²⁵Ac, ²²⁷Th.

23. Pharmaceutical composition according to Claim 20, where the at least one anticancer agent is selected from: alkylating agents, including altretamine, bendamustine, busulfan, carmustine, chlorambucil, chlormethine, cyclophosphamide, dacarbazine, ifosfamide, improsulfan tosylate, lomustine, melphalan, mitobronitol, mitolactol, nimustine, ranimustine, temozolomide, thiotepa, treosulfan, mechlorethamine, carboquone, apaziquone, fotemustine, glufosfamide, palifosfamide, pipobroman, trofosfamide, uramustine; platinum compounds, including carboplatin, cisplatin, eptaplatin, miriplatin hydrate, oxaliplatin, lobaplatin, nedaplatin, picoplatin, satraplatin; topoisomerase inhibitors, including etoposide, irinotecan, razoxane, sobuzoxane; DNA-modifying agents, including amrubicin, bisantrene, decitabine, mitoxantrone, procarbazine, trabectedine, clofarabine, amsacrine, brostallicin, pixantrone, laromustine; anticancer antibiotics, including bleomycin, dactinomycin, doxorubicin, epirubicin, idarubicin, levamisol, miltefosine, mitomycin C, romidepsin, streptozocin, valrubicin, zinostatin, zorubicin, daunurobicin, plicamycin, aclarubicin, peplomycin, pirarubicin; alpha emitters, including alpharadin (²²³Ra dichloride, Xofigo), ²¹¹At, ²¹³Bi, ²²⁵Ac, ²²⁷Th.

24. Kit according to Claim 21, where the at least one anticancer agent is selected from: alkylating agents, including altretamine, bendamustine, busulfan, carmustine, chlorambucil, chlormethine, cyclophosphamide, dacarbazine, ifosfamide, improsulfan tosylate, lomustine, melphalan, mitobronitol, mitolactol, nimustine, ranimustine, temozolomide, thiotepa, treosulfan, mechlorethamine, carboquone, apaziquone, fotemustine, glufosfamide, palifosfamide, pipobroman, trofosfamide, uramustine; platinum compounds, including carboplatin, cisplatin, eptaplatin, miriplatin hydrate, oxaliplatin, lobaplatin, nedaplatin, picoplatin, satraplatin; topoisomerase inhibitors, including etoposide, irinotecan, razoxane, sobuzoxane; DNA-modifying agents, including amrubicin, bisantrene, decitabine, mitoxantrone, procarbazine, trabectedine, clofarabine, amsacrine, brostallicin, pixantrone, laromustine; anticancer antibiotics, including bleomycin, dactinomycin, doxorubicin, epirubicin, idarubicin, levamisol, miltefosine, mito-mycin C, romidepsin, streptozocin, valrubicin, zinostatin, zorubicin, daunurobicin, plicamycin, aclarubicin, peplomycin, pirarubicin; alpha emitters, including alpharadin (²²³Ra dichloride, Xofigo), ²¹¹At, ²¹³Bi, ²²⁵Ac, ²²⁷Th.

## Revendications

1. Composé de formule (I) 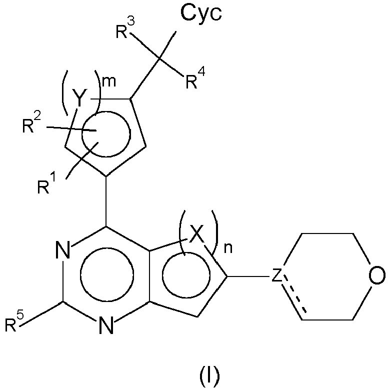 dans lequel
X est CH, CF, S ou N,
Y est CH, S ou N,
Z est C ou N,
---- forme, si Z = C, une double liaison conjointement avec la liaison simple,
est absent si Z = N,
n vaut 1 ou 2, où
si n = 1, X = S,
et si n = 2, les deux X = CH, ou le X lié au cycle pyrimidine est CF et le X non lié au cycle pyrimidine est CH, ou l'un des X est CH et l'autre X est N ;
m vaut 1 ou 2, où
si m = 1, Y = S,
et si m = 2, les deux Y = CH, ou l'un des Y est CH et l'autre Y est N ;
R¹, R², R³, R⁴, indépendamment les uns des autres, sont H, Hal, CN, OH, CONH₂, CONH(LA) ou LA ;
R⁵ est H, Hal, CN ou C≡CH ;
Cyc est phényle, qui peut être non substitué ou mono- ou disubstitué, indépendamment les uns des autres, par R⁶, ou est Hét¹ ;
Hét¹ est un hétérocycle mono- ou bicyclique, de 5-10 chaînons, ayant 1-3 atomes de N, O et/ou S, ou 1-4 atomes de N, qui peut être non substitué ou mono-, di- ou trisubstitué, indépendamment les uns des autres, par R⁶, ou peut être monosubstitué par Hét² ;
R⁶ est Hal, LA, oxo, CN, ou NH₂ ;
LA est alkyle non ramifié ou ramifié ayant 1-5 atomes de C, qui peut être saturé ou partiellement insaturé, où 1-3 atomes de H peuvent être remplacés par Hal, et/ou un atome de H peut être remplacé par CN ou Hét², et/ou un ou deux groupements CH₂ peuvent être remplacés par O, NH, NH₂, N(CH₃) ou CO ;
Hét² est un homo- ou hétérocycle aliphatique de 3-5 chaînons ayant 0, 1, 2 ou 3 atomes de N, O et/ou S, qui est non substitué ;
Hal est F, Cl, Br ou I ;
où H, C, N, O, P, S, F et Cl comportent également les isotopes plus lourds de ces atomes,
et/ou un sel, tautomère et/ou stéréoisomère physiologiquement acceptable de celui-ci, y compris des mélanges de ceux-ci en toutes proportions.

2. Composé selon la revendication 1, qui répond à la formule (Ib), 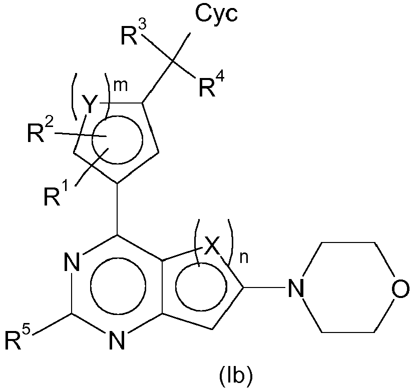 dans lequel tous les substituants revêtent la signification indiquée pour la formule (I), et/ou un sel, tautomère et/ou stéréoisomère physiologiquement acceptable de celui-ci, y compris des mélanges de ceux-ci en toutes proportions.

3. Composé selon la revendication 1 ou 2, qui répond à la formule (II) 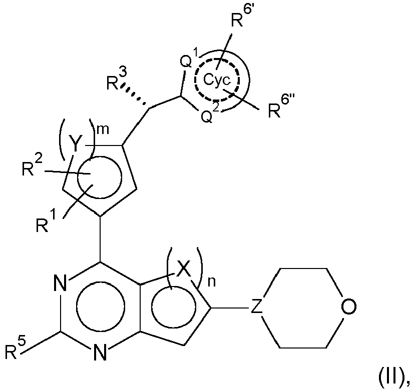 dans lequel
R³ est Hal, CN, OH, CONH₂, CONH(LA) ou LA ;
R^{6'},R^{6"}, indépendamment l'un de l'autre, sont H, Hal, LA, oxo, CN, NH₂ ou Hét² ;
Q¹,Q², indépendamment l'un de l'autre, sont CH, N ou NH et sont dans chaque cas non substitués ;
---- désigne la présence ou l'absence de doubles liaisons dans Cyc ;
et les autres substituants revêtent la signification indiquée pour la formule (I),
et/ou un sel, tautomère et/ou stéréoisomère physiologiquement acceptable de celui-ci, y compris des mélanges de ceux-ci en toutes proportions.

4. Composé selon la revendication 1 ou 2, qui répond à la formule (III) 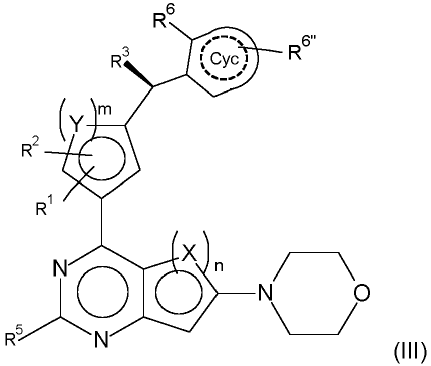 dans lequel
R³ est Hal, CN, OH, CONH₂, CONH(LA) ou LA ;
R⁶ est Hal, LA, oxo, CN, NH₂ ou Hét² ;
R^{6"} est H, Hal, LA, oxo, CN, NH₂ ou Hét² ;
---- désigne la présence ou l'absence de doubles liaisons dans Cyc ;
et les autres substituants revêtent la signification indiquée pour la formule (I), et/ou un sel, tautomère et/ou stéréoisomère physiologiquement acceptable de celui-ci, y compris des mélanges de ceux-ci en toutes proportions.

5. Composé selon la revendication 3, qui répond à la formule (IIa) 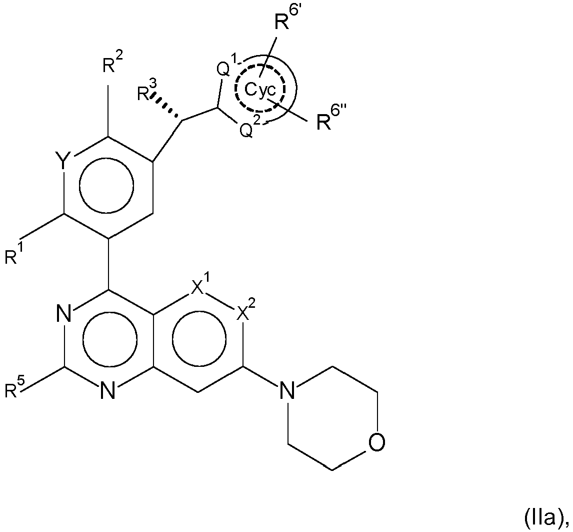 dans lequel
R², R³, indépendamment l'un de l'autre, sont Hal, CN, OH, CONH₂, CON(LA) ou LA ;
R^{6'},R^{6"}, indépendamment l'un de l'autre, sont H, Hal, LA, oxo, CN, NH₂ ou Hét² ;
Q¹,Q², indépendamment l'un de l'autre, sont CH, N ou NH et sont dans chaque cas non substitués ;
X¹ est CH, CF ou N ;
X² est CH ou N,
où X¹, X² ne sont pas simultanément N ;
Y est CH ou N ;
---- désigne la présence ou l'absence de doubles liaisons dans Cyc ;
et les autres substituants revêtent la signification indiquée pour la formule (I),
et/ou un sel, tautomère et/ou stéréoisomère physiologiquement acceptable de celui-ci, y compris des mélanges de ceux-ci en toutes proportions.

6. Composé selon la revendication 3, qui répond à la formule (IIb) 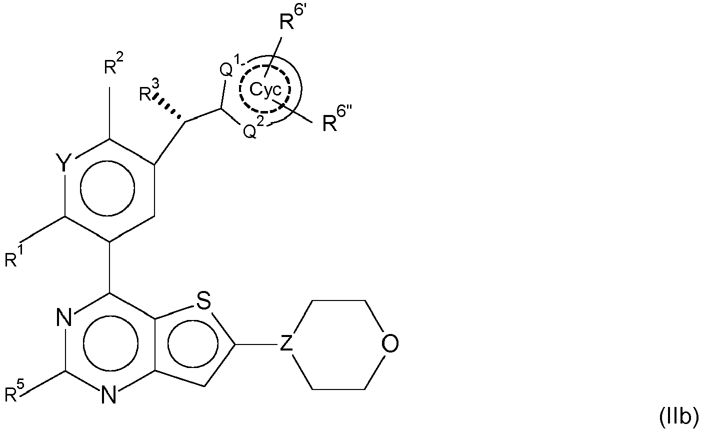 dans lequel
R², R³, indépendamment l'un de l'autre, sont Hal, CN, OH, CONH₂, CON(LA) ou LA ;
R^{6'},R^{6"}, indépendamment l'un de l'autre, sont H, Hal, LA, oxo, CN, NH₂ ou Hét² ;
Q¹,Q², indépendamment l'un de l'autre, sont CH, N ou NH et sont dans chaque cas non substitués ;
Y est CH ou N,
---- désigne la présence ou l'absence de doubles liaisons dans Cyc ;
et tous les autres substituants revêtent la signification indiquée pour la formule (I),
et/ou un sel, tautomère et/ou stéréoisomère physiologiquement acceptable de celui-ci, y compris des mélanges de ceux-ci en toutes proportions.

7. Composé selon la revendication 4, qui répond à la formule (IIIa) 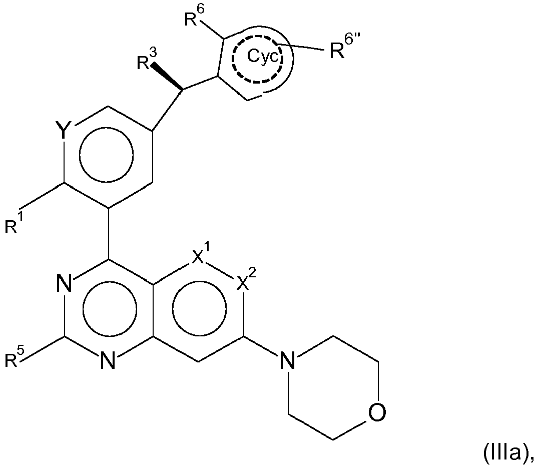 dans lequel
R³ est Hal, CN, OH, CONH₂, CON(LA) ou LA ;
R⁶ est Hal, LA, oxo, CN, NH₂ ou Hét² ;
R^{6"} est H, Hal, LA, oxo, CN, NH₂ ou Hét² ;
X¹ est CH, CF ou N ;
X² est CH ou N, où X¹, X² ne sont pas simultanément N ;
Y est CH ou N ;
---- désigne la présence ou l'absence de doubles liaisons dans Cyc ;
et les autres substituants revêtent la signification indiquée pour la formule (I),
et/ou un sel, tautomère et/ou stéréoisomère physiologiquement acceptable de celui-ci, y compris des mélanges de ceux-ci en toutes proportions.

8. Composé selon la revendication 4, qui répond à la formule (IIIb) 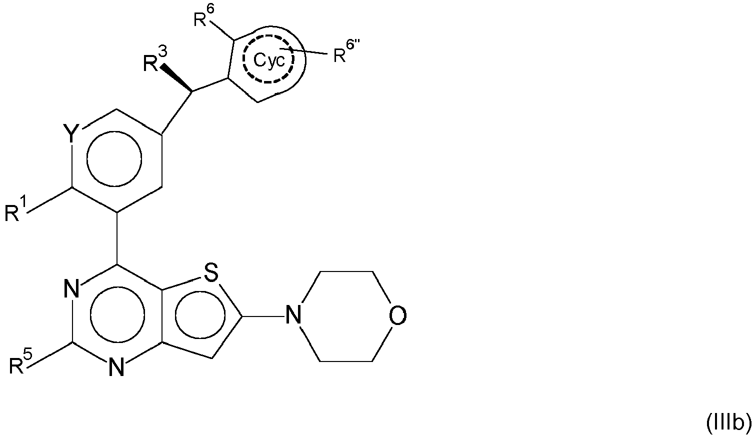 dans lequel
R³ est Hal, CN, OH, CONH₂, CON(LA) ou LA ;
R⁶ est Hal, LA, oxo, CN, NH₂ ou Hét² ;
R^{6"} est H, Hal, LA, oxo, CN, NH₂ ou Hét² ;
Y est CH ou N,
---- désigne la présence ou l'absence de doubles liaisons dans Cyc ;
et tous les autres substituants revêtent la signification indiquée pour la formule (I),
et/ou un sel, tautomère et/ou stéréoisomère physiologiquement acceptable de celui-ci, y compris des mélanges de ceux-ci en toutes proportions.

9. Composé selon la revendication 5, dans lequel les radicaux non désignés plus en détail revêtent la signification indiquée pour la formule (IIa), mais dans lequel dans le cas de la sous-formule (IIa-A)
X¹ est CH,
R¹ est F ou Cl,
R² est F ou Cl,
dans le cas de la sous-formule (IIa-B)
R¹ est F,
R² est F ou Cl,
dans le cas de la sous-formule (IIa-C)
X¹, X² sont CH,
dans le cas de la sous-formule (IIa-D)
X¹ est CH,
R⁵ est H,
dans le cas de la sous-formule (IIa-E)
R³ est H, OH,
dans le cas de la sous-formule (IIa-F)
X¹ est CH,
R³ est OH,
dans le cas de la sous-formule (IIa-G)
X¹ est CH,
Y est CH,
dans le cas de la sous-formule (IIa-H)
X¹ est CH,
Cyc est pyridine, pyrazine ou pyridazine, ou pyrazolo[1,5-a]pyrimidinyle ou imidazo-[1,2-b]pyridazinyle,
dans le cas de la sous-formule (IIa-J)
Cyc est pyridine, pyrazine, pyridazine, pyrazolo[1,5-a]pyrimidinyle, imidazo[1,2-b]-pyridazinyle, furo[2,3-c]pyridinyle, furo[2,3-d]pyridazinyle, thiéno[2,3-d]pyridazinyle, thiéno[2,3-d]pyrimidinyle ou imidazo[4,5-c]pyridinyle, chacun d'entre eux pouvant être non substitué, ou pouvant être mono- ou disubstitué par méthoxy, méthyle, oxo, Cl ou CHF₂O,
dans le cas de la sous-formule (IIa-K)
R¹ est F ou Cl,
R² est F ou Cl,
R³ est OH,
R⁵ est H,
X¹, X² sont CH,
dans le cas de la sous-formule (IIa-L)
R¹ est F,
R² est F ou Cl,
R³ est H ou OH,
R⁵ est H,
dans le cas de la sous-formule (IIa-M)
R¹ est F ou Cl,
R² est F ou Cl,
R³ est OH,
R⁵ est H,
X¹, X² sont CH,
Cyc est pyridine, pyrazine ou pyridazine, ou pyrazolo[1,5-a]pyrimidinyle ou imidazo-[1,2-b]pyridazinyle,
dans le cas de la sous-formule (IIa-N)
R¹ est F,
R² est F ou Cl,
R³ est H ou OH,
R⁵ est H,
Cyc est pyridine, pyrazine, pyridazine, pyrazolo[1,5-a]pyrimidinyle, imidazo[1,2-b]-pyridazinyle, furo[2,3-c]pyridinyle, furo[2,3-d]pyridazinyle, thiéno[2,3-d]pyridazinyle, thiéno[2,3-d]pyrimidinyle ou imidazo[4,5-c]pyridinyle, chacun d'entre eux pouvant être non substitué, ou pouvant être mono- ou disubstitué par méthoxy, méthyle, oxo, Cl ou CHF₂O,
dans le cas de la sous-formule (IIa-O)
R¹ est F,
R² est F ou Cl,
R³ est H ou OH,
R⁵ est H,
Cyc est 5-méthoxypyridazin-3-yle, imidazo[1,2-b]pyridazin-6-yle, 3-chloro-6-méthoxy-pyrazin-2-yle, 3-chloropyrazin-2-yle, pyridazin-4-yle, 3-méthoxypyrazin-2-yle, 6-méthoxypyridazin-3-yle, 3-difluorométhoxypyridin-2-yle, 3-méthylpyrazin-2-yle, thiéno-[2,3-d]pyrimidin-4-yle, 1-méthyl-1H-pyridin-2-on-6-yle, 1H-pyridazin-6-on-3-yle, furo-[2,3-d]pyridazin-7-yle, thiéno[2,3-d]pyridazin-7-yle, 3,5-diméthylpyrazin-2-yle, furo-[2,3-d]pyrimidin-4-yle, 3-méthyl-3H-imidazo[4,5-c]pyridin-4-yle,
et/ou un sel, tautomère et/ou stéréoisomère physiologiquement acceptable de celui-ci, y compris des mélanges de ceux-ci en toutes proportions.

10. Composé selon la revendication 7, dans lequel les radicaux non désignés plus en détail revêtent la signification indiquée pour la formule (IIIa), mais dans lequel dans le cas de la sous-formule (IIIa-B)
R¹ est F,
dans le cas de la sous-formule (IIIa-C)
X¹, X² sont CH,
dans le cas de la sous-formule (IIIa-D)
X¹ est CH,
R⁵ est H,
dans le cas de la sous-formule (IIIa-E)
R³ est H, OH,
dans le cas de la sous-formule (IIIa-F)
X¹ est CH,
R³ est OH,
dans le cas de la sous-formule (IIIa-G)
X¹ est CH,
Y est CH,
dans le cas de la sous-formule (IIIa-H)
X¹ est CH,
Cyc est pyridine, pyrazine ou pyridazine, ou pyrazolo[1,5-a]pyrimidinyle ou imidazo[1,2-b]-pyridazinyle,
dans le cas de la sous-formule (IIIa-J)
Cyc est pyridine, pyrazine, pyridazine, pyrazolo[1,5-a]pyrimidinyle, imidazo[1,2-b]-pyridazinyle, furo[2,3-c]pyridinyle, furo[2,3-d]pyridazinyle, thiéno[2,3-d]pyridazinyle, thiéno[2,3-d]pyrimidinyle ou imidazo[4,5-c]pyridinyle, chacun d'entre eux pouvant être non substitué, ou pouvant être mono- ou disubstitué par méthoxy, méthyle, oxo, Cl ou CHF₂O,
dans le cas de la sous-formule (IIIa-K)
R¹ est F ou Cl,
R³ est OH,
R⁵ est H,
X¹, X² sont CH,
dans le cas de la sous-formule (IIIa-L)
R¹ est F,
R³ est H ou OH,
R⁵ est H,
dans le cas de la sous-formule (IIIa-M)
R¹ est F ou Cl,
R³ est OH,
R⁵ est H,
X¹, X² sont CH,
Cyc est pyridine, pyrazine ou pyridazine, ou pyrazolo[1,5-a]pyrimidinyle ou imidazo[1,2-b]-pyridazinyle,
dans le cas de la sous-formule (IIIa-N)
R¹ est F,
R³ est H ou OH,
R⁵ est H,
Cyc est pyridine, pyrazine, pyridazine, pyrazolo[1,5-a]pyrimidinyle, imidazo[1,2-b]-pyridazinyle, furo[2,3-c]pyridinyle, furo[2,3-d]pyridazinyle, thiéno[2,3-d]pyridazinyle, thiéno[2,3-d]pyrimidinyle ou imidazo[4,5-c]pyridinyle, chacun d'entre eux pouvant être non substitué, ou pouvant être mono- ou disubstitué par méthoxy, méthyle, oxo, Cl ou CHF₂O,
dans le cas de la sous-formule (IIIa-O)
R¹ est F,
R³ est H ou OH,
R⁵ est H,
Cyc est 5-méthoxypyridazin-3-yle, imidazo[1,2-b]pyridazin-6-yle, 3-chloro-6-méthoxy-pyrazin-2-yle, 3-chloropyrazin-2-yle, pyridazin-4-yle, 3-méthoxypyrazin-2-yle, 6-méthoxypyridazin-3-yle, 3-difluorométhoxypyridin-2-yle, 3-méthylpyrazin-2-yle, thiéno-[2,3-d]pyrimidin-4-yle, 1-méthyl-1H-pyridin-2-one-6-yle, 1H-pyridazin-6-one-3-yle, furo[2,3-d]pyridazin-7-yle, thiéno[2,3-d]pyridazin-7-yle, 3,5-diméthylpyrazin-2-yle, furo[2,3-d]pyrimidin-4-yle, 3-méthyl-3H-imidazo[4,5-c]pyridin-4-yle,
et/ou un sel, tautomère et/ou stéréoisomère physiologiquement acceptable de celui-ci, y compris des mélanges de ceux-ci en toutes proportions.

11. Composé selon la revendication 6, dans lequel les radicaux non désignés plus en détail revêtent la signification indiquée pour la formule (IIb), mais dans lequel
dans le cas de la sous-formule (IIb-Q)
R¹ est F ou Cl,
R² est F ou Cl,
R³ est OH,
R⁵ est H,
Y est CH,
dans le cas de la sous-formule (IIb-R)
R¹ est F,
R² est F ou Cl,
R³ est OH,
R⁵ est H,
Y est CH,
dans le cas de la sous-formule (IIb-S)
Cyc est pyridine, pyrazine ou pyridazine,
dans le cas de la sous-formule (IIb-T)
R¹ est F ou Cl,
R² est F ou Cl,
R³ est OH,
R⁵ est H,
Cyc est pyridine, pyrazine ou pyridazine,
dans le cas de la sous-formule (IIb-U)
R¹ est F,
R² est F ou Cl,
R³ est OH,
R⁵ est H,
Cyc est pyridine, pyrazine, pyridazine ou 3-méthylpyrazin-2-yle,
et/ou un sel, tautomère et/ou stéréoisomère physiologiquement acceptable de celui-ci, y compris des mélanges de ceux-ci en toutes proportions.

12. Composé selon la revendication 8, dans lequel les radicaux non désignés plus en détail revêtent la signification indiquée pour la formule (IIIb), mais dans lequel
dans le cas de la sous-formule (IIIb-Q)
R¹ est F ou Cl,
R³ est OH,
R⁵ est H,
Y est CH,
dans le cas de la sous-formule (IIIb-R)
R¹ est F,
R³ est OH,
R⁵ est H,
Y est CH,
dans le cas de la sous-formule (IIIb-S)
Cyc est pyridine, pyrazine ou pyridazine,
dans le cas de la sous-formule (IIIb-T)
R¹ est F ou Cl,
R³ est OH,
R⁵ est H,
Cyc est pyridine, pyrazine ou pyridazine,
dans le cas de la sous-formule (IIIb-U)
R¹ est F,
R³ est OH,
R⁵ est H,
Cyc est pyridine, pyrazine, pyridazine ou 3-méthylpyrazin-2-yle,
et/ou un sel, tautomère et/ou stéréoisomère physiologiquement acceptable de celui-ci, y compris des mélanges de ceux-ci en toutes proportions.

13. Composé selon l'une des revendications 1 à 12, choisi dans le groupe constitué par :
le [2-chloro-4-fluoro-5-(7-morpholin-4-ylquinazolin-4-yl)phényl]-(5-méthoxypyridazin-3-yl)-méthanol,
le [4-fluoro-3-(7-morpholin-4-ylquinazolin-4-yl)phényl]-(5-méthoxypyridazin-3-yl)méthanol,
le [2,4-difluoro-5-(7-morpholin-4-ylquinazolin-4-yl)phényl]imidazo[1,2-b]pyridazin-6-yl-méthanol,
le (3-chloro-6-méthoxypyrazin-2-yl)-[4-fluoro-3-(7-morpholin-4-ylquinazolin-4-yl)phényl]-méthanol,
le (R)-(3-chloropyrazin-2-yl)-[4-fluoro-3-(7-morpholin-4-ylquinazolin-4-yl)phényl]méthanol,
le [2-chloro-4-fluoro-5-(7-morpholin-4-ylquinazolin-4-yl)phényl]pyridazin-4-ylméthanol,
le [4-fluoro-3-(7-morpholin-4-ylquinazolin-4-yl)phényl]-(3-méthoxypyrazin-2-yl)méthanol,
le [4-fluoro-3-(7-morpholin-4-ylquinazolin-4-yl)phényl]-(6-méthoxypyridazin-3-yl)méthanol,
le (3-difluorométhoxypyridin-2-yl)-[4-fluoro-3-(7-morpholin-4-ylquinazolin-4-yl)phényl]-méthanol,
le (R)-[4-fluoro-3-(7-morpholin-4-ylquinazolin-4-yl)phényl]-(3-méthylpyrazin-2-yl)méthanol,
le [2,4-difluoro-5-(7-morpholin-4-ylquinazolin-4-yl)phényl]-(3-méthylpyrazin-2-yl)méthanol,
le [2,4-difluoro-5-(7-morpholin-4-ylquinazolin-4-yl)phényl]thiéno[2,3-d]pyrimidin-4-ylméthanol,
la 6-{[4-fluoro-3-(7-morpholin-4-ylquinazolin-4-yl)phényl]hydroxyméthyl}-1-méthyl-1H-pyridin-2-one,
la 3-[[2-chloro-4-fluoro-5-(7-morpholinoquinazolin-4-yl)phényl]hydroxyméthyl]-1H-pyridazin-6-one,
le (S)-[2-chloro-4-fluoro-5-(7-morpholin-4-ylquinazolin-4-yl)phényl]-(6-méthoxypyridazin-3-yl)méthanol,
le (R)-[4-fluoro-3-(7-morpholin-4-ylpyrido[3,2-d]pyrimidin-4-yl)phényl]-(3-méthylpyrazin-2-yl)-méthanol,
la 4-(4-chloro-2-fluoro-5-imidazo[1,2-b]pyridazin-6-ylméthylphényl)-7-morpholin-4-yl-quinazoline,
le [4-fluoro-3-(6-morpholin-4-ylthiéno[3,2-d]pyrimidin-4-yl)phényl]-(3-méthylpyrazin-2-yl)-méthanol,
le (R)-[4-fluoro-3-(5-fluoro-7-morpholin-4-ylquinazolin-4-yl)phényl]-(3-méthylpyrazin-2-yl)-méthanol,
le [2-chloro-4-fluoro-5-(5-fluoro-7-morpholin-4-ylquinazolin-4-yl)phényl]-(3-méthoxypyrazin-2-yl)méthanol,
le [4-fluoro-3-(5-fluoro-7-morpholin-4-ylquinazolin-4-yl)phényl]-(3-méthoxypyrazin-2-yl)-méthanol,
le [4-fluoro-3-(5-fluoro-7-morpholin-4-ylquinazolin-4-yl)phényl]-(6-méthoxypyridazin-3-yl)-méthanol,
le [4-fluoro-3-[7-(2,2,3,3,5,5,6,6-octadeutériomorpholin-4-yl)quinazolin-4-yl]phényl]-(3-méthyl-pyrazin-2-yl)méthanol,
le [4-fluoro-3-(5-fluoro-7-morpholin-4-ylquinazolin-4-yl)phényl]-(6-méthoxypyridazin-3-yl)-méthanol,
le [2-chloro-4-fluoro-5-[7-(2,2,3,3,5,5,6,6-octadeutériomorpholin-4-yl)quinazolin-4-yl]phényl]-(6-méthoxypyridazin-3-yl)méthanol,
le [2-chloro-4-fluoro-5-(6-morpholin-4-ylthiéno[3,2-d]pyrimidin-4-yl)phényl]-(3-méthylpyrazin-2-yl)méthanol,
le [4-fluoro-3-(6-morpholin-4-ylthiéno[3,2-d]pyrimidin-4-yl)phényl]-(3-méthylpyrazin-2-yl)-méthanol,
le [2-chloro-4-fluoro-5-(5-fluoro-7-morpholin-4-ylquinazolin-4-yl)phényl]-(3-méthoxypyrazin-2-yl)méthanol,
le [4-fluoro-3-(5-fluoro-7-morpholin-4-ylquinazolin-4-yl)phényl]-(3-méthoxypyrazin-2-yl)-méthanol,
le [4-fluoro-3-(5-fluoro-7-morpholin-4-ylquinazolin-4-yl)phényl]-(3-méthylpyrazin-2-yl)-méthanol,
le [2-chloro-4-fluoro-5-(5-fluoro-7-morpholin-4-ylquinazolin-4-yl)phényl]-(6-méthoxypyridazin-3-yl)méthanol,
le [4- fluoro-3-(7-morpholin-4-ylquinazolin-4-yl)phényl]furo[2,3-d]pyridazin-7-ylméthanol,
le [2,4-difluoro-5-(7-morpholin-4-ylquinazolin-4-yl)phényl]furo[2,3-d]pyridazin-7-ylméthanol,
le [2,4-difluoro-5-(7-morpholin-4-ylquinazolin-4-yl)phényl]thiéno[2,3-d]pyridazin-7-ylméthanol,
le (3,5-diméthylpyrazin-2-yl)-[4-fluoro-3-(7-morpholin-4-ylquinazolin-4-yl)phényl]méthanol,
la 6-{[2-chloro-5-(7-morpholin-4-ylquinazolin-4-yl)phényl]hydroxyméthyl}-1-méthyl-1H-pyridin-2-one,
la 6-{[4-fluoro-3-(7-morpholin-4-ylquinazolin-4-yl)phényl]hydroxyméthyl}-2H-pyridazin-3-one,
la 6-{[4-fluoro-3-(7-morpholin-4-ylquinazolin-4-yl)phényl]hydroxyméthyl}-1-méthyl-1H-pyridin-2-one,
la 6-{[2-chloro-4-fluoro-5-(7-morpholin-4-ylquinazolin-4-yl)phényl]hydroxyméthyl}-1-méthyl-1H-pyridin-2-one,
le [4-fluoro-3-(7-morpholin-4-ylquinazolin-4-yl)phényl]furo[2,3-d]pyrimidin-4-ylméthanol,
le [4-fluoro-3-(7-morpholin-4-ylquinazolin-4-yl)phényl]furo[3,2-d]pyrimidin-4-ylméthanol,
le [2,4-difluoro-5-(7-morpholin-4-ylquinazolin-4-yl)phényl]-(3-méthoxypyrazin-2-yl)méthanol,
le 4-fluoro-3-(7-morpholin-4-ylquinazolin-4-yl)phényl]-(3-méthyl-3H-imidazo[4,5-c]pyridin-4-yl)méthanol,
le [4-fluoro-3-(7-morpholin-4-ylquinazolin-4-yl)phényl]furo[3,2-d]pyrimidin-4-ylméthanol,
et/ou un sel, tautomère et/ou stéréoisomère physiologiquement acceptable de celui-ci, y compris des mélanges de ceux-ci en toutes proportions.

14. Procédé de préparation d'un composé de formule (I) selon la revendication 1 et/ou d'un sel, tautomère et/ou stéréoisomère physiologiquement acceptable de celui-ci, comportant les étapes suivantes :
(a) la réaction d'un composé de formule (V) 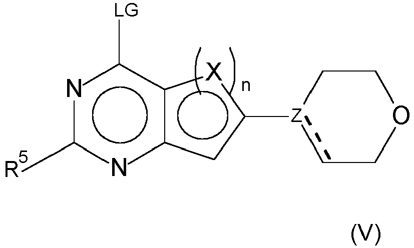 où LG est un groupement partant classique, tel que Hal,
avec un composé de formule (IV) 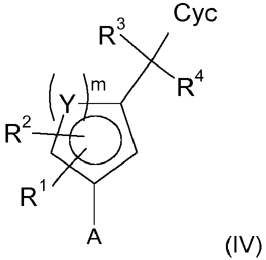 où A est un acide boronique ou un ester d'acide boronique,
pour donner le composé de formule (I), et éventuellement
(b) la conversion d'une base ou d'un acide du composé de formule (I) en l'un de ses sels.

15. Composé selon l'une des revendications 1 à 13 et/ou un sel, tautomère et/ou stéréoisomère physiologiquement acceptable de celui-ci, y compris des mélanges de ceux-ci en toutes proportions, pour une utilisation comme médicament.

16. Composé selon l'une des revendications 1 à 13 et/ou un sel, tautomère et/ou stéréoisomère physiologiquement acceptable de celui-ci, y compris des mélanges de ceux-ci en toutes proportions, pour une utilisation comme médicament pour la sensibilisation de cellules cancéreuses vis-à-vis d'agents anticancéreux et/ou d'un rayonnement ionisant.

17. Composé selon l'une des revendications 1 à 13 et/ou un sel, tautomère et/ou stéréoisomère physiologiquement acceptable de celui-ci, y compris des mélanges de ceux-ci en toutes proportions, pour une utilisation dans la prophylaxie et/ou la thérapie du cancer, des tumeurs ou des métastases en combinaison avec une radiothérapie et/ou avec au moins un agent anticancéreux.

18. Médicament comprenant au moins un composé selon l'une des revendications 1 à 13 et/ou un sel, tautomère et/ou stéréoisomère physiologiquement acceptable de celui-ci, y compris des mélanges de ceux-ci en toutes proportions.

19. Composition pharmaceutique comprenant, comme composé actif, une quantité efficace d'au moins un composé selon l'une des revendications 1 à 13 et/ou d'un sel, tautomère et/ou stéréoisomère physiologiquement acceptable de celui-ci, y compris des mélanges de ceux-ci en toutes proportions, conjointement avec des adjuvants pharmaceutiquement tolérés.

20. Composition pharmaceutique comprenant, comme composé actif, une quantité efficace d'au moins un composé selon l'une des revendications 1 à 13 et/ou d'un sel, tautomère et/ou stéréoisomère physiologiquement acceptable de celui-ci, y compris des mélanges de ceux-ci en toutes proportions, conjointement avec des adjuvants pharmaceutiquement tolérés en combinaison avec une quantité efficace d'au moins un agent anticancéreux.

21. Kit, constitué de conditionnements séparés (a) d'une quantité efficace d'au moins un composé selon l'une des revendications 1 à 13 et/ou d'un sel, tautomère et/ou stéréoisomère physiologiquement acceptable de celui-ci, y compris des mélanges de ceux-ci en toutes proportions, et (b) d'une quantité efficace d'au moins un agent anticancéreux.

22. Composé selon l'une des revendications 1 à 13 et/ou un sel, tautomère et/ou stéréoisomère physiologiquement acceptable de celui-ci, y compris des mélanges de ceux-ci en toutes proportions, pour une utilisation selon la revendication 16 ou 17, où l'agent anticancéreux est choisi parmi : des agents alkylants, y compris l'altrétamine, la bendamustine, le busulfan, la carmustine, le chlorambucil, la chlorméthine, la cyclophosphamide, la dacarbazine, l'ifosfamide, le tosylate d'improsulfan, la lomustine, le melphalan, le mitobronitol, le mitolactol, la nimustine, la ranimustine, le témozolomide, le thiotépa, le tréosulfan, la méchloréthamine, la carboquone, l'apaziquone, la fotémustine, le glufosfamide, le palifosfamide, le pipobroman, le trofosfamide, l'uramustine ; les composés de platine, y compris le carboplatine, le cisplatine, l'eptaplatine, l'hydrate de miriplatine, l'oxaliplatine, le lobaplatine, le nédaplatine, le picoplatine, le satraplatine ; les inhibiteurs de topoisomérase, y compris l'étoposide, l'irinotécan, le razoxane, le sobuzoxane ; les agents de modification de l'ADN, y compris l'amrubicine, le bisantrène, la décitabine, la mitoxantrone, la procarbazine, la trabectédine, la clofarabine, l'amsacrine, la brostallicine, la pixantrone, la laromustine ; les antibiotiques anticancéreux, y compris la bléomycine, la dactinomycine, la doxorubicine, l'épirubicine, l'idarubicine, le lévamisole, la miltéfosine, la mitomycine C, la romidepsine, la streptozocine, la valrubicine, la zinostatine, la zorubicine, la daunorubicine, la plicamycine, l'aclarubicine, la péplomycine, la pirarubicine ; les émetteurs alpha, y compris l'alpharadine (dichlorure de ²²³Ra, Xofigo), ²¹¹At, ²¹³Bi, ²²⁵Ac, ²²⁷Th.

23. Composition pharmaceutique selon la revendication 20, dans laquelle le au moins un agent anticancéreux est choisi parmi : des agents alkylants, y compris l'altrétamine, la bendamustine, le busulfan, la carmustine, le chlorambucil, la chlorméthine, la cyclophosphamide, la dacarbazine, l'ifosfamide, le tosylate d'improsulfan, la lomustine, le melphalan, le mitobronitol, le mitolactol, la nimustine, la ranimustine, le témozolomide, le thiotépa, le tréosulfan, la méchloréthamine, la carboquone, l'apaziquone, la fotémustine, le glufosfamide, le palifosfamide, le pipobroman, le trofosfamide, l'ura-mustine ; les composés de platine, y compris le carboplatine, le cisplatine, l'eptaplatine, l'hydrate de miriplatine, l'oxaliplatine, le lobaplatine, le nédaplatine, le picoplatine, le satraplatine ; les inhibiteurs de topoisomérase, y compris l'étoposide, l'irinotécan, le razoxane, le sobuzoxane ; les agents de modification de l'ADN, y compris l'amrubicine, le bisantrène, la décitabine, la mitoxantrone, la procarbazine, la trabectédine, la clofarabine, l'amsacrine, la brostallicine, la pixantrone, la laromustine ; les antibiotiques anticancéreux, y compris la bléomycine, la dactinomycine, la doxorubicine, l'épirubicine, l'idarubicine, le lévamisole, la miltéfosine, la mitomycine C, la romidepsine, la streptozocine, la valrubicine, la zinostatine, la zorubicine, la daunorubicine, la plicamycine, l'aclarubicine, la péplomycine, la pirarubicine ; les émetteurs alpha, y compris l'alpharadine (dichlorure de ²²³Ra, Xofigo), ²¹¹At, ²¹³Bi, ²²⁵Ac, ²²⁷Th.

24. Kit selon la revendication 21, dans lequel le au moins un agent anticancéreux est choisi parmi : des agents alkylants, y compris l'altrétamine, la bendamustine, le busulfan, la carmustine, le chlorambucil, la chlorméthine, la cyclophosphamide, la dacarbazine, l'ifosfamide, le tosylate d'improsulfan, la lomustine, le melphalan, le mitobronitol, le mitolactol, la nimustine, la ranimustine, le témozolomide, le thiotépa, le tréosulfan, la méchloréthamine, la carboquone, l'apaziquone, la fotémustine, le glufosfamide, le palifosfamide, le pipobroman, le trofosfamide, l'uramustine ; les composés de platine, y compris le carboplatine, le cisplatine, l'eptaplatine, l'hydrate de miriplatine, l'oxaliplatine, le lobaplatine, le nédaplatine, le picoplatine, le satraplatine ; les inhibiteurs de topoisomérase, y compris l'étoposide, l'irinotécan, le razoxane, le sobuzoxane ; les agents de modification de l'ADN, y compris l'amrubicine, le bisantrène, la décitabine, la mitoxantrone, la procarbazine, la trabectédine, la clofarabine, l'amsacrine, la brostallicine, la pixantrone, la laromustine ; les antibiotiques anticancéreux, y compris la bléomycine, la dactinomycine, la doxorubicine, l'épirubicine, l'idarubicine, le lévamisole, la miltéfosine, la mitomycine C, la romidepsine, la streptozocine, la valrubicine, la zinostatine, la zorubicine, la daunorubicine, la plicamycine, l'aclarubicine, la péplomycine, la pirarubicine ; les émetteurs alpha, y compris l'alpharadine (dichlorure de ²²³Ra, Xofigo), ²¹¹At, ²¹³Bi, ²²⁵Ac, ²²⁷Th.
